(19) Europäisches Patentamt
European Patent Office
Office européen des brevets

(11) EP 1 633 870 B1

(12) # EUROPEAN PATENT SPECIFICATION

(45) Date of publication and mention of the grant of the patent:
**27.03.2013 Bulletin 2013/13**

(21) Application number: **04735607.6**

(22) Date of filing: **01.06.2004**

(51) Int Cl.:
***C12N 15/11*** *(2006.01)* ***C12Q 1/68*** *(2006.01)*

(86) International application number:
**PCT/GB2004/002316**

(87) International publication number:
**WO 2005/049829 (02.06.2005 Gazette 2005/22)**

(54) **PROCESS FOR SCREENING A DRUG RESPONSE IN CANCER PATIENTS**

PROZESS ZUM SCREENING EINER MEDIKAMENTENANSPRACHE IN KREBSPATIENTEN

PROCEDE DE CRIBLAGE D'UNE REPONSE MEDICAMENTEUSE DES PATIENTS ATTEINTS DE CANCER

(84) Designated Contracting States:
**AT BE BG CH CY CZ DE DK EE ES FI FR GB GR HU IE IT LI LU MC NL PL PT RO SE SI SK TR**
Designated Extension States:
**HR LT LV**

(30) Priority: **30.05.2003 GB 0312451**
**26.09.2003 GB 0322636**
**21.11.2003 GB 0327132**

(43) Date of publication of application:
**15.03.2006 Bulletin 2006/11**

(60) Divisional application:
**10012191.2 / 2 348 110**

(73) Proprietor: **OncoTherapy Science, Inc.**
**Kawasaki-shi**
**Kanagawa 213-0012 (JP)**

(72) Inventors:
- **TSURUO, Takashi**
  **Tokyo 156-0051 (JP)**
- **NAKAMURA, Yusuke**
  **Yokohama-shi,**
  **Kanagawa 225-0011 (JP)**
- **SONE, Saburo**
  **Tokushima 770-0037 (JP)**
- **FUKUOKA, Masahiro**
  **Nara 631-0007 (JP)**

(74) Representative: **Harding, Charles Thomas et al**
**D Young & Co LLP**
**120 Holborn**
**London**
**EC1N 2DY (GB)**

(56) References cited:
- **DATABASE GENEBANK [Online] 18 March 2001 (2001-03-18), XP002290893 retrieved from GENEBANK Database accession no. NM_024829 cited in the application**
- **DATABASE GENEBANK [Online] 5 July 2001 (2001-07-05), XP002304184 retrieved from GENEBANK Database accession no. BC009799 cited in the application**
- **DATABASE GENEBANK [Online] 26 April 2000 (2000-04-26), XP002304185 retrieved from GENEBANK Database accession no. NM_014325 cited in the application**
- **DATABASE GENEBANK [Online] 12 July 2001 (2001-07-12), XP002304186 retrieved from GENEBANK Database accession no. BC010488 cited in the application**
- **DATABASE GENEBANK [Online] 7 May 1999 (1999-05-07), XP002304187 retrieved from GENEBANK Database accession no. NM_004090 cited in the application**
- **DATABASE GENEBANK [Online] 20 May 1998 (1998-05-20), XP002304188 retrieved from GENEBANK Database accession no. AI026836 cited in the application**
- **DATABASE GENEBANK [Online] 11 September 2002 (2002-09-11), XP002304189 retrieved from GENEBANK Database accession no. BU500509 cited in the application**
- **DATABASE GENEBANK [Online] 8 September 2000 (2000-09-08), XP002304190 retrieved from GENEBANK Database accession no. NM_016090 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 633 870 B1

- DATABASE GENEBANK [Online] 22 January 2003 (2003-01-22), XP002304191 retrieved from GENEBANK Database accession no. BX092512 cited in the application
- DATABASE GENEBANK [Online] 12 February 1999 (1999-02-12), XP002304192 retrieved from GENEBANK Database accession no. AI436027 cited in the application
- DATABASE GENEBANK [Online] 25 August 1999 (1999-08-25), XP002304193 retrieved from GENEBANK Database accession no. AI971137 cited in the application
- DATABASE GENEBANK [Online] 27 March 2002 (2002-03-27), XP002304194 retrieved from GENEBANK Database accession no. BQ024877 cited in the application
- DE BONO JOHANN S ET AL: "The ErbB receptor family: a therapeutic target for cancer." TRENDS IN MOLECULAR MEDICINE. 2002, vol. 8, no. 4 Suppl, 2002, pages S19-S26, XP002290892 ISSN: 1471-4914

## Description

Field of the Invention

The present invention relates to a method of personalized cancer therapy which employs a set of marker genes to predict whether a patient will respond to a chemotherapeutic agent and a kit for use in said method.

In particular, the method predicts patient response to erbB tyrosine kinase inhibitors. More particularly the method relates to those patients with cancers mediated alone or in part by erbB tyrosine kinase, especially patients with advanced Non-small Cell Lung Cancer (NSCLC), for example adenocarcinoma, using the levels of a set of marker genes having differential expression between responders and non responders to the erbB tyrosine kinase inhibitor.

Background to the Invention

Lung cancer is the leading cause of cancer death and is therefore a major health problem worldwide. In the treatment of this disease, chemotherapy is the mainstay, because the majority has locally advanced stage 3 (44%) or metastatic stage 4 (32%) disease at diagnosis [1]. Nevertheless, the findings of large meta-analysis revealed that platinum-based chemotherapy contributed to prolong the median survival time of patients with advanced non-small cell lung cancer (NSCLC) by only about 6 weeks compared with best supportive care [2].

In the last decade, many new cytotoxic agents have been developed including paclitaxel, docetaxel, gemcitabine, and vinorelbine, and have offered multiple choices for patients with advanced lung cancer. However, each regimen served only modest survival benefit compared with the cisplatin-based therapies [3], [4]. More recently, new therapeutic strategies including a number of molecular-targeted agents have been developed in an effort to overcome the limitations of conventional cytotoxic agents [5] [6].

In recent years it has been discovered that certain growth factor tyrosine kinase enzymes are important in the transmission of biochemical signals which initiate cell replication. They are large proteins which span the cell membrane and possess an extracellular binding domain for growth factors such as epidermal growth factor (EGF) and an intracellular portion which functions as a kinase to phosphorylate tyrosine amino acids in proteins and hence to influence cell proliferation.

Various classes of receptor tyrosine kinases are known (Wilks, Advances in Cancer Research, 1993, 60, 43-73) based on families of growth factors which bind to different receptor tyrosine kinases. The classification includes Class I receptor tyrosine kinases comprising the EGF family of receptor tyrosine kinases. This includes receptors for the ligands EGF, TGF$\alpha$ (also referred to as TGFA), amphiregulin (also referred to as AREG), betacellulin, heparin binding EGF, epiregulin and the neuregulins (including NRG-1, NRG-2, NRG-3 and NRG-4). More specifically, these receptors include those with a functional kinase domain called erbB1 (EGFR), erbB2 (Neu, Her2) and erbB4 (Her 4), and erbB3 (her3), which does not), Class II receptor tyrosine kinases comprising the insulin family of receptor tyrosine kinases such as the insulin and IGFI receptors and insulin-related receptor (IRR) and Class III receptor tyrosine kinases comprising the platelet-derived growth factor (PDGF) family of receptor tyrosine kinases such as the PDGF$\alpha$, PDGF$\beta$ and colony-stimulating factor 1 (CSF1) receptors.

It is known that the erbB family of receptor tyrosine kinases, which include EGFR, erbB2, erbB3 and erbB4, are frequently involved in driving the proliferation and survival of tumour cells (reviewed in Olayioye et al., EMBO J., 2000, 19, 3159). One mechanism by which this can occur is over expression of the receptor at the protein level, generally as a result of gene amplification. This has been observed in many common human cancers (reviewed in Klapper et al., Adv. Cancer Res., 2000, 77, 25) such as, non-small cell lung cancers (NSCLCs) including adenocarcinomas (Cemy et al., Brit. J. Cancer, 1986, 54, 265; Reubi et al., Int. J. Cancer, 1990, 45, 269; Rusch et al., Cancer Research, 1993, 53, 2379; Brabender et al, Clin. Cancer Res., 2001, 7, 1850) as well as other cancers of the lung (Hendler et al., Cancer Cells, 1989, 7, 347. As

a consequence of the mis-regulation of one or more of these receptors, it is widely believed that many tumours become clinically more aggressive and so correlate with a poorer prognosis for the patient (Brabender et al, Clin. Cancer Res., 2001, 7, 1850; Ross et al, Cancer Investigation, 2001, 19, 554, Yu et al., Bioessays, 2000, 22.7, 673). In addition to these clinical findings, a wealth of pre-clinical information suggests that the erbB family of receptor tyrosine kinases are involved in cellular transformation. In addition to this, a number of pre-clinical studies have demonstrated that anti-proliferative effects can be induced by knocking out one or more erbB activities by small molecule inhibitors, dominant negatives or inhibitory antibodies (reviewed in Mendelsohn et al., Oncogene, 2000, 19, 6550).

Thus it has been recognised that inhibitors of these receptor tyrosine kinases should be of value as a selective inhibitor of the proliferation of mammalian cancer cells (Yaish et al. Science, 1988, 242, 933, Kolibaba et al, Biochimica et Biophysica Acta, 1997, 133, F217-F248; Al-Obeidi et al, 2000, Oncogene, 19, 5690-5701; Mendelsohn et al, 2000, Oncogene, 19, 6550-6565). In addition to this pre-clinical data, findings using inhibitory antibodies against EGFR and erbB2 (c-225 and trastuzumab respectively) have proven to be beneficial in the clinic for the treatment of selected solid tumours (reviewed in Mendelsohn et al, 2000, Oncogene, 19, 6550-6565).

A number of small molecule inhibitors of erbB family of receptor tyrosine kinases are known, particularly inhibitors of EGF and erbB2 receptor tyrosine kinases. For example European Patent Application No. 0566226 and International Patent Applications WO 96/33980 and WO 97/30034 disclose that certain quinazoline derivatives which possess an anilino substituent at the 4-position possess EGFR tyrosine kinase inhibitory activity and are inhibitors of the proliferation of cancer tissue including prostate cancer. It has been disclosed by J R Woodburn et al. in Proc. Amer. Assoc. Cancer Research, 1997, 38, 633 and Pharmacol. Ther., 1999, 82, 241-250 that the compound N-(3-chloro-4-fluorophenyl)-7-methoxy-6-(3-morpholinopropoxy)quinazolin-4-amine is a potent EGFR tyrosine kinase inhibitor. This compound is also known as Iressa (registered trade mark), gefitinib (United States Adopted Name), by way of the code number ZD1839 and Chemical Abstracts Registry Number 184475-35-2. The compound is identified hereinafter as gefitinib. Gefitinib has recently been approved in Japan for the treatment of inoperable or recurrent non-small cell lung cancer (NSCLC) and in the USA as a monotherapy for the treatment of patients with locally advanced metastatic NSCLC after failure of both platinum and docetaxel chemotherapies.

It is further known from International Patent Application WO 96/30347 that certain structurally-related quinazoline derivatives possessing an anilino substituent at the 4-position also possess EGFR tyrosine kinase inhibitory activity. It has been disclosed in WO 99/55683 that the compound N-(3-ethynylphenyl)-6,7-bis(2-methoxyethoxy)quinazolin-4-amine, or a pharmaceutically-acceptable salt thereof (linked to the code numbers CP 358774 and OSI-774, identified hereinafter by the code number OSI-774) is an EGFR TKI.

It is further known from International Patent Application WO 97/38983 that certain other structurally-related quinazoline derivatives possessing an anilino substituent at the 4-position also possess EGFR tyrosine kinase inhibitory activity. It has been disclosed in J.Med. Chem., 1999, 42,1803-1815 and WO 00/31048 that the compound 6-acrylamido-N-(3-chloro-4-fluorophenyl)-7-(3 morpholinopropoxy) quinazolin-4 - amine (linked to the code numbers PD 183805 and CI 1033, identified hereinafter by the code number CI 1033) is an EGFR TKI.

It is further known from International Patent Application WO 97/02266 that certain other structurally-related heterocyclic derivatives also possess EGFR tyrosine kinase inhibitory activity. For example, the compound 4-[(1R)-1-phenylethylamino]-6-(4-hydroxyphenyl)-7H-pyrrolo[2,3-d]pyrimidine (linked to the code numbers PKI-166, CGP 75166 and CGP 59326, identified hereinafter by the code number PKI-166) is an EGFR TKI.

It is further known from European Patent Application No. 0787722 and International Patent Applications WO 98/50038, WO 99/09016 and WO 99/24037 that certain other structurally-related quinazoline derivatives possessing an anilino substituent at the 4-position also possess EGFR tyrosine kinase inhibitory activity. For example, the compound N-[4-(3-bromoanilino)quinazolin-6-yl]but-2-ynamide (linked to the code numbers CL-387785 and EKB-785, identified hereinafter by the code number CL-387785) is an EGFR TKI.

It is further known from Nature Medicine, 2000, 6, 1024-1028 and United States Patent No. 6,002,008 that certain other structurally-related quinoline derivatives possessing an anilino substituent at the 4-position also possess EGFR tyrosine kinase inhibitory activity. For example, the compound 4-(3-chloro-4-fluoroanilino)-3-cyano-6-(4-dimethylaminobut-2(E)-enamido)-7-ethoxyquinoline (identified hereinafter by the code number EKB-569) is an EGFR TKI.

It is also known from WO 99/35146 and WO 01/04111 that certain other quinazoline derivatives are inhibitors of one or more of the erbB receptor tyrosine kinase inhibitors. For example the compound N-{3-chloro-4-[(3-fluorobenzyl) oxy]phenyl}-6-[5-({[2-(methylsulfonyl)ethyl]amino}methyl)-2-furyl]quinazolin-4-amine (also identified as lapatinib or GW2016 identified hereinafter by the code GW2016) is thought to be an inhibitor of both EGF and erbB2 receptor tyrosine kinases.

Novartis AE788 is another suitable inhibitor compound.

Inhibition of erbB receptor tyrosine kinase may also be achieved by inhibition of the extracellular ligand binding to a receptor using suitable antibodies against an erbB receptor. For example using the anti-erbB2 antibody trastuzumab [Herceptin™] and the anti-erbb1 antibody cetuximab [C225]). The use of such inhibitory antibodies have proven to be beneficial in the clinic for the treatment of selected solid tumours (reviewed in Mendelsohn et al, 2000, Oncogene, 19, 6550-6565).

As mentioned above, gefitinib is an oral active inhibitor of epidermal growth factor receptor-tyrosine kinase (EGFR-TK), which blocks signalling pathways responsible for driving proliferation, invasion, and survival of cancer cells [7]. Potent anti-tumour effects as well as rapid improvements in NSCLC-related symptoms and quality of life have been observed in clinical studies that enrolled patients with advanced NSCLC who did not respond to platinum-based chemotherapy. In the randomized double-blind phase II monotherapy trial (the IDEAL 1 trial), use of gefitinib as 2nd or 3rd line of chemotherapy to advanced NSCLC achieved tumour response rate of 18.4% (95%CI: 11.0-25.9%), and in the IDEAL 2 trial, use as 3rd or 4th line of chemotherapy achieved that of 11.8% (95%CI: 6.2-19.7%) [8],[27],[28].

Moreover in these trials, the treatment of this drug achieved high disease control rate (54.4% in IDEAL 1, 42.2% in IDEAL 2) and overall symptom improvement rate (40.3% in IDEAL 1, 43.1% in IDEAL 2).

Those results were promising when compared with responses to conventional cytotoxic agents, but the fact remained that about half of the patients enrolled in these studies received non-effective treatment with no improvement in symptoms. Moreover, the medication exposed non-responders to adverse effects, including life threatening ones such as interstitial pneumonia [11].

Patients responses to the various chemotherapy treatments differ, therefore there is a need to find methods of predicting which treatment regimes best suit a particular patient.

There is an increasing body of evidence that suggests that patients responses to numerous drugs may be related to a patients genetic profile and that determination of the genetic factors that influence, for example, response to a particular drug could be used to provide a patient with a personalised treatment regime. Such personalised treatment regimes offer the potential to maximise therapeutic benefit to the patient, whilst minimising, for example side effects that may be associated with alternative and less effective treatment regimes. There is therefore a need for methods that can predict a patients response to a drug.

Summary of the Invention

It has been found that the sensitivity of certain cancers to chemotherapeutic agents can be predicted by gene expression and hence that the suitability of cancer patients for treatment with such chemotherapeutic agents can be determined by measuring the relative levels of particular genes in patient tissue.

The present invention provides a set of isolated marker genes comprising genes identified as having differential expression as between patients who are responders and non responders to an erbB receptor tyrosine kinase inhibitor; said gene set comprising all of the first 12 genes listed in Table 4 herein or gene-specific oligonucleotides derived from said genes; wherein the first 12 genes listed in Table 4 are the genes FLJ22662 shown in Table 4A, AREG shown in Table 4A, CORO1C shown in Table 4A, AVEN shown in Table 4A, DUSP3 shown in Table 4A, DJ473B4 shown in Table 4A, PHLDA2 shown in Table 4A, RBM7 shown in Table 4A, EST shown in Table 4A, OSMR shown in Table 4A, GCLC shown in Table 4A, and COL4A3BP shown in Table 4A; and wherein the gene-specific oligonucleotides derived from said genes are fragments of said genes that uniquely identify said genes.

In another aspect, the present invention provides an *in vitro* method of predicting the responsiveness of a patient with cancer to treatment with an erbB receptor kinase inhibitor, or for selecting patients that will respond to an erbB receptor kinase inhibitor comprising comparing the differential expression of the marker genes of the gene set according to the present invention.

In a further aspect, the present invention provides a diagnostic kit for use in the method of the present invention comprising a marker gene set of the present invention on a suitable support medium.

In a further aspect, the present invention provides the use of the set of isolated marker genes according to the present invention to measure the expression level of said genes in a tissue sample from a patient having NSCLC.

In another aspect, the present invention provides a diagnostics kit comprising means for determining the level of expression of the first 12 genes listed in Table 4 herein in a tissue sample from a NSCLC patient, comprising a support material comprising a set of isolated marker genes, according to the present invention, each gene thereof attached thereto; wherein the first 12 genes listed in Table 4 are the genes FLJ22662 shown in Table 4A, AREG shown in Table 4A, CORO1C shown in Table 4A, AVEN shown in Table 4A, DUSP3 shown in Table 4A, DJ473B4 shown in Table 4A, PHLDA2 shown in Table 4A, RBM7 shown in Table 4A, EST shown in Table 4A, OSMR shown in Table 4A, GCLC shown in Table 4A, and COL4A3BP shown in Table 4A.

In a further aspect, the present invention provides the use of all of the marker genes from a gene set according to the present invention in the manufacture of a diagnostic wherein said diagnostic is used *in vitro* to predict the responsiveness of a patient with cancer to treatment with an erbB receptor kinase inhibitor, or to select patients that will respond to an erbB receptor kinase inhibitor by comparing the differential expression of the marker genes from a gene set according to the present invention.

In another aspect, the present invention provides the use of a gene set according to the present invention in the manufacture of a diagnostic for assessing *in vitro* if an erbB tyrosine kinase receptor inhibitor modulates gene expression of at least one of the gene from the marker gene set according to the present invention relative to a relevant control.

In a further aspect, the present invention provides the use of a gene set according to the present invention in the manufacture of a diagnostic for carrying out a clinical trial measuring the effect or effectiveness of erbB receptor tyrosine kinase inhibition or inhibitors comprising measuring *in vitro* the relative levels of expression of a gene set according to the present invention in a patient.

Accordingly, the present description provides an isolated set of marker genes comprising at least one gene identified as having differential expression as between patients who are responders and non responders to an erbB receptor tyrosine kinase inhibitor, said gene set comprising one or more genes selected from at least the group consisting of the 51 genes listed in Table 4 herein including gene-specific oligonucleotides derived from said genes. In Table 4, accession numbers are given for the genes on the GenBank database. As will be appreciated by those skilled in the art, sequences available at the given accession numbers represent only examples of sequences of the genes referred to in the table; alternative sequences, including sequences which comprise sequencing error corrections, allelic or other variations, splice mutants and the like are also included in the definition of the gene represented by the name usesd. In a most preferred ebodiment, the sequences referred to are the sequences set forth at the accession numbers and specific sequences given and set out in detail in Table 4a.

In a further aspect the present description provides a set of isolated marker genes comprising at least one gene identified as having differential expression as between patients who are responders and non responders to an erbB receptor tyrosine kinase inhibitor; said gene set selected from the group consisting of the 51 genes listed in Table 4 herein including gene-specific oligonucleotides derived from said genes.

The present invention permits the improved prognosis and hence quality of life of cancer patients by matching the treatments to individual patients and so making more effective use of the types of drug available.

A preferred set is at least one or more of the first 40 genes listed in Table 4 herein.

A further preferred set is at least one or more of the first 20 genes listed in Table 4 herein.

A further preferred set is at least one or more of the first 12 genes listed in Table 4 herein.

A preferred set is at least one or more of the first 5 genes listed in Table 4 herein.

An especially preferred set is the first 12 genes listed in Table 4a herein, namely FLJ22662 (e.g. GenBank NM_024829), AREG (e.g. GenBank BC009799), CORO1C (e.g. GenBank NM_014325), AVEN (e.g. GenBank BC010488), DUSP3 (e.g. GenBank NM_004090, DJ473B4 (e.g. GenBank AI026836), PHLDA2 (e.g. GenBank BU500509), RBM7 (e.g. GenBank NM_016090), EST (GenBank BX092512), OSMR (e.g. GenBank AI436027), GCLC (e.g. GenBank AI971137), COL4A3BP (e.g. GenBank BQ024877).

Preferably the inhibitor is selected from gefitinib, OSI-774, PKI-166, EKB-569, GW2016, CI-1033 and an anti-erbB antibody such as trastuzumab and cetuximab.

Most preferably the inhibitor is gefitinib.

The present invention is particularly suitable for use in predicting the response to the aforementioned chemotherapeutic agents in those patients or patient population with a cancer mediated alone , or in part, by an erbB tyrosine kinase. Such cancers include, for example, non-solid tumours such as leukaemia, multiple myeloma or lymphoma, and also solid tumours, for example bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval tumours.

The present invention is particularly suitable for identifying those patients with NSCLC, more particularly advanced NSCLC including advanced adenocarcinoma that will respond to treatment with chemotherapeutic agents such as an erbB receptor tyrosine kinase inhibitor as hereinbefore defined.

The present invention offers considerable advantages in the treatment of cancers such as NSCLC, especially advanced NSCLC by identifying "individual cancer profiles" of NSCLC and so determining which tumours would respond to gefitinib. This includes 1$^{st}$ line treatment and any other treatment regimen, such as, for example chemotherapy failed patients.

The present invention is particularly useful in the treatment of patients with advanced NSCLC who have failed previous chemotherapy, such as platinum-based chemotherapy.

The present invention is also particularly useful in the treatment of patients with locally advanced (stage IIIB) or metastasized (stage IV) NSCLC who have received previous chemotherapy, such as platinum-based chemotherapy.

The present description also provides a method of predicting the responsiveness of a patient or patient population with cancer-, for example lung cancer, to treatment with chemotherapeutic agents, especially erbB receptor tyrosine kinase inhibitors, comprising comparing the differential expression of a set of marker genes said marker genes selected from the gene sets as defined above.

Preferably the assessment of expression is performed by gene expression profiling using oligonucleotide-based arrays or cDNA-based arrays of any type; RT-PCR (reverse transcription- Polymerase Chain Reaction), real-time PCR, *in-situ* hybridisation, Northern blotting, Serial analysis of gene expression (SAGE) for example as described by Velculescu et al Science 270 (5235): 484-487, or differential display. Details of these and other methods can be found for example in Sambrook et al, 1989, Molecular Cloning: A Laboratory Manual). Preferably the assessment uses a microarray assay.

Alternatively, or in addition, the assessment uses an immunohistochemical assay.

In a further aspect, the present description provides a kit for use in a method of predicting the responsiveness of a patient or patient population with cancer, to treatment with chemotherapeutic agents, especially erbB receptor tyrosine kinase inhibitors, comprising a marker gene set as defined above on a suitable support medium. Preferably the marker gene is attached to a support material or membrane such as nitrocellulose, or nylon or a plastic film or slide.

Preferably the kit comprises a microarray.

## Detailed Description Including Preferred Embodiments

The invention will be described in more detail and illustrated by the following examples which are meant to serve to assist one of ordinary skill in the art in carrying out the invention and are not intended in any way to limit the scope of the invention. Certain elements of the invention are also described in more detail below.

"Set of isolated marker genes"

These are, according to the context of the embodiments described herein, a group of genes which can be used in classification or categorisation of patent response according to the description.

"Differential expression"

Genes that are either expressed at a higher or lower level as between groups of responders or nonresponders.

"Responders/Non responders"

Objective tumour responses according to Union International Contre le Cancer/World Health Organization (U ICC/WHO) Criteria are categorised as follows: complete response (CR): no residual tumour in all evaluable lesions; partial response (PR): residual tumour with evidence of chemotherapy-induced 50% or greater decrease under baseline in the sum of all measurable lesions and no new lesions; stable disease (SD) residual tumour not qualified for CR; and progressive disease (PD): residual tumour with evidence of 25% or greater increase under baseline in the sum of all measurable lesions or appearance of new lesions. As defined herein, non responders are PD.
The present invention is particularly effective for determining those patients which are CR or PR
"ErbB receptor inhibitors including, without limitation, ErbB receptor tyrosine kinase inhibitors"
This family includes EGF, erbB2 (HER), erbB3 (note that erbB3 does not have a functional kinase domain) and erbB4 as described in the background to the invention above.

"Gene-specific oligonucleotides"

These are intended to be unique to the respective genes so that, for example, fragments of the gene that uniquely identify the gene. Advantageously, a gene-specific oligonucleotide is between 5 and 50 nucleotides in length, preferably about 15 to 30 nucleotides, and most preferably about 23 nucleotides.

"Arrays or microarrays"

Array technology and the various techniques and applications associated with it are described generally in numerous textbooks and documents. Gene array technology is particularly suited to the practice of the present invention. Methods for preparing micoarrays are well known in the art. These include Lemieux et al., (1998), Molecular Breeding 4, 277-289, Schena and Davis. Parallel Analysis with Biological Chips. in PCR Methods Manual (eds. M. Innis, D. Gelfand, J. Sninsky), Schena and Davis, (1999), Genes, Genomes and Chips. In DNA Microarrays: A Practical Approach (ed. M. Schena), Oxford University Press, Oxford, UK, 1999), *The Chipping Forecast (Nature Genetics* special issue; January 1999 Supplement), Mark Schena (Ed.), Microarray Biochip Technology, (Eaton Publishing Company), Cortes, 2000, The Scientist 14[17]:25, Gwynne and Page, Microarray analysis: the next revolution in molecular biology, Science, 1999 August 6; and Eakins and Chu, 1999, Trends in Biotechnology, 17, 217-218.
The technology is described in PCT/US01/10063 and US 2002 090979 and references therein.
Commercial suppliers include Affymetrix (California) and Clontech Laboratories (California).
Major applications for array technology include the identification of sequence (nucleotide sequence/nucleotide sequence mutation) and the determination of expression level (abundance) of nucleotide sequences. Gene expression profiling may make use of array technology, optionally in combination with proteomics techniques (Celis et al, 2000, FEBS Lett, 480(1):2-16; Lockhart and Winzeler, 2000, Nature 405(6788):827-836; Khan et al., 1999, 20(2):223-9). Other applications of array technology are also known in the art; for example, nucleotide sequence discovery, cancer research (Marx, 2000, Science 289: 1670-1672; Scherf, et al, 2000, Nat Genet;24(3):236-44; Ross et al, 2000, Nat Genet. 2000 Mar;24(3):227-35), SNP analysis (Wang et al, 1998, Science, 280(5366):1077-82), drug discovery, pharmacogenomics, disease diagnosis (for example, utilising microfluidics devices: Chemical & Engineering News, February 22, 1999, 77 (8):27-36), toxicology (Rockett and Dix (2000), Xenobiotica, 30(2):155-77; Afshari et al., 1999, Cancer Res1;59(19): 4759-60) and toxicogenomics (a hybrid of functional genomics and molecular toxicology). The goal of toxicogenomics is to find correlations between toxic responses to toxicants and changes in the nucleotide sequencetic profiles of the objects exposed to such toxicants (Nuwaysir, et al (1999), Molecular Carcinonucleotide sequencesis, 24:153-159).
In general, any library may be arranged in an orderly manner into an array, by spatially separating the members of the library. Examples of suitable libraries for arraying include nucleic acid libraries (including DNA, nucleotide sequence, oligonucleotide, etc libraries), peptide, polypeptide and protein libraries, as well as libraries comprising any molecules, such as ligand libraries, among others. Accordingly, where reference is made to a "library" such reference includes reference to a library in the form of an array.

The members of a library are generally fixed or immobilised onto a solid phase, preferably a solid substrate, to limit diffusion and admixing of the samples. In particular, the libraries may be immobilised to a substantially planar solid phase, including membranes and non-porous substrates such as plastic and glass. Furthermore, the samples are preferably arranged in such a way that indexing (i.e. reference or access to a particular sample) is facilitated. Typically the samples are applied as spots in a grid formation. Common assay systems may be adapted for this purpose. For example, an array may be immobilised on the surface of a microplate, either with multiple samples in a well, or with a single sample in each well. Furthermore, the solid substrate may be a membrane, such as a nitrocellulose or nylon membrane (for example, membranes used in blotting experiments). Alternative substrates include glass, or silica based substrates. Thus, the samples are immobilised by any suitable method known in the art, for example, by charge interactions, or by chemical coupling to the walls or bottom of the wells, or the surface of the membrane. Other means of arranging and fixing may be used, for example, pipetting, drop-touch, piezoelectric means, ink-jet and bubblejet technology, electrostatic application, etc. In the case of silicon-based chips, photolithography may be utilised to arrange and fix the samples on the chip. The samples may be arranged by being "spotted" onto the solid substrate; this may be done by hand or by making use of robotics to deposit the sample. In general, arrays may be described as macroarrays or microarrays, the difference being the size of the sample spots. Macroarrays typically contain sample spot sizes of about 300 microns or larger and may be easily imaged by existing gel and blot scanners. The sample spot sizes in microarrays are typically less than 200 microns in diameter and these arrays usually contain thousands of spots. Thus, microarrays may require specialised robotics and imaging equipment, which may need to be custom made. Instrumentation is described generally in a review by Cortese, 2000, The Scientist 14[11]:26.

Techniques for producing immobilised libraries of DNA molecules have been described in the art. Generally, most prior art methods describe how to prepare single-stranded nucleic acid molecule libraries, using for example masking techniques to build up various permutations of sequences at the various discrete positions on the solid substrate. US 5,837,832 describes an improved method for producing DNA arrays immobilised to silicon substrates based on very large scale integration technology. In particular, US 5,837,832 describes a strategy called "tiling" to prepare specific sets of probes at spatially-defined locations on a substrate which may be used to produced the immobilised DNA libraries of the present invention. US 5,837,832 also provides references for earlier techniques that may also be used.

To aid detection, targets and probes may be labelled with any readily detectable reporter such as a fluorescent, bioluminescent, phosphorescent, radioactive reporter. Labelling of probes and targets is disclosed in Shalon et al., 1996, Genome Res 6(7):639-45.

The materials for use in the methods of the present invention are ideally suited for preparation of kits. A set of instructions will typically be included.

## GENERAL RECOMBINANT DNA METHODOLOGY TECHNIQUES

The present invention employs, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press.

In a specific embodiment, a cDNA microarray system representing 27, 648 genes was used to select a set of genes predicating the responsiveness to gefitinib for advanced NSCLC. Statistical analysis of the expression profiles identified dozens of genes differentially expressed between responders and non-responders to gefitinib. A drug response scoring (DRS) system based on the expression of these genes successfully predicted the response to gefitinib therapy.

## Description of the Figures

**Figure 1:** Images illustrating laser-microbeam microdissection of four representative lung adenocarcinomas. The upper row shows the samples before dissection; the lower row, dissected cancer cells (H.E. stain X100). TBB indicates transbronchial biopsy; LN, lymph-node.

**Figure 2:** Establishing a scoring system to predict the efficacy of gefitinib treatment.

**A.** Different prediction scores appear when the number of discriminating genes is changed. The number of the discriminating gene sets (from 5 to 51) corresponds to the number of selected genes from the top of the rank-ordered list in table 4. A larger value of classification score (CS) indicates better separation of the two groups.
**B.** Hierarchical clustering of 17 "learning" cases using 51 candidate genes for gefitinib-sensitivity (left), and 12 prediction genes that were finally selected for the GRS (right). The dendrograms represent similarities in expression patterns among individual cases; longer branches indicate greater differences. The two groups were most clearly separated by the 12-gene set.
**C.** Schematic distinction of responder, non-responder and "test cases" verified on the basis of the GRS. Red diamonds denote prediction scores for learning PR cases and blue diamonds represent learning PD cases. A pink triangle indicates a test PR case that had not been used for establishing GRS, and blue triangles indicate test PD cases. Yellow triangles indicate test SD cases that kept the SD status throughout the 4-month observation period, and green triangles indicate test cases once judged as SD at a certain-time point of the study but showed progression of the disease within three or four months after the start of treatment.

**Figure 3:** Validation of GRS with semi-quantitative RT-PCR and immunohistochemical analyses.

**A.** Representative image of semi-quantitative RT-PCR analysis of RNAs from the PR and PD groups. *OSMR* and *GCLC* genes were over-expressed in non-responders (PD). The integrity of each cDNA template was controlled through amplification of *ACTB.*
**B.** Immunohistochemical staining of representative samples from fiberscopic transbronchial biopsy (TBB) and lymph-node (LN) biopsy from the same PD-patient (No. LC21), using anti-AREG antibody (X 200).
**C.** Immunohistochemical staining of representative samples from PD patients, using antibodies for other 4 prediction markers (TGFA, ADAM9, CD9, and OSMR) (X200).

**Figure 4:** Serologic concentration of TGFA determined by ELISA in 5 PR, 10 SD, and 20 PD adenocarcinoma cases. The averaged serum levels of TGFA were shown as black bars: 19·0 $\pm$ 2·8 pg/ml (mean$\pm$SE) in PD patients, 13·9 $\pm$ 1·9 pg/ml in SD patients, and 12·8 $\pm$ 1·4 pg/ml in PR patients.

**Figure 5:** Anti-apoptotic effect of secreted AREG on gefitinib-sensitive PC-9 cells.

**A.** Expression of *AREG* transcript examined by semi-quantitative RT-PCR in lung-adenocarcinoma cell lines PC-9, NCI-H358, and -H522.
**B.** PC-9 cells cultured in medium supplemented with 10% FCS, in serum-free medium, or in serum-free conditioned medium (CM) obtained from cultures of NCI-H358 or -H522 cells. Each medium was replaced once with the same medium at the 48-hour time point; 72 hours after adding gefitinib at concentrations of 0·5 or 1·0 $\mu$M, cell viability was measured by MTT assays. The experiments were done in triplicate. The Y-axis indicates the relative MTT value (MTT in the presence of 0·5 or 1·0 $\mu$M gefitinib/MTT in the absence of gefitinib) of the cells incubated in different media.
**C.** Effect of AREG, secreted in an autocrine manner, on the resistance of NSCLC cells to gefitinib. At the start of culture, PC-9 cells were inoculated into medium containing 1·0 $\mu$M gefitinib and recombinant AREG protein (final concentrations of 1-100 ng/ml); 72 hours later, cell viability was measured by triplicate MTT assays (blue bars). The Y-axis indicates the relative MTT values (MTT at individual concentrations of AREG/MTT without AREG) of the cells.
Effect of AREG on the viability of NSCLC cells in the absence of 1·0 $\mu$M gefitinib was also studied. Individual PC-9 cells were added to medium containing recombinant AREG protein but no gefitinib; 72 hours later, viability was measured by triplicate MTT assays (red bars).

**Figure 6:** Immunohistochemical analysis of amphiregulin expression in sections derived from PD and PR patients.

Materials and Methods

***Patients and tissue samples***

A phase II clinical study was carried out comprising a multi-center trial to explore the dominant biological factors responsible for clinical anti-tumor effect, adverse drug reactions (ADR) and pharmacokinetics of ZD1839 dosed 250mg daily in patients with advanced non-small-cell lung cancer who have failed previous chemotherapy. The primary endpoint was to clarify a gene-expression profile that could determine in advance a potential anti-tumor effect of gefitinib. At the start of the study, the sample size was estimated using studies conducted thus far as a rationale.[12,13] Since the response rate for gefitinib has been less than 20% in patients with lung cancer,[8-10] about 50 patients were estimated to be required to obtain learning cases estimated above. Patients whose locally advanced (stage IIIB) or metastasized (stage IV) NSCLCs were resistant to one or more regimens of conventional chemotherapy were enrolled in this trial. Inclusion criteria were (1) age greater than 20 years, (2) Performance Status (PS) 0-2, (3) adequate liver and kidney function tests. All patients were treated with 250mg of gefitinib orally once a day at the Tokushima University or Kinki University hospitals in Japan. The treatment was continued until the patient was dropped from the study due to (1) progression of disease, (2) intolerable toxicity, or (3) withdrawal of consent.

Objective tumor responses were assessed every 4 weeks after the beginning of treatment, according to criteria outlined by the *Union International Contre le Cancer*/World Health Organization (UICC/WHO). Response categories were as follows: complete response (CR), no residual tumor in any evaluable lesion; partial response (PR), residual tumor with evidence of 50% or greater decrease under baseline in the sum of all measurable lesions, and no new lesions; progressive disease (PD), residual tumor with evidence of 25% or greater increase under baseline in the sum of all measurable lesions, or appearance of new lesions; and stable disease (SD), residual tumor not qualified for CR, PR, or PD. All evaluable lesions were measured bi-dimensionally (sum of products of longest diameter and its longest perpendicular of measurable lesions) using the same techniques as baseline, e.g. plain X-ray, CT, or MRI.

At the end of 4-month treatment (or withdrawal), the best overall response was evaluated for each patient based on definitions as follows: CR, patients who qualified for CR at two sequential examination points with an interval of at least 28 days between them; PR, patients judged as PR or better at two sequential examination points with an interval of at least 28 days between them; SD, patients who were SD or better at two sequential examination points at least 28 days apart but who did not qualify as CR or PR. The first judgment of an SD case must be done at or after the first tumor assessment point (28 days after randomization); PD, the patients determined as PD at or before the first tumor assessment point (28 days after randomization); Unknown, the patient does not qualify for a best response of increased disease, and all objective statuses after baseline (before randomization) and before progression are unknown.

Prior to the gefitinib treatment, tumor specimens were taken by transbronchial (TBB), skin, or lymph-node biopsy with written informed consent from each patient. Ethics approval was obtained from the ethics committee of the individual institutes. Biopsy samples were frozen immediately, embedded in TissueTek OCT medium (Sakura, Tokyo, Japan), and stored at -80 °C. All samples were examined microscopically, and samples from 28 patients (17 learning and 11 test cases) that contained enough cancer cells for analysis of expression profiles were initially selected for further analysis. For validation of the prediction system, a blinded set of samples from 5 newly enrolled cases (4 PD and 1 SD) were also added to the 11 test cases. Clinical and histological information about these patients is summarized in Table 1-3.

***Microdissection***

In view of significant differences in the proportions of cancer cells and various types of parenchymal cells that are present from one tumor to another, microdissection is a necessary means of obtaining precise gene-expression profiles on cDNA microarrays. Therefore we stained 8 μm-thick frozen sections with hematoxylin and eosin and collected cancer cells selectively, using the μCUT laser-microbeam microdissection system (Molecular Machines & Industries AG, Glattbrugg, Switzerland).[14] In this system tissue sections are mounted on a thin supporting polyethylene membrane that will be cut together with the target tissue; a pulsed-ultraviolet (UV) narrow-beam-focus laser cuts out cancer cells along a pre-selected track that can be observed on a video screen. The material to be extracted is never directly exposed to the laser but only circumscribed by it; unlike other LMM systems, this one allows recovery of dissected cells to proceed without radiation. Moreover, the membrane protects the tissue on the slide against cross-contamination. Using this system we were able to isolate small areas of tissue rapidly, and to isolate single cells from histological sections (figure 1).

***RNA extraction and T7-based RNA amplification***

Total RNA was extracted from individual microdissected populations of cancer cells using RNeasy mini kits and RNase-free DNase kits (QIAGEN, Hilden, Germany) according to the manufacturer's protocols. Total RNAs were subjected to T7-based RNA amplification, as described previously.[15] Two rounds of amplification yielded 40-200 μg of aRNA (amplified RNA) (>100,000-fold) from each sample. As a control probe, normal human lung poly(A)$^+$RNA (BD Biosciences Clontech, Palo Alto, CA and BIOCHAIN, Hayward, CA, USA) was amplified in the same way. Aliquots (2·5 μg) of aRNA from individual samples and from the control were reversely transcribed in the presence of Cy5-dCTP and Cy3-dCTP respectively.

***cDNA microarray***

Our "genome-wide" cDNA microarray system contains 27,648 cDNAs selected from the UniGene database of the National Center for Biotechnology Information.[15] Fabrication of the microarray, hybridization, washing, and detection of signal intensities were described previously.[15] To normalize the amount of mRNA between tumors and controls, the Cy5/Cy3 ratio for each gene's expression was adjusted so that the averaged Cy5/Cy3 ratio of 52 housekeeping genes was equal to one. We assigned a cutoff value to each microarray slide using analysis of variance, and the Cy5/Cy3 ratio of the gene was calculated as follows: (1) if Cy5 (cancer sample) was lower than the cut off level, then the Cy5/Cy3 ratio of the gene was substituted by 2·5 percentile among the Cy5/Cy3 ratios of other genes whose Cy5 and Cy3 were higher than the cut off level; (2) if Cy3 (control sample) was lower than the cut off level, then the Cy5/Cy3 ratio of the gene was substituted by 97·5 percentile among the Cy5/Cy3 ratios of other genes whose Cy5 and Cy3 were higher than the cut off level; (3) if both Cy5 and Cy3 were lower than the cut off level, then the Cy5/Cy3 ratio of the gene was left blank.

*Extraction of genes for predicting responsiveness to gefitinib*

To discover genes that might be associated with sensitivity to gefitinib, individual measurements of about 27,648 genes were compared between the two groups of patients, one classified as responders to gefitinib (PR) and the other as non-responders (PD). To reduce the dimensionality of the number of potent genes that could discriminate between the two classes, we extracted only genes that fulfilled two criteria: 1) signal intensities were higher than the cut-off level in at least 60% of either group, and 2) $| MED_{PR} - MED_{PD} |$□1, where MED indicates the median calculated from log-transformed relative expression ratios in each group. Then random-permutation tests were applied to estimate the ability of individual genes to distinguish between the two classes (PR and PD); mean ($\mu$) and standard deviations ($\sigma$) were calculated from the log-transformed relative expression ratios of each gene in both groups. A discrimination score (DS) for each gene was defined as follows:

$$DS = (\mu_{PR} - \mu_{PD}) / (\sigma_{PR} + \sigma_{PD}).$$

The samples were randomly permutated 10,000 times for each pair of groups. Since the DS dataset of each gene showed a normal distribution, we calculated a *p*-value for the user-defined grouping.

***Calculation of drug-response scores***

We calculated gefitinib response scores (GRS) reflecting the expression levels of candidate prediction-genes according to procedures described previously.[16-18] Each gene (gi) votes for either responder (PR) or non-responder (PD) depending on whether the expression level (xi) in the sample is closer to the mean expression level of one group or the other in reference samples. The magnitude of the vote (vi) reflects the deviation of the expression level in the sample from the average of the two classes:

$$Vi = | xi - (\mu_{PR} + \mu_{PD}) /2 |.$$

We summed the votes to obtain total votes for responders ($V_{PR}$) and non-responders ($V_{PD}$), and calculated GRS values as follows:

$$GRS = ((V_{PR} - V_{PD}) / (V_{PR} + V_{PD})) \times 100,$$

where the GRS value reflects the margin of victory in the direction of either responder or non-responder. GRS values range from -100 to 100; the higher an absolute value of GRS, the stronger the prediction.

***Cross-validation of scores and evaluation of the prediction system***

The prediction scores of all samples were obtained by a leave-one-out approach, in which one sample at a time was removed from the sample set; permutational *p*-values and mean values of the two classes were calculated for each gene using the remaining samples. The drug-response of the withheld sample was predicted by calculating the prediction score. These procedures were repeated for each sample.[16-17]

To evaluate the reliability of the prediction system, we calculated a "classification score" (CS) using the GRS values of responders and non-responders in each gene set, as follows:

$$CS = (\mu_{GRSpr} - \mu_{GRSpd})/(\sigma_{GRSpr} + \sigma_{GRSpd}).^{17}$$

A larger value of CS indicates better separation of the two groups by the prediction system.

***Hierarchical clustering***

We used web-available software ("Cluster" and "TreeView") written by M. Eisen (http://genome-www5.stanford.edu/MicroArray/SMD/restech.html) to create a graphic representation of the microarray data and to create a dendrogram of hierarchical clustering. Before the clustering algorithm was applied, the fluorescence ratio for each spot was first log-transformed and then the data for each sample were median-centered to remove experimental biases.

***Semi-quantitative RT-PCR analysis***

*Aliquots (5·0 µg) of the same aRNA hybridized to the microarray slides from individual samples and from the normal control lung were reversely transcribed using oligo(dT)$_{12-18}$ primer and Super-Script II reverse transcriptase (Invitrogen, Carlsbad, CA, USA). Semi-quantitative RT-PCR experiments were carried out with the following sets of synthesized primers specific to the 12 top-ranked genes used for establishing a GRS or with beta-actin (ACTB)-specific primers as an internal control:* FLJ22662, *5'-GCCATAAGTGGTCCCACAGT-3' and 5'-GTCTTCTAGTCCGTCATCTCCCT-3'; Amphiregulin* (AREG), *5'-CCATAGCTGCCTTTATGTCTGC-3' and 5'-CTTTTTACCTTCGTGCACCTTT-3'; coronin, actin binding protein, 1C* (CORO1C), *5'-TAATCTGCTGAGGACCTTTTGTC-3' and 5'-TAATTCACTGTCCTCTTCTGGGA-3'; apoptosis, caspase activation inhibitor* (AVEN), *5'-GCTCACAGCAGTAAATGCCTA-3' and 5'-TGCTATGCTGTAAACACTGGCTA-3'; dual specificity phosphatase 3* (DUSP3), *5'-GGATCCTTTATTGGTGGTAGAGC-3'and 5'-CCAGAGTGACCCTGAAGATAAAT-3';* DJ473B4, *5'-ACCTGATTCTCTAGGTGCAGTTT-3' and 5'-GTCGTTTCAACCAGGTAGTTTTG-3'; pleckstrin homology-like domain, family A, member 2* (PHLDA2), *5'-GGGCGCCTTAAGTTATTGGA-3' and 5'-GGATGGTAGAAAAGCAAACTGG-3'; RNA binding motif protein 7* (RBM7), *5'-TGTAATGGAGATTGTACAGGTTG-3' and 5'-AGGAACAGTACAAATGCTGTGGT-3';* BX092512 *(EST), 5'-GCACTCCTTGAAGGTACACTAAC-3' and 5' ATTTGTATTCACTCAGCCATGC-3'; oncostatin M receptor* (OSMR), *5'-ACCCAACTTCAAAACTAGGACTC-3' and 5'-ACAGCTTGATGTCCTTTCTATGC-3'; glutamate-cysteine ligase, catalytic subunit (GCLC), 5'-TCATGAAAGGCACTGAGTTTTG-3' and 5'-GTTAGCTGAAGCAGCTTTATTGG3'; collagen, type IV, alpha 3 binding protein* (COL4A3BP), *5'-ATATGCACAATCCTGGAAGTGA-3' and 5'-TGCCTTACTAGCATTACCACCAT-3';* ACTB, *5'-GAGGTGATAGCATTGCTTTCG-3' and 5'-CAAGTCAGTGTACAGGTAAGC-3'. PCR reactions were optimized for the number of cycles to ensure product intensity within the logarithmic phase of amplification. We did phosphorimager quantification analysis (Molecular Imager FX: Bio-Rad Laboratories, Hercules, CA, USA), and RT-PCR band intensities were quantitatively compared with normalized Cy5/Cy3 ratio of gene expression from the microarray data.*

RT-PCR was performed to screen the mutation at entire region of codon 709 - 870 (from p-loop to activation loop) of *EGFR* which was recently reported as a hot spot of mutation,[18] using three primer sets: fragment-1, 5'-TCTTACACCCAGTGGAGAAGC-3' and 5'-GTCTTTGTGTTCCCGGACAT-3'; fragment-2, 5'-ACTATGTCCGGGAACACAAA-3' and 5'-TTCCGTCATATGGCTTGG-3'; fragment-3, 5'-CGTCGCTATCAAGGAATTAAGAG-3' and 5'-GTAGCTCCAGACATCACTCTGGT-3'. RT-PCR products from 19 NSCLC patients treated with gefitinib were analyzed by direct sequencing.

***Immunohistochemical analysis***

To confirm the differential expression of AREG and transforming growth factor-alpha (TGFA) proteins, both of which encode the ligand for EGFR and other ERBB members, and other 3 candidate markers (a disintegrin and metalloproteinase domain 9 (ADAM9), CD9 antigen (p24), and OSMR), which are also known to relate to the EGFR signaling, for predicting responders vs non-responders to gefitinib, we stained clinical tissue sections obtained by fiberscopic transbronchial biopsy (TBB) and lymph-node biopsy using ENVISION+ Kit/HRP (DakoCytomation, Glostrup Denmark). Briefly, after endogenous peroxidase and protein blocking reactions, anti-human AREG polyclonal antibody (Neo Markers, Fremont, CA, USA), anti-human TGFA monoclonal antibody (Calbiochem, Darmstadt, Germany), anti-human ADAM9 monoclonal antibody (R&D Systems Inc. Minneapolis, MN, USA), anti-human CD9 monoclonal antibody (Novocastra Laboratories Ltd, Newcastle upon Tyne, UK), or anti-human OSMR monoclonal antibody (Santa Cruz Biotechnology, Inc., Santa Cruz, CA, USA), was added, and then HRP-labeled anti-rabbit or antimouse IgG as the secondary antibody. Substrate-chromogen was then added and the specimens were counterstained with hematoxylin.

Frozen tissue samples from 11 patients were selected for analysis of immunohistochemistry. Positivity of immunostaining was assessed semi-quantitatively by scoring intensity as absent or positive by three independent investigators without prior knowledge of the clinical follow-up data. Cases were accepted only as positive if reviewers independently defined them thus.

***ELISA***

Serum was obtained from an independent set of 35 lung-ADC patients who were treated with gefitinib based on the same protocol as this clinical study at Hiroshima University hospital in Japan (5 for PR, 10 for SD, and 20 for PD). The sera of all the patients were obtained with informed consent at the time of diagnosis and every 4 weeks after the beginning of treatment, and stored at -80 °C. The serum TGFA levels were measured by an ELISA using a commercially available enzyme test kits (TGF-alpha ELISA kit: Oncogene Rsearch Products, San Diego, CA, USA).

***In vitro gefitinib treatment and AREG-autocrine assay***

Human NSCLC (adenocarcinoma) cell lines PC-9, NCI-H358, and NCI-H522 were purchased from the American Type Culture Collection (ATCC; Rockville, MD, USA). To detect expression of *AREG* in these NSCLC cells, total RNA from each line was reverse-transcribed for single-stranded cDNAs using oligo(dT)$_{12-18}$ primer and Superscript II (Invitrogen). Semi-quantitative reverse transcriptase-PCR (RT-PCR) was carried out as described previously.[14] gefitinib (4-(3-chloro-4-fluoroanilino)-7-methoxy-6-(3-morpholinopropoxy) quinazoline: ZD1839, Iressa), an inhibitor of epidermal growth factor receptor tyrosine kinase, was provided by AstraZeneca Pharmaceuticals (Macclesfield, UK). The drug was dissolved in DMSO at a concentration of 10mM and kept at -20°C.

We performed flow-cytometry to determine the sensitivity of lung adenocarcinoma cell lines to gefitinib treatment. Cells were plated at densities of 5 X $10^5$ cells/100-mm dish and treated with 1·0 μM of gefitinib in appropriate serum-free medium. The cells were trypsinized 72 hours after the treatment, collected in PBS, and fixed in 70% cold ethanol for 30 min. After treatment with 100 μg/ml RNase (Sigma-Aldrich Co., St. Louis, MO, USA), the cells were stained with 50 μg/ml propidium iodide (Sigma-Aldrich Co.) in PBS. Flow cytometry was performed on a Becton Dickinson FACScan and analyzed by ModFit software (Verity Software House, Inc., Topsham, ME, USA). The percentages of nuclei in G0/G1, S, and G2/M phases of the cell cycle and sub-G1 population were determined from at least 20,000 ungated cells.

To investigate whether AREG functions as an autocrine anti-apoptotic factor in lung adenocarcinoma cells treated with gefitinib, we carried out the following assay. First, gefitinib-sensitive PC-9 cells, which do not express *AREG,* were cultured in serum-free medium for at least 8 hours prior to gefitinib treatment. These cells were then incubated with 0·5 or 1·0 μM of gefitinib for 72 hours in media that were either serum-free or supplemented with 10% FCS, or in serum-free conditioned medium collected from 72-hour cultures of *AREG*-expressing cells (NCI-H358 or NCI-H522). Each medium was replaced once with the same medium containing gefitinib at the 48-hour time point. To detect the response of each cell line to gefitinib, viability was evaluated by MTT assays using Cell Counting Kits (WAKO, Osaka, Japan).

To confirm the autocrine effect of AREG on the gefitinib-resistance of NSCLC cells, we cultured PC-9 cells for 72 hours in serum-free medium containing 1·0 μM of gefitinib and recombinant AREG protein (Genzyme-Techne, Minneapolis, MN, USA) in final concentrations of 1-100 ng/ml. Cell viability was evaluated by MTT assays. A possible effect of AREG itself on the viability of NSCLC cells was evaluated also, by culturing the PC-9 cells in serum- and gefitinib-free medium containing only recombinant AREG protein. MTT assays were performed as above.

**Results**

***Response to gefitinib treatment***

Of the 53 patients enrolled in this trial, 46 had tumors diagnosed as adenocarcinomas (86.8%); five were squamous-cell carcinomas (9·4%); two were large cell carcinomas (3·8%). Fifteen patients achieved a PR and nobody revealed a CR; 17 patients were classified as SD, and 19 as PD. No clinical-response data were available for two of the patients. The tumor-response rate (CR+PR/CR+PR+SD+PD) for this treatment was 29·4%, and the disease control rate (CR+PR+SD/CR+PR+SD+PD) was 62-8% (table 1).

Tumor samples were collected from 43 patients. Samples from 32 of those 43 contained sufficient numbers of cancer cells for analysis of expression profiles on our cDNA microarray. The numbers of samples that were judged to be suitable for further microarray analysis, were 8 for PR, 7 for SD, and 13 for PD (table 2). 17 of the 28 samples were analyzed as learning cases (7 for PR and 10 for PD), and 11 were as test cases (1 for PR, 3 for PD, and 7 for SD) for establishing a predictive scoring system for the efficacy of gefitinib treatment. For further validation of the prediction system, another blinded set of samples from 5 newly enrolled test-cases (4 for PD and 1 for SD) were obtained and added finally to the initial 11 test cases above.

***Identification of genes associated with sensitivity to gefitinib***

We attempted to extract genes that were differentially expressed between tumors from seven patients in the PR group (defined as responders) and those from 10 patients in the PD group (defined as non-responders) by comparing expression levels of 27,648 genes. (tables 2, 3).

We carried out a random-permutation test to distinguish between the two subclasses defined by tumor response, and identified 51 genes whose permutational *p*-values were less than 0·001 (table 4). Expression levels of 40 genes were higher, and those of the other 11 were lower, in the non-responders.

Establishment of a predictive scoring system for the efficacy of gefitinib treatment

Based on the expression profiles of the 51 genes selected above, we tried to establish a predictive scoring system for the efficacy of gefitinib treatment. Prediction scores, termed gefitinib response score (GRS), were calculated according to procedures described previously (see Methods). To determine the number of candidates that provided the best separation of the two groups, we ranked the 51 genes on the basis of the significance of their permutational *p*-values and calculated prediction scores by the leave-one-out test, in decrements of 1 starting from the bottom of the rank-ordered list (51, 50, 49, 48 etc.). We calculated a classification score (CS), a standard we had previously defined for evaluation of the ability to discriminate two classes, for each set of genes.[17]

As shown in Figure 2A, we obtained different prediction scores when the number of discriminating genes was changed. We obtained the best CS, meaning the best separation of responders from non-responders, when we calculated the scores using only the 12 top-ranked genes in our candidate list.

Hierarchical clustering analyses using all 51 genes, or only the top 12, classified all 17 cases into one of two groups according to the response to gefitinib (figure 2, B). The two groups were most clearly separated when we used the top 12 genes for cluster analysis. Finally, we established a numerical drug-response-scoring algorithm that might be clinically applicable for predicting sensitivity of an individual NSCLC to gefitinib, on the basis of expression levels of the 12 selected genes.

To validate this prediction system we investigated 8 additional ("test") NSCLC cases (1 for PR and 7 for PD) that were completely independent of the 17 "learning" cases used for establishing the system. We examined gene-expression profiles in each of those samples and then calculated GRS on the basis of the expression levels of the 12 discriminating genes. As shown in Figure 2C, scores obtained by the GRS system were concordant with the clinical responses to gefitinib in all eight "test" cases.

***GRS values for patients with SD in tumor response***

GRS values for the eight test-SD patients were calculated according to the predictive scoring system established above. Although the values were widely distributed from - 83·0 (predicted as non-responder) to 61·6 (responder), the scores of patients who retained SD status throughout the observation period were likely to be higher than those of patients who had been judged as SD at a certain time-point of the study but showed progression of the disease within three or four months after the start of treatment (figure 2, C). Although the GRS system was established on the basis of gene-expression profiles that distinguished between patients with PR and patients with PD (without SD) in tumor response, these results suggest that the GRS serves in classifying SD patients into groups according to their response to gefitinib.

***Validation of GRS with semi-quantitative RT-PCR analysis***

To confirm differential expression of the top 12 predictive genes between PR and PD cases, expression values derived from microarray data were correlated with values from semi-quantitative RT-PCR of RNAs from the same patients (5 PR and 7 PD) (figure 3, A, table 5, A). Spearman rank correlations were positive for all of the 12 genes and significantly positive for seven of 12 genes.

***Immunohistochemical validation of GRS***

To validate differential expression of the predictive protein markers between PR and PD cases, we carried out immunohistochemical staining with five different antibodies for AREG, TGFA, ADAM9, CD9, and OSMR, all of which were known to be involved in the ligand-EGFRs signaling and whose permutational *p*-values were less than 0·01. We first stained paired tumor tissue sections obtained by TBB and lymph-node biopsy from the same patients using these 5 antibodies. No intra-patient differences on protein expression of these five markers were observed in three different patients (figure 3, B). We also validated the microarray data with the five markers in 11 NSCLC samples (5 for PR and 6 for PD). The results were consistent with the microarray data (figure 3, C, table 5, B).

***Serum levels of TGFA***

To further evaluate the availability of the prediction system in routine clinical situations, we detected TGFA protein using ELISA in serum samples from 5 PR, 10 SD, and 20 PD patients that were independently collected for serological test and were not enrolled in microarray analysis. The serum levels of TGFA were 19·0 ± 2·8 pg/ml (mean±SE) in PD patients, 13·9 ± 1·9 pg/ml in SD patients, and 12·8 ± 1·4 pg/ml in PR patients (figure 4). Twelve of 20 serum samples from PD patients were positive for TGFA and all samples from PR patients were negative, when 16·0 pg/ml was used as a cutoff.

***In vitro gefitinib treatment and AREG-autocrine assay***

AREG, a ligand for EGFR and other ERBB members was significantly over-expressed in non-responders but not (or hardly) detectable in responders. To investigate whether AREG protein leads to resistance of NSCLCs to gefitinib therapy when it is secreted in an autocrine manner, we performed the following biological analyses. We initially identified expression of AREG mRNA in lung-adenocarcinoma cell lines NCI-H358 and -H522, but not in PC-9, by means of RT-PCR experiments (figure 5, A). Next, we performed flow-cytometric analysis 72 hours after treatment of PC-9 cells with 1·0 μM of gefitinib, and found that gefitinib increased the percentages of nuclei in sub-G1 (24%) compared with cells with no treatment (6%) (data not shown). This result suggested that gefitinib might induce apoptosis in PC-9 cells.

We then analyzed the viability of PC-9 cells, which are gefitinib-sensitive and do not express *AREG,* after culture in serum-free medium or in serum-free, conditioned medium obtained from NCI-H358 or -H522 cells grown in the presence or absence of 0·5 or 1·0 μM of gefitinib. As shown in Figure 5B, the viability of PC-9 cells incubated in the serum-free, conditioned medium containing gefitinib was greater than that of PC-9 cells grown in serum-free medium with the same concentrations of gefitinib. As the supplier of gefinitib has reported previously, the anti-tumor effect of gefitinib decreases in the presence of 10% FCS, suggesting that this assay should be suitable for quantitative measurement of gefitinib dosage and activity.

To investigate whether AREG, secreted in an autocrine manner, inhibits apoptosis of NSCLC cells treated with gefitinib, we cultured PC-9 cells in serum-free medium containing recombinant AREG protein at final concentrations of 1-100 ng/ml, in the presence or absence of 1·0 μM gefitinib. The viability of PC-9 cells incubated with both AREG and 1·0 μM gefitinib was increased in comparison to cells incubated with 1·0 μM gefitinib only, in an AREG-dose-dependent manner (figure 5, C). On the other hand, recombinant AREG alone had no effect on the viability of PC-9 cells (figure 5, C). This observation appeared to indicate that AREG inhibits the apoptosis induced by gefitinib, but does not in itself affect cell viability. Immunostaining for AREG is shown in Figure 6.

**Discussion**

A large body of evidence supports the view that molecules in the EGFR autocrine pathway are involved in a number of processes important to cancer formation and progression, including cell proliferation, angiogenesis, and metastatic spread.[5] Therapeutic blockade of specific signaling, therefore, could be a promising strategy for cancer treatment. Gefitinib, a synthetic anilinoquinazoline, inhibits the tyrosine kinase activity of EGFR by competing with adenosine triphosphate for a binding site on the intracellular domain of the receptor.[7] In phase II trials (IDEAL 1 and IDEAL 2), use of gefitinib as a 2nd-, 3rd-, or 4th-line monotherapy for advanced NSCLC achieved tumor-response rates of nearly 20%,[8-10] which were superior to those achieved with conventional cytotoxic agents. Multivariate analysis of patients in the IDEAL 1 study suggested that the response rate in females might be higher than in males, and higher in patients with adenocarcinomas than in patients with squamous-cell carcinomas (odds ratios 2·7 and 3·5 respectively).[9] Recent study suggested that individuals in whom gefitinib is efficacious are more likely to have adenocarcinomas of the bronchioloalveolar subtype and to be never smokers (odds ratios 13·5 and 4·2 respectively).[19] The higher tumor-response rate (29·4%) documented in the clinical trial reported here might reflect a higher proportion of patients with adenocarcinoma (46 adenocarcinomas, five squamous-cell carcinomas and two large-cell carcinomas) than has been the case in other studies. The clinicopathological determinants of gefitinib sensitivity including bronchioloalveolar carcinoma (BAC) features are predictve to a certan extent,[9,10,19,20] however, previous reports and our observations obviously suggest that no factors can perfectly predict the response of NSCLC to gefitinib treatment. Therefore novel methods to discriminate responders from non-responders in advance could allow a more focused use of gefitinib in clinical settings.

By statistical analysis of gene-expression profiles of advanced NSCLCs obtained on cDNA microarrays, we identified dozens of genes associated with sensitivity to gefitinib. We introduced a prediction-scoring system based on expression of the 12 genes that had shown the most significant differences in expression levels between responder (PR) and non-responder (PD) groups. This set of genes was selected from expression profiles of lung adenocarcinomas; however, the GRS system successfully classified all eight of our "test" PR and PD cases in accord with their clinical responses to gefitinib, and one of them was a squamous-cell carcinoma. Moreover, this system was likely to separate intermediate tumor responses (SD) into two groups, one representing patients who succeeded in maintaining the tumor-static effect for a long period and the other representing patients who failed to do so.

In practical terms, we need to predict the chemosensitivity of individual tumors using the minimally invasive techniques available at every hospital, because patients with advanced NSCLCs are rarely candidates for surgical resection of their tumors. Therefore we have tried to establish a prediction system that requires only the amount of cancerous tissue that can be obtained by, for example, flexible bronchofiberscopy. By verifying individual steps of the method, we were able to precisely profile gene expression in biopsy specimens as small as 1 mm. Relevant microarray results were confirmed by semi-quantitative RT-PCR for 12 genes that showed the most significant differences to establish a GRS system. Furthermore, we validated the effectiveness of antibodies for 5 different biomarkers (AREG, TGFA, ADAM9, CD9, and OSMR), all of which were reported to be involved in the ligand-EGFR signaling, for discriminating potential responders from non-responders, in both TBB and lymph-node biopsy samples. Moreover, we were able to detect serum TGFA proteins in lung-ADC patients by ELISA. Further evaluation of these markers for clinical use are necessary, however, the limited number of genes required for prediction should eventually enable laboratories to diagnose in advance the efficacy of gefitinib treatment for an NSCLC patient, using routine procedures such as serological examinations of blood, PCR experiments, or immunohistochemical analysis of biopsy specimens.

To our knowledge, this is the first report about gene-expression profiles of unresectable "advanced" lung cancers, although profiles of surgically resected specimens of "early" lung cancers have been reported.[21,22] However, about 70% of tumors in patients diagnosed with NCSLC are already locally advanced or metastatic, which generally renders them resistant to conventional therapeutic modalities. Therefore the genes listed here should be useful for disclosing molecular mechanisms of lung-cancer progression and may be potential targets for drug development.

Gefitinib was developed as a "selective" inhibitor of EGFR-TK; however, no clear association between the level of EGFR activation and response to gefitinib has been found *in vitro* or *in vivo*.[7,23] In clinical trials, gefitinib has been more effective against adenocarcinomas than against squamous-cell carcinomas,[9,10] although over-expression of EGFR is less frequent in adenocarcinomas.[24] Therefore, it is important to identify which individual tumors are good targets for this treatment. In our analysis using clinical samples, the difference in EGFR protein expression between responders and non-responders were not statistically significant. On the other hand, amphiregulin *(AREG)* and transforming growth factor alpha *(TGFA),* both of which encode the ligand for EGFR and other ERBB members, were significantly over-expressed in non-responders but not (or hardly) detectable in responders ($p$=0·0000000000093 and 0·0095 respectively; table 4).

The significance of the ligands and the EGFR autocrine loop in growth and survival of lung-cancer cells is indisputable,[24-26] but the role of AREG in formation and progression of cancers is poorly understood. However, several lines of evidence suggest that over-expression of *AREG* is associated with shortened survival of patients with NSCLC.[24] Moreover, anti-apoptotic activity of AREG in human lung-adenocarcinoma cells was reported recently.[25] To investigate whether the anti-apoptotic activity of AREG leads to resistance of NSCLC cells to gefitinib therapy, we performed a biological assay using a gefitinib-sensitive but *AREG*-non-expressing NSCLC cell line, PC-9. We found that the anti-tumor activity of gefitinib on PC-9 cells was dramatically decreased by autocrine secretion of AREG. This evidence strongly suggests that although growth-factor signaling by the EGFR is markedly complicated at every step because of the multiplicity of ligands, dimerization partners, effectors, and downstream pathways,[26] AREG might be a principal activator of the ligands-receptor autocrine growth pathway that leads to cancer progression and resistance to gefitinib.

Several elements associated with the EGFR-TK pathway are present on our list of differentially-expressed genes. For example, genes encoding dual specificity phosphatase 3 *(DUSP3), ADAM9, CD9,* and *OSMR* were expressed predominantly in non-responders ($p$=0·00000000094, 0·01, 0·000022, and 0.0000011, respectively). *DUSP3* gene modulates EGFR signaling by dephosphorylating mitogen activated protein kinase (MAPK), a key mediator of signal transduction,[27] and ADAM9 is involved in activation of EGFR signaling by shedding the ectodomain of proHB-EGF (pro Heparin-binding epidermal growth factor-like growth factor).[28] CD9 physically interacts with transmembrane TGFA. CD9 expression strongly decreases the growth factor- and PMA- induced proteolytic conversions of transmembrane to soluble TGFA and strongly enhances the TGFA-induced EGFR activation.[29] OSMR is reported to be constitutively associated with ERBB2 in breast cancer cells.[30] Although other target molecules for gefitinib have been suggested, our results suggest that EGFR signaling is at least one of the important processes involved in response to this drug.

Since gefitinib can induce apoptosis of some cancer cells *in vivo,* other molecules with anti-apoptotic activity, as well as AREG, may contribute to a tumor's resistance to the drug. *AVEN* (apoptosis, caspase-activation inhibitor), which was specifically expressed in our non-responders ($p$=0·00000000042), is known to enhance the anti-apoptotic activity of Bcl-xL and to suppress Apaf-1-mediated caspase activation.[31] On the other hand, mechanisms regulating drug transport should also affect drug resistance. *GCLC* (glutamate-cysteine ligase, catalytic subunit), which plays an important role in cellular detoxification of anticancer drugs such as cisplatin, etoposide and doxorubicin,[32] was over-expressed in our group of non-responders (*p*=0·00000012). As these genes correlated negatively with responses to chemotherapy in our panel of tumors (i.e. the higher the expression of these genes, the greater the resistance to gefitinib), they might be involved in the mechanism(s) leading to that resistance. It should be noted also that the functions of nearly half of our candidate prediction-genes are unknown. Therefore further investigations will be needed to reveal more clearly the biological events underlying responses of NSCLCs to gefitinib.

In summary, we identified 51 genes whose expression differed significantly between responders and non-responders to gefitinib among human lung carcinomas, and established a numerical scoring system, based on expression patterns of 12 of those genes, to predict the response of individual tumors to this drug. Although further validation using a larger set of clinical cases will be necessary, the data presented here may yield valuable insights into the molecular events underlying signal-suppressing strategies and provide important information about gefitinib treatment for individual NSCLC patients by testing a set of genes with high predictive values.

<u>References</u>

1. Fossella, F.V., et al., Randomized phase III trial of docetaxel versus vinorelbine or ifosfamide in patients with advanced non-small-cell lung cancer previously treated with platinum-containing chemotherapy regimens. The TAX 320 Non-Small Cell Lung Cancer Study Group. J Clin Oncol, 2000. 18(12): p. 2354-62.

2. Non-small Cell Lung Cancer Collaborative Group. Chemotherapy in non-small cell lung cancer: a meta-analysis using updated data on individual patients from 52 randomised clinical trials. Bmj, 1995. 311(7010): p. 899-909.

3. Schiller, J.H., et al., Comparison of four chemotherapy regimens for advanced non-small-cell lung cancer. N Engl J Med, 2002. 346(2): p. 92-8.

4. Kelly, K., et al., Randomized phase III trial of paclitaxel plus carboplatin versus vinorelbine plus cisplatin in the treatment of patients with advanced nonsmall-cell lung cancer: a Southwest Oncology Group trial. J Clin Oncol, 2001. 19(13): p. 3210-8.

5. Baselga, J., Why the epidermal growth factor receptor? The rationale for cancer therapy. Oncologist, 2002. 7 Suppl 4: p. 2-8.

6. Traxler, P., Tyrosine kinases as targets in cancer therapy - successes and failures. Expert Opin Ther Targets, 2003. 7(2): p. 215-34.

7. Wakeling, A.E., et al., ZD1839 (Iressa): an orally active inhibitor of epidermal growth factor signaling with potential for cancer therapy. Cancer Res, 2002. 62(20): p. 5749-54.

8. Herbst, R.S., Dose-comparative monotherapy trials of ZD1839 in previously treated non-small cell lung cancer patients. Semin Oncol, 2003. 30(1 Suppl 1): p. 30-8.

9. Fukuoka, M., et al., Final results from a phase □ trial of ZD1839 ('Iressa') for patients with advanced non-small cell lung cancer (IDEAL 1). Pro Am Soc Clin Oncol 2002. 21;298a(A1188).

10. Kris, MG., et al., A phase II trial of ZD1839 ('Iressa') in advanced non-small cell lung cancer (NSCLC) patients who had failed platinum-and docetaxel-based regimens (IDEAL 2). Pro Am Soc Clin Oncol 2002. 21;292a(A1166).

11. Inoue, A., et al., Severe acute interstitial pneumonia and gefitinib. Lancet, 2003. 361(9352): p. 137-9.

12. Bohm, M., et al., Microbeam MOMeNT: non-contact laser microdissection of membrane-mounted native tissue. Am J Pathol, 1997. 151(1): p. 63-7.

13. Okabe, H., et al., Genome-wide analysis of gene expression in human hepatocellular carcinomas using cDNA microarray: identification of genes involved in viral carcinogenesis and tumor progression. Cancer Res, 2001. 61(5): p. 2129-37.

14. Kitahara, O., et al., Alterations of gene expression during colorectal carcinogenesis revealed by cDNA microarrays after laser-capture microdissection of tumor tissues and normal epithelia. Cancer Res, 2001. 61(9): p. 3544-9.

15. Golub, T.R., et al., Molecular classification of cancer: class discovery and class prediction by gene expression monitoring. Science, 1999. 286(5439): p. 531-7.

16. MacDonald, T.J., et al., Expression profiling of medulloblastoma: PDGFRA and the RAS/MAPK pathway as therapeutic targets for metastatic disease. Nat Genet, 2001. 29(2): p. 143-52.

17. Kaneta.Y., et al., Prediction of sensitivity to STI571 among chronic myeloid leukemia patients by genome-wide cDNA microarray analysis. Jpn J Cancer Res 2002. 93, p. 849-856.

18. Pavelic, K., et al., Evidence for a role of EGF receptor in the progression of human lung carcinoma. Anticancer Res, 1993. 13(4): p. 1133-7.

19. Kikuchi, T., et al., Expression profiles of non-small cell lung cancers on cDNA microarrays: Identification of genes for prediction of lymph-node metastasis and sensitivity to anti-cancer drugs. Oncogene, 2003. 22(14): p. 2192-205.

20. Heighway, J., et al., Expression profiling of primary non-small cell lung cancer for target identification. Oncogene, 2002. 21(50): p. 7749-63.

21. Beer, D.G., et al., Gene-expression profiles predict survival of patients with lung adenocarcinoma. Nat Med, 2002. 8(8): p. 816-24.

22. Miura, K., et al., Laser capture microdissection and microarray expression analysis of lung adenocarcinoma reveals tobacco smoking- and prognosis-related molecular profiles. Cancer Res, 2002. 62(11): p. 3244-50.

23. Moasser, M.M., et al., The tyrosine kinase inhibitor ZD1839 ("Iressa") inhibits HER2-driven signaling and suppresses the growth of HER2-overexpressing tumor cells. Cancer Res, 2001. 61(19): p. 7184-8.

24. Rusch, V., et al., Overexpression of the epidermal growth factor receptor and its ligand transforming growth factor alpha is frequent in resectable non-small cell lung cancer but does not predict tumor progression. Clin Cancer Res, 1997. 3(4): p. 515-22.

25. Fontanini, G., et al., Evaluation of epidermal growth factor-related growth factors and receptors and of neoangiogenesis in completely resected stage I-IIIA non-small-cell lung cancer: amphiregulin and microvessel count are independent prognostic indicators of survival Clin Cancer Res, 1998. 4(1): p. 241-9.

26. Brundage, M.D., D. Davies, and W.J. Mackillop, Prognostic factors in non-small cell lung cancer: a decade of progress. Chest, 2002. 122(3): p. 1037-57.

27. Hurbin, A., et al., Inhibition of apoptosis by amphiregulin via an insulin-like growth factor-1 receptor-dependent pathway in non-small cell lung cancer cell lines. J Biol Chem, 2002. 277(51): p. 49127-33.

28. Yarden, Y. and M.X. Sliwkowski, Untangling the ErbB signaling network. Nat Rev Mol Cell Biol, 2001. 2(2): p. 127-37.

29. Prenzel, N., et al., EGF receptor transactivation by G-protein-coupled receptors requires metalloproteinase cleavage of proHB-EGF. Nature 1999. 402(6764):884-8

30. Nelson, Chau, et al., Aven, a novel inhibitor of caspase activation, binds Bcl-xL and Apaf-1. Molec Cell 2000. 6: p. 31-41.

31. Tipnis, SR., et al., Overexpression of the regulatory subunit of r-glutamylcysteine synthetase in Hela cells increases r-glutamylcysteine synthetase activity and confers drug resistance. Biochem J 1999. 337, p. 559-566.

Table 1: Summary of baseline patient characteristics and response

| Characteristics | Percentage (%) | Number of Patient |
|---|---|---|
| Sex | | |
| male | 58·5 | (31) |
| female | 41·5 | (22) |
| Age | | |
| median | 59 | |
| range | 35-80 | |
| Histology | | |
| adenocarcinoma | 86·8 | (46) |
| squamous cell carcinoma | 9·4 | (5) |
| large cell carcinoma | 3·8 | (2) |
| Stage | | |
| IIIA | 1·9 | (1) |
| IIIB | 7·5 | (4) |
| IV | 90·6 | (48) |
| Performance Status | | |
| 0 | 26·4 | (14) |
| 1 | 60·4 | (32) |
| 2 | 13·2 | (7) |
| Number of Prior Regimen | | |
| 1 | 24·5 | (13) |
| 2 | 35·9 | (19) |
| 3 | 28·3 | (15) |
| 4 | 0 | (0) |
| 5 | 7·5 | (4) |
| 6 | 3·8 | (2) |
| Response to Gefitinib Therapy | | |
| CR | 0 | (0) |
| PR | 28·3 | (15) |
| SD | 32·1 | (17) |
| PD | 35·8 | (19) |
| unknown | 3·8 | (2) |
| Tumor Response Rate (%) (CR+PR/CR+PR+SD+PD) | 29·4 | (15) |
| Disease Control Rate (%) (CR+PR+SD/CR+PR+SD+PD) | 62·8 | (32) |

Table 2: Number of cases suitable for analysis and their best overall responses

| | Best Overall Response | | | | |
|---|---|---|---|---|---|
| Number of Cases | PR | SD | PD | Unknown | Total |
| All cases enrolled | 15 | 17 | 19 | 2 | 53 |
| Cases that consented to the study | 15 | 14 | 13 | 1 | 43 |
| Cases suitable for analysis | 8 | 10 | 13 | 1 | 32 |
| Learning cases (1) | 7 | 0 | 10 | 0 | 17 |

(continued)

| | Best Overall Response | | | | |
|---|---|---|---|---|---|
| Number of Cases | PR | SD | PD | Unknown | Total |
| Test cases (1,2) | 1 | 7 | 3 | 0 | 11 |

(1) Learning cases were used for developing the GRS, whereas test cases were used for validation of the
(2) Another blinded set or samples from 5 newly enrolled cases (4 PD and 1 SD) were also added to these 11 test cases later.

Table 3: Clinicopathological features of patients

| Case No. (*) | Sex | Age | Histology Type (1) | T | N | M | Stage Classification (2) | Number ot Previous Chemotherapy | EGFR Stained Tumour Cell (%) | EGFR mutation (3) | Plasma Gefitinib Concentration (ng/ml) | Response to Gefitinib (4) 1st month | 2nd month | 3rd month | 4th month | Best Overall Response (5) | Use tor Prediction (6) | GRS (7) |
|---|---|---|---|---|---|---|---|---|---|---|---|---|---|---|---|---|---|---|
| LC01 | female | 36 | ADC | 1 | 0 | 1 | N | 1 | | None detected | 258·9 | PR | PR | PR | PR | PR | learning | 100 |
| LC02 | male | 64 | ADC | 2 | 3 | 1 | N | 3 | 80 | | 140·3 | PR | PR | PR | PR | PR | learning | 100 |
| LC03 | female | 54 | ADC | 2 | 0 | 1 | N | 3 | 80 | | 167·0 | PR | PR | PR | PR | PR | learning | 100 |
| LC04 | female | 75 | ADC | 2 | 1 | 1 | N | 1 | 20 | None detected | 169·7 | PR | PR | PR | PR | PR | learning | 100 |
| LC05 | female | 73 | ADC | 0 | 2 | 1 | N | 5 | 30 | 46 A750del (2481 24950 | 300·6 | PR | PR | PR | PR | PR | learning | 100 |
| LC06 | female | 75 | ADC | 4 | 1 | 1 | N | 3 | | None detected | 874·0 | SD | PR | PR | PR | PR | learning | 100 |
| LC07 | female | 70 | ADC | 2 | 1 | 1 | N | 3 | 80 | 17_A750del (2485_2496d | 460·8 | SD | PR | PR | PR | PR | learning | 100 |
| LC08 | female | 47 | ADC | 4 | 3 | 1 | N | 2 | 95 | L858R (2819T>G) | 306·5 | PR | PR | PR | PR | PR | test | 54.8 |
| mean | (range) | 62 | (36-75) | | | | | 2·6 (1-5) | 64 (20-95) | | 334·7 (140·3-874·0) | | | | | | | |
| LC09 | female | 63 | ADC | 4 | 0 | 1 | N | 3 | 90 | | 743·4 | SD | SD | SD | SD | SD | test | 61.6 |
| LC10 | male | 56 | ADC | 2 | 0 | 1 | N | 6 | 70 | | 511·8 | SD | SD | SD | SD | SD | test | -9.8 |
| LC11 | male | 67 | ADC | 4 | 0 | 1 | N | 2 | 0 | | 631·3 | SD | SD | SD | SD | SD | test | -5.3 |
| LC12 | male | 53 | ADC | 4 | 3 | 1 | IV | 2 | | None detected | 306·1 | SD | SD | SD | PD | SD | test | -23.8 |
| LC13 | female | 56 | ADC | 4 | 2 | 0 | IIIB | 2 | 40 | | 364·8 | SD | SD | PD | | SD | test | -58.5 |
| LC14 | female | 62 | ADC | 4 | 2 | 1 | N | 3 | 60 | | 322·4 | SD | SD | PD | | SD | test | -83 |
| LC15 | male | 61 | ADC | 0 | 0 | 1 | N | 5 | 60 | | 278·9 | SD | SD | PD | | SD | test | -40.5 |
| mean | (range) | 60 | (53-67) | | | | | 3·3 (2-6) | 53 (0-90) | | 451·2 (278·9-631·3) | | | | | | | |
| LC16 | male | 42 | ADC | 4 | 3 | 1 | N | 5 | 90 | None detected | 212·6 | SD | PD | | | PD | learning | -63.9 |
| LC17 | female | 54 | ADC | 2 | 3 | 1 | IV | 2 | 50 | None detected | 320·6 | SD | PD | | | PD | learning | -86 |
| LC18 | female | 61 | ADC | 1 | 3 | 0 | IIIB | 2 | | None detected | 229·3 | SD | PD | | | PD | learning | -67.8 |
| LC19 | male | 59 | ADC | 0 | 2 | 1 | IV | 2 | 30 | W817C (2697G>T) | 150·7 | SD | PD | | | PD | learning | -57.1 |
| LC20 | male | 65 | ADC | 0 | 3 | 1 | N | 3 | | None detected | 167·8 | SD | PD | | | PD | learning | -59.1 |

(continued)

| Case No. (*) | Sex | Age | Histology Type (1) | T | N | M | Stage Classification (2) | Number ot Previous Chemotherapy | EGFR Stained Tumour Cell (%) | EGFR mutation (3) | Plasma Gefitinib Concentration (ng/ml) | Response to Gefitinib (4) 1st month | 2nd month | 3rd month | 4th month | Best Overall Response (5) | Use tor Prediction (6) | GRS (7) |
|---|---|---|---|---|---|---|---|---|---|---|---|---|---|---|---|---|---|---|
| LC21 | male | 55 | ADC | 4 | 3 | 1 | IV | 3 | 80 | None detected | | PD | | | | PD | learning | -73.1 |
| LC22 | male | 80 | ADC | 4 | 3 | 1 | N | 2 | 80 | Q787Q (2607G>A) | | PD | | | | PD | learning | -55.5 |
| LC23 | male | 35 | ADC | 4 | 0 | 1 | N | 5 | | None detected | | PD | | | | PD | learning | -100 |
| LC24 | male | 57 | ADC | 4 | 3 | 1 | IV | 1 | 0 | None detected | | PD | | | | PD | learning | -46.7 |
| | | | | | 2 | | | | | | | | | | | | | |
| LC25 | female | 65 | ADC | | 0 | 1 | IV | 1 | | None detected | 356·3 | PD | | | | PD | learning | -86.1 |
| LC26 | male | 64 | SCC | 3 | 3 | 1 | N | 2 | | None detected | 405·6 | SD | PD | | | PD | test | -67.7 |
| LC27 | female | 65 | ADC | 4 | 2 | 1 | N | 1 | | L858R(2819T>G) | | PD | | | | PD | test | -69.4 |
| LC28 | male | 74 | ADC | 2 | 1 | 1 | N | 1 | 10 | | | PD | | | | PD | test | -64.8 |
| mean | (range) | 60 | (35-80) | | | | | 2·3 (1-5) | 49 (0-90) | | 263·2 (150·7-405·6) | | | | | | | |

(1) ADC, adenocarcinoma; SCC, squamous-cell carcinoma.
(2) TNM clinical classification and stage grouping were assessed based on the UICC/WHO classification.
(3) Mutation at codon position 709 - 870 (from p-loop to activation loop) of *EGFR* (GenBank Accession No. NM005228).
(4) Objective Tumor Response to Gefitinib was assessed every 4 weeks after the start of treatment using UICC/WHO Criteria. PR, partial response; SD, stable disease; PD, progressive disease
(5) Overall Best Response was evaluated based on the definitions as mentioned in materials and methods.
(6) learning, samples used for developing the GRS; test, samples used for validation of the GRS.
(7) GRS: gefitinib response score determined by prediction system
(*) For further validation of the GRS, another blinded set of samples from 5 newly enrolled cases (4 PD and 1 SD) were also added to these 28 cases later.

Table 4. List of 51 candidate genes for discriminating responder (PR) from non-responder (PD) to gefitinib (*)

| Rank Orc | GenBank | Symbol | Gene Name | Predominantly Expressed Clas | Permutational *p*-value | Median-fold Difference (log2) |
|---|---|---|---|---|---|---|
| 1 | NM_0248 | *FLJ2266* | hypothetical protein FLJ22662 | PD | 8.1E-12 | 2.0 |
| 2 | BC00979 | *AREG* | amphiregulin (schwannoma-derived growth factor) | PD | 9.3E-12 | 8.0 |
| 3 | NM_0143 | *CORO1C* | coronin, actin binding protein, 1C | PD | 2.3E-10 | 4.6 |
| 4 | BC01048 | *AVEN* | apoptosis, caspase activation inhibitor | PD | 4.2E-10 | 4.3 |
| 5 | NM_0040 | *DUSP3* | dual specificity phosphatase 3 (vaccinia virus phosphatase VH1-rel | PD | 9.4E-10 | 4.4 |
| 6 | AI026836 | *DJ473B4* | hypothetical protein dJ473B4 | PD | 1.7E-09 | 8.0 |
| 7 | BU50050 | *PHLDA2* | pleckstrin homology-like domain, family A, member 2 | PD | 1.8E-09 | 8.0 |
| 8 | NM_0160 | *RBM7* | RNA binding motif protein 7 | PD | 1.8E-08 | 2.9 |
| 9 | BX092512 | | EST | PD | 7.7E-08 | 3.0 |
| 10 | AI436027 | *OSMR* | oncostatin M receptor | PD | 1.1E-07 | 3.7 |
| 11 | AI971137 | *GCLC* | glutamate-cysteine ligase, catalytic subunit | PD | 1.2E-07 | 3.9 |
| 12 | BQ02487 | *COL4A3* | collagen, type IV, alpha 3 (Goodpasture antigen) binding protein | PD | 2.0E-07 | 3.6 |
| 13 | U52522 | *ARFIP2* | ADP-ribosylation factor interacting protein 2 (arfaptin 2) | PD | 2.6E-07 | 2.8 |
| 14 | BM99605 | *C10orf9* | chromosome 10 open reading frame 9 | PD | 4.2E-07 | 2.5 |
| 15 | AK025452 | *NIP30* | NEFA-interacting nuclear protein NIP30 | PD | 5.1E-07 | 3.7 |
| 16 | N52048 | *KIAA077* | KIAA0776 protein | PD | 5.4E-07 | 7.2 |
| 17 | AA50700 | *SLC35F2* | solute carrier family 35, member F2 | PD | 6.0E-07 | 5.8 |
| 18 | AA22624 | *GAMLG* | calcium modulating ligand | PD | 6.8E-07 | 5.0 |
| 19 | AF005888 | *NOC4* | neighbor of COX4 | PD | 1.1E-06 | 4.0 |
| 20 | AF012281 | *PDZK1* | PDZ domain containing 1 | PD | 1.3E-06 | 4.5 |
| 21 | AI188190 | *DIS3* | mitotic control protein dis3 homolog | PD | 1.7E-06 | 3.8 |
| 22 | BC00153 | *CGI-48* | CGI-48 protein | PD | 2.0E-06 | 3.5 |
| 23 | NM_0070 | *CPSF6* | cleavage and polyadenylation specific factor 6, 68kDa | PD | 2.2E-06 | 3.4 |
| 24 | NM_0022 | *KIF3C* | kinesin family member 3C | PD | 2.2E-06 | 3.5 |
| 25 | BQ13523 | *CD9* | CD9 antigen (p24) | PD | 2.2E-06 | 1.7 |
| 26 | BC05132 | *LRRC8* | leucine rich repeat containing 8 | PD | 2.5E-06 | 3.4 |
| 27 | BC03850 | *SNF1LK* | SNF1-like kinase | PD | 2.6E-06 | 2.8 |
| 28 | U78556 | *CRA* | cisplatin resistance associated | PD | 2.7E-06 | 3.7 |
| 29 | BC03562 | *EGR2* | early growth response 2 (Krox-20 homolog, Drosophila) | PD | 3.4E-06 | 3.0 |
| 30 | X52426 | *KRT13* | keratin 13 | PD | 1.9E-05 | 3.4 |
| 31 | NM_0055 | *BCAT1* | branched chain aminotransferase 1, cytosolic | PD | 2.3E-05 | 1.7 |
| 32 | NM_0066 | *SDCCAG* | serologically defined colon cancer antigen 3 | PR | 2.6E-05 | 3.7 |
| 33 | AA46409 | *PIGK* | phosphatidylinositol glycan, class K | PD | 3.2E-05 | 1.1 |
| 34 | AA96118 | *MRPS9* | mitochondrial ribosomal protein S9 | PD | 9.8E-05 | 2.3 |
| 35 | NM_0181 | *ASPM* | asp (abnormal spindle)-like, microcephaly associated (Drosophila) | PR | 2.3E-04 | 2.8 |
| 36 | NM_0227 | *ACBD3* | acyl-Coenzyme A binding domain containing 3 | PD | 2.4E-04 | 3.8 |
| 37 | AA160544 | *ZNF325* | zinc finger protein 325 | PR | 2.7E-04 | 4.5 |
| 38 | AK05765 | *LOC285* | hypothetical protein LOC285513 | PD | 2.7E-04 | 3.8 |
| 39 | NM_0033 | *TSSC1* | tumor suppressing subtransferable candidate 1 | PD | 2.9E-04 | 4.7 |
| 40 | BC007451 | *XAB1* | XPA binding protein 1 | PD | 3.0E-04 | 1.3 |
| 41 | BC03546 | *HNLF* | putative NFkB activating protein HNLF | PR | 3.5E-04 | 1.1 |
| 42 | CK00409 | *EIF4EBP* | eukaryotic translation initiation factor 4E binding protein 2 | PR | 3.6E-04 | 1.4 |
| 43 | NM_1446 | *MGC232* | hypothetical protein MGC23280 | PR | 4.2E-04 | 2.3 |
| 44 | NM_0046 | *SSA2* | Sjogren syndrome antigen A2 (60kDa, ribonucleoprotein autoantige | PR | 4.2E-04 | 1.2 |
| 45 | NM_0027 | *PRKACA* | protein kinase, cAMP-dependent, catalytic, alpha | PR | 5.0E-04 | 1.2 |
| 46 | NM_0051 | *FEZ2* | fasciculation and elongation protein zeta 2 (zygin II) | PD | 6.1E-04 | 3.3 |
| 47 | NM_0058 | *SRRM1* | serine/arginine repetitive matrix 1 | PR | 7.0E-04 | 1.4 |
| 48 | NM_0062 | *PDGFRL* | platelet-derived growth factor receptor-like | PD | 7.0E-04 | 2.4 |
| 49 | AI096936 | *SNX13* | sorting nexin 13 | PR | 8.4E-04 | 1.6 |
| 50 | NM_0147 | *KIAA025* | KIAA0258 gene product | PD | 8.9E-04 | 2.5 |
| 51 | BF973104 | *TOM7* | homolog of Tom7 (S. cerevisiae) | PR | 1.0E-03 | 1.5 |

(*) The 12 and 51 gene sets were listed as the rank-order of permutational *p*-values that were less than 0.001.

| Table 4A List of 12 Candidate Genes for Discriminating Responder (PR) from Non-responder (PD) to Gefitinib | | | | |
|---|---|---|---|---|
| | | | | |
| Rank Order | GenBank ID | Gene Symbol | Gene Name | Nucleotides |
| | 1 NM_024829 | *FLJ22662* | hypothetical protein FlJ22662 | ATACGGCATCCATGAAATATAT<br>CATGCGATACAACAATTATAAG<br>AAGGATCCTTACAGTAGAGGTG<br>ACCCCTGTAATACCATCTGCTG<br>CCGTGAGGACCTGAACTCACCT<br>AACCCAAGTCCTGGAGGTTGTT<br>ATGACACAAAGGTGGCAGATAT<br>CTACCTAGCATCTCAGTACACA<br>TCCTATGCCATAAGTGGTCCCA<br>CAGTACAAGGTGGCCTCCCTGT<br>TTTTCGCTGGGACCGTTTCAAC<br>AAAACTCTACATCAGGGCATGC<br>CAGAGGTCTACAACTTTGATTT<br>TATTACCATGAAACCAATTTTG<br>AAACTTGATATAAAATGAAGGA<br>GGGAGATGACGGACTAGAAGAC |

(continued)

| Rank Order | GenBank ID | Gene Symbol | Gene Name | Nucleotides |
|---|---|---|---|---|
| | 2 BC009799 | *AREG* | amphiregulin (<u>schwannoma-derived growth factor</u>) | CTCCACTCGCTCTTCCAACACC<br>CGCTCGTTTTGCGGCAGCTCGT<br>GTCCCAGAGACCGAGTTGCCCC<br>AGAGACCGAGACGCCGCCGCTG<br>CGAAGGACCAATGAGAGCCCCG<br>CTGCTACCGCCGGCGCCGGTGG<br>TGCTGTCGCTCTTGATACTCGG<br>CTCAGGCCATTATGCTGCTGGA<br>TTGGACCTCAATGACACCTACT<br>CTGGGAAGCGTGAACCATTTTC<br>TGGGGACCACAGTGCTGATGGA<br>TTTGAGGTTACCTCAAGAAGTG<br>AGATGTCTTCAGGGAGTGAGAT<br>TTCCCCTGTGAGTGAAATGCCT<br>TCTAGTAGTGAACCGTCCTCGG<br>GAGCCGACTATGACTACTCAGA<br>AGAGTATGATAACGAACCACAA<br>ATACCTGGCTATATTGTCGATG<br>ATTCAGTCAGAGTTGAACAGGT<br>AGTTAAGCCCCCCCAAAACAAG<br>ACGGAAAGTGAAAATACTTCAG<br>ATAAACCCAAAAGAAAGAAAAA<br>GGGAGGCAAAAATGGAAAAAAT<br>AGAAGAAACAGAAAGAAGAAAA<br>ATCCATGTAATGCAGAATTTCA<br>AAATTTCTGCATTCACGGAGAA<br>TGCAAATATATAGAGCACCTGG<br>AAGCAGTAACATGCAAATGTCA<br>GCAAGAATATTTCGGTGAACGG<br>TGTGGGGAAAAGTCCATGAAAA<br>CTCACAGCATGATTGACAGTAG<br>TTTATCAAAAATTGCATTAGCA<br>GCCATAGCTGCCTTTATGTCTG<br>CTGTGATCCTCACAGCTGTTGC |

(continued)

| Rank Order | GenBank ID | Gene Symbol | Gene Name | Nucleotides |
|---|---|---|---|---|
| | 3 NM_014325 | *CORO1C* | coronin, actin binding protein, 1C | GATAGGCCACATTCCAGTAAGA<br>ACTCAATTTGTCTCCCAAATTT<br>GCAGAAACAAAACGTGATTTAA<br>AAGCTGAGCTTTTTATCAGAAA<br>GCTTTTTTGATGTTTTAAGTGT<br>TATGTGACTTGTTGAACTTTTT<br>AAAAAGTGCTACTTTTAAAATC<br>CCAGATACTCTGAATTTTAGAA<br>AACAAACTAATTCTGATTGTGT<br>CGTGCCCAAGTACCCTTTTTTT<br>TTTAATGAGTAGGGACCAATGC<br>CACATTGCTTTTTATATTTCTT<br>TCTTTTTTAATGTTGCCAAAAC<br>CAAAAGTAGCTTTGTTTTCCTT<br>TGTATTTTGCTACTTTGCAGTA<br>TTTGTGTGTGTGGTTTTTTTTC<br>CTTAATTTGAAAGGGACAGCAC<br>TGTGTATGTTTA |
| | 4 BC010488 | *A VEN* | apoptosis, caspase activation inhibitor | AGGAGACCATTTGGAAGAAGAA<br>CTAGATCTGTTGCTTAATTTAG<br>ATGCACCTATAAAAGAGGGAGA<br>TAACATCTTACCAGATCAGACG<br>TCTCAGGACCTGAAATCCAAGG<br>AAGATGGGGAGGTGGTCCAAGA<br>GGAAGAAGTTTGTGCAAAACCA<br>TCTGTGACTGAAGAAAAAAACA<br>TGGAACCTGAGCAACCAAGTAC<br>CTCCAAAAATGTTACCGAGGAA<br>GAGCTGGAAGACTGGTTGGACA<br>GCATGATTTCCTAAAAAGGGGA<br>AAAAAAGTGCCTGAAGCAAATC<br>TTGGTTGCCTTCTAACGGCAGG<br>TGGGCATAAGGCTGTCCTTCAG<br>GACCAGCCAGTTTACAAGCATG<br>TCTCAAGCTAGTGTGTTCCATT<br>ATGCTCACAGCAGTAAATGCCT<br>ACCTCTGTGTTTGACATCTGAA<br>AGAATACATTGAAGCAGCTTGT<br>TGCATTTGTTTTTCTGGCTTAG<br>TAATCTAATAGATTTCCTTAAG<br>GGCAGGAGATAGACTCTGGCCC<br>TTGTTTCTAGCCTCCTTCCTTG<br>CAGTGTTTACAACATAGCCAGT<br>GTTTACAGCATAGCA |

(continued)

| Rank Order | GenBank ID | Gene Symbol | Gene Name | Nucleotides |
|---|---|---|---|---|
| | 5 NM_ 004090 | *DUSP3* | 3 virusdual specificity phosphatase (vaccinia VH1-related) phosphatase | GGATCCTTTATTGGTGGTAGAG<br>CAAAAAAACCCAAACACGATAA<br>ACCTTTCAAAAGACTTTCTAAG<br>GATGATATTGGAATGCACCAGC<br>CCTCACATGTGTATGCACATTT<br>GCCAGAATATAAGAGTTTTGTT<br>TTAAATACAGTCTTGTTAGGAT<br>TTTACGTTATTGTTATTATGGA<br>AAGTGATTGTGATGCTATTTAT<br>CTTCAGGGTCACTCTGG |
| | 6 AI026836 | *DJ473B4* | hypothetical protein dJ473B4 | GCAGTCGTTTCAACCAGGTAGT<br>TTTGGGTTGTTTTTAAAGCCCT<br>TTTGAGGTCTTACACATTATTA<br>ACTTTAAAATAATCAGGCAGCT<br>AAGAATAATTACTAGAAAAATC<br>ATCTACCACTTCAAACATGGTC<br>AACTACTTCAAAACTGCACCTA<br>GAGAATCAGGTACCTGAAGTAG<br>AACAAGAAGCCTGGAGGTGGAC<br>TTTGAGAGGAGGGAATACCC |
| | 7 BU500509 | *PHLDA2* | Pleckstrin homology-like domain, family A, member 2 | TACGTGTACTTCACCATCGTCA<br>CCACCGACCACAAGGAGATCGA<br>CTTCCGCTGCGCGGGCGAGAGC<br>TGCTGGAACGCGGCCATCGCGC<br>TGGCGCTCATCGATTTCCAGAA<br>CCGCCGCGCCCTGCAGGACTTT<br>CGCAGCCGCCAGGAACGCACCG<br>CACCCGCCGCACCCGCCGAGGA<br>CGCCGTGGCTGCCGCGGCCGCC<br>GCACCCTCCGAGCCCTCGGAGC<br>CCTCCAGGCCATCCCCGCAGCC<br>CAAACCCCGCACGCCATGAGCC<br>CGCCGCGGGCCATACGCTGGAC<br>GAGTCGGACCGAGGCTAGGACG<br>TGGCCGGCGCTCTCCAGCCCTG<br>CAGCAGAAGAACTTCCCGTGCG<br>CGCGGATCCTCGCTCCGTTGCA<br>CGGGCGCCTTAAGTTATTGGAC<br>TATCTAATATCTATGTATTTAT<br>TTCGCTGGTTCTTTGTAGTCAC<br>ATATTTTATAGTCTTAATATCT<br>TGTTTTTGCATCACTGTGCCCA<br>TTGCAAATAAATCACTTGGCCA<br>GTTTGCTTTTCTACCATCC |

(continued)

| Rank Order | GenBank ID | Gene Symbol | Gene Name | Nucleotides |
|---|---|---|---|---|
| | 8 NM_016090 | *RBM7* | RNA binding motif protein 7 | CTGTGACATGCTCTTGAGCTTT<br>ACCCTAGTTGAACATACATGTG<br>TAGATTTACACATACTGTTTCA<br>TTNNNNAATTTAGAAATTGTTC<br>ATTAAATCCCATTTGAGGTATA<br>AGTCACTCAGGAAGTTAAAATA<br>TCTCTACACGTATATTTTTACA<br>TTAAAAATACAGTGTTAGCATA<br>ANNNNCCCTTTNNNNNGAAGAA<br>CAAAAATGTCAGTGCATAGTTA<br>GATAAAATGGTAAAATGTTTTA<br>CTGAAAGCATACTTTTTTGGAA<br>AATAGATTCATGAAGCCTTTAA<br>GTGCTGCTTCTGTCAGTCAAAC<br>GTTAAAAACTTTAACATTTTCA<br>AAGTGCCCAGACTGTGTACAAA<br>GACACATGTAATGGAGATTGTA<br>CAGGTTGTTTTTTTGTTTGAAC<br>CTTTGAAAGAGTTTAATCTTAA<br>CGTTTTCTAATTTTAAAATTTT<br>AAAATCTTGTTTAACAAAAGCT<br>TGTATTAAGATACTGTTTTCAT<br>TTCATTACAGAATTGTTTATAA<br>AAGTTCATTTGTTGAAAANNNA<br>GGATCCTTTTTAATACCACAGC<br>ATTTGTACTGTTCCT |
| | 9 BX092512 | | EST | ATATGTGCACACACACACTCAC<br>ACCCACACCCATAAAGATTTTG<br>CACTCCTTGAAGGTACACTAAC<br>TCACCATTTTTATCATACTTAT<br>CCCAGTGTGCCACAGTTACTGG<br>CTTATATGCCTGTCTCTGCTAT<br>CTTATTTTATCTGTCTCCACAA<br>CACAGCAAACTACCTGGCCTTC<br>AATAAAGGGCTTATGAATTATT<br>CATGAATCCATTTTGCCAGGTG<br>CCTAGCCCTGTGTCTGGCTTGA<br>AGCAGGTGTTCCCAAGGTGTGG<br>CATGGCTGAGTGAATACAAAT |

(continued)

| Rank Order | GenBank ID | Gene Symbol | Gene Name | Nucleotides |
|---|---|---|---|---|
| | 10 AI436027 | *OSMR* | oncostatin M receptor | CACCAATGAGCTTACTACCCAA<br>CTTCAAAACTAGGACTCTAACA<br>ATAACTTCTGTCATATCTCATC<br>CTGTAACGCCCCCACCTTCGCT<br>CCTTCCGCCAAGATAATTATCA<br>CTTTAAATTGTGTGCGTGTGTA<br>TTCTCATTTCTTATGTGATGGT<br>AAAAATGCCTTTATTTTGTTTG<br>GTTTTAATGCATAGAAAGGACA<br>TCAAGCTGT |
| | 11 AI971137 | *CCLC* | glutamate-cysteine ligase, catalytic subunit | CTCTAAAAGCCATTCACTCCAG<br>ATTTTACCTGGGGAATATTCTA<br>CATACTGCTTACTTTCTCTATA<br>AAACTCATCAATAAATCATGAA<br>AGGCACTGAGTTTTGTAAATCA<br>GGACCCTAAATGTTTAATTGTA<br>AATAAGTTTCAGATAATTATTA<br>TAGCTTTGCGTTGAAGTTNNNN<br>NNNNNTTTTTCTCAACTAGTTA<br>AGTCAACTGCTTCTGAAATAAC<br>TCTGTATTGTAGATTATGCAGA<br>TCTTTACAGGCATAAATATTTA<br>AACTGTAATATGCTAACTTGAA<br>GAGATTGCAATAAAGCTGCTTC<br>AGCTAAC |

(continued)

| Rank Order | GenBank ID | Gene Symbol | Gene Name | Nucleotides |
|---|---|---|---|---|
| 12 | BQ024877 | *COL4A3BP* | collagen type IV, alpha 3 (Goodpasture antigen) binding protein | CTCACTGAAGTTGAAATGACTG CCCACTTCAAAATCTTCATTGT GTTTACACACCAGTGTATTTAT ACAAATCAGAGGCATTTTGTAG ATGCTTTGCTGACTTGTTCAGC TCTGTAAAAACACAGAAATCAG ACCCATTTTGTAAAGCGGAAAA TCATGTTACATGGAACATGTCC TGTATATATCACATACATGGTA ATGGAGTCTTAATGATAAGTGC AAGATAATAATTTAATGATGGG ATTAGTCTGATCGCTTAATATG CACAATCCTGGAAGTGAATTAC TTGCATCAGATATAGTGATATT TATTATTCTGTACAGAGAGAAA AATACATATAAAACATATGCTT ACATTACATGCACGCGGATTTC ATGCTCCATAATCTTTTCTATT TTTTAATTTACCTTTCTGTAAA TGATGTGCATGGAATATGCCTT ATAGAAAAATGCTGTTCATAAT TTGACTACGTGGAAAAGTGCCT ATATGGTGGTAATGCTAGTAAG GCA |

Table 5A: Correlation of cDNA microarrav data with semi-quantatative RT-F

| | | Spearman rank correlation | |
|---|---|---|---|
| Rank Order | Gene Symbol | ρ | *p*-value |
| 1 | *FLJ22662* | 0.69 | 0.02 |
| 2 | *AREG* | 0.53 | 0.08 |
| 3 | *CORO1C* | 0.35 | 0.24 |
| 4 | *AVEN* | 0.63 | 0.04 |
| 5 | *DUSP3* | 0.63 | 0.04 |
| 6 | *DJ473B4* | 0.45 | 0.14 |
| 7 | *PHLDA2* | 0.84 | 0.01 |
| 8 | *RBM7* | 0.83 | 0.01 |
| 9 | *EST(BX092512)* | 0.63 | 0.04 |
| 10 | *OSMR* | 0.67 | 0.03 |
| 11 | *GCLC* | 0.46 | 0.13 |
| 12 | *COL4A3BP* | 0.27 | 0.24 |

Correlations positive for all 12 genes and significantly positive for 7 of 12 ge

Table 5B: Result of immunohistochemical staining

| | PR | PD |
|---|---|---|
| AREG | 1/5 | 5/6 |
| TGFA | 2/5 | 6/6 |
| ADAM9 | 1/5 | 4/6 |
| CD9 | 2/5 | 5/6 |
| OSMR | 2/5 | 6/6 |

## Claims

**1.** A set of isolated marker genes comprising genes identified as having differential expression as between patients who are responders and non responders to an erbB receptor tyrosine kinase inhibitor; said gene set comprising all of the first 12 genes listed in Table 4 herein or gene-specific oligonucleotides derived from said genes; wherein the first 12 genes listed in Table 4 are the genes FLJ22662 shown in Table 4A, AREG shown in Table 4A, CORO1C shown in Table 4A, AVEN shown in Table 4A, DUSP3 shown in Table 4A, DJ473B4 shown in Table 4A, PHLDA2 shown in Table 4A, RBM7 shown in Table 4A, EST shown in Table 4A, OSMR shown in Table 4A, GCLC shown in Table 4A, and COL4A3BP shown in Table 4A; and wherein the gene-specific oligonucleotides derived from said genes are fragments of said genes that uniquely identify said genes.

**2.** A set according to claim 1, wherein the genes comprise the sequences set forth in Table 4A.

**3.** A set according to claim 1, wherein the set comprises gene-specific oligonucleotides, said oligonucleotides comprising 5 to 50 nucleotides of the sequences set forth in Table 4A.

**4.** A set according to any preceding claim wherein the inhibitor is selected from gefitinib, OSI-774, PKI-166, EKB-569, GW2016 and CI-1033.

**5.** A set according to claim 4 wherein the inhibitor is gefitinib.

**6.** A set according to any of claims 1 to 3 wherein the inhibitor is an anti-erbB antibody.

**7.** A set according to claim 6 wherein the antibody is trastuzumab or cetuximab.

**8.** An *in vitro* method of predicting the responsiveness of a patient with cancer to treatment with an erbB receptor kinase inhibitor, or for selecting patients that will respond to an erbB receptor kinase inhibitor comprising comparing the differential expression of the marker genes of the gene set as defined in any one of claims 1 to 7.

9. A method according to claim 8 wherein the responsiveness of the patients is represented by the generation of a Drug Response Score.

10. A method according to claim 8 or 9 wherein the comparison is performed by microarray assay.

11. A method according to claim 8 or claim 9, wherein the comparison is performed by immunohistochemistry.

12. A method according to claim 11, said method comprises detecting the differential expression of amphiregulin.

13. A method according to any of claims 8 to 12 wherein the inhibitor is as defined in any of claims 4 to 7.

14. A diagnostic kit for use in the method of any one of claims 8 to 13 comprising a marker gene set as defined in any one of claims 1 to 7 on a suitable support medium.

15. A kit according to claim 14 which comprises a microarray.

16. Use of the set of isolated marker genes according to any one of claims 1 to 7 to measure the expression level of said genes in a tissue sample from a patient having NSCLC.

17. A diagnostics kit comprising means for determining the level of expression of the first 12 genes listed in Table 4 herein in a tissue sample from a NSCLC patient, comprising a support material comprising a set of isolated marker genes, as defined in any of claims 1 to 3, each gene thereof attached thereto; wherein the first 12 genes listed in Table 4 are the genes FLJ22662 shown in Table 4A, AREG shown in Table 4A, CORO1C shown in Table 4A, AVEN shown in Table 4A, DUSP3 shown in Table 4A, DJ473B4 shown in Table 4A, PHLDA2 shown in Table 4A, RBM7 shown in Table 4A, EST shown in Table 4A, OSMR shown in Table 4A, GCLC shown in Table 4A, and COL4A3BP shown in Table 4A.

18. Use of all of the marker genes from a gene set as defined in any one of claims 1 to 7 for the manufacture of a diagnostic comprising said gene set wherein said diagnostic is used *in vitro* to predict the responsiveness of a patient with cancer to treatment with an erbB receptor kinase inhibitor, or to select patients that will respond to an erbB receptor kinase inhibitor by comparing the differential expression of the marker genes from a gene set as defined in any one of claims 1 to 7.

19. Use of a gene set as defined in any one of claims 1 to 3 for the manufacture of a diagnostic comprising said gene set for assessing *in vitro* if an erbB tyrosine kinase receptor inhibitor modulates gene expression of at least one of the gene from the marker gene set according to any of claims 1 to 8 relative to a relevant control.

20. Use of a gene set as defined in any one of claims 1 to 3 for the manufacture of a diagnostic comprising said gene set for carrying out a clinical trial measuring the effect or effectiveness of erbB receptor tyrosine kinase inhibition or inhibitors comprising measuring *in vitro* the relative levels of expression of a gene set as defined in any one of claims 1 to 3 in a patient.

**Patentansprüche**

1. Satz isolierter Markergene, umfassend Gene, bei denen eine unterschiedliche Expression zwischen auf einen erbB-Rezeptor-Tyrosinkinase-Inhibitor ansprechenden und nicht ansprechenden Patienten nachgewiesen ist; wobei der Gensatz die gesamten ersten 12 hierin in Tabelle 4 aufgeführten Gene oder von den genannten Genen abgeleitete genspezifische Oligonukleotide umfasst; wobei es sich bei den ersten 12 in Tabelle 4 aufgeführten Genen um die Gene FLJ22662, dargestellt in Tabelle 4A, AREG, dargestellt in Tabelle 4A, CORO1C, dargestellt in Tabelle 4A, AVEN, dargestellt in Tabelle 4A, DUSP3, dargestellt in Tabelle 4A, DJ473B4, dargestellt in Tabelle 4A, PHLDA2, dargestellt in Tabelle 4A, RBM7, dargestellt in Tabelle 4A, EST, dargestellt in Tabelle 4A, OSMR, dargestellt in Tabelle 4A, GCLC, dargestellt in Tabelle 4A, und COL4A3BP, dargestellt in Tabelle 4A, handelt; und wobei es sich bei den von den genannten Genen abgeleiteten genspezifischen Oligonukleotiden um Fragmente der Gene handelt, mit denen die Gene in einzigartiger Weise identifiziert werden.

2. Satz nach Anspruch 1, wobei die Gene die in Tabelle 4A angegebenen Sequenzen umfassen.

3. Satz nach Anspruch 1, wobei der Satz genspezifische Oligonukleotide umfasst, wobei die Oligonucleotide 5 bis 50 Nukleotide der in Tabelle 4A angegebenen Sequenzen umfassen.

4. Satz nach einem vorhergehenden Anspruch, wobei der Inhibitor aus Gefitinib, OSI-774, PKI-166, EKB-569, GW2016 und CI-1033 ausgewählt ist.

5. Satz nach Anspruch 4, wobei es sich bei dem Inhibitor um Gefitinib handelt.

6. Satz nach einem der Ansprüche 1 bis 3, wobei es sich bei dem Inhibitor um einen Anti-erbB-Antikörper handelt.

7. Satz nach Anspruch 6, wobei es sich bei dem Antikörper um Trastuzumab oder Cetuximab handelt.

8. In-vitro-Verfahren zur Vorhersage der Ansprechbarkeit eines an Krebs leidenden Patienten auf die Behandlung mit einem erbB-Rezeptorkinase-Inhibitor oder zur Auswahl von Patienten, die auf einen erbB-Rezeptorkinase-Inhibitor ansprechen, wobei man die unterschiedliche Expression der Markergene des Gensatzes mit der in einem der Ansprüche 1 bis 7 angegebenen Bedeutung vergleicht.

9. Verfahren nach Anspruch 8, wobei die Ansprechbarkeit der Patienten durch die Erzeugung eines Drug Response Score repräsentiert wird.

10. Verfahren nach Anspruch 8 oder 9, wobei der Vergleich mittels Mikroarray-Test erfolgt.

11. Verfahren nach Anspruch 8 oder 9, wobei der Vergleich mittels Immunhistochemie erfolgt.

12. Verfahren nach Anspruch 11, bei dem man die unterschiedliche Expression von Amphiregulin nachweist.

13. Verfahren nach einem der Ansprüche 8 bis 12, wobei der Inhibitor die in einem der Ansprüche 4 bis 7 angegebene Bedeutung aufweist.

14. Diagnostisches Kit zur Verwendung bei dem Verfahren gemäß einem der Ansprüche 8 bis 13, umfassend einen Markergensatz mit der in einem der Ansprüche 1 bis 7 angegebenen Bedeutung auf einem geeigneten Trägermedium.

15. Kit nach Anspruch 14, das einen Mikroarray umfasst.

16. Verwendung des Satzes isolierter Markergene nach einem der Ansprüche 1 bis 7 zum Messen des Expressionsniveaus der Gene in einer Gewebeprobe aus einem Patienten mit NSCLC.

17. Diagnostisches Kit, umfassend Mittel zur Bestimmung des Expressionsniveaus der ersten 12 hierin in Tabelle 4 aufgeführten Gene in einer Gewebeprobe aus einem NSCLC-Patienten, umfassend ein Trägermaterial, umfassend einen Satz isolierter Markergene mit der in einem der Ansprüche 1 bis 3 angegebenen Bedeutung, wobei dessen Gene jeweils daran gebunden sind; wobei es sich bei den ersten 12 in Tabelle 4 aufgeführten Genen um die Gene FLJ22662, dargestellt in Tabelle 4A, AREG, dargestellt in Tabelle 4A, CORO1C, dargestellt in Tabelle 4A, AVEN, dargestellt in Tabelle 4A, DUSP3, dargestellt in Tabelle 4A, DJ473B4, dargestellt in Tabelle 4A, PHLDA2, dargestellt in Tabelle 4A, RBM7, dargestellt in Tabelle 4A, EST, dargestellt in Tabelle 4A, OSMR, dargestellt in Tabelle 4A, GCLC, dargestellt in Tabelle 4A, und COL4A3BP, dargestellt in Tabelle 4A, handelt.

18. Verwendung aller Markergene aus einem Gensatz mit der in einem der Ansprüche 1 bis 7 angegebenen Bedeutung zur Herstellung eines den Gensatz umfassenden Diagnostikums, wobei das Diagnostikum in vitro zur Vorhersage der Ansprechbarkeit eines an Krebs leidenden Patienten auf die Behandlung mit einem erbB-Rezeptorkinase-Inhibitor oder zur Auswahl von Patienten, die auf einen erbB-Rezeptorkinase-Inhibitor ansprechen, verwendet wird, indem die unterschiedliche Expression der Markergene aus einem Gensatz mit der in einem der Ansprüche 1 bis 7 angegebenen Bedeutung verglichen wird.

19. Verwendung eines Gensatzes mit der in einem der Ansprüche 1 bis 3 angegebenen Bedeutung zur Herstellung eines den Gensatz umfassenden Diagnostikums zur In-vitro-Beurteilung, ob ein erbB-Tyrosinkinase-Rezeptor-Inhibitor die Genexpression wenigstens eines Gens aus dem Markergensatz nach einem der Ansprüche 1 bis 8 relativ zu einer entsprechenden Kontrolle moduliert.

**20.** Verwendung eines Gensatzes mit der in einem der Ansprüche 1 bis 3 angegebenen Bedeutung zur Herstellung eines den Gensatz umfassenden Diagnostikums zur Durchführung einer klinischen Versuchsreihe, bei der die Wirkung oder Wirksamkeit der erbB-Rezeptor-Tyrosinkinase-Hemmung bzw. -Inhibitoren gemessen wird, wobei man in vitro die relativen Expressionsniveaus eines Gensatzes mit der in einem der Ansprüche 1 bis 3 angegebenen Bedeutung bei einem Patienten misst.

**Revendications**

**1.** Ensemble de gènes marqueurs isolés comprenant des gènes identifiés comme ayant une expression différentielle entre des patients qui sont des répondeurs et des non-répondeurs à un inhibiteur de la tyrosine kinase du récepteur erbB ; ledit ensemble de gènes comprenant la totalité des 12 premiers gènes mentionnés dans le Tableau 4 de la présente invention, ou des oligonucléotides spécifiques de gènes dérivant desdits gènes ; dans lequel les 12 premiers gènes mentionnés dans le Tableau 4 sont les gènes FLJ22662 présenté dans le Tableau 4A, AREG présenté dans le Tableau 4A, CORO1C présenté dans le Tableau 4A, AVEN présenté dans le Tableau 4A, DUSP3 présenté dans le Tableau 4A, DJ473B4 présenté dans le Tableau 4A, PHLDA2 présenté dans le Tableau 4A, RBM7 présenté dans le Tableau 4A, EST présenté dans le Tableau 4A, OSMR présenté dans le Tableau 4A, GCLC présenté dans le Tableau 4A et COL4A3BP présenté dans le Tableau 4A ; et dans lequel les oligonucléotides spécifiques de gènes dérivant desdits gènes sont des fragments desdits gènes qui identifient sans ambiguïté lesdits gènes.

**2.** Ensemble selon la revendication 1, dans lequel les gènes comprennent les séquences présentées dans le Tableau 4A.

**3.** Ensemble selon la revendication 1, lequel ensemble comprend des oligonucléotides spécifiques de gènes, lesdits oligonucléotides comprenant 5 à 50 nucléotides des séquences présentées dans le Tableau 4A.

**4.** Ensemble selon l'une quelconque des revendications précédentes, dans lequel l'inhibiteur est choisi parmi le géfitinib, l'OSI-774, le PKI-166, l'EKB-569, le GW2016 et le CI-1033.

**5.** Ensemble selon la revendication 4, dans lequel l'inhibiteur est le géfitinib.

**6.** Ensemble selon l'une quelconque des revendications 1 à 3, dans lequel l'inhibiteur est un anticorps anti-erbB.

**7.** Ensemble selon la revendication 6, dans lequel l'anticorps est le trastuzumab ou le cétuximab.

**8.** Procédé in *vitro* de prédiction de la réactivité d'un patient présentant un cancer à un traitement avec un inhibiteur de la kinase du récepteur erbB, ou pour la sélection de patients qui vont répondre à un inhibiteur de la kinase du récepteur erbB, comprenant la comparaison de l'expression différentielle des gènes marqueurs de l'ensemble de gènes tel que défini dans l'une quelconque des revendications 1 à 7.

**9.** Procédé selon la revendication 8, dans lequel la réactivité des patients est représentée par la production d'un score de réponse au médicament.

**10.** Procédé selon la revendication 8 ou 9, dans lequel la comparaison est réalisée par analyse sur microréseau.

**11.** Procédé selon la revendication 8 ou la revendication 9, dans lequel la comparaison est réalisée par immunohistochimie.

**12.** Procédé selon la revendication 11, lequel procédé comprend la détection de l'expression différentielle de l'amphiréguline.

**13.** Procédé selon l'une quelconque des revendications 8 à 12, dans lequel l'inhibiteur est tel que défini dans l'une quelconque des revendications 4 à 7.

**14.** Trousse diagnostique pour utilisation dans le procédé de l'une quelconque des revendications 8 à 13, comprenant un ensemble de gènes marqueurs tel que défini dans l'une quelconque des revendications 1 à 7 sur un milieu support approprié.

**15.** Trousse selon la revendication 14, qui comprend un microréseau.

**16.** Utilisation de l'ensemble de gènes marqueurs isolés selon l'une quelconque des revendications 1 à 7 pour mesurer le niveau d'expression desdits gènes dans un échantillon de tissu provenant d'un patient présentant un NSCLC.

**17.** Trousse diagnostique comprenant des moyens pour déterminer le niveau d'expression des 12 premiers gènes mentionnés dans le Tableau 4 de la présente invention dans un échantillon de tissu provenant d'un patient NSCLC, comprenant un matériau support comprenant un ensemble de gènes marqueurs isolés tel que défini dans l'une quelconque des revendications 1 à 3, chacun de ses gènes y étant attaché ; dans laquelle les 12 premiers gènes mentionnés dans le Tableau 4 sont les gènes FLJ22662 présenté dans le Tableau 4A, AREG présenté dans le Tableau 4A, CORO1C présenté dans le Tableau 4A, AVEN présenté dans le Tableau 4A, DUSP3 présenté dans le Tableau 4A, DJ473B4 présenté dans le Tableau 4A, PHLDA2 présenté dans le Tableau 4A, RBM7 présenté dans le Tableau 4A, EST présenté dans le Tableau 4A, OSMR présenté dans le Tableau 4A, GCLC présenté dans le Tableau 4A et COL4A3BP présenté dans le Tableau 4A.

**18.** Utilisation de la totalité des gènes marqueurs provenant d'un ensemble de gènes tel que défini dans l'une quelconque des revendications 1 à 7 pour la fabrication d'un diagnostic comprenant ledit ensemble de gènes, pour laquelle ledit diagnostic est utilisé in vitro pour prédire la réactivité d'un patient présentant un cancer à un traitement avec un inhibiteur de la kinase du récepteur erbB, ou pour sélectionner les patients qui vont répondre à un inhibiteur de la kinase du récepteur erbB, par comparaison de l'expression différentielle des gènes marqueurs provenant d'un ensemble de gènes tel que défini dans l'une quelconque des revendications 1 à 7.

**19.** Utilisation d'un ensemble de gènes tel que défini dans l'une quelconque des revendications 1 à 3 pour la fabrication d'un diagnostic comprenant ledit ensemble de gènes, pour évaluer *in vitro* si un inhibiteur de la tyrosine kinase du récepteur erbB module l'expression génique d'au moins l'un des gènes provenant de l'ensemble des gènes marqueurs selon l'une quelconque des revendications 1 à 8, par comparaison avec un témoin approprié.

**20.** Utilisation d'un ensemble de gènes tel que défini dans l'une quelconque des revendications 1 à 3 pour la fabrication d'un diagnostic comprenant ledit ensemble de gènes, pour mettre en oeuvre un essai clinique mesurant l'effet ou l'efficacité de l'inhibition ou d'inhibiteurs de la tyrosine kinase du récepteur erbB, comprenant la mesure in vitro des niveaux relatifs d'expression d'un ensemble de gènes tel que défini dans l'une quelconque des revendications 1 à 3 chez un patient.

**Figure 1**

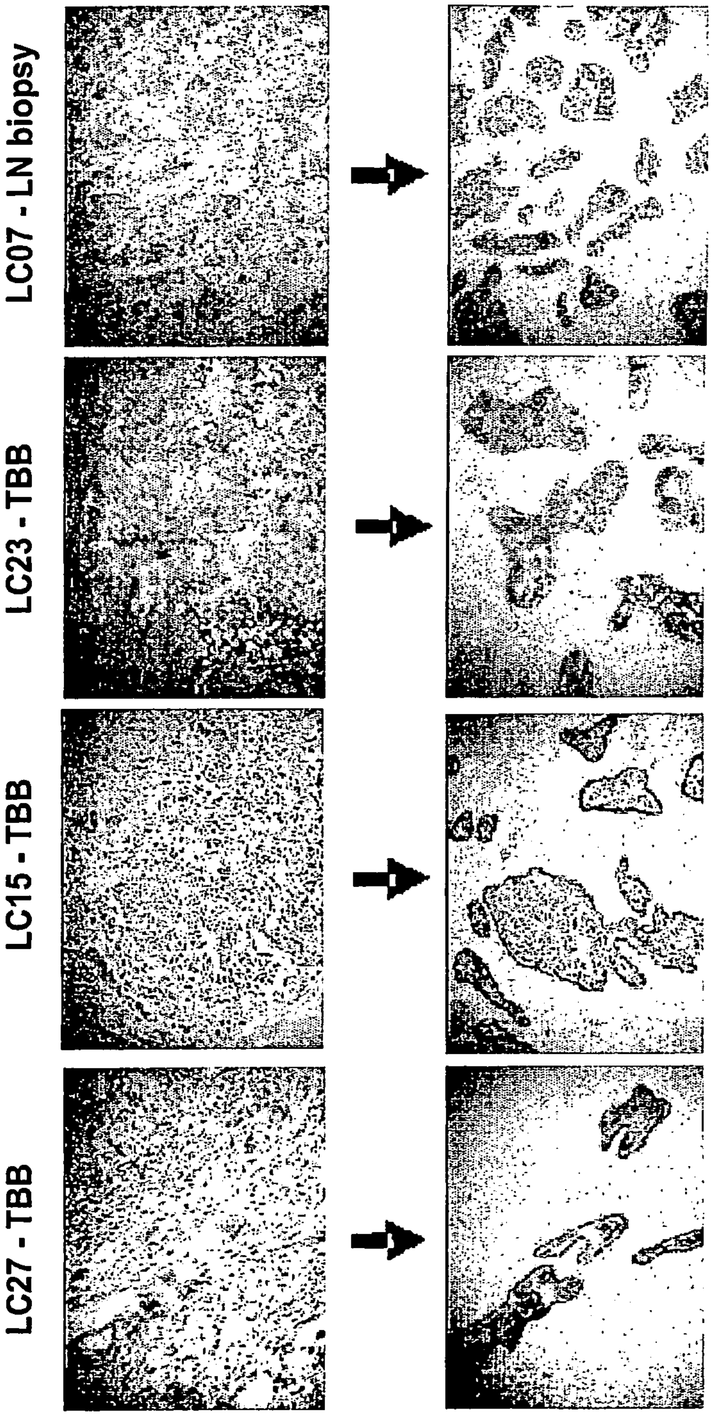

# Figure 2

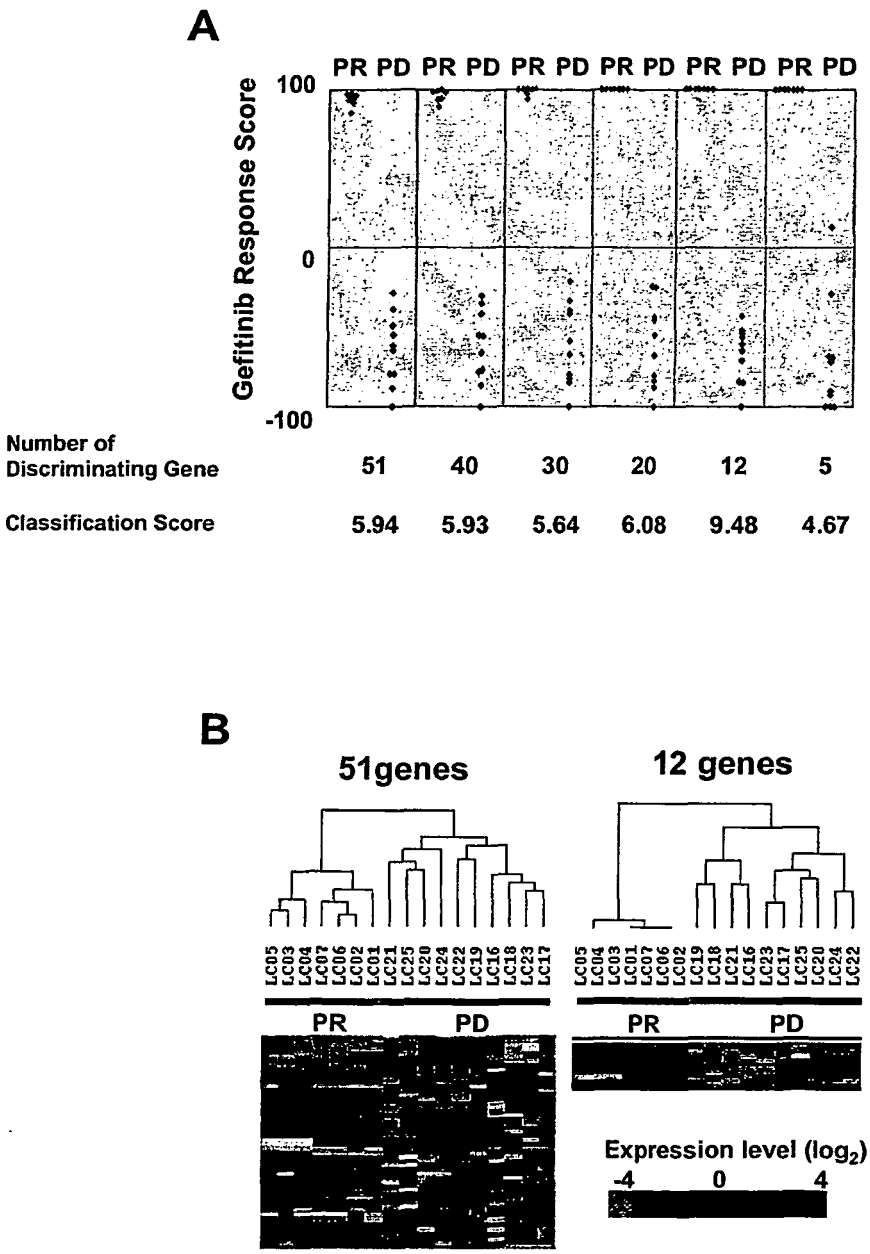
A
PR PD PR PD PR PD PR PD PR PD PR PD
Gefitinib Response Score
100
0
-100
Number of Discriminating Gene
51 40 30 20 12 5
Classification Score
5.94 5.93 5.64 6.08 9.48 4.67
B
51genes
12 genes
LC05 LC03 LC04 LC07 LC06 LC02 LC01 LC21 LC25 LC20 LC24 LC22 LC19 LC16 LC18 LC23 LC17
LC05 LC04 LC03 LC01 LC07 LC06 LC02 LC19 LC18 LC21 LC16 LC23 LC17 LC25 LC20 LC24 LC22
PR
PD
PR
PD
Expression level ($log_2$)
-4 0 4

# Figure 2 cont.

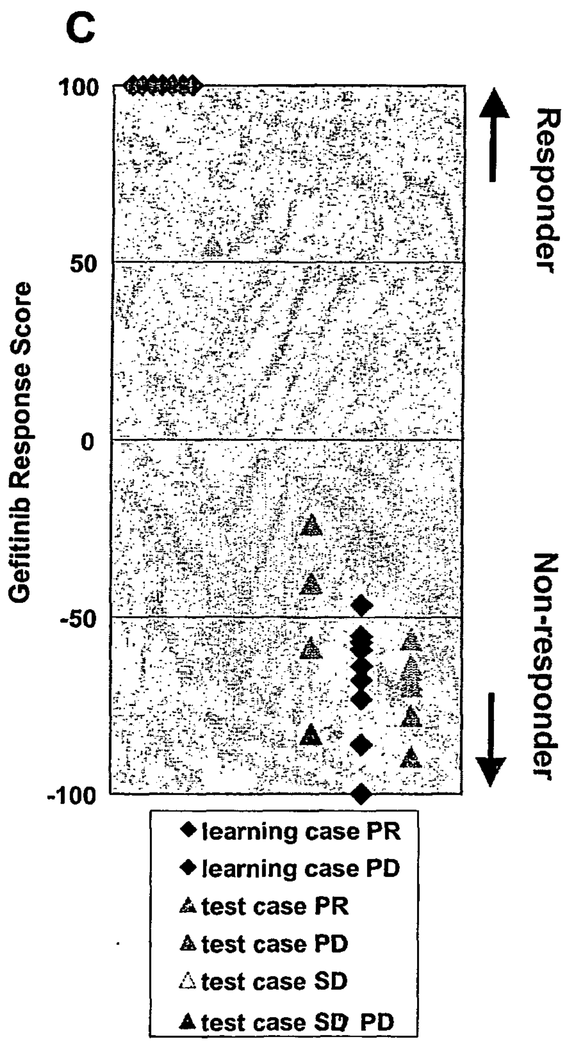

Figure 3

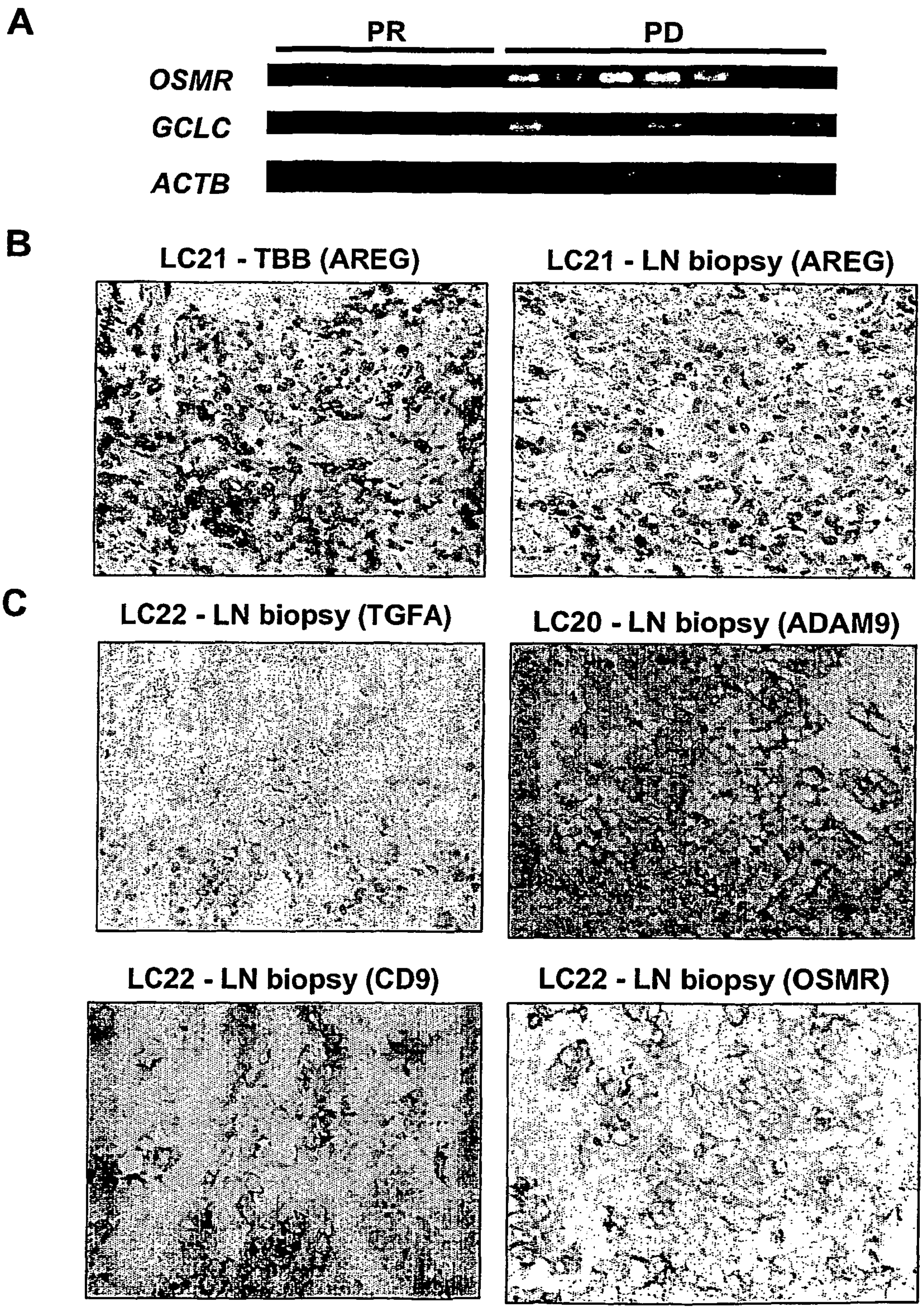
A
PR
PD
OSMR
GCLC
ACTB
B
LC21 - TBB (AREG)
LC21 - LN biopsy (AREG)
C
LC22 - LN biopsy (TGFA)
LC20 - LN biopsy (ADAM9)
LC22 - LN biopsy (CD9)
LC22 - LN biopsy (OSMR)

# Figure 4

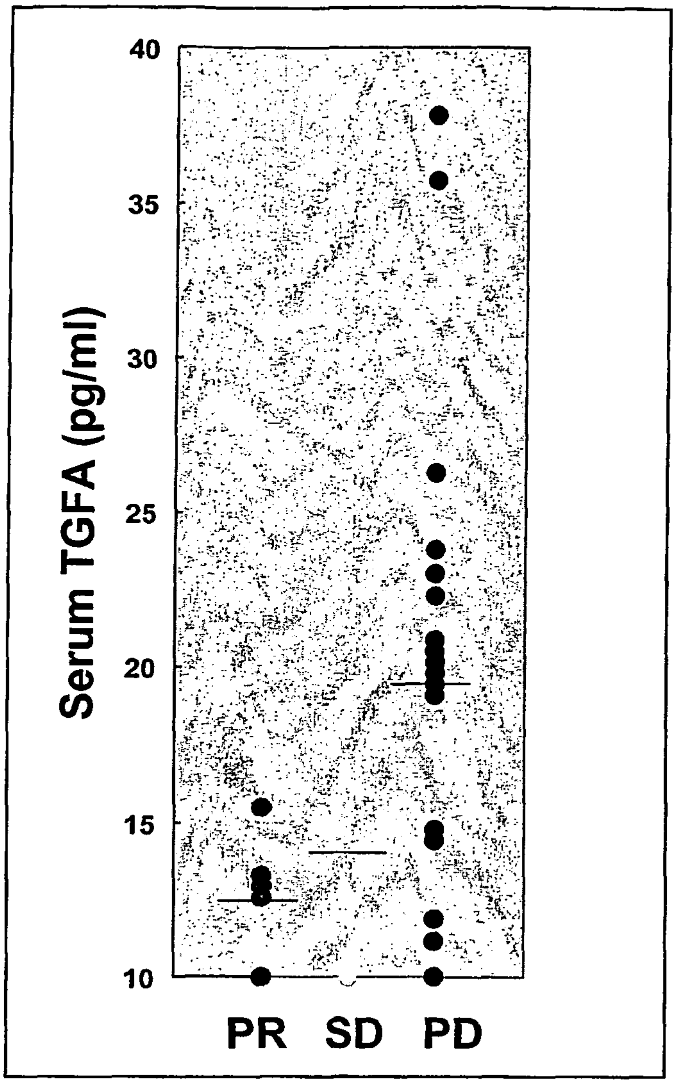

# Figure 5

A

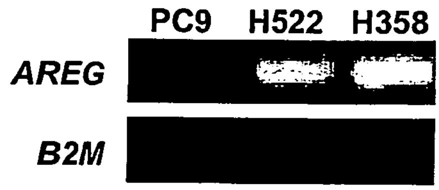
PC9
H522
H358
AREG
B2M

B

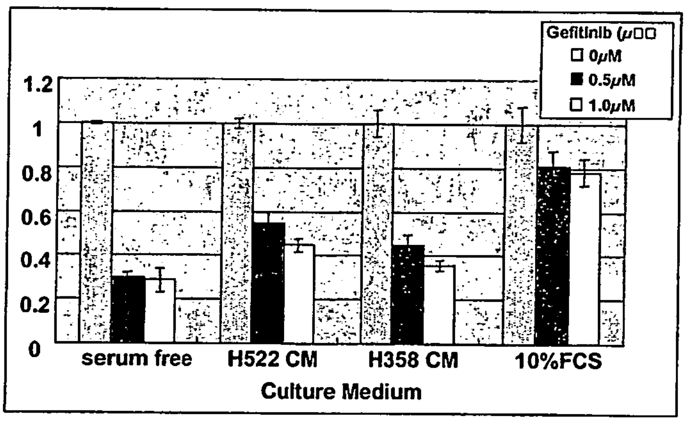
Gefitinib (μ
0μM
0.5μM
1.0μM
1.2
1
0.8
0.6
0.4
0.2
0
serum free
H522 CM
H358 CM
10%FCS
Culture Medium

C

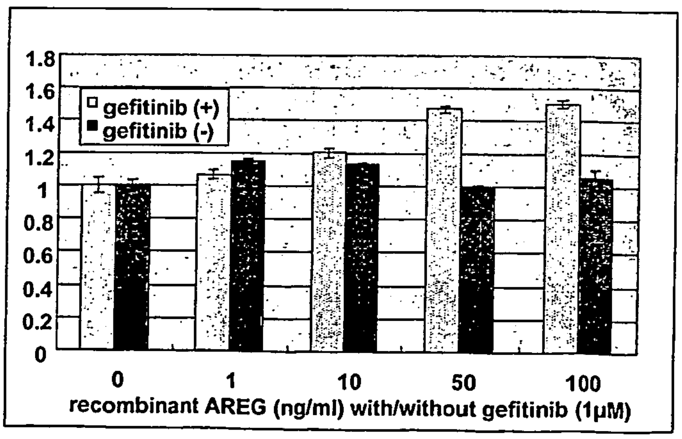
1.8
1.6
1.4
1.2
1
0.8
0.6
0.4
0.2
0
gefitinib (+)
gefitinib (-)
0
1
10
50
100
recombinant AREG (ng/ml) with/without gefitinib (1μM)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0566226 A
- WO 9633980 A
- WO 9730034 A
- WO 9630347 A
- WO 9955683 A
- WO 9738983 A
- WO 0031048 A
- WO 9702266 A
- EP 0787722 A
- WO 9850038 A
- WO 9909016 A
- WO 9924037 A
- US 6002008 A
- WO 9935146 A
- WO 0104111 A
- US 0110063 W
- US 2002090979 A
- US 5837832 A
- GB 2004002316 W
- GB 0312451 A
- GB 0322636 A
- GB 0327132 A


### Non-patent literature cited in the description


- **WILKS.** *Advances in Cancer Research,* 1993, vol. 60, 43-73
- **OLAYIOYE et al.** *EMBO J.,* 2000, vol. 19, 3159
- **KLAPPER et al.** *Adv. Cancer Res.,* 2000, vol. 77, 25
- **CEMY et al.** *Brit. J. Cancer,* 1986, vol. 54, 265
- **REUBI et al.** *Int. J. Cancer,* 1990, vol. 45, 269
- **RUSCH et al.** *Cancer Research,* 1993, vol. 53, 2379
- **BRABENDER et al.** *Clin. Cancer Res.,* 2001, vol. 7, 1850
- **HENDLER et al.** *Cancer Cells,* 1989, vol. 7, 347
- **ROSS et al.** *Cancer Investigation,* 2001, vol. 19, 554
- **YU et al.** *Bioessays,* 2000, vol. 22.7, 673
- **MENDELSOHN et al.** *Oncogene,* 2000, vol. 19, 6550
- **YAISH et al.** *Science,* 1988, vol. 242, 933
- **KOLIBABA et al.** *Biochimica et Biophysica Acta,* 1997, vol. 133, F217-F248
- **AL-OBEIDI et al.** *Oncogene,* 2000, vol. 19, 5690-5701
- **MENDELSOHN et al.** *Oncogene,* 2000, vol. 19, 6550-6565
- **J R WOODBURN et al.** *Proc. Amer. Assoc. Cancer Research,* 1997, vol. 38, 633
- *Pharmacol. Ther.,* 1999, vol. 82, 241-250
- *CHEMICAL ABSTRACTS,* 184475-35-2
- *J.Med. Chem.,* 1999, vol. 42, 1803-1815
- *Nature Medicine,* 2000, vol. 6, 1024-1028
- **VELCULESCU et al.** *Science,* vol. 270 (5235), 484-487
- **SAMBROOK et al.** Molecular Cloning: A Laboratory Manual. 1989
- **LEMIEUX et al.** *Molecular Breeding,* 1998, vol. 4, 277-289
- Parallel Analysis with Biological Chips. **SCHENA ; DAVIS.** PCR Methods Manual. 1999
- Genes, Genomes and Chips. **SCHENA ; DAVIS.** DNA Microarrays: A Practical Approach. Oxford University Press, 1999
- Microarray Biochip Technology. Eaton Publishing Company
- **CORTES.** *The Scientist,* 2000, vol. 14 (17), 25
- **GWYNNE ; PAGE.** *Microarray analysis: the next revolution in molecular biology, Science,* 06 August 1999
- **EAKINS ; CHU.** *Trends in Biotechnology,* 1999, vol. 17, 217-218
- **CELIS et al.** *FEBS Lett,* 2000, vol. 480 (1), 2-16
- **LOCKHART ; WINZELER.** *Nature,* 2000, vol. 405 (6788), 827-836
- **MARX.** *Science,* 2000, vol. 289, 1670-1672
- **SCHERF et al.** *Nat Genet,* 2000, vol. 24 (3), 236-44
- **ROSS et al.** *Nat Genet.,* March 2000, vol. 24 (3), 227-35
- **WANG et al.** *Science,* 1998, vol. 280 (5366), 1077-82
- *Chemical & Engineering News,* 22 February 1999, vol. 77 (8), 27-36
- **ROCKETT ; DIX.** *Xenobiotica,* 2000, vol. 30 (2), 155-77
- **AFSHARI et al.** *Cancer Res,* 1999, vol. 59 (19), 4759-60

- **NUWAYSIR et al.** *Molecular Carcinonucleotide sequencesis,* 1999, vol. 24, 153-159
- **CORTESE.** *The Scientist,* 2000, vol. 14 (11), 26
- **SHALON et al.** *Genome Res,* 1996, vol. 6 (7), 639-45
- **J. SAMBROOK ; E. F. FRITSCH ; T. MANIATIS.** *Molecular Cloning: A Laboratory Manual,* 1989
- **AUSUBEL, F. M. et al.** Current Protocols in Molecular Biology. John Wiley & Sons, 1995
- **B. ROE ; J. CRABTREE ; A. KAHN.** DNA Isolation and Sequencing: Essential Techniques. John Wiley & Sons, 1996
- Oligonucleotide Synthesis: A Practical Approach. Irl Press, 1984
- **D. M. J. LILLEY ; J. E. DAHLBERG.** Methods of Enzymology: DNA Structure Part A: Synthesis and Physical Analysis of DNA Methods in Enzymology. Academic Press, 1992
- **FOSSELLA, F.V. et al.** Randomized phase III trial of docetaxel versus vinorelbine or ifosfamide in patients with advanced non-small-cell lung cancer previously treated with platinum-containing chemotherapy regimens. The TAX 320 Non-Small Cell Lung Cancer Study Group. *J Clin Oncol,* 2000, vol. 18 (12), 2354-62
- Non-small Cell Lung Cancer Collaborative Group. Chemotherapy in non-small cell lung cancer: a meta-analysis using updated data on individual patients from 52 randomised clinical trials. *Bmj,* 1995, vol. 311 (7010), 899-909
- **SCHILLER, J.H. et al.** Comparison of four chemotherapy regimens for advanced non-small-cell lung cancer. *N Engl J Med,* 2002, vol. 346 (2), 92-8
- **KELLY, K. et al.** Randomized phase III trial of paclitaxel plus carboplatin versus vinorelbine plus cisplatin in the treatment of patients with advanced nonsmall-cell lung cancer: a Southwest Oncology Group trial. *J Clin Oncol,* 2001, vol. 19 (13), 3210-8
- **BASELGA, J.** Why the epidermal growth factor receptor? The rationale for cancer therapy. *Oncologist,* 2002, vol. 4 (7), 2-8
- **TRAXLER, P.** Tyrosine kinases as targets in cancer therapy - successes and failures. *Expert Opin Ther Targets,* 2003, vol. 7 (2), 215-34
- **WAKELING, A.E. et al.** ZD1839 (Iressa): an orally active inhibitor of epidermal growth factor signaling with potential for cancer therapy. *Cancer Res,* 2002, vol. 62 (20), 5749-54
- **HERBST, R.S.** Dose-comparative monotherapy trials of ZD1839 in previously treated non-small cell lung cancer patients. *Semin Oncol,* 2003, vol. 30 (1), 30-8
- **FUKUOKA, M. et al.** Final results from a phase □ trial of ZD1839 ('Iressa') for patients with advanced non-small cell lung cancer (IDEAL 1). *Am Soc Clin Oncol,* 2002, vol. 21, 298
- **KRIS, MG. et al.** A phase II trial of ZD1839 ('Iressa') in advanced non-small cell lung cancer (NSCLC) patients who had failed platinum-and docetaxel-based regimens (IDEAL 2. *Pro Am Soc Clin Oncol,* 2002, vol. 21, 292
- **INOUE, A. et al.** Severe acute interstitial pneumonia and gefitinib. *Lancet,* 2003, vol. 361 (9352), 137-9
- **BOHM, M. et al.** Microbeam MOMeNT: non-contact laser microdissection of membrane-mounted native tissue. *Am J Pathol,* 1997, vol. 151 (1), 63-7
- **OKABE, H. et al.** Genome-wide analysis of gene expression in human hepatocellular carcinomas using cDNA microarray: identification of genes involved in viral carcinogenesis and tumor progression. *Cancer Res,* 2001, vol. 61 (5), 2129-37
- **KITAHARA, O. et al.** Alterations of gene expression during colorectal carcinogenesis revealed by cDNA microarrays after laser-capture microdissection of tumor tissues and normal epithelia. *Cancer Res,* 2001, vol. 61 (9), 3544-9
- **GOLUB, T.R. et al.** Molecular classification of cancer: class discovery and class prediction by gene expression monitoring. *Science,* 1999, vol. 286 (5439), 531-7
- **MACDONALD, T.J. et al.** Expression profiling of medulloblastoma: PDGFRA and the RAS/MAPK pathway as therapeutic targets for metastatic disease. *Nat Genet,* 2001, vol. 29 (2), 143-52
- **KANETA.Y. et al.** Prediction of sensitivity to STI571 among chronic myeloid leukemia patients by genome-wide cDNA microarray analysis. *Jpn J Cancer Res,* 2002, vol. 93, 849-856
- **PAVELIC, K. et al.** Evidence for a role of EGF receptor in the progression of human lung carcinoma. *Anticancer Res,* 1993, vol. 13 (4), 1133-7
- **KIKUCHI, T. et al.** Expression profiles of non-small cell lung cancers on cDNA microarrays: Identification of genes for prediction of lymph-node metastasis and sensitivity to anti-cancer drugs. *Oncogene,* 2003, vol. 22 (14), 2192-205
- **HEIGHWAY, J. et al.** Expression profiling of primary non-small cell lung cancer for target identification. *Oncogene,* 2002, vol. 21 (50), 7749-63
- **BEER, D.G. et al.** Gene-expression profiles predict survival of patients with lung adenocarcinoma. *Nat Med,* 2002, vol. 8 (8), 816-24
- **MIURA, K. et al.** Laser capture microdissection and microarray expression analysis of lung adenocarcinoma reveals tobacco smoking- and prognosis-related molecular profiles. *Cancer Res,* 2002, vol. 62 (11), 3244-50
- **MOASSER, M.M. et al.** The tyrosine kinase inhibitor ZD1839 (''Iressa'') inhibits HER2-driven signaling and suppresses the growth of HER2-overexpressing tumor cells. *Cancer Res,* 2001, vol. 61 (19), 7184-8

- **RUSCH, V. et al.** Overexpression of the epidermal growth factor receptor and its ligand transforming growth factor alpha is frequent in resectable non-small cell lung cancer but does not predict tumor progression. *Clin Cancer Res,* 1997, vol. 3 (4), 515-22
- **FONTANINI, G. et al.** Evaluation of epidermal growth factor-related growth factors and receptors and of neoangiogenesis in completely resected stage I-IIIA non-small-cell lung cancer: amphiregulin and microvessel count are independent prognostic indicators of survival. *Clin Cancer Res,* 1998, vol. 4 (1), 241-9
- **BRUNDAGE, M.D. ; D. DAVIES ; W.J. MACKILLOP.** Prognostic factors in non-small cell lung cancer: a decade of progress. *Chest,* 2002, vol. 122 (3), 1037-57
- **HURBIN, A. et al.** Inhibition of apoptosis by amphiregulin via an insulin-like growth factor-1 receptor-dependent pathway in non-small cell lung cancer cell lines. *J Biol Chem,* 2002, vol. 277 (51), 49127-33
- **YARDEN, Y. ; M.X. SLIWKOWSKI.** Untangling the ErbB signaling network. *Nat Rev Mol Cell Biol,* 2001, vol. 2 (2), 127-37
- **PRENZEL, N. et al.** EGF receptor transactivation by G-protein-coupled receptors requires metalloproteinase cleavage of proHB-EGF. *Nature,* 1999, vol. 402 (6764), 884-8
- **NELSON ; CHAU et al.** Aven, a novel inhibitor of caspase activation, binds Bcl-xL and Apaf-1. *Molec Cell,* 2000, vol. 6, 31-41
- **TIPNIS, SR. et al.** Overexpression of the regulatory subunit of r-glutamylcysteine synthetase in Hela cells increases r-glutamylcysteine synthetase activity and confers drug resistance. *Biochem J,* 1999, vol. 337, 559-566

SEQUENCE LISTING

<110> ASTRAZENECA UK LIMITED & THE UNIVERSITY OF TOKYO

<120> PROCESS

<130> P017248WO

<140> PCT/GB2004/002316
<141> 2004-06-01

<150> GB 0312451.8
<151> 2003-05-30

<150> GB 0322636.2
<151> 2003-09-26

<150> GB 0327132.7
<151> 2003-11-21

<160> 83

<170> PatentIn version 3.3

<210> 1
<211> 352
<212> DNA
<213> Homo sapiens

<400> 1

```
atacggcatc catgaaatat atcatgcgat acaacaatta taagaaggat ccttacagta      60
gaggtgaccc ctgtaatacc atctgctgcc gtgaggacct gaactcacct aacccaagtc     120
ctggaggttg ttatgacaca aaggtggcag atatctacct agcatctcag tacacatcct     180
atgccataag tggtcccaca gtacaaggtg gcctccctgt ttttcgctgg gaccgtttca     240
acaaaactct acatcagggc atgccagagg tctacaactt tgattttatt accatgaaac     300
caattttgaa acttgatata aaatgaagga gggagatgac ggactagaag ac             352
```

<210> 2
<211> 1039
<212> DNA
<213> Homo sapiens

<400> 2

```
ctccactcgc tcttccaaca cccgctcgtt ttgcggcagc tcgtgtccca gagaccgagt      60
tgccccagag accgagacgc cgccgctgcg aaggaccaat gagagccccg ctgctaccgc     120
cggcgccggt ggtgctgtcg ctcttgatac tcggctcagg ccattatgct gctggattgg     180
acctcaatga cacctactct gggaagcgtg aaccattttc tggggaccac agtgctgatg     240
gatttgaggt tacctcaaga agtgagatgt cttcagggag tgagatttcc cctgtgagtg     300
aaatgccttc tagtagtgaa ccgtcctcgg gagccgacta tgactactca gaagagtatg     360
ataacgaacc acaaatacct ggctatattg tcgatgattc agtcagagtt gaacaggtag     420

ttaagccccc ccaaaacaag acggaaagtg aaaatacttc agataaaccc aaaagaaaga     480
aaaagggagg caaaaatgga aaaaatagaa gaaacagaaa gaagaaaaat ccatgtaatg     540
cagaatttca aaatttctgc attcacggag aatgcaaata tatagagcac ctggaagcag     600
taacatgcaa atgtcagcaa gaatatttcg gtgaacggtg tggggaaaag tccatgaaaa     660
ctcacagcat gattgacagt agtttatcaa aaattgcatt agcagccata gctgccttta     720
tgtctgctgt gatcctcaca gctgttgctg ttattacagt ccagcttaga agacaatacg     780
tcaggaaata tgaaggagaa gctgaggaac gaaagaaact tcgacaagag aatggaaatg     840
tacatgctat agcataactg aagataaaat tacaggatat cacattggag tcactgccaa     900
gtcatagcca taaatgatga gtcggtcctc tttccagtgg atcataagac aatggaccct     960
ttttgttatg atggttttaa actttcaatt gtcacttttt atgctatttc tgtatataaa    1020
ggtgcacgaa ggtaaaaag                                                 1039
```

<210> 3
<211> 386
<212> DNA
<213> Homo sapiens

<400> 3

```
gataggccac attccagtaa gaactcaatt tgtctcccaa atttgcagaa acaaaacgtg      60
atttaaaagc tgagcttttt atcagaaagc ttttttgatg ttttaagtgt tatgtgactt     120
gttgaacttt ttaaaaagtg ctacttttaa aatcccagat actctgaatt ttagaaaaca     180
aactaattct gattgtgtcg tgcccaagta cccttttttt tttaatgagt agggaccaat     240
gccacattgc tttttatatt tctttctttt ttaatgttgc caaaaccaaa agtagctttg     300
ttttcctttg tattttgcta ctttgcagta tttgtgtgtg tggttttttt tccttaattt     360
gaaagggaca gcactgtgta tgttta                                          386
```

<210> 4
<211> 565
<212> DNA
<213> Homo sapiens

<400> 4

```
aggagaccat ttggaagaag aactagatct gttgcttaat ttagatgcac ctataaaaga      60
gggagataac atcttaccag atcagacgtc tcaggacctg aaatccaagg aagatgggga     120
ggtggtccaa gaggaagaag tttgtgcaaa accatctgtg actgaagaaa aaaacatgga     180
acctgagcaa ccaagtacct ccaaaaatgt taccgaggaa gagctggaag actggttgga     240
cagcatgatt tcctaaaaag gggaaaaaaa gtgcctgaag caaatcttgg ttgccttcta     300
acggcaggtg ggcataaggc tgtccttcag gaccagccag tttacaagca tgtctcaagc     360
tagtgtgttc cattatgctc acagcagtaa atgcctacct ctgtgtttga catctgaaag     420
```

aatacattga agcagcttgt tgcatttgtt tttctggctt agtaatctaa tagatttcct 480
taagggcagg agatagactc tggcccttgt ttctagcctc cttccttgca gtgtttacaa 540
catagccagt gtttacagca tagca 565

<210> 5
<211> 215
<212> DNA
<213> Homo sapiens

<400> 5

ggatccttta ttggtggtag agcaaaaaaa cccaaacacg ataaaccttt caaaagactt 60
tctaaggatg atattggaat gcaccagccc tcacatgtgt atgcacattt gccagaatat 120
aagagttttg ttttaaatac agtcttgtta ggattttacg ttattgttat tatggaaagt 180
gattgtgatg ctatttatct tcagggtcac tctgg 215

<210> 6
<211> 218
<212> DNA
<213> Homo sapiens

<400> 6

gcagtcgttt caaccaggta gttttgggtt gtttttaaag ccctttttgag gtcttacaca 60
ttattaactt taaaataatc aggcagctaa gaataattac tagaaaaatc atctaccact 120
tcaaacatgg tcaactactt caaaactgca cctagagaat caggtacctg aagtagaaca 180
agaagcctgg aggtggactt tgagaggagg gaataccc 218

<210> 7
<211> 525
<212> DNA
<213> Homo sapiens

<400> 7

tacgtgtact tcaccatcgt caccaccgac cacaaggaga tcgacttccg ctgcgcgggc 60
gagagctgct ggaacgcggc catcgcgctg gcgctcatcg atttccagaa ccgccgcgcc 120
ctgcaggact ttcgcagccg ccaggaacgc accgcacccg ccgcacccgc cgaggacgcc 180
gtggctgccg cggccgccgc accctccgag ccctcggagc cctccaggcc atccccgcag 240
cccaaacccc gcacgccatg agcccgccgc gggccatacg ctggacgagt cggaccgagg 300
ctaggacgtg gccggcgctc tccagccctg cagcagaaga acttcccgtg cgcgcggatc 360
ctcgctccgt tgcacgggcg ccttaagtta ttggactatc taatatctat gtatttattt 420
cgctggttct ttgtagtcac atattttata gtcttaatat cttgtttttg catcactgtg 480
cccattgcaa ataaatcact tggccagttt gcttttctac catcc 525

<210> 8
<211> 565
<212> DNA
<213> Homo sapiens

<220>
<221> misc_feature
<222> (69)..(72)
<223> n is a, c, g, or t

<220>
<221> misc_feature
<222> (178)..(181)
<223> n is a, c, g, or t

<220>
<221> misc_feature
<222> (188)..(192)
<223> n is a, c, g, or t

<220>
<221> misc_feature
<222> (525)..(527)
<223> n is a, c, g, or t

<400> 8

```
ctgtgacatg ctcttgagct ttaccctagt tgaacataca tgtgtagatt tacacatact     60
gtttcattnn nnaatttaga aattgttcat taaatcccat ttgaggtata agtcactcag    120
gaagttaaaa tatctctaca cgtatatttt tacattaaaa atacagtgtt agcataannn    180
nccctttnnn nngaagaaca aaaatgtcag tgcatagtta gataaaatgg taaaatgttt    240
tactgaaagc atactttttt ggaaaataga ttcatgaagc ctttaagtgc tgcttctgtc    300
agtcaaacgt taaaaacttt aacattttca aagtgcccag actgtgtaca aagacacatg    360
taatggagat tgtacaggtt gttttttgt ttgaaccttt gaaagagttt aatcttaacg    420
ttttctaatt ttaaaatttt aaaatcttgt ttaacaaaag cttgtattaa gatactgttt    480
tcatttcatt acagaattgt ttataaaagt tcatttgttg aaaannnagg atccttttta    540
ataccacagc atttgtactg ttcct                                          565
```

<210> 9
<211> 285
<212> DNA
<213> Homo sapiens

<400> 9

```
atatgtgcac acacacactc acacccacac ccataaagat tttgcactcc ttgaaggtac     60
actaactcac catttttatc atacttatcc cagtgtgcca cagttactgg cttatatgcc    120
tgtctctgct atcttatttt atctgtctcc acaacacagc aaactacctg gccttcaata    180
aagggcttat gaattattca tgaatccatt ttgccaggtg cctagccctg tgtctggctt    240
gaagcaggtg ttcccaaggt gtggcatggc tgagtgaata caaat                    285
```

<210> 10
<211> 207
<212> DNA
<213> Homo sapiens

<400> 10

```
caccaatgag cttactaccc aacttcaaaa ctaggactct aacaataact tctgtcatat     60
ctcatcctgt aacgccccca ccttcgctcc ttccgccaag ataattatca ctttaaattg    120
tgtgcgtgtg tattctcatt tcttatgtga tggtaaaaat gcctttattt tgtttggttt    180
taatgcatag aaaggacatc aagctgt                                        207
```

<210> 11

<211> 315
<212> DNA
<213> Homo sapiens

<220>
<221> misc_feature
<222> (173)..(181)
<223> n is a, c, g, or t

<400> 11

```
ctctaaaagc cattcactcc agattttacc tggggaatat tctacatact gcttactttc     60
tctataaaac tcatcaataa atcatgaaag gcactgagtt ttgtaaatca ggaccctaaa    120
tgtttaattg taaataagtt tcagataatt attatagctt tgcgttgaag ttnnnnnnnn    180
ntttttctca actagttaag tcaactgctt ctgaaataac tctgtattgt agattatgca    240
gatctttaca ggcataaata tttaaactgt aatatgctaa cttgaagaga ttgcaataaa    300
gctgcttcag ctaac                                                     315
```

<210> 12
<211> 509
<212> DNA
<213> Homo sapiens

<400> 12

```
ctcactgaag ttgaaatgac tgcccacttc aaaatcttca ttgtgtttac acaccagtgt     60
atttatacaa atcagaggca ttttgtagat gctttgctga cttgttcagc tctgtaaaaa    120
cacagaaatc agacccattt tgtaaagcgg aaaatcatgt tacatggaac atgtcctgta    180
tatatcacat acatggtaat ggagtcttaa tgataagtgc aagataataa tttaatgatg    240
ggattagtct gatcgcttaa tatgcacaat cctggaagtg aattacttgc atcagatata    300
gtgatattta ttattctgta cagagagaaa aatacatata aaacatatgc ttacattaca    360
tgcacgcgga tttcatgctc cataatcttt tctatttttt aatttacctt tctgtaaatg    420
atgtgcatgg aatatgcctt atagaaaaat gctgttcata atttgactac gtggaaaagt    480

gcctatatgg tggtaatgct agtaaggca                                      509
```

<210> 13
<211> 1654
<212> DNA
<213> Homo sapiens

<400> 13

```
tggagcccga ggtccccgcg cggcccgggc ctggcgccct gaggggaaga gcggcccggc      60
ccgagccatg acggacggga tcctagggaa ggcagccaca atggagatcc ctatccacgg     120
gaacggcgaa gccaggcagc ttcctgaaga tgatgggctg gagcaggacc tccagcaggt     180
gatggtgtca ggacccaacc tcaatgaaac cagcattgtg tctggtggct atgggggctc     240
tggtgatgga ctcatcccca cagggtctgg ccgccatcca tctcacagca ccactccttc     300
tggccctgga gatgaggtgg ctcggggcat tgctggagaa aagtttgaca tcgtcaagaa     360
atggggcatc aacacctata agtgcacaaa gcaactgtta tcagaacgat ttggtcgagg     420
ctcacggact gtggacctgg agctagagct gcagattgag ttgctgcgtg agacgaagcg     480
caagtatgag agtgtcctgc agctgggccg ggcactgaca gcccacctct acagcctgct     540
gcagacccag catgcactgg gtgatgcctt tgctgacctc agccagaagt ccccagagct     600
tcaggaggaa tttggctaca atgcagagac acagaaacta ctatgcaaga atggggaaac     660
gctgctagga gccgtgaact tctttgtctc tagcatcaac acattggtca ccaagaccat     720
ggaagacacg ctcatgactg tgaaacagta tgaggctgcc aggctggaat atgatgccta     780
ccgaacagac ttagaggagc tgagtctagg cccccgggat gcagggacac gtggtcgact     840
tgagagtgcc caggccactt tccaggccca tcgggacaag tatgagaagc tgcggggaga     900
tgtggccatc aagctcaagt tcctggaaga aaacaagatc aaggtgatgc acaagcagct     960
gctgctcttc cacaatgctg tgtccgccta ctttgctggg aaccagaaac agctggagca    1020
gaccctgcag cagttcaaca tcaagctgcg gcctccagga gctgagaaac cctcctggct    1080
agaggagcag tgagctgctc ccagcccaac ttggctatca agaaagacat tgggaagggc    1140
agccccaggg tgtgggagat tggacatggt acatcctttg tcacttgccc tctggcttgg    1200
gctccttttt ctggctgggg cctgacacca gttttgccca cattgctatg gtgggaagag    1260
tgcctggagg cccagaagtt gctgccctgt ctatcttcct ggccacaggg cttcattccc    1320
agatcttttc cttccacttc acagccaacg gctatgacaa aaccactccc tggccaatgg    1380
catcactctt caggctgggg tgtgctccct gaccaatgac agagcctgaa aatgccctgt    1440
cagccaatgg cagctcttct cggactcccc tgggccaatg atgttgcgtc taataccctt    1500
tgtctctcct ctatgcgtgc ccattgcaga gaaggggact gggaccaaag gggtggggat    1560
aatggggagc cccattgctg gccttgcatc tgaataggcc taccctcacc cacccaccca    1620

gtttaattgt gcttagagcc caagaagatt ggga                                 1654
```

<210> 14
<211> 652
<212> DNA
<213> Homo sapiens

<220>
<221> misc_feature
<222> (188)..(188)
<223> n is a, c, g, or t

<400> 14

```
tttttttttt ttttttttaa agtagtttaa taaactccac aaaataatag cagatgcatt      60
gaaatattta cataattcga ttttcaaatc tctcattcaa ataaaaggga taaaaataaa     120
atttctgcct ttacggcagc agaacctctt tcctgaaatg gattggtaaa ataagatact     180
tcactggnag aggaactaat ttatgtttaa gaggtattca tattcagcta agaaaataca     240
accctttttc agctatatag attagggaat ataaaatgat attttctaca ttttttgacc     300
tgtattcaaa gttctaaatt caactttgac ttgaagagag aaggtgattt tggtacccat     360
acagagtaga tcatcacaat tacaatggaa agataattaa cgttttatat gctgtttatt     420
tgcttttgaa agtttgggtc agaaaggctg tgataataat tctggcccaa acaggtatgc     480
ttatacctga cacaaatttc actaaaacct aacacttttg gcttggagtt cttgggattt     540
cgactttctg agtcccttcc atttccaaag catgtttcat tgagagcagg caatgtttgg     600
ggatcaggtg tatgattcaa gactaattaa gatgccaaag ttttccaagc tc             652
```

<210> 15
<211> 1990
<212> DNA
<213> Homo sapiens

<400> 15

```
attgtggcgg tgaggaacag gaagccctga agggtcaaaa gaaatacaaa agcaaaggct      60
atttcttttt ttttttctt tctttcattc cttccttcct ctgtttcttt ctttcttcct     120
ttcatttttt tttcttttt aagagcgagc ggctctgcgg tggcggtttg gggtgggcgc     180
cgccgaggtg aggtcgtctc gcctcccgcg cgccggtaga ttggttgttt cattatggat     240
ggaggggatg atggtaacct tattatcaaa aagaggtttg tgtctgaggc agaactagat     300
gaacggcgca aaaggaggca agaagaatgg gagaaagttc gaaaacctga agatccagaa     360
gaatgtccag aggaggttta tgaccctcga tctctatatg aaaggctaca ggaacagaag     420
gacaggaagc agcaggagta cgaggaacag ttcaaattca aaaacatggt aagaggctta     480
gatgaagatg agaccaactt ccttgatgag gtttctcgac agcaggaact aatagaaaag     540
```

```
caacgaagag aagaagaact gaaagaactg aaggaataca gaaataacct caagaaggtt     600
ggaatttctc aagagaacaa gaaggaagtg gaaaagaaac tgactgtgaa gcctatagaa     660
accaagaaca agttctccca ggcgaagctg ttggcaggag ctgtgaagca taagagctca     720
gagagtggca acagtgtgaa aagactgaaa ccggaccctg agccagatga caagaatcaa     780
gagccctcat cctgcaagtc tctcggaaac acctccctga gtggcccctc catccactgc     840
ccctctgctg cagtatgtat cggcatcctc ccaggcctgg gtgcctactc tgggagcagc     900
gactccgagt ccagctcaga cagcgaaggc accatcaatg ccaccggaaa gattgtctcc     960
tccatcttcc gaaccaacac cttcctcgag gccccctagt ttctccgtcc ctacacaggg    1020
agctcctccc caagggtaga tcggaccgtt catgctgcct ataggcatta tgtccctcaa    1080
aaaaaaactc ctttgcctgc atcctgtgta caacatgaca tttttaacca atccaatcta    1140
aaaatgtgcc agaatccacc tgtggcccga atcgtgtttg gttcctcttt ctactccact    1200
gcagatgacc aaacctgtcc cgctgccact ttcctcactg atattgggag gagggcaagg    1260
cccagccgaa gttccactaa aaatgcccca ggagaatagg caccggctgg cttgccaaag    1320
ggtttgggtt ttattgcttt ctgtttttttc ttttcccgac agcacaaaga agtaagggca   1380
gttattggac aggtgttatt taaacattct attgtaaatg aatgtgttgt ttggttctac    1440
tgcattgtgg agcatgcggg ggaagagaac tgacccaggt aatgaaatgg agcccttccc    1500
tggaactaac cagtccttga tgttgtgtga ctaagtaaag atgataaacc ccatctgctg    1560
ggggtgtcac ttcacactcg gcatgcattg tgaaagcttt ccataccctt ggccattccc    1620
tctctcctct ctctccaacc ccatttatgc aggaggggac tgctaacaag aacgcttcca    1680
tctcaaacct tttctctgcc tgggaaatta ttttatgttt gtttttgaaa taaaggattt    1740
agtttaagat tctaaatttt agagaaacaa acgtaggcct tgtttactaa tagccagaca    1800
tcagaactgc aggtaggtat gttaatgaga tgacttattt ctggcagctc ctggaatcct    1860
aatattgtaa atgagtggga cacacttgca tattgtgacc attctattga ggcccttctc    1920
tgtttaatgc atattatact tgtgctttta actgtggaat ctatttctaa cctaaaaaaa    1980
aaaaaaaaaa                                                            1990
```

<210> 16
<211> 450
<212> DNA
<213> Homo sapiens

<220>
<221> misc_feature
<222> (355)..(355)
<223> n is a, c, g, or t

<220>
<221> misc_feature
<222> (361)..(361)
<223> n is a, c, g, or t

<220>
<221> misc_feature
<222> (375)..(375)
<223> n is a, c, g, or t

<220>
<221> misc_feature
<222> (380)..(380)
<223> n is a, c, g, or t

<220>
<221> misc_feature
<222> (404)..(404)
<223> n is a, c, g, or t

<220>
<221> misc_feature
<222> (437)..(437)
<223> n is a, c, g, or t

<220>
<221> misc_feature
<222> (445)..(445)
<223> n is a, c, g, or t

<400> 16

```
aaatttctgt attttagttt tgaagtgctt tctatttaaa aataaaaaac atgatttatt     60
ttcatttctg acacagaagt gtttctttta aaaaaaaaag accacatttt aaatttctgc    120
ttaaatgtat ataaagtata catttaagta tactggcact cgcaacaaat gaatccttcc    180
ccagggataa atggattgga aaatttgttt ttcattcaac atttggaaag agaacaaacc    240
tgaaatatgt aatttttaaa attatgtgaa aataatgtga aaaatttcat atagtatttg    300
tgtgaaaatc aggtggaaaa aaacttccat gaagaaccca atttaccaaa attcnccatc    360
nttttaagat ttacnttttn aataccatac tactggtttt aacnggaatt ggggtgtggt    420
atgagggggg ttttgcnggg gagangggga                                     450
```

<210> 17
<211> 508
<212> DNA
<213> Homo sapiens

<400> 17

```
tatttccatg aattcaaagc cttttaatga tgtgaacact tactccccat ttcttttttta     60
cattgttaca aaaaatttac atacagtttt ctgaaagtgg cattttgttg gttgttatta    120
tactgatgac acatattaac actttgtatt gaagaagtat cataaaaatc acagggcatt    180
acagattttt gataagaagt agtaatagca ttgtctttta acagctggag gctcccaggc    240
atactctttg gtgagaaatg attaatttta tattttcatt ttgatgagaa tcttttcttg    300

tttttaccag ttataaaaac aaagcttttt ctttgttgtg atactgtgca ctaagactta    360
gtttcttgag ctgatgctaa ataaaatgag atcaatagga atattccggg aggtcgtgag    420
aagtttttag aaaggatggc atctacatat atatggagct ctgaaaactg ttggagagta    480
tgacctggga ctgaaactgt ggagcaca                                       508
```

<210> 18
<211> 475
<212> DNA
<213> Homo sapiens

<220>
<221> misc_feature
<222> (475)..(475)
<223> n is a, c, g, or t

<400> 18

```
tcgacatcta atcctcattc ttatgaactt ggtattatgt gaatccattg ggaaatgaag      60
actcagagag gttaaaccat ttgcccaagg tcacacagct actaagcggt gctaggatta     120
aaccctggca gtttcagtcc ttaaccaaag ggttaagcgc tttacatata tatacctgta     180
tattatcaat tttcaaataa tttgaaatag taaaatgcag ttcctctggt cctgaatatg     240
aggtaatctt cctatggttc agaagacatt tcagcatttc ctaatatatc ataatgagtc     300
cattttggtt gtaccatgat gtagtcattt tgttttatag tatattaaag aatgcagaaa     360
agcatagctc atagttccag tgtcttgctc tgaggttttt ccattcagta gacattttaa     420
gtatatgtac caggcacata aatatccaga taattaaagt ttatatcatt aagcn          475
```

<210> 19
<211> 1061
<212> DNA
<213> Homo sapiens

<400> 19

```
gcgccatcaa tcgccgccgc ctcgtcccgc ttctcggctg aggcgccgcg cggccaggca      60
gcgggtccag gcctcagccg cgcgcccagg ggcctccggg gccctcccgg gtcagcatgc     120
ccggggtgaa actgaccacc caggcctact gcaagatggt gctgcacggc gccaagtacc     180
cgcactgcgc cgtcaacggg ctcctggtgg ccgagaagca gaagccgcgt aaggagcacc     240
tccccctggg cggccccggc gcccaccaca ccctcttcgt ggactgcatc cccctcttcc     300
acggcaccct ggccctcgcc cccatgctgg aggtggctct caccctgatt gattcatggt     360
gcaaagatca tagctacgtg attgctggtt attatcaagc taatgagcga gtaaaggatg     420
ccagtccaaa ccaggttgca gagaaggtgg cctccagaat cgccgagggc ttcagcgaca     480
ctgcgctcat catggtagac aacaccaagt ttacgatgga ctgcgtagcg cctacgatcc     540
acgtgtacga gcaccatgag aacagatggc ggtgcagaga cccacaccat gactactgtg     600

aagactggcc agaggcacag aggatctcag cctcgctcct ggacagccgg tcctacgaga     660
cgctcgtgga tttcgataac cacctggatg acattcggaa tgactggaca aacccagaga     720
tcaataaagc tgtcctacac ttgtgctagg caggcaccgc tgtgactggg ctccgggcct     780
ttcccactac gttgaagaag aaaacctatt tttaaatgta aataaaatat ctggtagcct     840
gtgtggaaag ctgaccgttt taagaagtgg catgtgcctt gaaagggggc agaatgttca     900
gtcggtcgtg tttttaacac agagtctcta gaagaggtgc agacatcccg tctgactgtc     960
cctgtggact ctctcagttg tatgttgcta taatcctcca aatcaaagct ctttctgctt    1020
gtgcaagatt gttcctatta aacagtttta actaaccttt a                        1061
```

<210> 20
<211> 2040
<212> DNA
<213> Homo sapiens

<400> 20

```
gaattccggg cagctcctct tccatctcca gaaatgacct ccaccttcaa cccccgagaa      60
tgtaaactgt ccaagcaaga agggcaaaac tatggcttct tcctgcgaat tgagaaggac     120
accgagggcc acctggtccg ggtggttgag aagtgtagcc cagcagagaa ggctggcctt     180
caagatggag acagagttct taggatcaat ggtgtctttg tggacaaaga agaacatatg     240
caggttgtgg atctggtcag aaagagtggg aattcagtga ctttactagt tctggatggg     300
gattcctatg agaaagcagt gaaaacacgg gtggacttga aagagttggg tcaaagtcag     360
aaggagcaag gtttgagtga taatatactt tcccctgtga tgaatggagg tgtgcaaact     420
tggacccagc cccggctctg ctatctcgtg aaggaaggag gcagctatgg cttctctctg     480
aaaactgtcc aaggtaaaaa gggggtgtac atgactgata ttacacctca aggtgtggct     540
atgagagctg gagttctggc tgatgatcac ttgattgaag tgaatggaga gaatgtagag     600
gatgccagcc atgagaaagt ggttgaaaag gtgaagaagt caggaagccg tgtcatgttc     660
ctgctggtgg acaaagaaac tgacaagcgt catgttgagc agaagataca attcaaaaga     720
gaaacagcca gtttgaaact gttaccccac cagccccgaa ttgtggagat gaagaaagga     780
agcaatggct atggtttcta tctgagggca ggctcagaac agaaaggtca aatcatcaag     840
gacatagatt ctggaagtcc agcagaggag gctggcttga agaacaatga tctggtagtt     900
gctgtcaacg gcgagtctgt ggaaaccctg gatcatgaca gtgtggtaga aatgattaga     960
aagggtggag atcagacttc actgttggtg gtagacaaag agacggacaa catgtacaga    1020
ctggctcatt tttctccatt tctctactat caaagtcaag aactgcccaa tggctctgtc    1080
aaggaggctc cagctcctac tcccacttct ctggaagtct caagtccacc agatactaca    1140
gaggaagtag atcataagcc taaactctgc aggctggcta aaggtgaaaa tggctatggc    1200

tttcacttaa atgcgattcg ggtctgccca ggctcattca tcaaagaggt acagaagggc    1260
ggtcctgctg acttggctgg gctagaggat gaggatgtca tcattgaagt gaatggggtg    1320
aatgtgctag atgaacccta tgagaaggtg gtggatagaa tccagagcag tgggaagaat    1380
gtcacacttc tagtctgtgg aaagaaggcc tatgattatt tccaagctaa gaaaatccct    1440
attgtttcct ccctggctga tccacttgac acccctccag attctaaaga aggaatagtg    1500
gtggagtcaa accatgactc gcacatggca aaagaacggg cccacagtac agcctcacat    1560
tcttcttcca attctgaaga tacagagatg tgatgaaaac aagtaatagc tttggctgtt    1620
tatttgatag ctgtttctgg gtatttaata ggaatccttt ctcaaggaat gagttgtgac    1680
ctgtttactg tctctttaga agaaaaactc cactggaaac cattcaccat gtgtgactgt    1740
cttctgttat catttgtctt acaggcggct attgcagacg gctaatttat gcttaactta    1800
ggaagagata aggcaagagc tagatttttt tcatgtgatc ttttccaagc ttcaacttaa    1860
cttaactaca tttctctgta tgatgatgtc tcttacttct acaggttcct tgagcaccaa    1920
agatgattca taactctgta taggtgacag ctgcttataa aagcatctta gcagataagc    1980
ctattaaaat tgtgcttttg taacaatgtt gtggttgcta gaataaatac catgaacccg    2040
```

<210> 21
<211> 633
<212> DNA
<213> Homo sapiens

<400> 21

```
tttaaaagcc actaattatc tgttttttat tttgtaagta acaagatata gacatttgaa      60
tgccaatgtc ttattctgga gagacactgg agctgaagtt caacaatgat cacacttatt     120
acctggcaat aaaaacacaa ccatctttcc agtcaggtca aaatatccta ctttttgcct     180
ttctaccaaa tcccaaacat tcacagtttt tcaaggacca ctaataaaat acaggaagct     240
tttaaagaca gtaagagaac acctagtgta agttaggtga attaaagatg gcaaaggaga     300
ttacatcctc aacactgaca gcttccaaga cttagaaaag agattgttcc ttgcttctaa     360
aattgttcta ttttcctctg taggaaaatg aaagtttttt cttacaaata ttaaataatc     420
aaagtactta cgcaaaatta atctgctcct caatgagatg agcactccat ttaaatgatc     480
tttacagatc cctgaagttg ctgtcctgtc actgtattta agtgatggat attcaattga     540
attattctgc ataaataatt ctggtcaacc cagacgtata gtagtatgat gggtcagata     600
cagtcaactg ttcaataaaa atgcagatgt ctg                                   633
```

<210> 22
<211> 1878
<212> DNA
<213> Homo sapiens

<400> 22

```
ctgcgtttct cctcaaacct aacgatgccg ccggagcgga ggagacgaat gaaactggac      60
cggagaaccg gagcgaagcc gaagcggaag cccggaatga ggccggactg gaaagccgga     120
gcggggccag gcgggcctcc ccaaaagcct gccccttcat cccagcggaa accgccggcc     180
cggccgagcg cggcggccgc tgcgattgca gtcgcggcgg cggaggaaga gagacggctc     240
cggcagcgga accgcctgag gctggaggag gacaaaccgg ccgtggagcg gtgcttggag     300
gagctggtct tcggcgacgt cgagaacgac gaggatgcgt tgctgcggcg tctgcgaggc     360
ccgagggttc aagaacatga agactcgggt gactcagaag tggagaatga agcaaaaggt     420
aattttccac ctcaaaagaa gccagtttgg gtggatgaag aagatgaaga tgaggaaatg     480
gttgacatga tgaacaatcg gtttcggaag gatatgatga aaaatgctag tgaaagtaaa     540
ctttcgaaag acaaccttaa aaagagactt aaagaagaat tccaacatgc catgggagga     600
gtacctgcct gggcagagac tactaagcgg aaaacatctt cagatgatga aagtgaagag     660
gatgaagatg atttgttgca aaggactggg aatttcatat ccacatcaac ttctcttcca     720
agaggaatct tgaagatgaa gaactgccag catgcgaatg ctgaacgtcc tactgttgct     780
cggatctcat ctgtgcagtt ccatcccggt gcacagattg tgatggttgc tggattagat     840
aatgctgtat cactatttca ggttgatggg aaaacaaatc ctaaaattca gagcatctat     900
ttggaaaggt ttccaatctt taaggcttgt tttagtgcta atggggaaga agttttagcc     960
acgagtaccc acagcaaggt tctttatgtc tatgacatgc tggctggaaa gttaattcct    1020
gtgcatcaag tgagaggttt gaaagagaag atagtgagga gctttgaagt ctccccagat    1080
gggtccttct tgctcataaa tggcattgct ggatatttgc atttgctagc aatgaagacc    1140
aaagaactga ttggaagcat gaaaattaat ggaagggttg cagcatccac attctcttca    1200
gatagtaaga aagtatacgc ctcttcgggg gatggagaag tttatgtttg ggatgtgaac    1260
tcaaggaagt gccttaacag atttgttgat gaaggcagtt tatatggatt aagcattgcc    1320
acatctagga atggacagta tgttgcttgt ggttctaatt gtggagtggt aaatatatac    1380
aatcaagatt cttgtctcca agaaacaaac ccaaagccaa taaaagctat aatgaacttg    1440
gttacaggtg ttacttctct gaccttcaat cctactacag aaatcttggc aattgcttca    1500
gaaaaaaatg aaagaagcag tcagattggt tcatcttcct tcctgtacag tattttcaaa    1560
cttcccagtc attaaaaata agaatatttc tcatgttcat accatggatt tttctccgag    1620
aagtggatac tttgccttgg ggaatgaaaa gggcaaggcc ctgatgtata ggttgcacca    1680
ttactcagac ttctaaagag actatttgaa gtccagttga gtcacaagag aagcctgtct    1740
tgatatatca tctcagaaac tttcctgaat atgtgataat atatggaaaa tgatttatag    1800
atccagctgt gcttaagagc cagtaatgtc ttaataaaca tgtggcagct tttgtttgaa    1860

aaaaaaaaaa aaaaaaaa                                                    1878
```

<210> 23
<211> 3426
<212> DNA
<213> Homo sapiens

<400> 23

```
aattccgggc ggcggcggcc gaggctgaag gaagatggcg gacggcgtgg accacataaa     60
catttacgcg gatgtcggcg aagagttcaa ccaggaagct gaatatggtg ggcatgatca    120
gatagatttg tatgacgatg tcatatctcc atctgcaaat aatggagatg ccccagaaga    180
ccgagattac atggatactc tcccaccaac tgttggtgat gatgtgggta aaggagcagc    240
accaaatgtt gtctatacat atactggaaa gagaattgca ttatatattg gaaatctaac    300
atggtggaca acagatgaag acttaactga agcagttcat tctttgggag taaatgatat    360
tttggagata aaattttttg aaaatcgagc aaatggccag tcaaaggggt ttgcccttgt    420
tggtgttgga tctgaagcat cttcaaaaaa gttaatggat ctgttaccta aaagagaact    480
tcatggtcag aatcctgttg taactccatg caataaacag ttcctgagtc aatttgaaat    540
gcagtccagg aaaactacac aatcaggaca aatgtctggg gaaggtaaag ctggtcctcc    600
aggaggcagt tcccgtgcag catttccaca aggtggtaga ggacggggcc gttttccagg    660
ggctgttcct ggtggggaca gatttcctgg gccagcagga ccaggagggc cacccccacc    720
ttttccagct ggacagactc caccacgtcc acccttaggt cctccaggcc cacctggtcc    780
accaggtcct ccacctcctg gtcaggttct gcctcctcct ctagctgggc ctcctaatcg    840
aggagatcgc cctccaccac cagttctttt tcctggacaa ccttttgggc agcctccatt    900
gggtccactt cctcctggcc ctccacctcc agttccaggc tacggccccc ctcctggccc    960
accacctcca caacagggac cacctccacc tccaggcccc tttccacctc gtccacccgg   1020
tccacttggg ccacccctta cactagctcc tcctccgcat cttcctggac cacctccagg   1080
tgccccaccg ccagctccgc atgtgaaccc agctttcttt cctccaccaa ctaacagtgg   1140
catgcctaca tcagatagcc gaggtccacc accaacagat ccatatgggc gacctccacc   1200
atatgatagg ggtgactatg gcccccctgg aagggaaatg gatactgcaa gaacgccatt   1260
gagtgaagct gaatttgaag aaatcatgaa tagaaatagg gcaatctcaa gcagtgctat   1320
ttcgagagct gtgtctgatg ccagtgctgg tgattatggg agtgctattg agacactggt   1380
aactgcaatt tcttttaatta aacaatccaa agtatctgct gatgatcgtt gcaaagttct   1440
tattagttct ttgcaagatt gccttcatgg aattgagtcc aagtcttatg gttctggatc   1500
aagacgtgaa cgatcaagag agagggacca tagtagatca cgagaaaaga gtcgacgtca   1560
taaatcccgt agtagagacc gtcatgacga ttattacaga gagagaagca gagaacgaga   1620
```

gaggcaccgg gatcgtgacc gagaccgtga ccgagagcgt gaccgagagc gcgaatatcg 1680
tcatcgttag aagctgaagg aagaggatca ccttccaaga caaaacagtc ttcatgggcc 1740
aaaaatgacg cttgtccagc agtttgcttc ttgtgattga actgaacctg taaggattca 1800
tggataaaat gaacaggaat agatctgaat aaagcaaatc tgcataaatg gtaaccagta 1860
gctctacttt tattttttat gttgcttaac tgttttattt gaaggaaacc tgtgtgattt 1920
aaaaagttat agcttttgca actttattac tggttatata catttggcca ttatgatgtg 1980
caagcaattg gaaaaaaagt caagtaaatg cttgtttttg tagtagtttg ttcttgttaa 2040
aaatgtttat atgataatgt ctgtaaacag catcactttg attacaatag atgtagtgtt 2100
gtaataaact gtttaatggg gctgatgtgt aaagctgttc aagttatttg atgtttacac 2160
ctcagggaaa gtcttgtgtt cagcaatatc taaagataat gttactatga caacattttt 2220
actgtccttt aaagcattgc aatagcgttt ttggatatgc ctcaatctaa tcttgcgttc 2280
agtgaattaa acatagtaat taagtgtctt ttgcccttga ttttgatatt agaataggtg 2340
attacatgga tatttaatat ttctatattc tgcttttcta gctgttttta cctagttagc 2400
ttgtgacttt gctgaatggt atgtaaactt gtaaaaatag agatttgaca gacatagcaa 2460
tctagtcaat gtgtaagggg tcaaaaaaaa cagaggtttt aacacataag taaaaacccg 2520
tacatatttg atgtgtaatg caggttaatt acaacacaga tgtaccgaaa cacttaattg 2580
tgaaccgcta acattgaaga aattttgaca attccgattt gatgctgcaa ttacttgctg 2640
tttttattga tcttatggtt tatttcttaa gccatagtca gtgtaaatac agccctgcag 2700
caggtaaatg tgagtaaaga gagccttata ttttccaatt ggtataaaat ttttgaagga 2760
tgtgatgttc attaacattc ggttgtattc cccagtattt gtaatgggaa attacagata 2820
aaccgtgtct gcacagttta aggaatacta tgtatattca tgcaccgtat tgattcatgc 2880
tatagttact taatcaaaga ttttttttcaa acctgcctta catataggcc cactttaaaa 2940
gcacctgact agcatgtgtt cttgattgca aaattggcag aggcagggtg tcaacttgat 3000
taggtgtttt tatgggaatg taatttgaaa tcactacttc agaaatttga cttaaaattc 3060
ttgagcacgt taatatgttt ttaagatctg attatctttg agagatcttc tgttaataca 3120
cattggttgt taaagagtac ccaaattcta ggacaatgct taaagtgtta aaataccta 3180
gatactgtgt tatgtgcaac tgtagaaacc ctccagaaat ttccactgct gttcttcact 3240
ttcatcttgt ctgctatcaa accacttctg acaaaattag ctgttttgaa ttacccatat 3300
cactgccagt tttattttaa aatattttgt gtttgaagta tctgtgcatg ggatcgttga 3360
tgtttatcag aactgttcac tttcagaaat gattttttaa agcattttgt tgaaatgcgg 3420
ttgctt 3426

<210> 24
<211> 5401
<212> DNA
<213> Homo sapiens

<400> 24

```
tcactctcgc tgccgctgct ccgccccatc cccttctgtt tttctctctc attctccagt     60
ggcggcggcg gggaaggcgg aggcagaggc agcagcagcc gcgctggctg caatgaatga    120
tcccccagct tggggggagg actccaggtg agcctctgcc ctcgggaggc ccgggacccc    180
cggccgccca cgaccggcag cccacgctat ggatccctag aggaaggagg agaagacagc    240
tcgccgccca cccccatccc attttcctct tcctttatct cattgttgcc gaagctgttt    300
acggcagcgc tccctctgct ccagcatggg gcgggctccg ggcacggctg ctcggcaggc    360
gctgctcccg cggcgactgg gggattctgc ctaattcacc tcccagccgg tgcagagagg    420
accggagagc ggtggaggcc cggactgcag cagcgttggg gccacctccc agcgtcccca    480
ccctaggagg ctgcatgcgg attgaagagc tgcgcctggg ggctgggccg gccccgctga    540
tcccgaccta gcgagcagga tagcaggacc gcccaggctg cggaggggct cgggggcagg    600
aaggtcagag cagcaagatg gccagtaaga ccaaggccag cgaggccctc aaggtggtgg    660
cccggtgccg ccccctcagc aggaaggagg aggctgctgg tcacgagcag atcctgacca    720
tggacgtgaa actgggccag gtgaccctgc ggaacccccg cgccgccccg ggggagctgc    780
ccaagacctt cacctttgac gccgtgtatg atgccagctc caagcaggcc gacctgtatg    840
acgaaaccgt gaggcccctg atagactccg tgctccaggg tttcaatggc acggtgtttg    900
cctatggcca gacgggcact ggcaagacct ataccatgca ggggacctgg gtggagcccg    960
agctgcgcgg ggtcatcccg aatgcctttg agcacatctt cacccacatc tcccgctccc   1020
agaaccaaca gtacctggtc cgggcctcct atttggagat ctaccaggaa gagattcgag   1080
acctgctctc caaggagccg ggcaagaggc tagagctgaa agagaacccc gagactggcg   1140
tctacatcaa ggacctctcc tccttcgtca ccaagaatgt caaggagatt gagcatgtga   1200
tgaacctggg gaaccagacc cggcctgtgg gcagcaccca catgaatgag gtcagctccc   1260
gctcccatgc catcttcatc atcactgtgg agtgcagcga acgtggctct gatggccagg   1320
accacatccg agtgggcaag ctcaacctcg tggacctggc tggcagcgag aggcagaaca   1380
aggcaggccc caacacagcg ggaggggcag ccacaccatc ctcgggtggc ggtggtggcg   1440
gtggaggcag tggtggtggt gctggtggag agaggcctaa ggaagcctcc aaaatcaacc   1500
tctcattatc tgccctgggc aacgtgattg ctgccctggc gggcaacagg agcacccaca   1560
ttccctaccg ggactccaag ctgacccggc tgctccagga ctccctgggg gggaatgcca   1620
agaccatcat ggtagccaca ctggggccag cttctcacag ctacgatgag agcctctcca   1680
```

ccttgcgctt tgccaaccga gccaagaaca tcaagaacaa gccccgggtg aacgaggacc 1740
ccaaggacac actgctgcgg gaattccaag aggagattgc ccgcctgaag gcccagctgg 1800
agaagagggg gatgctgggg aagcggcccc ggaggaagag cagccgcagg aagaaggccg 1860
tgtccgcccc gcctgggtac cctgagggcc cagtgattga ggcctgggtg gcagaagagg 1920
aggatgacaa caacaacaac caccgcccgc cccagcccat cctggagtca gccttggaga 1980
agaacatgga gaattacctg caggaacaga aggagcggct ggaggaggag aaggcagcca 2040
tccaggatga ccgcagcctg gtgagcgagg agaagcagaa gctgctggag gagaaggaga 2100
agatgctgga ggacctgcgg cgggaacagc aggccacaga gctgcttgcg gccaagtaca 2160
aggccatgga gagcaagctc ctcatcgggg gcaggaacat catggatcac accaacgaac 2220
agcagaagat gttggaactg aagaggcagg agattgccga gcagaaacgt cgtgagcggg 2280
agatgcagca ggagatgatg ctccgggacg aggagactat ggagctccgg ggcacctaca 2340
catccctgca gcaggaggtg gaggtcaaaa ccaagaaact caagaagctc tacgccaagc 2400
tgcaggcggt gaaggcggag atccaggacc agcatgatga gtatatccgc gtgcggcagg 2460
acctggagga ggcgcagaac gagcagaccc gcgaactcaa gctcaagtac ctaatcatcg 2520
agaacttcat cccgccggag gagaagaaca agatcatgaa ccggcttttc ctggactgtg 2580
aggaggagca gtggaagttc cagccactgg tgccagccgg cgtcagtagc agccagatga 2640
agaagcggcc aacatctgca gtgggctaca agaggcctat cagccagtat gctcgggttg 2700
ccatggcaat ggggtcccac cccaggtaca gggctgaaaa cataatgttt ctggagttgg 2760
atgtgtcccc tccagctgtc tttgagatgg aattctctca cgaccaagaa caagaccctc 2820
gtgcgctaca catggagagg ctcatgcgat tggacagctt tctggaaaga ccttccacgt 2880
ctaaagtccg aaagtccaga tcctggtgcc agagtcctca gcggcctcca ccttccacca 2940
cacatgcctc cctggcctct gcttctctgc gccctgcaac agtggcggac catgagtgac 3000
aaccatcacg tcaggctgcc catccaatag actcctggga tggggcagcc aaccctggct 3060
catctcatct gccgcttggt gcgtgtgcgt gtgcgtgcat gtgcgtgtgc gtgtgtgcag 3120
gggtgagaat ctggcagatg gtgcctctgc ctgctcttct tcgcctcctt tatttaattc 3180
atgttattta ttcgcggagc tctgttcgtg ttggggagat gccctcgcct gagccgtctg 3240
ggcctaccgt ggtcactgcg tagcctcttt ttcttctgac ttgagagctc ccccagtcag 3300
atctcaggct tgtccccctg tcagctgcct ccagaaggga aggtagccag tgcctgagaa 3360
gacagtccct tttctaccca ccgcactcca taacctccat cttctcccac actgatggcg 3420
agcagcccct gagcactttc tgggactggg agactgcttg gtgttccctg aggacaagag 3480
acatcctgac agtgttgggc atctgctccc cgtggacaca gccccactct ccactttctg 3540
agcctcagac aacctcattc agcctcttgg gctccttttc aaggacatta ataacctcac 3600

```
caacatagct catgcccttc agctttgaca agaactcacg gcttcccaaa ctctgctttc   3660
tgcccacctt ggatgggaac tgtggaccaa gcaattacca tcgccttgga acctgcagga   3720
aatggaacag caattgagac aacttgaaca gtcatcaacg gaagtccctc cactggattc   3780
ctttgtttct gtcccctccg aggagtcatt ttggtcgaca ggctctcaag gcaactcccc   3840
attttcaaga ggctgctcct gcctgcttcg atcatttctc cctgcagctg cctagacccc   3900
gttcacagtg ggaggagtca atgtcattct acccctcgct aaacgaagat attaacatct   3960
attgcttttt cccttcatct gtcacaggaa acagaagccc aggcacaatc ttttccagct   4020
ttgcctgtta cccctgtttc tgaattgcat ctttaaggta ttattttgtt gacaatagat   4080
cctttattca ctagttacgc aaattggttc ctagggggat actccttacc ttcctttgtg   4140
atggcccaaa atgtctctag gtatctcaag tgataagtaa atttctacaa aaaaaaatgg   4200
ttaatgttca ttgactggct ttttaagtgt atattttgga ggacgggtga agaggtcata   4260
acgaaagcaa gcgagtgaat taggatttca aagtgcccta atagtgtgag tctccagttc   4320
ctagaatatg aagagtgctg tcgttggggt gaaaccatga gactgacaga tctgcctgaa   4380
atggggggtg tgggaggtgg tggcgggggt tattctcttt ccttcaggaa atgaaccctt   4440
cttacatcat tcaagttctg ctctgaggat caagcttggg tctgatttaa ctcagcgaca   4500
ctgtcatttc tgcttcatta ctggactaga gggttgagcc acccacttgc catttgctcc   4560
tgtccttcca ggaaatcaca attttcatca gagcccaaga gattatttga gactcaggat   4620
tcagatcaga ggttcgactg tggctgggac aggagttgtg tgtagaaatt caccaggtgg   4680
cctgagcgca gggggacctc cagggctgcg ttgagcagcc tctcccactg acctctttct   4740
cgtttgtgga caaagcagca cgtatcacct cattcatcac ttggacacat cgcctttgca   4800
ttgtcttgtc acacctccct cacagtctta tagcacaata tacccaaatc agcccccca    4860
gtccgagggc tgggcccaag gtatggtcgg aggaggagct cctgcctgcg gttttgtgta   4920
tgtgtgtatg tgtgtgcgtg tttgtgtgcg tgtttacctc cacaggggac actctacact   4980
cagtgtaaga tctgctggga acagggccac caggagtggc tggatctcag tctctctgtc   5040
tctctttctc tccttttcct tttggtgtat caaatatttg attgacaaag taagggcctt   5100
gattaggacc aaattctcgt gtgttgctat ggtctttatt taggacaaca attaacaatg   5160
cagtggccca ttcttgtcac tctacacata tgactatacg ggacatatgt aatatataaa   5220
tatatatata aaacattccc ctctgtcccc ttggcttcgg atggaggcct ttctgttgag   5280
ctgaaatgca cctgcagctg ggtgctgcca gcagcttgca ggccccagcc ctgttccaat   5340
caatgcagtt gacaataaag gaatgagtat cgtcacggaa aaaaaaaaaa aaaaaaaaaa   5400
a                                                                   5401
```

<210> 25
<211> 1280
<212> DNA
<213> Homo sapiens

<400> 25

```
taaacaatgg tatcaacgca aagtaagcgg gcagccgcct gcatctgtat ccagcgccag     60
gtcccgccag tcccagtgcg cgcgcccccc agtcccgcac ccgttcggcc aggctaagtt    120
agccctcacc atgcggtcaa aggaggcagc aagtgcatca aatacctgct gttcggattt    180
aacttcatct tctggcttgc cgggattgct gtccttgcca ttggactatg ggtccgattc    240
gactctcaga ccaagagcat cttcgagcaa gaaatttcct aataataata attccagctt    300
ctacacagga gtctatattc tgatcggagc cggcgccctc atgatgctgg tgggcttcct    360
gggctgctgc ggggctgtgc aggagtccca gtgcatgctg ggactgttct tcggcttcct    420
cttggtgata ttcgccattg aaatagctgc ggccatctgg ggatattccc acaaggatga    480
ggtgattaag gaagtccagg agttttacaa ggacacctac aacaagctga aaaccaagga    540
tgagccccag cgggaaacgc tgaaagccat ccactatgcg ttgaactgct gtggtttggc    600
tgggggcgtg gaacagttta tctcagacat ctgccccaag aaggacgtac tcgaaacctt    660
caccgtgaag tcctgtcctg atgccatcaa agaggtcttc gaccaataaa ttccacatca    720
tcggcgcagt gggcatcggc attgccgtgg tcatgatatt tggcatgatc ttcagtatga    780
tcttgtgctg tgctatccgc aggaaccgcg agatggtcta gagtcagctt acatccctga    840
gcaggaaagt ttacccatga agattggtgg gattttttgt ttgtttgttt tgttttgttt    900
gttgtttgtt gtttgttttt ttgccactaa ttttagtatt cattctgcat tgctagataa    960
aagctgaagt tactttatgt ttgtctttta atgcttcatt caatattgac atttgtagtt   1020
gagcgggggg tttggtttgc tttggtttat attttttcag ttgtttgttt ttgcttgtta   1080
tattaagcag aaatcctgca atgaaaggta ctatatttgc tagactctag acaagatatt   1140
gtacataaaa gaattttttt gtctttaaat agatacaaat gtctatcaac tttaatcaag   1200
ttgtaactta tattgaagac aatttgatac ataataaaaa attatgacaa tggccaaaaa   1260
aaaaaaaaaa aaaaaaaaaa                                                1280
```

<210> 26
<211> 3345
<212> DNA
<213> Homo sapiens

<400> 26

```
gcggccgggg cctggggctg cctgccgggc ggccgggcgc ggcgagccca gggaggcagc     60
gtccatggag caaaaggaat gccaggatcc tgcacaggca gacgcgggcc agcctcagca    120
ccgacagccg acgcgcagat agcagagcca tccttggggt tgaaccatga ttccggtgac    180
```

agagctccgc tactttgcgg acacgcagcc agcataccgg atcctgaagc cgtggtggga 240
tgtgttcaca gactacatct ctatcgtcat gctgatgatt gccgtcttcg gggggacgct 300
gcaggtcacc caagacaaga tgatctgcct gccttgtaag tgggtcacca aggactcctg 360
caatgattcg ttccggggct gggcagcccc tggcccggag cccacctacc ccaactccac 420
cattctgccg acccctgaca cgggccccac aggcatcaag tatgacctgg accggcacca 480
gtacaactac gtggacgctg tgtgctatga gaaccgactg cactggtttg ccaagtactt 540
cccctacctg gtgcttctgc acacgctcat cttcctggcc tgcagcaact tctggttcaa 600
attcccgcgc accagctcga agctggagca ctttgtgtct atcctgctga agtgcttcga 660
ctcgccctgg accacgaggg ccctgtcgga gacagtggtg gaggagagcg accccaagcc 720
ggccttcagc aagatgaatg ggtccatgga caaaaagtca tcgaccgtca gtgaggacgt 780
ggaggccacc gtgcccatgc tgcagcggac caagtcacgg atcgagcagg gtatcgtgga 840
ccgctcagag acgggcgtgc tggacaagaa ggagggggag caagccaagg cgctgtttga 900
gaaggtgaag aagttccgga cccatgtgga ggagggggac attgtgtacc gcctctacat 960
gcggcagacc atcatcaagg tgatcaagtt catcctcatc atctgctaca ccgtctacta 1020
cgtgcacaac atcaagttcg acgtggactg caccgtggac attgagagcc tgacgggcta 1080
ccgcacctac cgctgtgccc accccctggc cacactcttc aagatcctgg cgtccttcta 1140
catcagccta gtcatcttct acggcctcat ctgcatgtat acactgtggt ggatgctacg 1200
gcgctccctc aagaagtact cgtttgagtc gatccgtgag gagagcagct acagcgacat 1260
ccccgacgtc aagaacgact tcgccttcat gctgcacctc attgaccaat acgacccgct 1320
ctactccaag cgcttcgccg tcttcctgtc ggaggtgagt gagaacaagc tgcggcagct 1380
gaacctcaac aacgagtgga cgctggacaa gctccggcag cggctcacca agaacgcgca 1440
ggacaagctg gagctgcacc tgttcatgct cagtggcatc cctgacactg tgtttgacct 1500
ggtggagctg gaggtcctca agctggagct gatccccgac gtgaccatcc cgcccagcat 1560
tgcccagctc acgggcctca aggagctgtg gctctaccac acagcggcca agattgaagc 1620
gcccgcgctg gccttcctgc gcgagaacct gcgggcgctg cacatcaagt tcaccgacat 1680
caaggagatc ccgctgtgga tctatagcct gaagacactg gaggagctgc acctgacggg 1740
caacctgagc gcggagaaca accgctacat cgtcatcgac gggctgcggg agctcaaacg 1800
cctcaaggtg ctgcggctca agagcaacct aagcaagctg ccacaggtgg tcacagatgt 1860
gggcgtgcac ctgcagaagc tgtccatcaa caatgagggc accaagctca tcgtcctcaa 1920
cagcctcaag aagatggcga acctgactga gctggagctg atccgctgtg acctggagcg 1980
catcccccac tccatcttca gcctccacaa cctgcaggag attgacctca aggacaacaa 2040

```
cctcaagacc atcgaggaga tcatcagctt ccagcacctg caccgcctca cctgccttaa    2100
gctgtggtac aaccacatcg cctacatccc catccagatc ggcaacctca ccaacctgga    2160
gcgcctctac ctgaaccgca acaagatcga gaagatcccc acccagctct tctactgccg    2220
caagctgcgc tacctggacc tcagccacaa caacctgacc ttcctccctg ccgacatcgg    2280
cctcctgcag aacctccaga acctagccat cacggccaac cggatcgaga cgctccctcc    2340
ggagctcttc cagtgccgga agctgcgggc cctgcacctg ggcaacaacg tgctgcagtc    2400
actgccctcc agggtgggcg agctgaccaa cctgacgcag atcgagctgc ggggcaaccg    2460
gctggagtgc ctgcctgtgg agctgggcga gtgcccactg ctcaagcgca gcggcttggt    2520
ggtggaggag gacctgttca acacactgcc acccgaggtg aaggagcggc tgtggagggc    2580
tgacaaggag caggcctgag cgaggccggc ccagcacagc aagcagcagg accgctgccc    2640
agtcctcagg cccggagggg caggcctagc ttctcccaga actcccggac agccaggaca    2700
gcctcgtggc tgggcaggag cctggggccg cttgtgagtc aggccagagc gagaggacag    2760
tatctgtggg gctggcccct tttctccctc tgagactcac gtcccccagg gcaagtgctt    2820
gtggaggaga gcaagtctca agagcgcagt atttggataa tcagggtctc ctccctggag    2880
gccagctctg ccccaggggc tgagctgcca ccagaggtcc tgggaccctc actttagttc    2940
ttggtattta tttttctcca tctcccacct ccttcatcca gataacttat acattcccaa    3000
gaaagttcag cccagatgga aggtgttcag ggaaaggtgg gctgcctttt ccccttgtcc    3060
ttatttagcg atgccgccgg gcatttaaca cccacctgga cttcagcaga gtggtccggg    3120
gcgaaccagc catgggacgg tcacccagca gtgccgggct gggctctgcg gtgcggtcca    3180
cgggagagca ggcctccagc tggaaaggcc aggcctggag cttgcctctt cagtatttgt    3240
ggcagtttta gttttttgtt tttttttttt taatcaaaaa acaatttttt taaaaaaaaa    3300
gctttgaaaa tggatggttt gggtattaaa aaaaaaaaaa aaaaa                    3345
```

<210> 27
<211> 4762
<212> DNA
<213> Homo sapiens

<400> 27

```
atgcggcgcg gccccggagg cagcagcagc ggcggcggca gccggagcag taggcacccg      60
agcagcgcca gcggccgagc gggcggcttc ctggcctggg cgctccggtg gcggcggagg     120
tgcgcgcgga gccatggtta tcatgtcgga gttcagcgcg gaccccgcgg gccagggtca     180
gggccagcag aagcccctcc gggtgggttt ttacgacatc gagcggaccc tgggcaaagg     240
caacttcgcg gtggtgaagc tggcgcggca tcgagtcacc aaaacgcagg ttgcaataaa     300
aataattgat aaaacacgat tagattcaag caatttggag aaaatctatc gtgaggttca     360
```

gctgatgaag cttctgaacc atccacacat cataaagctt taccaggtta tggaaacaaa 420
ggacatgctt tacatcgtca ctgaatttgc taaaaatgga gaaatgtttg attatttgac 480
ttccaacggg cacctgagtg agaacgaggc gcggaagaag ttctggcaaa tcctgtcggc 540
cgtggagtac tgtcacgacc atcacatcgt ccaccgggac ctcaagaccg agaacctcct 600
gctggatggc aacatggaca tcaagctggc agattttgga tttgggaatt tctacaagtc 660
aggagagcct ctgtccacgt ggtgtgggag ccccccgtat gccgccccgg aagtctttga 720
ggggaaggag tatgaaggcc cccagctgga catctggagc ctgggcgtgg tgctgtacgt 780
cctggtctgc ggttctctcc ccttcgatgg gcctaacctg ccgacgctga gacagcgggt 840
gctggagggc cgcttccgca tccccttctt catgtctcaa gactgtgaga gcctgatccg 900
ccgcatgctg gtggtggacc ccgccaggcg catcaccatc gcccagatcc ggcagcaccg 960
gtggatgcgg gctgagccct gcttgccggg acccgcctgc cccgccttct ccgcacacag 1020
ctacacctcc aacctgggcg actacgatga gcaggcgctg ggtatcatgc agaccctggg 1080
cgtggaccgg cagaggacgg tggagtcact gcaaaacagc agctataacc actttgctgc 1140
catttattac ctcctccttg agcggctcaa ggagtatcgg aatgcccagt gcgcccgccc 1200
cgggcctgcc aggcagccgc ggcctcggag ctcggacctc agtggtttgg aggtgcctca 1260
ggaaggtctt tccaccgacc ctttccgacc tgccttgctg tgcccgcagc cgcagacctt 1320
ggtgcagtcc gtcctccagg ccgagatgga ctgtgagctc cagagctcgc tgcagtggcc 1380
cttgttcttc ccggtggatg ccagctgcag cggagtgttc cggccccggc ccgtgtcccc 1440
aagcagcctg ctggacacag ccatcagtga ggaggccagg caggggccgg gcctagagga 1500
ggagcaggac acgcaggagt ccctgcccag cagcacgggc cggaggcaca ccctggccga 1560
ggtctccacc cgcctctccc cactcaccgc gccatgtata gtcgtctccc cctccaccac 1620
ggcaagtcct gcagagggaa ccagctctga cagttgtctg accttctctg cgagcaaaag 1680
ccccgcgggg ctcagtggca ccccggccac tcaggggctg ctgggcgcct gctccccggt 1740
caggctggcc tcgcccttcc tggggtcgca gtccgccacc ccagtgctgc aggctcaggg 1800
gggcttggga ggagctgttc tgctccctgt cagcttccag gagggacggc gggcgtcgga 1860
cacctcactg actcaagggc tgaaggcctt tcggcagcag ctgaggaaga ccacgcggac 1920
caaagggttt ctgggactga acaaaatcaa ggggctggct cgccaggtgt gccaggtccc 1980
tgccagccgg gccagcaggg gcggcctgag ccccttccac gcccctgcac agagcccagg 2040
cctgcacggc ggcgcagccg gcagccggga gggctggagc ctgctggagg aggtgctaga 2100
gcagcagagg ctgctccagt tacagcacca cccggccgct gcacccggct gctcccaggc 2160
cccccagccg gcccctgccc cgtttgtgat cgccccctgt gatggccctg ggctgccccc 2220
gctccccagc accctcctca cgtcggggct cccgctgctg ccgcccccac tcctgcagac 2280

```
cggcgcgtcc ccggtggcct cagcggcgca gctcctggac acacacctgc acattggcac   2340
cggccccacc gccctccccg ctgtgccccc accacgcctg gccaggctgg ccccaggttg   2400
tgagcccctg gggctgctgc agggggactg tgagatggag gacctgatgc cctgctccct   2460
aggcacgttt gtcctggtgc agtgagggca gccctgcatc ctggcacgga cactgactct   2520
tacagcaata acttcagagg aggtgaagac atctggcctc aaagccaaga actttctaga   2580
agcgaaataa gcaatacgtt aggtgttttg gctttttagt ttatttttgt tttatttttt   2640
tcttgcactg agtgacctca actttgagta gggactggaa actttaggaa gaaagataat   2700
tgaggggcgt gtctgggggc gggggcagga ggggagcggg gtggagggaa cacgtgcagt   2760
gccgtggtgt ggggatctcg gcccctctct ctgggttcgt cgtggttgag atgattacct   2820
cggacgtcta cggaaacgag cgggcgcatt gttgtccgct tgtgtgtgtg tgtgtgtgtg   2880
tgtgtgtgcg cgtgcattga ttactatcca tttctttagt caacgctctc cacttcctga   2940
tttctgcttt aaggaaaact gtgaactttc tgcttcatgt atcagtttta aagcagccca   3000
ggcaaagatc atctacagat tctaggaatt ctctcccctg aaatcaaaac ctggaagact   3060
tttttttctt attttagttg agaagtttca taaactgctc aaggattagt tttccaggac   3120
tctgcggagg aacggcagga agaacctcag agagggcaga ggtgacttca aagtgctggg   3180
gactccgtcc tgagggtcac ttggccctga gcccctgcgt gcccttgcgg aagcccagaa   3240
gcttcttcct gctgcacctc ccgtttccgc tgctgctgac gtttatgcat ttcatgatgg   3300
ggtccaacaa gaacacctga cttgggtgaa gttgtgcaat attggaggct gactgtaggg   3360
ctgggcagct gggagacagg ctcatggctc atggctcatg gctcagggcg gtgcctgcca   3420
tgggccggga caccccctccc caccccccac ctaggctttt tgggttttgt tcaaggaagg   3480
taaagtgaga ggtttaggtc agtgttttta agtttttgtt ttttttttaa agcaaatcct   3540
gtatatgtat ctacatggga gacaggtaga cactacttat ttgttacatt ttgtactaca   3600
cgtttgtgtt ccaggtttca gcttccctcg ctcctgttgt taagaagcgt ccctgtcagc   3660
acaggtgtgc attgaggaag gggccccagg gccttcgctc cctcagcact ggggtggagg   3720
cggcaggaag gggcggccct tacctggcag gtctgggcgc acctttagca ggtggactcc   3780
gtggggctcc accagccaga agcctttgga aggcaacgaa ggcaatgctg ctccctgagt   3840
ccagtccccg cccccaaacc cagcccaggt gccttcagct acttcggctt cttaaaccct   3900
gcagtgttaa acagaggcat tgagaaaggg gaaaggcggg tatttttaaa agccaaagat   3960
tgacccagtt acttgagggt agggaggcgg gcccagtgca ggaggctgca tccctggcct   4020
gctggtgccc accgggggct gtgcctgtgc cgggccgcag ggaagctggc tgccccccatt  4080
cctgctgctg ctgctgctgc tgctctgtgg ctgtttcaaa gactgggcga aaggctgtcc   4140

ggagggcaga ccaggtgcct tgccgcagag aaaacaccaa agtctcctgt tcgctcataa   4200
agaagttttt gggatgggag agaatccaga ccatcttggg gcagccaggc ccttgccttc   4260
atttttacag aggtagcaca attgattcca acacaaaact tcccctttttt aaaatgattt  4320
ctgttctaat gccatagatc aaaggcctca gaaaccattg tgtgtttcct ctttgaagca   4380
atgacaagca ctttactttc acggtggttt ttgtttttttc ttattgctgt ggaacctctt  4440
ttggaggacg ttaaaggcgt gttttacttg tttttttaag agtgtgtgat gtgtgttttg   4500
tagatttctt gacagtgctg taatacagac ggcaatgcaa tagcctattt aaagacacta   4560
cgtgatctga ttgagatgta catagttttt ttttttacca taactgaatt attttatctc   4620
ttatgttaac atgagaaatg tatgccaaat gattagttga tgtatgtttt ttaatttaat   4680
atttaaataa aatatttggg agtataaaaa aaaaaaaaaa aaaaaaaaaa aaaaaaaaaa   4740
aaaaaaaaaa aaaaaaaaaa aa                                              4762
```

<210> 28
<211> 3327
<212> DNA
<213> Homo sapiens

<400> 28

```
cacacctttc caaggacccc caaactctgc tccgtgcacg tcaaatgctc ctttcccttg     60
tgtccaaccc cctacccctc tccctaacac ccctcttctc aacaagactc agcctctccc    120
cgaggtgggt gagcatcctt gaggtttccc acccttaact gctgtgtccc cggatggagc    180
cagagaaatg tggtgggggg gccggggcag agtttcaaca ttgcccccca gaaggaggag    240
ccagagatgg ggtctgtcca ggaaaacagg atgccggagc ccaggagtcg tcagcctagc    300
agttgcctgg cctccagatg cctcccaggg gagcagatcc tagcatgggc cccaggggtg    360
aggaagggcc tggaaccaga attgtctgga accctgatct gtaccaactt tagggtcacc    420
ttccagccct gtggatggca gtggaatcag gacactccct tgaacagtga atacgatttt    480
gccctggtca acattggacg attagaggct gtgagcggct tgtcccgagt ccagctcctc    540
cgtccagggt ccctgcataa atttatccct gaggagattc tgattcatgg ccgagacttc    600
cggctgctca gagttggttt tgaggctgga ggcctagagc ctcaggcttt tcaggtgacc    660
atggccattg tccaagccag agctcagagc aatcaagccc aacagtattc ggggataacc    720
ctgagcaagg ctggccaggg ttctggctcc agaaaaccac caattcctct catggagaca    780
gcggaagact gggagactga gcggaagaag caggcagcca gaggctggag ggtcagcacg    840
gtcaacgaga ggttcgacgt agccaccagc ctcccccgtt acttctgggt ccctaaccga    900
attctggaca gtgaggtcag gagagcattt ggccactttc atcagggccg tggaccggtc    960
agtgtgatgg ttagggtaat ggctgtggat tagagggtca tgtgggccag ggacatcgtg   1020

gagggaggaa cctctgtgag gtcagtgtgg gggcaagggt agcgtggagc taggcatttc   1080
tcccacaatg accctcttct gccccatgtg aagcgcttgt cctggcatca ccctgggggc   1140
agtgatcttc tccgctgtgg aggcttctat acagccagtg accctaacaa ggaggatatc   1200
agagcagtgg agttgatgca ccaggctggg cattcagatg ttgtcctggt agacactatg   1260
gatgagctgc ccagccttgc agatgtccaa cttgcccacc tgaggctgag ggccctctgc   1320
ctgcctgatt catctgtagc tgaggataaa tgctttcagc cctggaagga acacgatggc   1380
tggactatgt cagggcttgt cttcgaaagg ccagtgacat ttcagtatta gtgacatcca   1440
gggttcgttc tgtaatactt caaggctccg gtgtttctcc tcttccttga ttgtgtctgg   1500
cagctcctcc agcagtttcc agctgatttt gaattctctg agtttttcct tcttgctctt   1560
catgacagtg tcagggttcc tgacaccctt accttcctga gaaatacccc ctgggagcgc   1620
ggaaagcaga gcggacaggt cagtgacttc tatttttgac tcgtgttttt ttttccattg   1680
agatgtactc tctgaagttt ggtcttgatt tgttttatga gaagtgaggt ctgtgagtgg   1740
ggagggggag atttattctc attttcagga cgagactttt gccctacatc tttcctagaa   1800
taagaggtga gaatctcatg atttgtctct agatgtggga ggattgtgtg taaccatcct   1860
ttttcttgct tcctctgtcc agttaaactc ctatacacaa gtctacaccc caggatactc   1920
cagcctccag ctgggaactc ttttaacctg cagctgtctg tctgggactg ggatttacgt   1980
tatagcaatg cacagatact acaattccag aatcctggct atgacccaga acactgtcca   2040
gattcctggc tccctagacc acagccaagc ttcatggttc ctggaccccc cagttttgtg   2100
tggctcttct ctagaggagc attgaccccc ctgaatcagc tctgtccttg gcgggacagt   2160
ccttccctgc tggcagtctc ttctcgttgg ctccctcgac ctgctatctc ctctgaaagc   2220
tggctgacca ggaatggggt ctcccctcac attggggagc ttgcccttta cctccagggc   2280
tgctgctgcc tgggtatctg ggaccccaga tcaggctctg gagacgctgc tacctgaggg   2340
gaaggcctga ggtccaggta agaagggaaa atagactggg agtgggacaa gggacttgac   2400
tctgctgaac cagatgaaca ggagctggaa aggcaaggag ctgaagcctc tgggagtctg   2460
ggaagtgaag ttctactcct cttggcatca aacaaggttt gggagtgtag gaggtgcggg   2520
aaagtgcttg tggcttagat taagtggaat ttagggcata gctgaaaggg gaaacagaat   2580
taaagacacc agaagtagca gagaagcagg gggccagagc tacaacagta ttcttctctg   2640
ttcctctttg cctcctcccc agatgggcct ctcatctccc acaatctctg gcctccagga   2700
tgagctatcc catcttcagg agttattacg gaaaggacac caagaatatc tcctgaggat   2760
cactccaaga aaagagatcc acataccatt ctcaatccca ctgaaattgc tggcattctc   2820
aaaggcaggg cagaggggga tctggggtag agggagggtt ctgtctaatc tttttttttt   2880
cttttgtatc tgcacttgca gcctcagctt tcatacttca gcccttaagt tcactaagaa   2940
```

ggtctgagtt tctgctgcag atagtggtgt taactgctcc aactcttgtc ttgcttagtt 3000
tctacaaata tttttgcttc ttgtcatttg aaggattaag aaacaaaaac aatccagaaa 3060
ttgatcggtt tttttaggcc aatcccatcc cttctggata accagatgtt aaatcatgag 3120
atcagagatg ctgttcatca gtcccaacaa gatggcctag aaatcgcatt ctcacctcgc 3180
cttgctgctg ctttaattcc aagttctatt tcttccctta tagttttcta tgggaatgag 3240
gcggatacag gaaacaccct atctcctctg tatttttgta gtggaatttc tatttaaggg 3300
gctcattaaa gcatagtatt tatacac 3327

<210> 29
<211> 3061
<212> DNA
<213> Homo sapiens

<400> 29

gagcaattga ttaatagctc ggcgagggga ctcactgact gttataataa cactacacca 60
gcaactcctg gcttcccagc agccggaaca cagacaggag agagtcagtg gcaaatagac 120
atttttctta tttcttaaaa aacagcaact tgtttgctac ttttatttct gttgattttt 180
ttttcttggt gtgtgtggtg gttgttttta agtgtggagg gcaaaaggag ataccatccc 240
aggctcagtc caacccctct ccaaaacggc ttttctgaca ctccaggtag cgagggagtt 300
gggtctccag gttgtgcgag gagcaaatga tgaccgccaa ggccgtagac aaaatcccag 360
taactctcag tggttttgtg caccagctgt ctgacaacat ctacccggtg gaggacctcg 420
ccgccacgtc ggtgaccatc tttcccaatg ccgaactggg aggccccttt gaccagatga 480
acggagtggc cggagatggc atgatcaaca ttgacatgac tggagagaag aggtcgttgg 540
atctcccata tcccagcagc tttgctcccg tctctgcacc tagaaaccag accttcactt 600
acatgggcaa gttctccatt gaccctcagt accctggtgc cagctgctac ccagaaggca 660
taatcaatat tgtgagtgca ggcatcttgc aaggggtcac ttccccagct tcaaccacag 720
cctcatccag cgtcacctct gcctccccca acccactggc cacaggaccc ctgggtgtgt 780
gcaccatgtc ccagacccag cctgacctgg accacctgta ctctccgcca ccgcctcctc 840
ctccttattc tggctgtgca ggagacctct accaggaccc ttctgcgttc ctgtcagcag 900
ccaccacctc cacctcttcc tctctggcct acccaccacc tccttcctat ccatccccca 960
agccagccac ggacccaggt ctcttcccaa tgatcccaga ctatcctgga ttctttccat 1020
ctcagtgcca gagagaccta catggtacag ctggcccaga ccgtaagccc tttccctgcc 1080
cactggacac cctgcgggtg ccccctccac tcactccact ctctacaatc cgtaagccct 1140
ttccctgccc actggacacc ctgcgggtgc cccctccact cactccactc tctacaatcc 1200
gtaactttac cctgggggc cccagtgctg ggtgaccgg accaggggcc agtggaggca 1260

```
gcgagggacc ccggctgcct ggtagcagct cagcagcagc agcagccgcc gccgccgccg   1320
cctataaccc acaccacctg ccactgcggc ccattctgag gcctcgcaag taccccaaca   1380
gacccagcaa gacgccggtg cacgagaggc cctacccgtg cccagcagaa ggctgcgacc   1440
ggcggttctc ccgctctgac gagctgacac ggcacatccg aatccacact gggcataagc   1500
ccttccagtg tcggatctgc atgcgcaact tcagccgcag tgaccacctc accacccata   1560
tccgcaccca caccggtgag aagcccttcg cctgtgacta ctgtggccga aagtttgccc   1620
ggagtgatga gaggaagcgc cacaccaaga tccacctgag acagaaagag cggaaaagca   1680
gtgccccctc tgcatcggtg ccagccccct ctacagcctc ctgctctggg ggcgtgcagc   1740
ctgggggtac cctgtgcagc agtaacagca gcagtcttgg cggagggccg ctcgcccctt   1800
gctcctctcg gacccggaca ccttgagatg agactcaggc tgatacacca gctcccaaag   1860
gtcccggagg ccctttgtcc actggagctg cacaacaaac actaccaccc tttcctgtcc   1920
ctctctccct ttgttgggca aagggctttg gtggagctag cactgccccc tttccaccta   1980
gaagcaggtt cttcctaaaa cttagcccat tctagtctct cttaggtgag ttgactatca   2040
acccaaggca aaggggaggc tcagaaggag gtggtgtggg gacccctggc caagagggct   2100
gaggtctgac cctgctttaa agggttgttt gactaggttt tgctacccca cttcccctta   2160
ttttgaccca tcacaggttt ttgaccctgg atgtcagagt tgatctaaga cgttttctac   2220
aataggttgg gagatgctga tcccttcaag tggggacagc aaaaagacaa gcaaaactga   2280
tgtgcacttt atggcttggg actgatttgg gggacattgt acagtgagtg aagtatagcc   2340
tttatgccac actctgtggc cctaaaatgg tgaatcagag catatctagt tgtctcaacc   2400
cttgaagcaa tatgtattat aaactcagag aacagaagtg caatgtgatg ggaggaacat   2460
agcaatatct gctccttttc gagttgtttg agaaatgtag gctatttttt cagtgtatat   2520
ccactcagat tttgtgtatt tttgatgtac actgttctct aaattctgaa tctttgggaa   2580
aaaatgtaaa gcatttatga tctcagaggt taacttattt aagggggatg tacatatatt   2640
ctctgaaact aggatgcatg caattgtgtt ggaagtgtcc ttggtgcctt gtgtgatgta   2700
gacaatgtta caaggtctgc atgtaaatgg gttgccttat tatggagaaa aaaatcactc   2760
cctgagttta gtatggctgt atatttctgc ctattaatat ttggaatttt ttttagaaag   2820
tatatttttg tatgctttgt tttgtgactt aaaagtgtta cctttgtagt caaatttcag   2880
ataagaatgt acataatgtt accggagctg atttgtttgg tcattagctc ttaatagttg   2940
tgaaaaaata aatctattct aacgcaaaac cactaactga agttcagata atggatggtt   3000
tgtgactata gtgtaaataa atacttttca acaataaaaa aaaaaaaaaa aaaaaaaaaa   3060
a                                                                   3061
```

<210> 30
<211> 1677
<212> DNA
<213> Homo sapiens

<400> 30

```
ggccaagcaa gcttctatct gcacctgctc tcaatcctgc tctcaccatg agcctccgcc      60
tgcagagctc ctctgccagc tatggaggtg gtttcggggg tggctcttgc cagctgggag     120
gaggccgtgg tgtctctacc tgttcaactc ggtttgtgtc tgggggatca gctgggggct     180
atggaggcgg cgtgagctgt ggttttggtg gaggggctgg tagtggcttt ggaggtggct     240
atggaggtgg ccttggaggt ggctatggag gtggccttgg aggtggcttt ggtgggggtt     300
ttgctggtgg ctttgttgac tttggtgctt gtgatggcgg cctcctcact ggcaatgaga     360
agatcaccat gcagaacctc aacgaccgcc tggcttccta cctggagaag gtgcgcgccc     420
tggaggaggc caacgctgac ctggaggtga agatccgtga ctggcacctg aagcagagcc     480
cagctagccc tgagcgggac tacagcccct actacaagac cattgaagag ctccgggaca     540
agatcctgac cgccaccatt gaaaacaacc gggtcatcct ggagattgac aatgccaggc     600
tggctgtgga cgacttcagg ctcaagtatg agaatgagct ggccctgcgc cagagcgtgg     660
aggccgacat caacggcctg cgccgggtgc tggatgagct cactctgtct aagactgacc     720
tggagatgca gatcgagagc ctgaatgaag agctagccta catgaagaag aaccatgaag     780
aggagatgaa ggaatttagc aaccaggtgg tcggccaggt caacgtggag atggatgcca     840
ccccaggcat tgacctgacc cgcgtgctgg cagagatgag ggagcagtac gaggccatgg     900
cagagaggaa ccgccgggat gctgaggaat ggttccacgc caagagtgca gagctgaaca     960
aggaggtgtc taccaacact gccatgattc agaccagcaa gacagagatc acggagctca    1020
ggcgcacgct ccaaggcctg gagattgagc tgcagtccca gctgagcatg aaagcggggc    1080
tggagaacac ggtggcagag acggagtgcc gctatgccct gcagctgcag cagatccagg    1140
gactcatcag cagcatcgag gcccagctga gcgagctccg cagtgagatg gagtgccaga    1200
accaagagta caagatgctg ctggacatca agacacgtct ggagcaggag atcgccacct    1260
accgcagcct gctcgagggc caggacgcca agaagcgtca gcccccgtag cacctctgtt    1320
accacgactt ctagtgcctc tgttaccacc acctctaatg cctctggtcg ccgcacttct    1380
gatgtccgta ggccttaaat ctgcctggcg tcccctccct ctgtcttcag cacccagagg    1440
aggagagagc cggcagttcc ctgcaggaga gaggaggggc tgctggaccc aaggctcagt    1500
ccctctgctc tcaggacccc ctgtcctgac tctctcctga tggtgggccc tctgtgctct    1560
tctcttccgg tcggatctct ctcctctctg acctggatac gctttggttt ctcaacttct    1620
ctaccccaaa gaaaagatta ttcaataaag tttcctgcct ttctgcaaac ataaaaa       1677
```

<210> 31
<211> 8191
<212> DNA
<213> Homo sapiens

<400> 31

```
tttgcttgca acactggcac ctctgccctg caccccggga gtgagcagtg agtgaggctc      60
gggtctgggc gctggctccg aatcttcggg ctgggagaga ctccaccatc tgggggcggc     120
ctgggggagc agccttagtg tcttcctgct gatgcaatcc gctaggtcgc gagtctccgc     180
cgcgagaggg ccggtctgca atccagcccg ccacgtgtac tcgccgccgc ctcgggcact     240
gccccaggtc ttgctgcagc cgggaccgcg ctctgcagcc gcagacccgg tccacacggc     300
caggggctac gacccttggg atctgccctc cgctcagctc gagcttccct cgtggccgac     360
ggaacaatga aggattgcag taacggatgc tccgcagagt gtaccggaga aggaggatca     420
aaagaggtgg tggggacttt taaggctaaa gacctaatag tcacaccagc taccatttta     480
aaggaaaaac cagaccccaa taatctggtt tttggaactg tgttcacgga tcatatgctg     540
acggtggagt ggtcctcaga gtttggatgg gagaaacctc atatcaagcc tcttcagaac     600
ctgtcattgc accctggctc atcagctttg cactatgcag tggaattatt tgaaggattg     660
aaggcatttc gaggagtaga taataaaatt cgactgtttc agccaaacct caacatggat     720
agaatgtatc gctctgctgt gagggcaact ctgccggtat ttgacaaaga agagctctta     780
gagtgtattc aacagcttgt gaaattggat caagaatggg tcccatattc aacatctgct     840
agtctgtata ttcgtcctac attcattgga actgagcctt ctcttggagt caagaagcct     900
accaaagccc tgctctttgt actcttgagc ccagtgggac cttatttttc aagtggaacc     960
tttaatccag tgtccctgtg ggccaatccc aagtatgtaa gagcctggaa aggtggaact    1020
ggggactgca agatgggagg gaattacggc tcatctcttt ttgcccaatg tgaagcagta    1080
gataatgggt gtcagcaggt cctgtggctc tatggagagg accatcagat cactgaagtg    1140
ggaactatga atctttttct ttactggata aatgaagatg gagaagaaga actggcaact    1200
cctccactag atggcatcat tcttccagga gtgacaaggc ggtgcattct ggacctggca    1260
catcagtggg gtgaatttaa ggtgtcagag agatacctca ccatggatga cttgacaaca    1320
gccctggagg ggaacagagt gagagagatg tttggctctg gtacagcctg tgttgtttgc    1380
ccagtttctg atatactgta caaaggcgag acaatacaca ttccaactat ggagaatggt    1440
cctaagctgg caagccgcat cttgagcaaa ttaactgata tccagtatgg aagagaagag    1500
agcgactgga caattgtgct atcctgaatg gaaaatagag gatacaatgg aaaatagagg    1560
ataccaactg tatgctactg ggacagactg ttgcatttga attgtgatag atttctttgg    1620
ctacctgtgc ataatgtagt ttgtagtatc aatgtgttac aagagtgatt gtttcttcat    1680
```

```
gccagagaaa atgaattgca atcatcaaat ggtgtttcat aacttggtag tagtaactta   1740
ccttacctta cctagaaaaa cattaatgta agccatataa catgggattt tcctcaatga   1800
ttttagtgcc tccttttgta cttcactcag atactaaata gtagtttatt ctttaatata   1860
agttacattc tgctcctcaa acaaatgcaa ttttttgtgt gtgtttgaaa gctaatttga   1920
gaaaatttca taggttacat ttcctgcagc ctatctttat ccacagaaag tgttttcttt   1980
tttttaaatc aagactttta aaactggatt tcctcccatc actgtttttt gaaggtcctc   2040
caagtccgtg ttaaggtaaa tatctgtttt cttcctgatg tcacagcctg agcatactct   2100
gtgcattagg aagacctgag tgcatttccc accattgtcc tttccacatt atgttgtagc   2160
tggctggctg tcaggcgact acaagactga gggtcttgtg ccttatagat ctttgtatcc   2220
cccatggctg acatatagta ggtactcagt aaatggtttt ataatgaatc agtgaacatt   2280
ttgcttctat agaagtgtac cttctttgtt tctatattat gaaacctctt tattagaatt   2340
tgtgattgat tctgacagtg tatagattta ccttatattg tctttatttt ccatgagcta   2400
ctaagtcatt agagatactc tgaagcatag ttagtttagg aaatcacttc atattgattg   2460
tattagaatt atcttggaat tgaagatata tccctagagc aggggacccc aacccccagg   2520
ccatgggcca cacagcagga agaggtgagt ggtgggccat tgaggagctt catctgtatt   2580
tatggctact tcccatcact cgaattacca cctgaactcc acctcttgtc agctcagtgg   2640
cagcattaga ttctcatagg agcacaaatc ctattgtgaa ctctgcatgc aagggatcta   2700
ggctatgcgc tccttatgag aatctaatgc ttgatgacct gaggtgtaac agtttcatcc   2760
tgaaaccacc cttcaccctg cagtctgtgg aaaaattgtc ttccacaaaa ctggtccctg   2820
gtgccaaaaa tgttggggac cactgctcta gagagaggtc atgatatcat accaaccaaa   2880
tggaaatgac aaatgtttta tgtcaagtgt taattgcaga aataaatctt tttttttttt   2940
ttttggtaga aaacaaagag gcatactctg atttttatac tctgtttttg caggtgctct   3000
tttctttgaa tggagatttg atgagcaagt ggttaggatg cagggagagc tactatgggt   3060
gatattttcc ttgtttagga gctgtgagtt aaaattgtat cctttgtggt ttatctaagg   3120
aaagtcaaat cttgacagaa aacatttttc cttggaaggt caactctcag acattgtatt   3180
ttggtttccc tcagtcctca taacttcctt cttgctgaac atattttatt ctcttttcag   3240
agaaggaaaa taaaaaggat tctaaaagtt tgatgcattg gaaaaatttc cttgaggcat   3300
ttagcaacac atagaaaatg ggctttgatt cttttccaaa acttttagcc atagggtctt   3360
ttatagacag ggatagtaaa atgaaaattg agaaatataa gatgaaaagg aatgataaaa   3420
atatctttta gggggctttt aattggtgat ctgaaatctt gggagaagct gttcttttca   3480
ggcctgaggt gctcttgact gtcgcctgcg cactgtgtac cccgagcaac attctaaggg   3540
tgtgctttcg ccttggctaa ctcctttgac ctcattcttc atatagtagt ctaggaaaaa   3600
```

gttgcaggta atttaaactg tctagtggta catagtaact aaatttctat tcctatgaga 3660
aatgagaatt atttatttgc catcaacaca ttttatactt tgcatctcca aatttattgt 3720
ggcgagactt gtccattgtg aaagttagag aacattatgt ttgtatcatt tctttcataa 3780
aacctcaaga gcatttttaa gccctttca tcagacccag tgaaaactaa ggatagatgt 3840
ttaaaaactg gaggtctcct gataaggaga acacaatcca ccattgtcat ttaagtaata 3900
agacaggaaa ttgaccttga cgctttcttg ttaaatagat ttaacaggaa catctgcaca 3960
tcttttttcc ttgtgcacta tttgtttaat tgcagtggat taatacagca agagtgccac 4020
attataacta ggcaattatc cattcttcaa gacttagtta ttgtcacact aattgatcgt 4080
ttaaggcata agatggtcta gcattaggaa catgtgaagc taatctgctc aaaaagatca 4140
acaaattaat attgttgctg atatttgcat aattggctgc aattatttaa tgtttaattg 4200
ggttgatcaa atgagattca gcaattcaca agtgcattaa tataaacaga actggtggca 4260
cttaaaatga taatgattaa cttatattgc atgttctctt cctttcactt ttttcagttt 4320
ctacatttca gaccgagctt gtcagctttt ttgaaaacac atcagtagaa accaagattt 4380
taaaatgaag tgtcaagaca aaggcaaaac ctgagcagtt cctaaaaaga tttgctgtta 4440
gaaattttct ttgtggcagt catttattaa ggattcaact cgtgatacac caaaagaaga 4500
gttgacttca gagatgtgtt ccatgctctc tagcacagga atgaataaat ttataacacc 4560
tgctttagcc tttgttttca aaagcacaaa ggaaaagtga aagggaaaga gaaacaagtg 4620
actgagaagt cttgttaagg aatcaggttt tttctacctg gtaaacattc tctattcttt 4680
tctcaaaaga ttgctgtaag aaaaaatgta agacaaaaaa aaaaaaaaaa aacaaacaga 4740
ggcagaggca ggcagtagca agaaagcaga gcgtaacatc agctagatgg taacatgcaa 4800
tgtcagctct cttgaagaca tgggaaacct aagttacacc ttgggttaaa attcttcacc 4860
atattagttt tgttgcttca taaaatttac ctaagcaagt ggtcttgctt gcctcaaatc 4920
caagcagtct tgaacacttg gaggcaatta atgagtatat cttagtcaaa agaattgttg 4980
gagcttttta ttaaagctac agtttcagtt ctgcttttgg ggaattgtgc tatgaaagca 5040
gctgccaaaa taagctcatt tattttcttc aatcccactc agtgctcagt cactatattc 5100
tgtttccttt ttttttttca agttgcatat ttggtttccc cttatgattg ggaaagatga 5160
attttcagca gaaaacattg tttgttcact ttcaaagagt gatagtttct aaaacattta 5220
gagcaataaa tattcatcag aggtaccaag taagccggca gaagagttaa gggttagaga 5280
aatcccttat ttcatgtctt gactctaaaa ttatcaaagt acttttcctt gtaatgtgga 5340
tttcttctta tgcggatatg caaaaacttc agttatacgt agtaatgcta gcaggtaatt 5400
ttagtagaca ttttataaca actgtcactt tgtttcgcca catgtagagt ttgttcagct 5460

attttccaga tatctcccca caaaaggagg caaagggtac cagcttttca atgagcatta 5520
cctattactt ggcaaagatg atgaagactc tattaatagt tcatttgata aatgttgaca 5580
taaccaacaa tagagattag gaagttagtt ttaagaaatc aatggcatat agacattacc 5640
ctcatggagt ttgtattcta ctacttgaac tgattgtagc tataaaagca tagttagata 5700
gctgaatagt tagatcataa gcaaagaagg ccagaacaca tctcttatca agaaatcaat 5760
gaatagttta tctcattttt aaagcaactt tatccttctt taattccttc ctttcttcta 5820
gtgcaaaact acttaataag gttggtgttt aggttagtgt tcacaccatt cctcatctgg 5880
tgtgaattac cttctctttc tttactattt actaccaacc tagtacatgt gttgactgaa 5940
ttcttttcaa acaatgttga gttatcatgg tgcacctaat aaattaacac cacagattac 6000
agcatccttg ctgattttct cagcaaagcc agattagatg gaaataaaca aagaaaatga 6060
tcctagagtg aatttttcta gaaaatatct attatgaacc atgctgttta aagtattagc 6120
ttgaaggtga tggatccagc tattcagaaa ataactttca tataaccatg attttgcaca 6180
gtatgaggtc ttaaatgtgt ggaaagagat aaatttttta tcattaccac aaaccccttt 6240
taaagattca aaggtggaag aaagtgattt atttttctc ttcagcatac atatataaaa 6300
gacttgtcag atgtttaatt tggggaggtt gataatgaaa catatcaaca gagtatagta 6360
gttatagtag tgtttgtggg taaataattt cctggggtca gacatatata aacatatttg 6420
cttcaaaatg ataaaggcat gaaatcagtc ttaaaaattg aaatgggggt gatgggggag 6480
aaaaagaaga acaaatttga agtgcccttt caaatctgct ggatacaagt attgaagttt 6540
taagtcatct tattctgtct gaaagtgtat ttttcattct acaatagacc caatcaacaa 6600
gacgtataac ttgagttgca tgatgttcag tttatgtaat ctactgttgg gatggtaaga 6660
attgatgtag gctgtggtgt aagaatgaat taaaatatag tttcactggc ttttctctac 6720
atatccacta tcacaatggc taggtttcct gttgctcact attggattct ggagaaaaat 6780
ttaatgaaag atgatatcag aggaagaata agtggaggta gagaagaaag gaatgataga 6840
ggaggggaaa aaaacaaaac atatttttgt gttatccaaa ggagcttttt ccttattctg 6900
tcaagcattg agatcttctt cagctttcaa tgtagttgct aaatacaaat aatgctacta 6960
ggtagtgact aaatatagca aacacttcat cagatattag aattaggtca cactattgag 7020
gttataatct gaaggttgtg ttacatagaa accactttag attattatca acttggacta 7080
ggctttattt tataatagca tagtaagtaa tatctattgt gtcatttctt caaccatttt 7140
attctaagat ccatgaagct tcttgaggcc aaataaaata ataagtttag acaagaagta 7200
gattgtgact tttttccctt agagatacta tttactatct cctatcctga taggtggaag 7260
gtttactgaa ttggaaattg gttgactatt agtttttaac taaaatgtgc aataacacat 7320
tgcagtttcc tcaaactagt ttcctatgat cattaaactc attctcaggg ttaagaaagg 7380

aatgtaaatt tctgcctcaa tttgtacttc atcaataagt ttttgaagag tgcagatttt 7440
tagtcaggtc ttaaaaataa actcacaaat ctggatgcat ttctaaattc tgcaaatgtt 7500
tcctggggtg acttaacaag gaataatccc acaatatacc tagctaccta atacatggag 7560
ctggggctca acccactgtt tttaaggatt tgcgcttact tgtggctgag gaaaaataag 7620
tagttcgagg aagtagtttt taaatgtgag cttatagata gaaacagaat atcaacttaa 7680
ttatgaaatt gttagaacct gttctcttgt atctgaatct gattgcaatt actattgtac 7740
tgatagactc cagccattgc aagtctcaga tatcttagct gtgtagtgat tcttgaaatt 7800
ctttttaaga aaaattgagt agaaagaaat aaaccctttg taaatgaggc ttggcttttg 7860
tgaaagatca tccgcaggct atgttaaaag gattttagct cactaaaagt gtaataatgg 7920
aaatgtggaa aatatcgtag gtaaaggaaa ctacctcatg ctctgaaggt tttgtagaag 7980
cacaattaaa catctaaaat ggctttgtta caccagagcc atctggtgtg aagaactcta 8040
tatttgtatg ttgagagggc atggaataat tgtattttgc tggcaataga cacattcttt 8100
attatttgca gattcctcat caaatctgta attatgcaca gtttctgtta tcaataaaac 8160
aaaagaatcc tgtttgtgtg gtttcatgaa a 8191

<210> 32
<211> 2191
<212> DNA
<213> Homo sapiens

<400> 32

```
cacgggcgga gccggggcca tggagccgcc gctgccgggc taggcaggtc gtgccccgcc      60
gggccggcgg cgatgtcggg ctaccagcgc cacccgggcg ccaccccgct gtcccgagcc     120
cggagcctcg ccattcccga cgctccagcg ttctatgagc gccggtcttg tctcccccag     180
ctaaattgtg agcgccccca tggcagggac ctggactccc ccttcttcgg cattcggccg     240
gcctttatgt gctatgtgcc cagcccggtg ctggcttccg tgggagacac agatgacaga     300
tttgaagatc tggaagaggc aaatccattc tcttttagag agtttctgaa gaccaagaac     360
ctcggcctct cgaaagagga tccggccagc agaatttatg caaaggaagc ctcgaggcat     420
tccctgggac ttgaccacaa ctccccaccc tcccaaaccg gcgggtatgg cctggagtat     480
cagcagccat tttcgagga tccgacaggg gctggtgacc tcctggatgg ggaggaggat     540
gaggacaccg gatggagtgg ggcctacctg ccgtccgcca tcgagcagac tcaccccgag     600
agggtccctg ccggcacgtc gccctgcagc acataccttt ccttttctc caccccgtcg     660
gagctggcag ggcctgagtc tctgccctcg tgggcgttga gtgacactga ttctcgcgtg     720
tctccggcct ctccggcagg gagtcctagc gcagactttg cggttcatgg agagtctctg     780
ggagacaggc acctgcggac gctgcagata agttacgacg cactgaaaga tgaaaattct     840

aagctgagaa gaaagctgaa tgaggttcag agcttctctg aagctcaaac agaaatggtg     900
aggacgcttg agcggaagtt agaagcaaaa atgatcaagg aggaaagcga ctaccacgac     960
ctggagtcgg tggttcagca ggtggagcag aacctggagc tgatgaccaa acgggctgta    1020
aaggcagaaa accacgtcgt gaaactaaaa caggaaatca gtttgctcca ggcgcaggtc    1080
tccaacttcc agcgagagaa tgaagccctg cggtgcggcc agggcgccag cctgaccgtg    1140
gtgaagcaga acgccgacgt ggccctgcag aacctccggg tggtcatgaa cagtgcacag    1200
gcttccatca agcaactggt ttccggagct gagacactga atcttgttgc cgaaatcctt    1260
aaatctatag acagaatttc tgaaattaaa gacgaggagg aagactcttg aggacccctg    1320
ggtgttctca gcatgaagct ccgtgtatac cctgaggtca ccaccgctcg atctaaatgt    1380
gcagttgtgt ccttaaatat gcagtcttca cccagagtaa agtgttgatc gcaagagtcc    1440
agtgtcgtgc cctcagccag ttcttggcca ccacaatggg agcagccctg gccgagttgt    1500
ctctgtggtt tctatgcagc ccttcttggc gaaattcctg cgatcttata gattctaatg    1560
agctcttgga agacattgtc ataaaagcca gtgattttaa gaaaaagagt ggttctggaa    1620
tcagtgtttt ccagtcccat cccagaacat cagttgtaag ataagtacaa ttggttgtcc    1680
ttgatttcat aagtagaaca aacactaaat gtgcctctga gatggccacc ccgggcaggg    1740
acctgtgcct tccaccgatg ctcagggctc cctctggctc ccgggtcact cttgtggccc    1800
cagtgggtgg tccctgcagt catggcctga gtgcgcaggg gccaccgcgt ggctgccgct    1860
gtcctcctcc gggacccacg gggaccaagg tcacacgttc cgtgctgtga agctgtccag    1920
atgtgcctct ttggctgggg gttctggtgg acgtttcaag tggcattttg tacaatgcag    1980
gttagaattc aggaatttca agtatgtgcc cgggtctgtc aggtcccagt tgcctttctg    2040
acggcccccc tcagagggac ggcgatgagc actaaatgct tttttgacta ttttcctata    2100
gatttttttt aaaacttttt tttcctcctg ttccaattga tagctttctt atttaataaa    2160
ttctgtagtt caccaaaaaa aaaaaaaaaa a                                   2191
```

<210> 33
<211> 463
<212> DNA
<213> Homo sapiens

<400> 33

atatattccc agctagttga aaatgatgat tcccacaaga agcataactc agcttgtttc 60
tgcttactga gtattttcta ctatggtata tattgataac atttcttcca ttatgtatgt 120
tgtataccag agttacagtt actgtgggaa tcataatttg aaattttgac tcctgtgttt 180
ctggaatctt tacaacaaat gttgcattaa catataactt ttttcagttg actttaccaa 240
aattaagccc atctttagta gatactgttt taacatgtga aagaaatacg ttataaacat 300

accacaagat atggctataa aacaatgaga tcagtatcca tttttgcttt aaagaattgg 360
ccttattgct tcagtgtcac atctcatact caagggcatt tactacaaag aaagagttct 420
ccaatattgc tgttctgttg ctgcctgccc tatttacaca tgt 463

<210> 34
<211> 393
<212> DNA
<213> Homo sapiens

<400> 34

tttacactta tagtagactt tatttagtga atccaaatga catgtgataa ttgtttggaa 60
aggcctattg attttatatc tgatcattca atccagagac attaaattca gttgattaat 120
ggagttcccc aactgtaaga cttctttacg agattatttt caagctttga aaagatcttc 180
tgagataaag gggatcagca aacagtaaga gtgtgttgct atacccaagc aaaagaaata 240
aatcttaatc tctcagcaaa tcattcaaaa tgtcagaaat gttagtgttt ctatatcttg 300
gtaaaatgga ttgattgaga agtatgaaaa gtataacagt ggcatgcaga atattgtttt 360
tatgaatatt cagaatttca gttgtttaca taa 393

<210> 35
<211> 10434
<212> DNA
<213> Homo sapiens

<400> 35

atggcgaacc ggcgagtggg gcgaggctgc tgggaagtga gcccgaccga gcggaggccg 60
cccgcggggc tgcggggccc cgcggccgag gaggaggcgt cttccccgcc ggtcctgtct 120
ctcagccact tctgcaggtc tcctttcctt tgcttcgggg acgttctcct gggagcctca 180
cggacgctgt ctctggccct agacaaccct aacgaggagg tggcagaagt gaagatctcc 240
cacttcccgg ccgcggacct gggcttcagt gtgtcgcagc gctgtttcgt gttgcagcct 300
aaagagaaaa ttgttatttc tgttaactgg acaccactca aagaaggccg agtaagagag 360
attatgacat ttcttgtaaa tgatgttctg aaacaccaag ctatattact aggaaatgca 420
gaagagcaga aaaagaaaaa gaggagtctt tgggatacca ttaaaaagaa gaaaatttca 480
gcctctacaa gtcacaacag aagggtttca aatattcaga atgttaataa aacatttagt 540
gtttcccaaa aagttgacag agttaggagc ccactacaag cttgtgaaaa cttggctatg 600
aatgaaggcg gtcccccaac agaaaacaat tctttaatac ttgaagaaaa taaaataccc 660
atatcaccta ttagccctgc tttcaatgaa tgccatggtg caacttgctt gccactctct 720
gtacgtcgat ctactaccta ctcatctctt catgcatcag aaaataggga actattaaat 780
gtacacagtg ccaacgtttc aaaagtttct tttaatgaga aagctgtaac tgaaacttcc 840

```
tttaattctg taaatgttaa tggccaaaga ggagagaata gtaaacttag tcttaccccc     900
aactgttctt caactttgaa cattacacaa agccaaatac attttctaag tccagattct     960
tttgtaaata atagtcatgg agctaataat gaactagaat tagtaacatg tctttcatca    1020
gatatgttta tgaaagataa ttcacagcct gtgcatttgg aatcaacaat tgcacatgaa    1080
atttatcaga aaattttaag tccagattct ttcataaaag ataattatgg actaaatcag    1140
gatctagaat cagagtcagt taatcctatt ttatccccta atcaattttt aaaagataac    1200
atggcatata tgtgtacatc tcagcaaaca tgtaaagtac cattatcaaa tgaaaattct    1260
caagtcccac agtctcctga agattggaga aaaagtgaag tttcgccacg tattcctgaa    1320
tgtcagggtt caaaatctcc caaagctatt tttgaagaac tagtagaaat gaagtcaaat    1380
tactacagtt ttataaaaca aaataatcct aaattttctg cagttcagga tatttctagt    1440
catagccaca ataaacaacc taagagacgt ccaatacttt ctgccactgt tactaaaagg    1500
aaggccacct gtaccagaga aaaccaaact gagattaata aaccaaaagc aaaaagatgt    1560
ctcaacagtg cagtgggtga acatgaaaaa gtaataaata atcaaaagga aaaagaagat    1620
tttcattctt atcttccaat tatagatcca atattaagta aatctaagag ttataaaaac    1680
gaggtaacac cctcttcgac aacagcttca gttgctcgga aaagaaagag cgatggaagc    1740
atggaagatg caaatgtgag agttgcaatt acagaacata cagaagtgcg agaaatcaaa    1800
agaatccatt tttctccctc agagcctaaa acatcagctg ttaagaaaac aaaaaatgtg    1860
acaacaccca tctcaaaacg tattagcaac agagagaaat taaacctgaa gaagaaaact    1920
gatttatcaa tattcagaac tccaatttct aaaacaaaca aaaggacaaa acccattatc    1980
gctgtggcac agtccagttt gaccttcata aaccattaa aaacagatat tcccagacac    2040
ccgatgccat ttgctgcaaa aaacatgttt tatgatgaac gctggaagga aaagcaggaa    2100
cagggcttca cttggtggtt aaattttata ttaacccctg atgacttcac tgtaaaaaca    2160
aatatttctg aagtaaatgc tgctactctt cttttgggaa tagagaatca acataaaata    2220
agtgttccta gagcacctac aaaagaggaa atgtctctca gagcttatac tgctcggtgt    2280
aggttaaaca gactacgtcg tgcagcatgc cgtttgttta cttctgaaaa aatggttaaa    2340
gctattaaaa agcttgaaat tgaaattgaa gctaggcggt taattgttcg aaaagataga    2400
cacctatgga aagatgtggg agaacgtcag aaagtcctga attggctgtt gtcctacaat    2460
cctttgtggc ttcgaattgg tctagagaca acttatggag aactcatatc tttggaagat    2520
aacagtgatg tcacagggtt ggctatgttt attctgaatc gcctactttg gaatcctgat    2580
atagcagctg agtatagaca ccccactgtt cctcacctgt atagagatgg tcatgaagaa    2640
gctttgtcca agtttacatt gaaaaagtta ttgttgttgg tctgttttct tgattatgct    2700
aaaatttcca gactcattga tcatgatcct tgtctcttct gtaaagatgc cgaattcaag    2760
```

```
gctagtaaag aaatcctttt ggctttttca cgagatttcc taagtggtga aggtgacctt   2820
tcccgtcacc ttggcttatt gggattacct gttaaccatg ttcagacacc atttgatgaa   2880
tttgattttg ccgttacaaa tcttgccgta gacttgcaat gtggagtgcg ccttgtgcga   2940
accatggaac ttctcacaca gaactgggac ctctcaaaga aactcaggat tccggcaata   3000
agtcgtcttc aaaagatgca caatgttgac attgttcttc aagttcttaa atcacgagga   3060
attgaattaa gtgatgagca tggaaataca attctatcta aggatattgt ggataggcac   3120
agagaaaaaa ctctcaggtt gctttggaaa atagcgtttg cttttcaggt ggatatttcc   3180
cttaacttag atcaattaaa ggaagaaatt gcctttctaa aacacacaaa gagtataaag   3240
aaaacaatat ctctactatc atgccattct gatgatctta ttaataagaa aaaaggcaaa   3300
agggatagtg gttcctttga acaatatagt gaaaacataa agttattgat ggattgggta   3360
aatgctgttt gtgccttcta taataaaaag gtggagaatt ttacagtgtc tttctcagac   3420
ggccgtgtgt tatgttacct gatccaccat taccatcctt gctatgtgcc atttgacgct   3480
atatgtcagc gtactactca aactgtggaa tgtacgcaaa ctggttcagt ggtattaaat   3540
tcatcatctg aatctgatga cagttctctg gatatgtcac ttaaagcatt tgatcatgaa   3600
aatacttcag agctatacaa agagctccta gaaaatgaaa agaaaaattt tcacttggtt   3660
aggtctgcag ttagagacct tggtggaata cctgctatga ttaatcattc agatatgtca   3720
aatacaattc cagatgaaaa ggtggttatt acctatttgt catttctttg tgcaaggctt   3780
ttggatcttc gtaaagaaat aagagctgct cgactcatac aaacaacatg gagaaaatat   3840
aaactaaaaa cagatctcaa acgccatcag gagagagaga aagctgcaag aattattcaa   3900
ttggctgtaa tcaattttct agcaaaacaa agattgagaa aaagagttaa tgcagcactc   3960
gtcattcaga aatattggcg aagagtctta gcacagagaa aattattaat gttaaaaaag   4020
gaaaagctgg aaaaagttca aaataaagca gcatcactta ttcagggata ttggagaaga   4080
tattccacta gacaaagatt tctgaaattg aaatattatt caatcatcct gcaatctagg   4140
ataagaatga taattgctgt tacatcttat aaacgatatc tttgggctac agttacaatt   4200
cagaggcatt ggcgtgctta tttaagaaga aaacaagatc aacaaagata tgaaatgcta   4260
aaatcatcaa ctcttataat ccaatctatg ttcagaaaat ggaagcaacg taaaatgcaa   4320
tcacaagtaa aagctacagt aatattgcaa agagctttta gagaatggca tttaagaaaa   4380
caagctaaag aagaaaattc tgctattatc atacaatcat ggtatagaat gcataaagaa   4440
ttacggaagt atatttatat tagatcttgt gttgttatca ttcagaaaag atttcggtgc   4500
tttcaagccc aaaagttata taaaagaaga aaagagtcca tactaaccat ccagaagtac   4560
tacaaagcat atctgaaagg aaagattgag cgcaccaact atttgcagaa acgagctgca   4620
```

gccattcaat tacaagctgc ttttaggaga ctgaaagctc ataatttatg tagacaaatt 4680
agagctgctt gtgttattca gtcatactgg agaatgagac aagacagagt tcgattttta 4740
aaccttaaga agactattat caaatttcag gcacatgtaa gaaaacatca acaacgacag 4800
aaatataaga agatgaagaa agcagctgtt ataattcaga ctcatttccg agcttatatt 4860
tttgccatga aagttctagc atcttaccag aaaacacgct ctgctgtcat tgtgctgcag 4920
tctgcatata gagggatgca agccaggaaa atgtatattc acatcctcac atctgttata 4980
aagattcaat catattatcg tgcttatgtt tctaaaaagg aatttttgag cctaaaaaat 5040
gctacaataa aattgcagtc aactgttaag atgaaacaaa cacgtaaaca atatttgcat 5100
ttaagagcag ctgcactatt tatccagcaa tgttaccgtt ccaaaaaaat agctgcacaa 5160
aagagagaag agtatatgca gatgcgggaa tcttgtatca aactgcaagc atttgttaga 5220
ggataccttg tccgaaagca gatgaggtta caaagaaaag ctgttatttc actacagtct 5280
tatttcagaa tgagaaaggc tcggcagtat tatctgaaaa tgtataaagc aattattgtc 5340
attcagaatt actatcatgc atacaaagca caggtcaatc agaggaagaa cttcttgcaa 5400
gtcaaaaaag cagctacttg cttgcaagca gcttacagag gttataaagt acgccagcta 5460
atcaaacaac aatctatagc tgctcttaaa attcagtctg cttttagagg ctataataaa 5520
agggtaaaat atcaatctgt gcttcaatct ataataaaga ttcagagatg gtacagggcg 5580
tacaagactc ttcatgatac aagaacacat tttttgaaga caaaggcagc tgtgatttcc 5640
ctccagtctg cttatcgtgg ctggaaggtt cggaaacaga ttagaaggga acatcaagct 5700
gccttgaaga ttcagtctgc ttttagaatg gccaaggccc agaaacagtt tagattgttt 5760
aaaacagcag cattagtcat ccagcaaaat ttcagagcat ggactgcagg aaggaagcaa 5820
tgtatggagt atattgaact ccgtcatgcg gtactggtgc ttcaatctat gtggaaggga 5880
aaaacactga gaagacagct tcaaaggcaa cataaatgtg ctatcatcat acagtcatac 5940
tatagaatgc atgtgcaaca aaagaagtgg aaaatcatga aaaaagctgc tcttctgatt 6000
caaaagtatt atagggctta cagtattgga agagaacaga atcatttata tttgaaaaca 6060
aaagcagctg tagtaacttt acagtcagct tatcgtggta tgaaagtgag aaaaagaata 6120
aaggattgca acaaagcagc agtcactata cagtctaaat acagagctta caaaaccaaa 6180
aagaaatatg caacctatag agcttcagct attataattc agagatggta tcgaggtatt 6240
aaaattacaa accatcagca taaggagtat cttaatttga agaagacagc aattaaaatc 6300
caatctgttt atagaggtat tagagttaga agacatattc aacacatgca cagggcagcc 6360
acttttatta aagccatgtt taaaatgcat cagtcaagaa taagttacca tacaatgaga 6420
aaagcagcta ttgttattca agtaagatgt agagcatatt atcaaggtaa aatgcagcgt 6480
gaaaagtacc tgacaatttt gaaagctgtt aaagtccttc aggcaagttt tagaggagta 6540

agagttagac ggactcttag aaagatgcag actgcagcaa cactcattca gtcaaactac 6600
agaagataca gacagcaaac atactttaat aagttaaaga aaataacaaa aacagtacag 6660
caaagatact gggcaatgaa agaaagaaac atacaatttc aaaggtataa caaactgagg 6720
cattctgtaa tatacattca ggctattttt aggggaaaga aagctagaag acatttaaaa 6780
atgatgcata tagccgcaac tctcattcag aggagattta gaactctaat gatgagaaga 6840
agattcctct ctctcaagaa aactgctatt ttgattcaga gaaaatatcg ggcacatctt 6900
tgtacaaagc atcacttaca gttccttcag gtacaaaatg cagttattaa aatccagtca 6960
tcatacagaa gatggatgat aaggaaaagg atgcgagaga tgcacagggc tgctactttc 7020
atccagtcta ctttcagaat gcacagatta catatgagat atcgagcttt gaaacaggcc 7080
tccgttgtga tccaacagca ataccaagca aatagagctg caaaactgca gaggcagcat 7140
tatctcagac aaagacactc tgctgtgatc cttcaggctg cattcagggg tatgaaaact 7200
agaagacatt tgaagagtat gcattcctct gcaaccctta ttcagagtag gtttagatca 7260
ttactggtga ggagaagatt catttccctc aaaaaagcta ctatttttgt tcagaggaaa 7320
tatcgagcca ccatttgtgc caaacataaa ttgtaccaat tcttgcactt aagaaaggca 7380
gccattacaa tacagtcatc ttacagaaga ctgatggtaa agaagaagtt acaagaaatg 7440
caaagggctg cagttctcat tcaggctact ttcaggatgc acagaacata tattacattt 7500
cagacttgga aacatgcttc aattctaatt cagcaacatt atcgaacata tagagctgca 7560
aaattgcaaa gagaaaatta tatcagacaa tggcattctg ctgtggttat tcaggctgca 7620
tataaaggaa tgaaagcaag acaactttta agggaaaaac acaaagcttc tatcgtaata 7680
caaagcacct acagaatgta taggcagtat tgtttctacc aaaagcttca gtgggctaca 7740
aaaatcatac aagaaaaata tagagcaaat aaaaagaaac agaaagtatt tcaacacaat 7800
gaacttaaga aagagacttg tgttcaggca ggttttcagg acatgaacat aaaaaaacag 7860
attcaggaac agcaccaggc tgccattatt attcagaagc attgtaaagc ctttaaaata 7920
aggaagcatt atctccacct tagagcaaca gtagtttcta ttcaaagaag atacagaaaa 7980
ctaactgcag tgcgtaccca agcagttatt tgtatacagt cttattacag aggctttaaa 8040
gtacgaaagg atattcaaaa tatgcaccgg gctgccacac taattcagtc attctatcga 8100
atgcacaggg ccaaagttga ttatgaaaca aagaaaactg caattgtggt tatacagaat 8160
tattataggt tgtatgttag agtaaaaaca gaaagaaaaa actttttagc agttcagaaa 8220
tctgtacgaa ctattcaggc tgcttttaga ggcatgaaag ttagacaaaa attgaaaaat 8280
gtatcagagg aaaagatggc agccattgtt aaccaatctg cactctgctg ttacagaagt 8340
aaaactcagt atgaagctgt tcaaagtgaa ggtgttatga ttcaagagtg gtataaagct 8400

```
tctggccttg cttgttcaca ggaagcagag tatcattctc aaagtagggc tgcagtaaca   8460
attcaaaaag ctttttgtag aatggtcaca agaaaactgg aaacacagaa atgtgctgcc   8520
ctacggattc agttcttcct tcagatggct gtgtatcgga gaagatttgt tcagcagaaa   8580
agagctgcta tcactttaca gcattatttt aggacgtggc aaaccagaaa acagttttta   8640
ctatatagaa aagcagcagt ggttttacaa aatcactaca gagcatttct gtctgcaaaa   8700
catcaaagac aagtctattt acagatcaga agcagtgtta tcattattca agctagaagt   8760
aaaggattta tacagaaacg gaagtttcag gaaattaaaa atagcaccat aaaaattcag   8820
gctatgtgga ggagatatag agccaagaaa tatttatgta aagtgaaagc tgcctgcaag   8880
attcaagcct ggtatagatg ttggagagca cacaaagaat atctagctat attaaaagct   8940
gttaaaatta ttcaaggttg cttctatacc aaactagaga gaacacggtt tttgaatgtg   9000
agagcatcag caattatcat tcagagaaaa tggagagcta tacttcctgc aaagatagct   9060
catgaacact tcttaatgat aaaaagacat cgagctgctt gtttgatcca agcacattat   9120
agaggatata aaggaaggca ggtctttctt cggcagaaat ctgctgcttt gatcatacaa   9180
aaatatatac gagccaggga ggctggaaag catgaaagga taaaatatat tgaatttaaa   9240
aaatctacag ttatcctaca agcactggtg cgtggttggc tagtacgaaa aagattttta   9300
gaacagagag ccaaaattcg acttcttcac ttcactgcag ctgcatatta tcacctgaat   9360
gctgttagaa ttcaaagagc ctataaactt tacctggctg tgaagaatgc taacaagcag   9420
gttaattcag tcatctgtat tcagagatgg tttcgagcaa gattacaaga aaagagattt   9480
attcagaaat atcatagcat caaaaagatt gagcatgaag gtcaagaatg tctgagccag   9540
cgaaataggg ctgcatcagt aatacagaaa gcagtgcgcc attttctcct ccgtaaaaag   9600
caggaaaaat tcactagtgg aatcattaaa attcaggcat tatggagagg ctattcttgg   9660
aggaagaaaa atgattgtac aaaaattaaa gctatacgac taagtcttca agttgttaat   9720
agggagattc gagaagaaaa caaactctac aaaagaactg cacttgcact tcattacctt   9780
ttgacatata agcacctttc tgccattctt gaggccttaa aacacctaga ggtagttact   9840
agattgtctc cactttgttg tgagaacatg gcccagagtg gagcaatttc taaaatattt   9900
gttttgatcc gaagttgtaa tcgcagtatt ccttgtatgg aagtcatcag atatgctgtg   9960
caagtcttgc ttaatgtatc taagtatgag aaaactactt cagcagttta tgatgtagaa  10020
aattgtatag atatactatt ggagcttttg cagatatacc gagaaaagcc tggtaataaa  10080
gttgcagaca aaggcggaag catttttaca aaaacttgtt gtttgttggc tattttactg  10140
aagacaacaa atagagcctc tgatgtacga agtaggtcca aagttgttga ccgtatttac  10200
agtctctaca aacttacagc tcataaacat aaaatgaata ctgaaagaat actttacaag  10260
caaaagaaga attcttctat aagcattcct tttatcccag aaacacctgt aaggaccaga  10320

atagtttcaa gacttaagcc agattgggtt ttgagaagag ataacatgga agaaatcaca  10380
aatcccctgc aagctattca aatggtgatg gatacgcttg gcattcctta ttag        10434
```

<210> 36
<211> 3581
<212> DNA
<213> Homo sapiens

<400> 36

```
atacgtggct gccgtctgtc cccgctgagg aggtgcagca gccggagatg gcggcggtgc     60
tgaacgcaga gcgactcgag gtgtccgtcg acggcctcac gctcagcccg gacccggagg    120
agcggcctgg ggcggagggc gccccgctgc tgccgccacc gctgccaccg ccctcgccac    180
ctggatccgg tcgcggcccg ggcgcctcag gggagcagcc cgagcccggg gaggcggcgg    240
ctgggggcgc ggcggaggag gcgcggcggc tggagcagcg ctggggtttc ggcctggagg    300
agttgtacgg cctggcactg cgcttcttca aagaaaaaga tggcaaagca tttcatccaa    360
cttatgaaga aaaattgaag cttgtggcac tgcataagca agttcttatg ggcccatata    420
atccagacac ttgtcctgag gttggattct ttgatgtgtt ggggaatgac aggaggagag    480
aatgggcagc cctgggaaac atgtctaaag aggatgccat ggtggagttt gtcaagctct    540
taaataggtg ttgccatctc ttttcaacat atgttgcgtc ccacaaaata gagaaggaag    600
agcaagaaaa aaaaaggaag gaggaagagg agcgaaggcg gcgtgaagag gaagaaagag    660
aacgtctgca aaaggaggaa gagaaacgta ggagagaaga agaggaaagg cttcgacggg    720
aggaagagga aaggagacgg atagaagaag aaaggcttcg gttggagcag caaaagcagc    780
agataatggc agctttaaac tcccagactg ccgtgcagtt ccagcagtat gcagcccaac    840
agtatccagg gaactacgaa cagcagcaaa ttctcatccg ccagttgcag gagcaacact    900
atcagcagta catgcagcag ttgtatcaag tccagcttgc acagcaacag gcagcattac    960
agaaacaaca ggaagtagta gtggctgggt cttccttgcc tacatcatca aaagtgaatg   1020
caactgtacc aagtaatatg atgtcagtta atggacaggc caaaacacac actgacagct   1080
ccgaaaaaga actggaacca gaagctgcag aagaagccct ggagaatgga ccaaaagaat   1140
ctcttccagt aatagcagct ccatccatgt ggacacgacc tcagatcaaa gacttcaaag   1200
agaagattca gcaggatgca gattccgtga ttacagtggg ccgaggagaa gtggtcactg   1260
ttcgagtacc cacccatgaa gaaggatcat atctcttttg ggaatttgcc acagacaatt   1320
atgacattgg gtttggggtg tattttgaat ggacagactc tccaaacact gctgtcagcg   1380
tgcatgtcag tgagtccagc gatgacgacg aggaggaaga agaaaacatc ggttgtgaag   1440
agaaagccaa aaagaatgcc aacaagcctt tgctggatga gattgtgcct gtgtaccgac   1500
gggactgtca tgaggaggtg tatgctggca gccatcaata tccagggaga ggagtctatc   1560
```

tcctcaagtt tgacaactcc tactctttgt ggcggtcaaa atcagtctac tacagagtct 1620
attatactag ataaaaatgt tgttacaaag tctggagtct agggttgggc agaagatgac 1680
atttaatttg gaaatttctt tttacttttg tggagcatta gagtcacagt ttaccttatt 1740
gatattggtc tgatggtttg tgaactcttg ctgggaatca aaatttcctt gagactcttt 1800
agcattcata ctttggggtt aaaggagatt cctcagactc atccagccct tgggtgctga 1860
ccagcagagt cactagtgga tgctgaagtt acatgagcta catgttaaat atttaaagtc 1920
tccaaaataa aacaccccaa cgttgacctt acccggctga tggttagccc cttgctgcct 1980
gctccatgtg tcttatgaga gcccgtagtt acagtgtcct ctaatttgaa atccataagt 2040
taacaagtct atatcaggtg cagctggctt tgattaaagg ccatttttaa aacttaaaaa 2100
ctcaacacct cacagattat aatagaaaaa gaaatggcct cagtttgatc tcgttcagaa 2160
tgacccagat tgtttctgct ttgggtgcag ctgtttagtt cagagttata ttacagagaa 2220
ttattttctg agataatctt aaactagaat gttcaaaact aattgataat tgaagtatca 2280
agatacgtag aacacctcag agatttttct tcaggaactt ccacaaactt tgaatccttg 2340
tatctttatt tggtattcat actactagta gcaaaataca ggttttttgt tttgttttgt 2400
tttgttttgg cttcatagag tatctcaaat tgaaactttt ctgcacaaag aataaaatta 2460
aggattttat aaactcaaat tggcacctac tgaattaaaa tacataaaat catttaaata 2520
taattcagca tatgggaagt aacattgcac taatatggaa atcactgcca gagacagtct 2580
attttctttt aatttgttac tacttagtca caaaccccac attattccag tttggaatta 2640
cttattaagg agaattggaa atacatatgc ccatgcttaa attttatagc tttaatttgt 2700
gttatttctt tattgacggg aagaggtaca tcttttttc cttactgaaa acaaatatgg 2760
attaattgcc tcaaatttgt ataagtgatt ggctagtgat tcttgttttc agaagggaga 2820
gtggtataga tagaaaatga caaagatggc aatatacact taatgttgtt attgtatgtt 2880
gttactgaag tacttagatt tttaaaattt caaatcctaa atcacttctt gtaggagggt 2940
tttcattaac tgcagtatat acagttcact acatatgggt tgtttgagtt ttttgtgtgc 3000
tgtatttctt tctgtttttt aatacctggt tttgtacata tctaactctg ttctcttttg 3060
gttgttcaga aactggattt tttttttctt aagcagtgct taatttgtgt tttttaattt 3120
tgattcagaa gtagtcccag ctcataggtg ttcatactgt tacatccaga acatttgtca 3180
ggctctctgt cagctttcat gtacatatgg tatagaaacc atggagttag gcacttcctg 3240
gatttttttt ttatgagaaa aatactgtat ttaaaatgta aaataaactt ttaaaaagca 3300
ggcactaata tatatttctt ccagcctttg attacaaatt tgtccttgca catgttaaga 3360
tgaattatct cctaaaaata tcattgttct tgggagcagt gtatgttact ttacatagca 3420

gcggttcctg tcatgtgttc atgtcagaat atttttggtt ttaaactttc ttattgcctt 3480
tggctgttga ttagtacagt acaagtgcga tttcaaaaag atcttgaaag taatatattt 3540
aatcaattaa aatgtttatc tgtaaaaaaa aaaaaaaaaa a 3581

<210> 37
<211> 609
<212> DNA
<213> Homo sapiens

<220>
<221> misc_feature
<222> (565)..(565)
<223> n is a, c, g, or t

<220>
<221> misc_feature
<222> (587)..(587)
<223> n is a, c, g, or t

<220>
<221> misc_feature

<222> (598)..(598)
<223> n is a, c, g, or t

<400> 37

```
tttttttaca gttttcaaat attttactga aaatgcatat tgtacaatta atgtataatg     60
acacaccagt gtgagaaacc tccataggta tcatttccac aaatatgcta tgaatataga    120
gttcctacac aaaactatac aacttaccag atgtaattcc tgttacgtac catactcaca    180
atcgtcttga agaatatgga gaaaaagtgc tgagtgacaa aaacaggagc catgtgtgat    240
tttaataaat ggaaaacacg gcatttcagc tcagtggtaa agcagtaaac caatcagatg    300
cttagctatc aagtaatcat gtgagaggaa acagaattag atcctacctc atactatatg    360
ttgtcagcta acactgtagc agtggtatat gaatcactaa attacctcca acaaaatgta    420
ttcctgtatt gaaaaaagga ggtatggcca acattgtgtc acgttccaag gtgaattttg    480
cggtcacgat atgacgttca ggaagctact tttattgttc agttgatttc tatgctcaac    540
tattaggtca attccgaaat aatcncatat cacagctaaa ataatgncta ccaagtcnct    600
ctgactgct                                                            609
```

<210> 38
<211> 2216
<212> DNA
<213> Homo sapiens

<400> 38

```
ctgttagcaa tgcttcctga tgttgtgcgt ggcccttttt ggttgattct ctccaaattc     60
gggtcagctg ctgccacctg gcaaataaca gaggatatgc tgaatctcct gtccatcctt    120
gtaacgatat ccttcttaat gaaattcttc aactggctga gcaattacaa atgtcatctg    180
```

```
tccagacaca tgggcttaag gatgtctaca aaattttaga catttttgca aatgggaaaa     240
aaaatagtct tgtaaatact gaaacagatt tccatgaact ttatcctact cttggaaaga     300
aaacaattct ccttggctgc agaaatcaaa taagctgggt ttgcaatgac caaggacata     360
aatgaagatg gattgaagtg gaaaaattct gtctcccaag tgatcagtga catctgccag     420
aggtcattac agctactttt aactgtgaac agtcaccagc taaactactc acttgccaca     480
acaaaataac ctctctcaaa gtaaatccag tgcatctgta tatatgtgta gatagcagca     540
acaaacaatc ctgaaacatt atttttggct gttaggtaag taaacgtgat gataattata     600
aacaacattc aaataacctt ggaccttggt gaaatgactt gtggtggcca gaatggtgca     660
acaagatgtt atttgcaagt ttttttaaga cacaaatatc tcagatacta ataatgagaa     720
taaagactgt tgaatatgaa attaaagcca agcaataatg tgccaaaaag aggcagttat     780
accagcaaat gcatctatta tgggcacacc attatataat gatggtttgc tttatgaaga     840
ctgactgtaa cccacaggat aaaataagca aaggcatagt ttctgctttc ttcctggaaa     900
aacttgttta gaagcttcat aaagaggtac agcactaatg agcattagtc aggatacagt     960
tggcatctat gtttttatgt gagcccagag ggaagaggag ccactcaaag tcttgctggt    1020
ttaaaactca agacagctgc aaccagaagt tttgttgaaa tggagacttt aaacttatgg    1080
taattactct ttctggacac tagcatgtag aaagcaattc agttaactct gcccagagga    1140
ttaccagctt tagctgtgaa aaaatgggct cccggatgta aaatcactaa aacatgagat    1200
cttgtatcca aagaggcttc aaatgatgcc ttacagaaaa cgatgctcca gatgggcact    1260
tctaaatgct aactcttcat caagtatctt tctggattca agctcaaaat taattggctg    1320
caaaatagta ggaataaaaa tcacatattt tacactttag aaaaggatat tgatgatcaa    1380
cctgcatggt gataattatg atgagatacc ccagtgattt aatgatgtta gaaagaatta    1440
aatgggagag aattgctaac agctttcttg atctcttaac tatggagatg tcattcattt    1500
atttctgggg tgaaaattat agcttgcttt ttgacattgc tgctagtatt gttctttgtt    1560
gctttaaaaa ttgtctctct ttagaaaaac tcttgagcag ttaaacagtt ctttttctga    1620
ttcatatcat tgcttttaat aacatgtaaa ggctgtgtgt agagcaaact atataaaatg    1680
agtagaaagg gcttgctcat gttaattggc atccttgatg attttagttg agattcctta    1740
acatttattt tagatcacat ctttacgtaa cttatttttc ctaatgtttt ccatcgtgtc    1800
ttaaaatgat gctggtatat caggagattg cagtattata gtcatactcc ccaatcccta    1860
gaggagagga aagactaatt cttgttttaa gggcccctgg agataccttt tattaaggtt    1920
gaaaaaggtc aacacagcct gaaaataaga aaaatatata ctagcaatta ctaattttct    1980
aaatgtgtgt atctctgctg tactaatgtg tgaacaatat gtcgtgcata atactgtagc    2040

tggtcgtggt atgtcaatac attctgtgag tgtgtacagt ctgagtgatc agttttctat    2100
ttttatgtgt aaaaaaaata acttgtcgta tcccatttaa aggccaattt ctgtattcag    2160
gcaggcatat gtacatacat gaataaagcc aacaaaagtg tgcacatgta ttcagt        2216
```

<210> 39
<211> 1705
<212> DNA
<213> Homo sapiens

<400> 39

```
aattcggcac gagaagactt ccagtttgga gtcgtttgct gcggggaggg aatgaatggg      60
cgctgggaac acgcccgcga ggtggggacg cgccggccgt agcgaggtcc ttagcgtgtg     120
agtggccggg gtcgggtcgc ttccccgcag catggaggac gatgcaccag tgatctacgg     180
gctggagttc caggcacgtg ccttaacacc tcaaactgca gaaacagatg ccattcggtt     240
tttggttggg acgcagtctc ttaaatatga taatcagatc catatcatag attttgacga     300
tgaaaacaac attataaata aaaatgtcct cctccatcaa gcgggtgaaa tctggcatat     360
tagcgctagc cctgcagaca gaggtgtgct gacgacctgc tacaacagaa cttcagacag     420
caaagtcctg acatgtgcag ccgtgtggag gatgccgaag gaattggaat caggcagcca     480
cgagtcccct gatgattcat ccagcactgc acagaccctg gagctgctct gtcaccttga     540
caacacagcc catggcaaca tggcctgtgt cgtgtgggag ccaatgggag atgggaagaa     600
aatcatttcc ttggctgata accatatcct gctgtgggat ttacaggaaa gctcgagcca     660
ggctgtgctg gccagctcag cgtccctgga agggaaggga caactgaagt tcacctcagg     720
acggtggagc ccacatcata actgcaccca ggtggccaca gcgaacgaca ccaccctccg     780
tggctgggac acccggagca tgagccagat ctactgcata gagaatgccc acggacagct     840
ggtgcgggac cttgacttta atcccaataa gcagtactac ttggccagct gcggagacga     900
ctgtaaggtg aagttctggg acacccgaaa tgtcaccgaa cccgtgaaga ccctggagga     960
gcactcccac tgggtgtgga acgtccgcta caaccactct catgaccagc tggtcctcac    1020
gggcagcagt gacagcagag tcatcctttc caacatggtg tccatctcgt cggagccctt    1080
cggccacttg gtagacgacg atgacatcag tgaccaggag gaccaccgtt ctgaagagaa    1140
gagcaaggag cccctgcagg acaacgtgat cgccacctac gaggagcacg aggacagcgt    1200
ctatgccgtg gactggtcct cggctgaccc gtggctgttt gcctccctga gctatgacgg    1260
gaggctcgtg atcaacaggg tgcccagggc cctgaagtac cacatcctgc tatgactccc    1320
gggcctgggt tatccaggtc ccattgagtg gttttcctct tggcagattc tcaaacagtc    1380
gcagctcttt ggaggtgact cgtgttccag gtggatccct ctctgggaga gccgctgttc    1440
ccttcctgta gcagcagcat ttatgaatgg ggtgaatggg gctattgtcg acggcacagc    1500

taatgcccga acccagcccc tgtcggcaga gacagagccc cacattatta tgtgaataac    1560
aatgttttct gttttaaggg tgtcaggagt ttcgcttttt aaaaaaatgt ctgttcctgc    1620
agtagtaact cttctttctc ttgagagtaa aaaatgaaat aaaataaatc cacgctgaca    1680
aaaaaaaaaa aaaaaaaaaa aaaaa                                           1705
```

<210> 40
<211> 1800
<212> DNA
<213> Homo sapiens

<400> 40

```
gaggaagatg gcggcgtccg cagctgccgc tgagctccag gcttctgggg gtccgcggca      60
cccagtgtgt ctgttggtgt tgggaatggc gggatccggg aaaaccactt ttgtacagag     120
gctcacagga cacctgcatg cccaaggcac tccaccgtat gtgatcaacc tggatccagc     180
agtacatgaa gttccctttc ctgccaatat tgatattcgt gatactgtaa agtataaaga     240
agtaatgaaa caatatggac ttggacccaa tggcggcata gtgacctcac tcaatctctt     300
tgctaccaga tttgatcagg tgatgaaatt tattgagaag gcccagaaca tgtccaaata     360
tgtgttgatt gacacacctg gacagattga ggtattcacc tggtcagctt ctgggacaat     420
tatcactgaa gcccttgcat cctcatttcc aacagttgtc atctatgtaa tggacacatc     480
gagaagtacc aacccagtga ccttcatgtc caacatgctc tatgcctgca gcatcttata     540
caaaaccaag ctgcctttca ttgtggtcat gaataaaact gacatcattg accacagctt     600
tgcagtggaa tggatgcagg attttgaggc tttccaagat gccttgaatc aagagactac     660
atacgtcagt aacctgactc gttcaatgag cctggtgtta gatgagtttt acagctcact     720
cagggtggtg ggtgtctctg ctgttctggg tactggatta gatgaactct ttgtgcaagt     780
taccagtgct gccgaagaat atgaaaggga gtatcgtcct gaatatgaac gtctgaaaaa     840
atcactggcc aacgcagaga gccaacagca gagagaacaa ctggaacgcc ttcgaaaaga     900
tatgggttct gtagccttgg atgcagggac tgccaaagac agcttatctc ctgtgctgca     960
cccttctgat ttgatcctga ctcgaggaac cttggatgaa gaggatgagg aagcagacag    1020
cgatactgat gacattgacc acagagttac agaggaaagc catgaagagc cagcattcca    1080
gaattttatg caagaatcga tggcacaata ctggaagaga aacaataaat aggagacttt    1140
agcacacttc acttgtttct agaagtccag aattttggac ctccacgtga aagaactgtt    1200
cttacctctg aactgggggc tcccataagg gataattttc ctcagagtag caaagtttct    1260
cttattagag aaatcttgtg actcagatga agtcagggat agaagaccct tggacctggc    1320
aggttaatgc tgattattcc ttggcctttc ccttgtattt atgcaaggaa ggatatactg    1380
agctgatact gttccaagcc tacaacttca agttttatca tttgaactca agtacttttg    1440

ctgctgagga atggaatcaa aagaacgtag tctcctggtg accacctcag atctctatta    1500
ttaggctaga tgtatagcct ctactccccc agcttcttgc tcttgaccct gcactgtaag    1560
ttgcccttct attagcagcc aaggaaaagg gaaacatgag cttatccaga acggtggcag    1620
agtctccttg gcaatcaacc aacgttgcta tgaaatatgc ctcacactgt atagctcatt    1680
ataggacgtc aggtttgttg aaaaaagtgg gcaagacatg attaatgaat cagaatcctg    1740
tttcattggt gacttggata aagacttttt aattttaaaa aaaaaaaaaa aaaaaaaaaa    1800
```

<210> 41
<211> 2297
<212> DNA
<213> Homo sapiens

<400> 41

```
ggctgaggcg cgatggcagg tgtcggggct gggcctctgc gggcgatggg gcggcaggcc      60
ctgctgcttc tcgcgctgtg cgccacaggc gcccaggggc tctacttcca catcggcgag     120
accgagaagc gctgtttcat cgaggaaatc cccgacgaga ccatggtcat cggcaactat     180
cgtacccaga tgtgggataa gcagaaggag gtcttcctgc cctcgacccc tggcctgggc     240
atgcacgtgg aagtgaagga ccccgacggc aaggtggtgc tgtcccggca gtacggctcg     300
gagggccgct tcacgttcac ctcccacacg cccggtgacc atcaaatctg tctgcactcc     360
aattctacca ggatggctct cttcgctggt ggcaaactgc gtgtgcatct cgacatccag     420
gttggggagc atgccaacaa ctaccctgag attgctgcaa aagataagct gacggagcta     480
cagctccgcg cccgccagtt gcttgatcag gtggaacaga ttcagaagga gcaggattac     540
caaaggtatc gtgaagagcg cttccgactg acgagcgaga gcaccaacca gagggtccta     600
tggtggtcca ttgctcagac tgtcatcctc atcctcactg gcatctggca gatgcgtcac     660
ctcaagagct tctttgaggc caagaagctg gtgtagtgcc ctctttgtat gacccttcct     720
ttttacctca tttatttggt actttcccca cacagtcctt tatccacctg gatttttagg     780
gaaaaaaatg aaaaagaata agtcacattg gttccatggc cacaaaccat tcagatcagc     840
cacttgctga ccctggttct taaggacaca tgacattagt ccaatctttc aaaatcttgt     900
cttagggctt gtgaggaatc agaactaacc caggactcag tcctgcttct tttgcctcga     960
gtgattttcc tctgtttttc actaaataag caaatgaaaa ctctctccat taccttctgc    1020
tttctctttg tccacttacg cagtaggtga ctggcatgtg ccacagagca ggccctgcct    1080
cactgtctgc tggtcagttc tgggttcact taatggcttt gtgaatgtaa ataaggggca    1140
ggtcttggcc ctagaggatt gagatgtttt tctatatctt agaactattt ttggataaat    1200
tatatatttt ccttcctagt agaagtgtta ctgcctgtaa ctagctcaaa ataccaatgc    1260
agtttctgca ttctgggttt tgtttttcct tttttttttt tttttttttt ttttgagttt    1320

tgctctcgtc gcccaggctg gagtgcaatg gcgtgatctc agctcactgg caacatctgc    1380
ctcccgggtt caaatgattc tcctgcctca gtctcctgag tagctgggat tacaggtgcc    1440
cgccaccacg ctcagctaat ttttgtattt ttagtagaga tggggtttta ccatgttggc    1500
caggctggtc ttagactcct gacctcagtt gatccacctg cctcagcctc tgcattcagt    1560
ttattcacat atttttggta actcccatgg cagctcctag gatttcagcg gtctgtgggc    1620
cagaaagcag gcaccagggc tgacctcaag gccgtatcag agggccaagc agagttcttt    1680
tggatacctg cttttcatcc cacagggcct tagagtcaga ggtaaggtag caacagagct    1740
agaatggggc aatgcactct taccctcctt ctcaactttt atttaagctg tgctaaatgt    1800
tttcttcaag ggaaccagat ttagttcttt acagaatttt ccagtgaaat aaaacatgtt    1860
gtaatagctg tgtttgagat gaaataagag gttgtgggta gaggggaggc acctaaagga    1920
aaagaggaaa ggtgcctggg ctacctatgc agataacctg gagtggactt cactgtggac    1980
tcgtggtact aaggcttggc ctggacaggc agtctagggg gtatgggaat acacggtgtg    2040
gttgttcaac tatttgcaaa ggtcaaccaa atagaccaca tgttcgcaaa gtatcatctg    2100
aggaaattaa gtaccttctt agccctctca gtcataaatt tgaacaaatt ttaatacact    2160
tccctcatgc ccttctatat aaaacttaat accattagtt ccccattctt gacattttat    2220
ttcagttttt attatatatt tatttgaaat atttattaaa ttatctgacc tacagaacta    2280
aaaaaaaaaa aaaaaaa                                                   2297
```

<210> 42
<211> 653
<212> DNA
<213> Homo sapiens

<220>
<221> misc_feature
<222> (621)..(621)
<223> n is a, c, g, or t

<220>
<221> misc_feature

<222> (653)..(653)
<223> n is a, c, g, or t

<400> 42

```
gggacatttc caagggtatt taaactctca ctctgccacc tttctaaggg tgggaggctg      60
gcagagatgc tgcaatgctt gataatcatt tggccacact gaaatttcca aagggagctc     120
ttgccggtgc ttaaaaccaa aactcctgga cacttagaaa attccatgaa tctagcacaa     180
aatatccatt cttgcccaag tgtatcccct ttctctccag cttaatcttt tttttttttt     240
tttttaaag cccaggccaa gggtactttt aactggaaac tggggaggag ggaagaacac      300
tagcagggag ctaagaggca ggttgctggg taagccatcc tgctcctacc tggtgcctgt     360

atctacattg ctgagtgctg tgcgccagtg cctttccttc atctgcagat ggagcccatc     420
tctttccacc tgggtgagga gaccctctgc tactccaggg gtaaacctta aagaaggtgt     480
cttgaagagc ccaaaggaca ctcacgtgct aaggtgtcca ttttatgcat ctttaaaata     540
ttttatttaa aaaaaaaaat agccctgccc tgtcttagtg ccactaacgg cccagattca     600
ttcattctga atggaaaaac ngagactgcc agcactttcc tttggtcctt ccn            653
```

<210> 43
<211> 1955
<212> DNA
<213> Homo sapiens

<400> 43

```
catggaggcg ctgctgctgg gcgcggggtt gctgctgggc gcttacgtgc ttgtctacta      60
caacctggtg aaggccccgc cgtgcggcgg catgggcaac ctgcggggcc gcacggccgt     120
ggtcacgggt gagtgcggag gcgggtgagt gcgagctggc ggggcgcgcg gagaggaggc     180
cgggccggcg gtagcagcgg cccgccgggc tcagctcagc tcggctcccg cccgcggtcc     240
gcaggcgcca acagcggcat cggaaagatg acggcgctgg agctggcgcg ccggggagcg     300
cgcgtggtgc tggcctgccg cagccaggag cgcggggagg cggctgcctt cgacctccgc     360
caggagagtg ggaacaatga ggtcatcttc atggccttgg acttggccag tctggcctcg     420
gtgcgggcct ttgccactgc ctttctgagc tctgagccac ggttggacat cctcatccac     480
aatgccggta tcagttcctg tggccggacc cgtgaggcgt ttaacctgct gcttcgggtg     540
aaccatatcg gtccctttct gctgacacat ctgctgctgc cttgcctgaa ggcatgtgcc     600
cctagccgcg tggtggtggt agcctcagct gcccactgtc ggggacgtct tgacttcaaa     660
cgcctggacc gcccagtggt gggctggcgg caggagctgc gggcatatgc tgacactaag     720
ctggctaatg tactgtttgc ccgggagctc gccaaccagc ttgaggccac tggcgtcacc     780
tgctatgcag cccacccagg gcctgtgaac tcggagctgt tcctgcgcca tgttcctgga     840
tggctgcgcc cacttttgcg cccattggct tggctggtgc tccgggcacc aagagggggt     900
gcccagacac ccctgtattg tgctctacaa gagggcatcg agcccctcag tgggagatat     960
tttgccaact gccatgtgga agaggtgcct ccagctgccc gagacgaccg ggcagcccat    1020
cggctatggg aggccagcaa gaggctggca gggcttgggc ctggggagga tgctgaaccc    1080
gatgaagacc cccagtctga ggactcagag gccccatctt ctctaagcac cccccaccct    1140
gaggagccca cagtttctca accttacccc agccctcaga gctcaccaga tttgtctaag    1200
atgacgcacc gaattcaggc taaagttgag cctgagatcc agctctccta accctcaggc    1260
caggatgctt gccatggcac ttcatggtcc ttgaaaacct cggatgtgtg cgaggccatg    1320
ccctggacac tgacgggttt gtgatcttga cctccgtggt tactttctgg ggccccaagc    1380
```

```
tgtgccctgg acatctcttt tcctggttga aggaataatg ggtgattatt tcttcctgag   1440
agtgacagta accccagatg gagagatagg ggtatgctag acactgtgct tctcggaaat   1500
ttggatgtag tattttcagg ccccaccctt attgattctg atcagctctg gagcagaggc   1560
agggagtttg caatgtgatg cactgccaac attgagaatt agtgaactga tccctttgca   1620
accgtctagc taggtagtta aattaccccc atgttaatga agcggaatta ggctcccgag   1680
ctaagggact cgcctagggt ctcacagtga gtaggaggag ggcctgggat ctgaacccaa   1740
gggtctgagg ccagggccga ctgccgtaag atgggtgctg agaagtgagt cagggcaggg   1800
cagctggtat cgaggtgccc catgggagta aggggacgcc ttccgggcgg atgcagggct   1860
ggggtcatct gtatctgaag cccctcggaa taaagcgcgt tgaccgccaa aaaaaaaaaa   1920
aaaaaaaaaa aaaaaaaaaa aaaaaaaaaa aaaaa                              1955
```

<210> 44
<211> 3098
<212> DNA
<213> Homo sapiens

<400> 44

```
tcctgcttgt cggcatcgct ccccacaggc cgacgtcgag agggcctgct ttactcctcc     60
tctttctcct ccttctcccg cggcttctgc gcggagaggc gtcgcccggg atctgggttt    120
tggaagaagg atctttgtgg gaagacaggg tgaatttatc acagaggaat aacgagggag    180
aggagaaagg tttcctaaag acaaaaaaaa aaatggagga atctgtaaac caaatgcagc    240
cactgaatga gaagcagata gccaattctc aggatggata tgtatggcaa gtcactgaca    300
tgaatcgact acaccggttc ttatgtttcg gttctgaagg tgggacttat tatatcaaag    360
aacagaagtt gggccttgaa aatgctgaag ctttaattag attgattgaa gatggcagag    420
gatgtgaagt gatacaagaa ataaagtcat ttagtcaaga aggcagaacc acaaagcaag    480
agcctatgct ctttgcactt gccatttgtt cccagtgctc cgacataagc acaaaacaag    540
cagcatttaa agctgtttct gaagtttgtc gcattcctac ccatctcttt acttttatcc    600
agtttaagaa agatctgaag gaaagcatga aatgtggcat gtggggtcgt gccctccgga    660
aggctatagc ggactggtac aatgagaaag gtggcatggc ccttgctctg gcagttacaa    720
aatataaaca gagaaatggc tggtctcaca aagatctatt aagattgtca catcttaaac    780
cttccagtga aggacttgca attgtgacca aatatattac aaagggctgg aaagaagttc    840
atgaattgta taaagaaaaa gcactctctg tggagactga aaaattatta aagtatctgg    900
aggctgtaga gaaagtgaag cgcacaagag atgagctaga agtcattcat ctaatagaag    960
aacatagatt agttagagaa catcttttaa caaatcactt aaagtctaaa gaggtatgga   1020
aggctttgtt acaagaaatg ccgcttactg cattactaag gaatctagga aagatgactg   1080
```

```
ctaattcagt acttgaacca ggaaattcag aagtatcttt agtatgtgaa aaactgtgta   1140
atgaaaaact attaaaaaag gctcgtatac atccatttca tattttgatc gcattagaaa   1200
cttacaagac aggtcatggt ctcagaggga aactgaagtg gcgccctgat gaagaaattt   1260
tgaaagcatt ggatgctgct ttttataaaa catttaagac agttgaacca actggaaaac   1320
gtttcttact agctgttgat gtcagtgctt ctatgaacca aagagttttg ggtagtatac   1380
tcaacgctag tacagttgct gcagcaatgt gcatggttgt cacacgaaca gaaaaagatt   1440
cttatgtagt tgctttttcc gatgaaatgg taccatgtcc agtgactaca gatatgacct   1500
tacaacaggt tttaatggct atgagtcaga tcccagcagg tggaactgat tgctctcttc   1560
caatgatctg ggctcagaag acaaacacac ctgctgatgt cttcattgta ttcactgata   1620
atgagacctt tgctggaggt gtccatcctg ctattgctct gagggagtat cgaaagaaaa   1680
tggatattcc agctaaattg attgtttgtg gaatgacatc aaatggtttc accattgcag   1740
acccagatga tagaggcatg ttggatatgt gcggctttga tactggagct ctggatgtaa   1800
ttcgaaattt cacattagat atgatttaac cataagcagc agcacgatcc agagatccat   1860
tgccatcagt gatctcacta aaaatataca gctacttccc agctaatctc cacccaatga   1920
atgatgatgg tatagtatgt gcataatgga aagttacctt actgaaaaaa aaaaaagaag   1980
gaaaaataag atgggcccaa aggtctatct actaaactag ctcttgggga aatagcttca   2040
ggatactgta gtttcctcta tctaatagag aactttttgt taacagacac tgtaaaatag   2100
ttttgctttg ttgaataata catgtgtacc taaaagaggt aagagcaaaa agtgtaattc   2160
cacatcatgt tacttgagaa gtgcttaacg ttttcttaaa tgttttcatt gggaaaggac   2220
agctttgata atgtccaaat actctgaaat gcactagacc atataactgt gatgaaatat   2280
gaaactcatc tgtaaacttt tataccaagg gggtaaaaaa aaaaactaag gcatttgatt   2340
aaattatgaa tgagttttac aaattccttt cagagtttta ctaagatcac acaaataaca   2400
gctttcttat tcagtgaaaa agatatttta tttctgatgt tttatttgca ctcgtggaat   2460
atgttaccat taatcagaaa catcatggca acccctaaga atagactaag tttgtgttgg   2520
ctgagggatt ctatttggtt tgcttttttt tttttgcttt gttatatttt attgctacaa   2580
ggggtgtgac ttgataatga tttcctctga attataataa catagccaga tgtagtctca   2640
cactgttttt catactctta agtgtaaata atataaaatg tttcaagcgc ttaactcccc   2700
ctcattcaca aagtataaca attaaaatct caactataac cagtttagct ttttccttac   2760
ttttaaaata aaattttttta cttttaacta ttttttagt taatattttt aaaagtatac   2820
atgtcaatgg cctctttgtc cattattcat tttgtggcaa aatattcttc tttgatagtg   2880
taaacaaata ataaagcaat ctaggtcctt taggtttgaa aggcaatttt tgagtagcat   2940

attaccagct agccagtcac taggaatttt tttcagtatt atttgtatgt attaaacttt   3000
tcattacact aaagtgcatt attttattga gcaagtatcc ttcattgtga ggtttgacat   3060
taaagcaatc tgttgaaatg ccaaaaaaaa aaaaaaaa                           3098
```

<210> 45
<211> 2689
<212> DNA
<213> Homo sapiens

<400> 45

```
gatcttgggc tgaggttccc gggcgggcgg gcgcggagag acgcgggaag caggggctgg      60
gcgggggtcg cggcgccgca gctagcgcag ccagcccgag ggccgccgcc gccgccgccc     120
agcgcgctcc ggggccgccg gccgcagcca gcacccgccg cgccgcagct ccgggaccgg     180
ccccggccgc cgccgccgcg atgggcaacg ccgccgccgc caagaagggc agcgagcagg     240
agagcgtgaa agaattctta gccaaagcca aagaagattt tcttaaaaaa tgggaaagtc     300
ccgctcagaa cacagcccac ttggatcagt ttgaacgaat caagaccctc ggcacgggct     360
ccttcgggcg ggtgatgctg gtgaaacaca aggagaccgg gaaccactat gccatgaaga     420
tcctcgacaa acagaaggtg gtgaaactga aacagatcga acacaccctg aatgaaaagc     480
gcatcctgca agctgtcaac tttccgttcc tcgtcaaact cgagttctcc ttcaaggaca     540
actcaaactt atacatggtc atggagtacg tgcccggcgg ggagatgttc tcacacctac     600
ggcggatcgg aaggttcagt gagccccatg cccgtttcta cgcggcccag atcgtcctga     660
cctttgagta tctgcactcg ctggatctca tctacaggga cctgaagccg gagaatctgc     720
tcattgacca gcagggctac attcaggtga cagacttcgg tttcgccaag cgcgtgaagg     780
gccgcacttg gaccttgtgc ggcacccctg agtacctggc ccctgagatt atcctgagca     840
aaggctacaa caaggccgtg gactggtggg ccctgggggt tcttatctat gaaatggccg     900
ctggctaccc gcccttcttc gcagaccagc ccatccagat ctatgagaag atcgtctctg     960
ggaaggtgcg cttcccttcc cacttcagct ctgacttgaa ggacctgctg cggaacctcc    1020
tgcaggtaga tctcaccaag cgctttggga acctcaagaa tggggtcaac gatatcaaga    1080
accacaagtg gtttgccaca actgactgga ttgccatcta ccagaggaag gtggaagctc    1140
ccttcatacc aaagtttaaa ggccctgggg atacgagtaa ctttgacgac tatgaggaag    1200
aagaaatccg ggtctccatc aatgagaagt gtggcaagga gttttctgag ttttaggggc    1260
atgcctgtgc cccatgggt tttctttttt ctttttttctt tttttggtc ggggggggtgg    1320
gagggttgga ttgaacagcc agagggcccc agagttcctt gcatctaatt tcacccccac    1380
cccaccctcc agggttaggg ggagcaggaa gcccagataa tcagagggac agaaacacca    1440
gctgctcccc ctcatcccct tcaccctcct gccccctctc ccacttttcc cttcctcttt    1500

ccccacagcc ccccagcccc tcagccctcc cagcccactt ctgcctgttt taaacgagtt    1560
tctcaactcc agtcagacca ggtcttgctg gtgtatccag ggacagggta tggaaagagg    1620
ggctcacgct taactccagc ccccacccac acccccatcc cacccaacca caggccccac    1680
ttgctaaggg caaatgaacg aagcgccaac cttcctttcg gagtaatcct gcctgggaag    1740
gagagatttt tagtgacatg ttcagtgggt tgcttgctag aattttttta aaaaaacaac    1800
aatttaaaat cttatttaag ttccaccagt gcctccctcc ctccttcctc tactcccacc    1860
cctcccatgt ccccccattc ctcaaatcca ttttaaagag aagcagactg actttggaaa    1920
gggaggcgct ggggtttgaa cctccccgct gctaatctcc cctgggcccc tccccgggga    1980
atcctctctg ccaatcctgc gagggtctag gccccctttag gaagcctccg ctctcttttt    2040
ccccaacaga cctgtcttca cccttgggct ttgaaagcca gacaaagcag ctgcccctct    2100
ccctgccaaa gaggagtcat cccccaaaaa gacagagggg gagccccaag cccaagtctt    2160
tcctcccagc agcgtttccc cccaactcct taattttatt ctccgctaga ttttaacgtc    2220
cagccttccc tcagctgagt ggggagggca tccctgcaaa agggaacaga agaggccaag    2280
tccccccaag ccacggcccg gggttcaagg ctagagctgc tggggagggg ctgcctgttt    2340
tactcaccca ccagcttccg cctcccccat cctgggcgcc cctcctccag cttagctgtc    2400
agctgtccat cacctctccc ccactttctc atttgtgctt ttttctctcg taatagaaaa    2460
gtggggagcc gctggggagc caccccattc atccccgtat ttccccctct cataacttct    2520
ccccatccca ggaggagttc tcaggcctgg ggtggggccc cgggtgggtg cgggggcgat    2580
tcaacctgtg tgctgcgaag gacgagactt cctcttgaac agtgtgctgt tgtaaacata    2640
tttgaaaact attaccaata aagttttgtt taaaaaaaaa aaaaaaaaa                2689
```

<210> 46
<211> 1936
<212> DNA
<213> Homo sapiens

<400> 46

```
ccggagcctc ctggaccagg agaactgtaa cgcgagcccc gagccatggg cgaaaggcgg      60
ggccgagacg ggttgggggc gccgacggtt tcccggccct ggctgcagct tggaggagaa     120
gctgagcctg tgcttccgcc cctcggatcc gggcgccgag cccgaggacg gccgtgcggc     180
catcacggag ctcaactcct gcagggggac gagatttgga atgccctgac agataattat     240
gggaatgtga tgcctgtaga ctggaagtca tcgcatacta ggaccttgca cttgcttact     300
ctgaacctct cagaaaaagg ggtaagtgac agtttgctct ttgatacatc agatgatgaa     360
gagctgagag aacagctgga tatgcactca atcatcgtct cctgtgttaa tgatgaaccc     420
ctcttcacgg cagaccaggt tattgaagaa attgaagaaa tgatgcagga atcaccggac     480

ccagaagatg atgaaacccc tacacagtca gatcggcttt caatgctttc ccaggaaatt     540
caaactctca agaggtctag taccggcagt tatgaagaga gagtgaaaag gctctcagtg     600
tctgagttaa atgaaatcct ggaagaaatt gagactgcca ttaaggagta ctctgaggag     660
ctggtgcagc agttggcttt acgagatgaa ctggagtttg aaaaggaagt gaaaaacagc     720
tttatttctg ttcttattga agtgcaaaac aaacagaaag agcacaaaga aacagcaaaa     780
aagaaaaaga aactaaaaaa tggcagctct cagaatggga agaatgagag aagtcatatg     840
cccggcacat atttgactac agtcattcct tatgagaaaa aaaacggacc accgtctgtt     900
gaagatcttc aaatattaac aaaaattctt cgtgccatga aggaggacag tgaaaaagtt     960
ccgagcttgt taactgatta tattctgaaa gttctgtgtc ctacatagag cagcaacttt    1020
atctgcggtg ggctccaagc tagatttccg acagcattat tctgagagct ggctaccatt    1080
acccttcttg ctattggaaa ctcagcacat ttgaacttgg gtttgattca gtattaacag    1140
atcttgacta cactaattct ttatattata gaaccaacgg aaatatgggc actattttga    1200
attctagaga tggtttttgt taaatctact aataaactgt tctcttagta gattaagaga    1260
gagtaatatt aattgtgcat gtgcagttgt atttctcatt aactgacagt atgcccattt    1320
gtttttatgg ctttcttatc taaactgcac tgatgaacta gattaaagcc ttgggagatt    1380
tatactataa attcagtgat ggcaagaacc aacactgttt ttttgtgaga attgtcagtg    1440
taactattac ctaccagtat tgttcagaga gattgaaaca gaataaacgg gctgttcttg    1500
aagaagcaaa accagaatat gcattacttt ggtttaatac ttagtgctaa cattgaaact    1560
gttggtggtg atggattttg tagcttgctg cttgtttcac cactggtcaa attttaacca    1620
ttaaattgcc attcactttt agaatcttgt atttaagtaa gttttgattt tcaaatgttc    1680
tgcttcatgt gtctgtgaag aattgtactt ttttaaaagt gtgtgtcctc tgaggtgctt    1740
gagaaagtgt acactgcaga actgcccatt ctcattactg tgtcctattt tattcatgcc    1800
tgtgtgtttt tcttaagtat gaattctaga tacagctact tatggattca tcaatatcat    1860
gagcactttt gctggttcca gtcaaatcaa tggcatttaa taaatttttt aagaagtaaa    1920
aaaaaaaaaa aaaaaa                                                    1936
```

<210> 47
<211> 3990
<212> DNA
<213> Homo sapiens

<400> 47

```
ggagtttagg gcctgacaga agcccgcccc cgctggcgct cgtgcgcacg cgtggcgggc      60
tctcggcgca ctgagcaggc gcggcctcgt gtcggccgga gggggcgggc gcaacgacgc     120
gcgctgcgtc ccggcgctcg gctttccctc cgccggtccc gccctccgtc gcggcggcgc     180
```

```
ggtgtaccct gggataggga gcgatctccg agcgaggcgg caagatggac gcgggatttt    240
tccgcggaac aagtgcagaa caggataatc ggttcagcaa caaacagaag aaactactga    300
agcagctgaa atttgcagaa tgcctagaaa aaaaggtgga catgagcaaa gtaaatttgg    360
aggttataaa gccttggata acaaaaagag taacggaaat ccttgggttt gaagatgatg    420
ttgtgattga gtttatattc aaccagctgg aagtgaagaa tccagactcc aaaatgatgc    480
aaatcaacct gactggattt ttgaatggaa aaaatgctcg agaatttatg ggagaactgt    540
ggcccctgct gctaagtgca caagaaaaca tcgcgggaat cccttctgct ttcctagaac    600
tgaagaaaga agaaataaaa caaagacaga ttgaacaaga aaaactggca tctatgaaaa    660
agcaagatga agacaaagat aaaagagata aggaagaaaa agaaagcagc agagaaaaaa    720
gggagcggtc tcgtagccca agaagacgca aatccagatc tccttcccct agaagacgat    780
cttcccctgt caggagagag agaaagcgca gtcattctcg atctccccgt cacagaacca    840
agagccggag tccttcccct gctccagaaa agaaggaaaa aactccagag ctcccagaac    900
cttcagtgaa agtaaaagaa ccttcagtac aagaggctac ttctactagt gacattctga    960
aagttcccaa acctgaacct ataccagagc ctaaagaacc ttctccggaa aaaaattcca   1020
aaaaagaaaa ggagaaggag aagacccgac cacgatctcg gtcacgctcc aaatcaagat   1080
cccggacgcg gtcccgctct ccttctcaca ctcgacctag acggcgccat agatcccgat   1140
caagatcgta ttcacctaga aggcggccaa gcccaagaag gcggccatct cctcgaagaa   1200
gaactccgcc aagaagaatg cctcctccac caaggcatag aaggagtaga tctccagtaa   1260
gacgaagaag acgttcgtca gcatccttgt ctgggagtag ctcatcatcc tcttcatctc   1320
gttcacggtc accaccaaag aagcctccca agaggacatc cagcccccct cggaaaactc   1380
gtaggttatc tccttcagca agtcctccaa ggcgaaggca caggccatca cctcctgcaa   1440
ctccaccacc caaaactcgg cattccccta caccccagca gtcaaaccgt acaagaaaaa   1500
gtcgtgtttc tgtgtctcca gggagaactt caggtaaagt gacaaaacat aaaggtactg   1560
agaaaagaga atccccttca ccagcaccga agcctagaaa agtagagtta tctgaatcgg   1620
aagaagataa aggtggcaaa atggctgcag cagattctgt gcagcagaga cgccaataca   1680
gacgacaaaa ccagcagtct tcatctgact ctggctcctc ctcctcctca gaagatgaac   1740
gacccaagag atcccatgtg aagaatggtg aggttggcag gcggcggaga cattcccctt   1800
cccggagtgc ttctccatca ccacgaaagc gccaaaaaga gacttcccct cgtggtagac   1860
ggaggagaag tccatcccca ccacccacca gaaggcgacg gtctccttct cccgcccctc   1920
ctcctcgacg gcgcaggact cccacaccac caccacgacg aaggactcct tctcctcccc   1980
cacgtcggcg ctcaccttct cctagaagat actctcctcc aatacagagg agatactctc   2040
cttctccacc tccaaagaga agaacggctt cacctcctcc ccctcctaaa cgaagagcat   2100
```

```
caccatctcc accaccaaag cggcgggtct cccattctcc acctcccaaa caaagaagct    2160
ccccagtcac caagagacgt tcaccttcat tatcatccaa gcataggaaa gggtcttccc    2220
caagccgctc tacccgggag gcccgatcac cacaaccaaa caaacggcat tcgccctcac    2280
cacggcctcg agctcctcag acctcctcaa gtcctccacc cgttcgaaga ggagcgtcgt    2340
catcacccca aagaaggcag tccccgtctc caagtactag gcccattagg agagtctcca    2400
ggactccgga acctaaaaag ataaaaaagg ctgcttcccc aagcccacag tctgtaagaa    2460
gggtctcatc ctcccgatct gtctccgggt ctcctgagcc agcagctaaa aagcccccag    2520
cacctccatc ccccgtccag tctcagtcac cgtctacaaa ctggtcacca gctgtaccgg    2580
tcaaaaaggc caaaagccca acaccgagcc catcaccgcc aagaaattca gatcaggaag    2640
gaggtggaaa gaaaaagaag aaaaagaagg acaagaaaca caaaaaggat aagaagcaca    2700
agaagcacaa aaaacacaag aaggaaaagg ctgtggctgc agctgctgca gctgctgtga    2760
cccctgcagc cattgcagct gccacaacca cattagcaca ggaagagcca gtggcagcgc    2820
cagagccgaa gaaggagact gaaagtgaag ctgaagataa ccttgatgat ttagaaaagc    2880
acctgcgtga aaaggccctg agatcaatga ggaaggccca agtgtcccca cagtcttagg    2940
gggaaatgtt tgttatgatg taaattttat ttggtttgta cgcagttcaa tttcaaaatt    3000
gctaaaatgt gtttgagctt tagactataa catttgttgt aataattgct aggttgaagt    3060
tcaacatgta aaaaaagggg gcatggattt acattgcaaa aggtgtccac agtgtattag    3120
tgacattctt tcattgacag ctgacataat tcattgagtg aaatatttta agccaaaaaa    3180
aaattccctt tttaaaaaag gggtttaaa tactgttggc atttttatgg ttcctttaaa     3240
tgccctagct attcccagag gggttttttt gtttgttttt ttggttttga ttttcttttt    3300
gtttttcttt cttcttctta tttttttcat ttgagtctta gctcccattt aagttatgct    3360
tctgaccttg tatggtctgt aagcttgccc agaaataaga ccactgtttt gaactaccac    3420
aaaagtataa atgaatattt taatgccaca atctttcctg ttgcctgtgg agtctctgct    3480
gaaatgaatc aggattcgag ctctaggatg agacagaaaa tgaaagcatg ttgtttgcca    3540
ggacactgtg ggtttatatt gatgtgtaac aagttgattt ggaacactgg actctcattc    3600
tgttattctg gttttgtttt ttttgttttg tttttttttct tttgtaaagg caatgagcta    3660
gtcccagaaa ggatccttca gttacataca atttgtttaa tgaaatgtca tggctctgtt    3720
catatttttg tcttgttctt ccaattggta tatacaactt tcagagcctc ttgtatttgg    3780
aaggctggaa gggcccagac tttggaatag tgtcttggtt tcactgtttt tgttttgatt    3840
tttttttgt tttgattttt tttaaactaa agctatataa agcttgtgga ttaaacagaa     3900
taaatttcta aatttaaaaa tttaaaaaaa aaaaaaaaaa aaaaaaaaaa aaaaaaaaaa    3960

aaaaaaaaaa aaggaaaaaa aaaaaaaaaa                                      3990
```

<210> 48
<211> 1502
<212> DNA
<213> Homo sapiens

<400> 48

```
cctgcgtccc cgccccgcgc agccgccgcg ctcctgcgct ccgaggtccg aggttcccga      60
gatgaaggtc tggctgctgc ttggtcttct gctggtgcac gaagcgctgg aggatgttac     120
tggccaacac cttcccaaga acaagcgtcc aaaagaacca ggagagaata gaatcaaacc     180
taccaacaag aaggtgaagc ccaaaattcc taaaatgaag gacagggact cagccaattc     240
agcaccaaag acgcagtcta tcatgatgca agtgctggat aaaggtcgct tccagaaacc     300
cgccgctacc ctgagtctgc tggcggggca aactgtagag cttcgatgta aagggagtag     360
aattgggtgg agctaccctg cgtatctgga cacctttaag gattctcgcc tcagcgtcaa     420
gcagaatgag cgctacggcc agttgactct ggtcaactcc acctcggcag acacaggtga     480
attcagctgc tgggtgcagc tctgcagcgg ctacatctgc aggaaggacg aggccaaaac     540
gggctccacc tacatctttt ttacagagaa aggagaactc tttgtacctt ctcccagcta     600
cttcgatgtt gtctacttga acccggacag acaggctgtg gttccttgtc gggtgaccgt     660
gctgtcggcc aaagtcacgc tccacaggga attcccagcc aaggagatcc cagccaatgg     720
aacggacatt gtttatgaca tgaagcgggg ctttgtgtat ctgcaacctc attccgagca     780
ccagggtgtg gtttactgca gggcggaggc cgggggcaga tctcagatct ccgtcaagta     840
ccagctgctc tacgtggcgg ttcccagtgg ccctccctca acaaccatct tggcttcttc     900
aaacaaagtg aaaagtgggg acgacatcag tgtgctctgc actgtcctgg gggagcccga     960
tgtggaggtg gagttcacct ggatcttccc agggcagaag gatgaaaggc ctgtgacgat    1020
ccaagacact tggaggttga tccacagagg actgggacac accacgagaa tctcccagag    1080
tgtcattaca gtggaagact tcgagacgat tgatgcagga tattacattt gcactgctca    1140
gaatcttcaa ggacagacca cagtagctac cactgttgag ttttcctgac ttggaaaagg    1200
aaatgtaatg aacttatgga aagcccattt gtgtacacag tcagctttgg ggttcctttt    1260
attagtgctt tgccagaggc tgatgtcaag caccacaccc caaccccagc gtctcgtgag    1320
tccgacccag acatccaaac taaaaggaag tcatccagtc tattcacaga agtgttaact    1380
tttctaacag aaagcatgat tttgattgct tacctacata cgtgttccta gtttttatac    1440
atgtgtaaac aattttatat aatcaatcat ttctattaaa tgagcacgtt tttgtaaaaa    1500
at                                                                   1502
```

<210> 49
<211> 385
<212> DNA
<213> Homo sapiens

<400> 49

```
aaaaaaatta aggctaacca agtgcatcca ttgttcaatg gcacaattga tttcagcaac      60
tatttggaat atcctaatta taggaaatgc ccatctaagt gatatattta aataatacaa     120
tcaatttttt aaggtgaata aactatgatg gtttctaaat agtgtacatg ttacctgaaa     180
aatcagaaaa cacaaagaat gattaatttc gaaagttctt gcctaaaggc accactgact     240
taaaaaacat tcaaaatcaa ataccacaag acataaagcc tcttcatgta tatattcata     300
tatgcaataa atgcattaaa tgtaacttta ttaaacatag tacactgtac ttgacttatg     360
gttaaatatt ttacacacag cttga                                           385
```

<210> 50
<211> 6313
<212> DNA
<213> Homo sapiens

<400> 50

```
gccaggtccc tgaggggcgg gcagatgagg cctaggggtg ccgatcccta gtgtcgacta      60
tgcgagatct gattccggag ctgccatgat tgaagtggta gcagagctca gccggggtcc     120
tgtatttttg gctggggagg cgctggagtg tgtagtgacc gtcaccaacc cccttccgcc     180
cacggccact tctgcatcca gtgaggccct ggcctgggcc agtgcccaaa tccactgcca     240
gttccatgcc agtgagagtc gagtagcact gcctcctcct gactctagtc agccagatgt     300
ccagcccgac agccagactg tctttctgcc acaccgaggt gagaggggcc agtgtatcct     360
ttctactcca ccgaaaattc tattctgtga cctgaggctt gatcctggag agtccaaatc     420
atactcctac agtgaagtgc tgcccataga gggaccaccc tcctttcggg gtcagtcagt     480
caagtacgtc tacaaactga ccattggctg ccagcgtgtc aactccccta tcactttact     540
cagagtccct ctgagggttc ttgtgctgac tggccttcag gatgtccggt ttccccagga     600
tgaggctgta gccccatcca gtccattctt ggaggaggat gaaggtggga agaaagattc     660
atggctagct gagctggctg gggaacgcct aatggctgcc acatcctgcc gcagcctcca     720
tctatacaat atcagtgatg gccgagggaa agttgggacg tttggcatct tcaaatctgt     780
gtacagactt ggcgaggacg tggtggggac cttaaactta ggggaaggaa ccgtagcttg     840
tttgcagttt tcagtcagct tacagaccga ggagcgtgta cagcctgagt accagcggcg     900
acgtggggca gggggtgtcc cctctgtgtc acatgtgact cacgcccggc accaggaatc     960
ctgcctacat acaactagaa ccagcttctc cctcccaatc cctctcagct ccaccccagg    1020
cttctgtaca gccattgtgt ccttgaagtg gagattgcat tttgaatttg taacgtcccg    1080
agaaccagga ttggtactcc taccccctgt ggaacagccc gaacctacca cctggacagg    1140

acctgagcaa gtacctgtag acaccttcag ctgggacctg cccatcaagg tgctgcctac    1200
tagccccacc ctggcctcat atgctgcccc aggccccagc accagcacca taaccatctg    1260
aaactggccc accctggtgc tagttccttc cggatactga gaactcagca cctggactct    1320
aatgggaccc actttttcca cctggggtcc aatgtcgtgg acagtgagag tcgggctttc    1380
agctatagca ttaatttatt tgttcagaat acattggcag ctgctagtgg tttccctgga    1440
agtggcagca gcagtgagca gtcagcagat ggatgatcag ttgagtttag ctggagtggg    1500
gagcaggagc cccaggaaca ggggtgttgg ctgagcccca ttctgggtca ggccctcccc    1560
ctttgcaggg cagccgaggg tcagattttt gcaccaagga gaactggcag gttcctgcct    1620
cctgacgtac ctcacaccca gccgggaagt cgatgggatg ctgggacctg gggaaccaag    1680
gataggggaa ggagtcagca cagtgaaagg ctgcctttat ccctgcccac atgttccctc    1740
tctcacagtt ttccccccac agagccccctt tcagtggccc cttggtcctc ctaactaagc   1800
tgtcacctac catatgtggg ccttttttgtt ttataacagg agtattttct ctccaggtcc   1860
accccaacct cccctgattt atagcctgaa gccttatctt tcacactagt gttggtccct    1920
tcaggtttgg cccatcttgt attgctcttc tgttcattct tacatcacag caatctagtc    1980
actccctggt catccctcag tcactcatat cagagtcatt ctctctggcc atctttggtc    2040
actcacgtgt cacagcagcc cacgccaaca ggatgcagac aggtgcaatg gaaacagtcc    2100
ttgcggagcc aagactcacc cagggtaaaa tatttcccct catagtgaca gggggctagg    2160
gaagaacggg aaatgttagt aggtgtagga gtgctgatga gaggcagagg ctcttctggt    2220
ctggggtgga gacagtaagt acgcactatc cccgtattta gtttgtcttt cctgtttcac    2280
agctggagga agcctgggta ttttgacacg ggatcatctg taaggcccca tcctccctgt    2340
gccctctctg ctgctcctcc attcctaacg cttcacccca ctttaccttg agcttggaag    2400
tagcacttgc tgtagactcc tgggtgctgg aggagtagag acatcaccaa gcagatgatc    2460
ccccagcctc ctaggatccc cttggcctgt ccagcccaga gcatccttag ggccattgct    2520
gctgcacagc cctctcagac ccttcttggc ctctgctcag ctactctggt cttgactcct    2580
tgactttgct ttgcgttgct ccttgagtct tagtttctgt ctttctcccc tgggctcctg    2640
tctcacacta tctccctgcc ctctgctctc acaggctggg gatgtttata aagtgaggac    2700
cctggccccc tgctgagtag agctggaaaa gttgtaactc tgtttcctga ggtgagggca    2760
tgaaaacaag aggtctagct ttaacaagct gtgagagctg attcatgccc cggcacagct    2820
agagggaggg aggtggccat ggagggggca ctggactggg cacttcccca gcaaggaggc    2880
aggaggggcg agggccccca ggtggtcccc agatctcttc cctgacctgg agagaaggaa    2940
gcattccacc ttccccttt ctcccccact gccaccacca ggggtgtgta tgctgggatc     3000
```

```
cctgcctgga ccggagggag gcatttcctg gggatggtta atcctgtgcc ccagccaaac    3060
ccaggagctg caatagggtg cgacggccag aagctccagg agagtgagca ggcacctgga    3120
gtggagactg tgtttccctc agatcctagg gcagggtttc cctaatgtat ccaagaaata    3180
gggctgcccc tcagagatgg tggggagggt ctcttttcct caggcattcc agaggtgaac    3240
tgtccattgc ttatcacctt caaacataca gcagatgtgg gatcacccca catctgggga    3300
tggttctttc ccctttcaaa gaggagcatc tctaagtgcc ctgatgggat gaatcactcc    3360
aggttcacag aggtgtcctc tctttcctcc catatataat ggagtgaggt ttttaggaat    3420
ttatcatttg gcatcctctg agtttcccac aggttctgga ggagcccagg atggattatt    3480
gagagcatgg gctgtagaga cagtcttctt ggattcagat cctgactcca cttagctatg    3540
taacctggtc agattacttc acctctctga gcctgtttcc tcatctataa attggggata    3600
gtaatgccaa ctcattgggc tgttatgagg attactgaga taatgcgtgc agtgctctta    3660
tcaccatctc tggtgcgtaa gcgtcaggaa atagcagttg ctgtgattgg ggctaaagct    3720
ctgaggcaaa atgggcgaca ttatttttctt tgaatgacat taagcagttt gtgcatagct    3780
gagggcttct attggggatg gctgtctcct ggcatagacc tctgcacctt tcacactcat    3840
actccttgtc agcagtcccc aacctttttg gtaccaggga ccggttttgt ggaaaacaat    3900
ttttccacca gtggatggag ggggatagca gcggggagat gattttggga tgaaactgtt    3960
tcatctcaga tcatcaggca ttagattctc ataaggagtg tgcaatctag atcccttgca    4020
tgcggagttc acagtggggt ttgcactcct gtgagaatct aatgcctctg ctgatctgcc    4080
aggaggagga gctcaggcgg taatgctcac tcgcctgccg cccacctcct gctttgtgct    4140
cccgcttcct aacaggccac agactggtac tggcctgtgg cctgggggat ggagacccct    4200
aatccatgtc acctttccca cctctttcaa aaacaggtac ctccaggaac attttggttt    4260
tggccccttgt attgacttct gaatgtctag tttgagaaac tgttcccaaa taagccttct    4320
tcccccagat ctgcaccctc gcctctaccc taggacaaga tgtccttttc tcatcatcct    4380
gccaggctaa ctttaagtct cctgcttttt ctcacttgga tttggatcca tttcttccta    4440
tttccgctca tgtgaactct ccagttctcc tttctcacca ctctcctgct agccatctct    4500
ttggcactaa aggccctggt caaattggat ttctttcatt tttccacact tcaaagaccc    4560
atgttctagg tattctccat agggatagtc tctttggcat ttatttggtt tttctacgtt    4620
ttcagtccca tttactccaa gactcactcc ctgccaccta gtgcatcaga tacagctact    4680
tctggctgac ttttcaaggg ggaccaccct acctgtcatc tcttcactgt tcagaaatga    4740
ctgtgtcagt gcacctcaaa ctcccttgct gtccttttcc aaggagacag ctaaggtgga    4800
tggagatgca gaatggacct cacgttcgcc ctagtcagga ctgataccct ttccgtttca    4860
gaggattgcc aagaaaaaac tcacagttga ggcagggtgc tctgaggtcg gctgcggtgt    4920
```

```
gggaggcacg gcctgggcct gctctctggg ctggagcagg tggattcgaa ggcctgtcta   4980
gcacgagggc ccaaaggtct tgtcagtggc cagtagctct gccgcctttc ccagagaggg   5040
ggtccagggg acatcctgga aggctgggcc ctgggccacc ttctgctctt gcaagctaga   5100
gccagcccaa taggggcgg atgtgagtgg ggagctgggg cgcatgaagg tgggggtgat   5160
gccgaagggg aagggatcgc cagtggggat tggtgcgtgt gcggaaacgg ggacagaagt   5220
gaaggttcat cgcctataac gaagatgagg taggcatata ggggcttctg gaaagctaga   5280
ggctgggctg agccaggagt cctctcccag aagttggggg gcggtgcaga ggtgtgggtc   5340
gagcccgcat gcgtgcctgc tggggagggg gtgagtggtg aggaccaggc ccgctgggtc   5400
ctgggggcgc ggtggctggc gcgcaggtcc cggagggggc ggctggcgcg cactacacgc   5460
ttgggaacaa ggaaaacatc cgccggaggc ccggccgggc ggcgctccag cctcggggca   5520
ggtgcgcgga gaggaagtga gagcattccg gcccccccac cccaaccccg gccgctggcc   5580
ctctggtgag tcacagccga cccccgccgc cggagggaga ggggagctgc gggccagagc   5640
cccggagggt ctggaggagc caggagggtt tctgggagca gagggtcact tagtgggctt   5700
ctgtcgtggt gtcgctacgg gcgcgaaacg gacactgaac acagtctgac tgtatggagg   5760
caggtgggga gggatcccct gggagaactt ggcgggccga gagcagaccc cagggcaagg   5820
aggggccccc gagggggaaa ccgggagtcg ggcaggtggc gtaacccaga aagggaagga   5880
gagccggatt gattggggtg agagaggaag gaagcacgcc aagttaggcc tgggagaact   5940
gagggacctg aggagggagg agggagacca acacagggtg ggaaggcgga aatggccaaa   6000
ccccaggcat caggtctgtc cagaggctga cgtagacagt gaagggtgaa gggtaggttt   6060
taggagtagg gggagttatg attatttggt tacattttgg gattatttgg tctcacaggt   6120
agaagggagc ctgctggtct ctgtgtaacg gatggcttaa aagcaaggtt gtctgcgtct   6180
tggattactg tctgccattc agcctttgcc aaaaaatttg gcactgatct gcacattttt   6240
atagtcattt aaaattgtat gactctgtca aatgatttaa gtaattttgg tggattttta   6300
aaaataaaaa aat                                                        6313
```

<210> 51
<211> 1133
<212> DNA
<213> Homo sapiens

<400> 51

```
ggtaaggggt cctccctgcg ccacacggcc gtcgccatgg tgaagctgag caaagaggcc     60
aagcagagac tacagcagct cttcaagggg agccagtttg ccattcgctg ggctttatc     120
cctcttgtga tttacctggg atttaagagg ggtgcagatc ccggaatgcc tgaaccaact    180
gttttgagcc tactttgggg ataaaggatt atttggtctt ctggatttgg aggcaatcag    240

cggacagcat ggaagatgtg tgctctggct cggataagag atgggacatc attcagtcac    300
tagttggatg gcacaaggct cttcacagac gcatctgtag cagagtggat cttgtactaa    360
cttatgatag aatgtatcag aataaatgtt tttaacagtg taaacaccac aaacaaaaaa    420
cacaacacac acatcataca cacaaaaaac acaaaaaaaa caaacaaatc acacaaaagc    480
tacggtagac ctactattat gcggtgggcc gaaacaagac gggtattata gacaagggaa    540
acgagtcgtc aaatcgtcgt agcctgacac acatcatatt gttagaccca gcgtgtgcaa    600
tatctcgccg gggtagctcc ctcatatgag ggacacgtta tatatgtctc agatagggcg    660
ccggggtata acctgcagtt ttatagatat gctggcaaca gaaaaaagcg atgtaaaaaa    720
aaaaatgaag acaacataaa cacacacaca aagatactat cacatatata ctataaccaa    780
aaaatctcaa agcgtaaatc aaaaatacac taaaacaatt cacagccata ttcactacac    840
cctatccacc ccacacaaaa aaaataagac acaaaacatc acacatatac acactaccta    900
tcattttata ctttaatcta atataattaa gtaacaaatc aacacaaata tacacacgat    960
cgatagatac actgataaaa ttcaacaaac aaaataccaa ataaaatata ctaaacacac   1020
cactagacga gcatcttata ttgcactttt acgtagacct ctgatcaata acaacagacc   1080
tactccacaa atatactact aacacacaaa caatgcaaac agcacagaat aac          1133
```

<210> 52
<211> 20
<212> DNA
<213> Artificial

<220>
<223> Oligonucleotide primer

<400> 52
gccataagtg gtcccacagt 20

<210> 53
<211> 23
<212> DNA
<213> Artificial

<220>
<223> Oligonucleotide primer

<400> 53
gtcttctagt ccgtcatctc cct 23

<210> 54
<211> 22
<212> DNA
<213> Artificial

<220>
<223> oligonucleotide primer

<400> 54
ccatagctgc ctttatgtct gc 22

<210> 55
<211> 22
<212> DNA
<213> Artificial

<220>
<223> oligonucleotide primer

<400> 55
ctttttacct tcgtgcacct tt 22

<210> 56
<211> 23
<212> DNA
<213> Artificial

<220>
<223> oligonucleotide primer

<400> 56
taatctgctg aggacctttt gtc 23

<210> 57
<211> 23
<212> DNA

<213> Artificial

<220>
<223> oligonucleotide primer

<400> 57
taattcactg tcctcttctg gga 23

<210> 58
<211> 21
<212> DNA
<213> Artificial

<220>
<223> oligonucleotide primer

<400> 58
gctcacagca gtaaatgcct a 21

<210> 59
<211> 23
<212> DNA
<213> Artificial

<220>
<223> oligonucleotide primer

<400> 59
tgctatgctg tadacactgg cta 23

<210> 60
<211> 23
<212> DNA
<213> Artificial

<220>
<223> oligonucleotide primer

<400> 60
ggatccttta ttggtggtag agc 23

<210> 61
<211> 23
<212> DNA
<213> Artificial

<220>
<223> oligonucleotide primer

<400> 61
ccagagtgac cctgaagata aat 23

<210> 62
<211> 23
<212> DNA
<213> Artificial

<220>

<223> Oligonucleotide primer

<400> 62
acctgattct ctaggtgcag ttt 23

<210> 63
<211> 23
<212> DNA
<213> Artificial

<220>
<223> oligonucleotide primer

<400> 63
gtcgtttcaa ccaggtagtt ttg 23

<210> 64
<211> 20
<212> DNA
<213> Artificial

<220>
<223> oligonucleotide primer

<400> 64
gggcgcctta agttattgga 20

<210> 65
<211> 22
<212> DNA
<213> Artificial

<220>
<223> oligonucleotide primer

<400> 65
ggatggtaga aaagcaaact gg 22

<210> 66
<211> 23
<212> DNA
<213> Artificial

<220>
<223> Oligonucleotide primer

<400> 66
tgtaatggag attgtacagg ttg 23

<210> 67
<211> 23
<212> DNA
<213> Artificial

<220>
<223> oligonucleotide primer

<400> 67

aggaacagta caaatgctgt ggt 23

<210> 68
<211> 23
<212> DNA
<213> Artificial

<220>
<223> oligonucleotide primer

<400> 68
gcactccttg aaggtacact aac 23

<210> 69
<211> 22
<212> DNA
<213> Artificial

<220>
<223> oligonucleotide primer

<400> 69
atttgtattc actcagccat gc 22

<210> 70
<211> 23
<212> RNA
<213> Artificial

<220>
<223> oligonucleotide primer

<400> 70
acccaacttc aaaactagga ctc 23

<210> 71
<211> 23
<212> DNA
<213> Artificial

<220>
<223> oligonucleotide primer

<400> 71
acagcttgat gtcctttcta tgc 23

<210> 72
<211> 22
<212> DNA
<213> Artificial

<220>
<223> oligonucleotide primer

<400> 72
tcatgaaagg cactgagttt tg 22

<210> 73

<211> 23
<212> DNA
<213> Artificial

<220>
<223> oligonucleotide primer

<400> 73
gttagctgaa gcagctttat tgc 23

<210> 74
<211> 22
<212> DNA
<213> Artificial

<220>
<223> oligonucleotide primer

<400> 74
atatgcacaa tcctggaagt ga 22

<210> 75
<211> 23
<212> DNA
<213> Artificial

<220>
<223> oligonucleotide primer

<400> 75
tgccttacta gcattaccac cat 23

<210> 76
<211> 21
<212> DNA
<213> Artificial

<220>
<223> oligonucleotide primer

<400> 76
gaggtgatag cattgctttc g 21

<210> 77
<211> 21
<212> DNA
<213> Artificial

<220>
<223> oligonucleotide primer

<400> 77
caagtcagtg tacaggtaag c 21

<210> 78
<211> 21
<212> DNA
<213> Artificial

<220>
<223> oligonucleotide primer

<400> 78
tcttacaccc agtggagaag c 21

<210> 79
<211> 20
<212> DNA
<213> Artificial

<220>
<223> oligonucleotide primer

<400> 79
gtctttgtgt tcccggacat 20

<210> 80
<211> 20
<212> DNA
<213> Artificial

<220>
<223> Oligonucleotide primer

<400> 80
actatgtccg ggaacacaaa 20

<210> 81
<211> 18
<212> DNA
<213> Artificial

<220>
<223> oligonucleotide primer

<400> 81
ttccgtcata tggcttgg 18

<210> 82
<211> 23
<212> DNA
<213> Artificial

<220>
<223> oligonucleotide primer

<400> 82
cgtcgctatc aaggaattaa gag 23

<210> 83
<211> 23
<212> DNA
<213> Artificial

<220>
<223> oligonucleotide primer

<400> 83
gtagctccag acatcactct ggt 23

SEQUENCE LISTING

<110> ASTRAZENECA UK LIMITED & THE UNIVERSITY OF TOKYO

<120> PROCESS

<130> P017248WO

<140> PCT/GB2004/002316
<141> 2004-06-01

<150> GB 0312451.8
<151> 2003-05-30

<150> GB 0322636.2
<151> 2003-09-26

<150> GB 0327132.7
<151> 2003-11-21

<160> 83

<170> PatentIn version 3.3

<210> 1
<211> 352
<212> DNA
<213> Homo sapiens

<400> 1

```
atacggcatc catgaaatat atcatgcgat acaacaatta taagaaggat ccttacagta     60
gaggtgaccc ctgtaatacc atctgctgcc gtgaggacct gaactcacct aacccaagtc    120
ctggaggttg ttatgacaca aaggtggcag atatctacct agcatctcag tacacatcct    180
atgccataag tggtcccaca gtacaaggtg gcctccctgt ttttcgctgg gaccgtttca    240
acaaaactct acatcagggc atgccagagg tctacaactt tgattttatt accatgaaac    300
caattttgaa acttgatata aaatgaagga gggagatgac ggactagaag ac            352
```

<210> 2
<211> 1039
<212> DNA
<213> Homo sapiens

<400> 2

```
ctccactcgc tcttccaaca cccgctcgtt ttgcggcagc tcgtgtccca gagaccgagt     60
tgccccagag accgagacgc cgccgctgcg aaggaccaat gagagccccg ctgctaccgc    120
cggcgccggt ggtgctgtcg ctcttgatac tcggctcagg ccattatgct gctggattgg    180
acctcaatga cacctactct gggaagcgtg aaccattttc tggggaccac agtgctgatg    240
gatttgaggt tacctcaaga agtgagatgt cttcagggag tgagatttcc cctgtgagtg    300
aaatgccttc tagtagtgaa ccgtcctcgg gagccgacta tgactactca gaagagtatg    360
ataacgaacc acaaatacct ggctatattg tcgatgattc agtcagagtt gaacaggtag    420

ttaagccccc ccaaaacaag acggaaagtg aaaatacttc agataaaccc aaaagaaaga    480
aaaagggagg caaaaatgga aaaaatagaa gaaacagaaa gaagaaaaat ccatgtaatg    540
cagaatttca aaatttctgc attcacggag aatgcaaata tatagagcac ctggaagcag    600
taacatgcaa atgtcagcaa gaatatttcg gtgaacggtg tggggaaaag tccatgaaaa    660
ctcacagcat gattgacagt agtttatcaa aaattgcatt agcagccata gctgccttta    720
tgtctgctgt gatcctcaca gctgttgctg ttattacagt ccagcttaga agacaatacg    780
tcaggaaata tgaaggagaa gctgaggaac gaaagaaact tcgacaagag aatggaaatg    840
tacatgctat agcataactg aagataaaat tacaggatat cacattggag tcactgccaa    900
gtcatagcca taaatgatga gtcggtcctc tttccagtgg atcataagac aatggaccct    960
ttttgttatg atggttttaa actttcaatt gtcacttttt atgctatttc tgtatataaa   1020
ggtgcacgaa ggtaaaaag                                                1039
```

<210> 3
<211> 386
<212> DNA
<213> Homo sapiens

<400> 3

```
gataggccac attccagtaa gaactcaatt tgtctcccaa atttgcagaa acaaaacgtg     60
atttaaaagc tgagcttttt atcagaaagc ttttttgatg ttttaagtgt tatgtgactt    120
gttgaacttt ttaaaaagtg ctacttttaa aatcccagat actctgaatt ttagaaaaca    180
aactaattct gattgtgtcg tgcccaagta ccctttttttt tttaatgagt agggaccaat    240
gccacattgc tttttatatt tctttctttt ttaatgttgc caaaaccaaa agtagctttg    300
ttttcctttg tattttgcta ctttgcagta tttgtgtgtg tggttttttt tccttaattt    360
gaaagggaca gcactgtgta tgttta                                         386
```

<210> 4
<211> 565
<212> DNA
<213> Homo sapiens

<400> 4

aggagaccat ttggaagaag aactagatct gttgcttaat ttagatgcac ctataaaaga 60
gggagataac atcttaccag atcagacgtc tcaggacctg aaatccaagg aagatgggga 120
ggtggtccaa gaggaagaag tttgtgcaaa accatctgtg actgaagaaa aaaacatgga 180
acctgagcaa ccaagtacct ccaaaaatgt taccgaggaa gagctggaag actggttgga 240
cagcatgatt tcctaaaaag gggaaaaaaa gtgcctgaag caaatcttgg ttgccttcta 300
acggcaggtg ggcataaggc tgtccttcag gaccagccag tttacaagca tgtctcaagc 360
tagtgtgttc cattatgctc acagcagtaa atgcctacct ctgtgtttga catctgaaag 420

aatacattga agcagcttgt tgcatttgtt tttctggctt agtaatctaa tagatttcct 480
taagggcagg agatagactc tggcccttgt ttctagcctc cttccttgca gtgtttacaa 540
catagccagt gtttacagca tagca 565

<210> 5
<211> 215
<212> DNA
<213> Homo sapiens

<400> 5

ggatccttta ttggtggtag agcaaaaaaa cccaaacacg ataaaccttt caaaagactt 60
tctaaggatg atattggaat gcaccagccc tcacatgtgt atgcacattt gccagaatat 120
aagagttttg ttttaaatac agtcttgtta ggattttacg ttattgttat tatggaaagt 180
gattgtgatg ctatttatct tcagggtcac tctgg 215

<210> 6
<211> 218
<212> DNA
<213> Homo sapiens

<400> 6

gcagtcgttt caaccaggta gttttgggtt gtttttaaag cccttttgag gtcttacaca 60
ttattaactt taaaataatc aggcagctaa gaataattac tagaaaaatc atctaccact 120
tcaaacatgg tcaactactt caaaactgca cctagagaat caggtacctg aagtagaaca 180
agaagcctgg aggtggactt tgagaggagg gaataccc 218

<210> 7
<211> 525
<212> DNA
<213> Homo sapiens

<400> 7

tacgtgtact tcaccatcgt caccaccgac cacaaggaga tcgacttccg ctgcgcgggc 60
gagagctgct ggaacgcggc catcgcgctg gcgctcatcg atttccagaa ccgccgcgcc 120
ctgcaggact ttcgcagccg ccaggaacgc accgcacccg ccgcacccgc cgaggacgcc 180
gtggctgccg cggccgccgc accctccgag ccctcggagc cctccaggcc atccccgcag 240
cccaaacccc gcacgccatg agcccgccgc gggccatacg ctggacgagt cggaccgagg 300
ctaggacgtg gccggcgctc tccagccctg cagcagaaga acttcccgtg cgcgcggatc 360
ctcgctccgt tgcacgggcg ccttaagtta ttggactatc taatatctat gtatttattt 420
cgctggttct ttgtagtcac atattttata gtcttaatat cttgtttttg catcactgtg 480
cccattgcaa ataaatcact tggccagttt gcttttctac catcc 525

<210> 8
<211> 565
<212> DNA
<213> Homo sapiens

<220>
<221> misc_feature
<222> (69)..(72)
<223> n is a, c, g, or t

<220>
<221> misc_feature
<222> (178)..(181)
<223> n is a, c, g, or t

<220>
<221> misc_feature
<222> (188)..(192)
<223> n is a, c, g, or t

<220>
<221> misc_feature
<222> (525)..(527)
<223> n is a, c, g, or t

<400> 8

ctgtgacatg ctcttgagct ttaccctagt tgaacataca tgtgtagatt tacacatact 60
gtttcattnn nnaatttaga aattgttcat taaatcccat ttgaggtata agtcactcag 120
gaagttaaaa tatctctaca cgtatatttt tacattaaaa atacagtgtt agcataannn 180
nccctttnnn nngaagaaca aaaatgtcag tgcatagtta gataaaatgg taaaatgttt 240
tactgaaagc atactttttt ggaaaataga ttcatgaagc ctttaagtgc tgcttctgtc 300
agtcaaacgt taaaaacttt aacattttca aagtgcccag actgtgtaca aagacacatg 360
taatggagat tgtacaggtt gtttttttgt ttgaaccttt gaaagagttt aatcttaacg 420
ttttctaatt ttaaaatttt aaaatcttgt ttaacaaaag cttgtattaa gatactgttt 480
tcatttcatt acagaattgt ttataaaagt tcatttgttg aaaannnagg atccttttta 540
ataccacagc atttgtactg ttcct 565

<210> 9
<211> 285
<212> DNA
<213> Homo sapiens

<400> 9

atatgtgcac acacacactc acacccacac ccataaagat tttgcactcc ttgaaggtac 60
actaactcac catttttatc atacttatcc cagtgtgcca cagttactgg cttatatgcc 120
tgtctctgct atcttatttt atctgtctcc acaacacagc aaactacctg gccttcaata 180
aagggcttat gaattattca tgaatccatt ttgccaggtg cctagccctg tgtctggctt 240
gaagcaggtg ttcccaaggt gtggcatggc tgagtgaata caaat 285

<210> 10
<211> 207
<212> DNA
<213> Homo sapiens

<400> 10

caccaatgag cttactaccc aacttcaaaa ctaggactct aacaataact tctgtcatat 60
ctcatcctgt aacgccccca ccttcgctcc ttccgccaag ataattatca ctttaaattg 120
tgtgcgtgtg tattctcatt tcttatgtga tggtaaaaat gcctttattt tgtttggttt 180
taatgcatag aaaggacatc aagctgt 207

<210> 11
<211> 315
<212> DNA
<213> Homo sapiens

<220>
<221> misc_feature
<222> (173)..(181)

<223> n is a, c, g, or t

<400> 11

```
ctctaaaagc cattcactcc agattttacc tggggaatat tctacatact gcttactttc      60
tctataaaac tcatcaataa atcatgaaag gcactgagtt ttgtaaatca ggaccctaaa     120
tgtttaattg taaataagtt tcagataatt attatagctt tgcgttgaag ttnnnnnnnn     180
ntttttctca actagttaag tcaactgctt ctgaaataac tctgtattgt agattatgca     240
gatctttaca ggcataaata tttaaactgt aatatgctaa cttgaagaga ttgcaataaa     300
gctgcttcag ctaac                                                      315
```

<210> 12
<211> 509
<212> DNA
<213> Homo sapiens

<400> 12

```
ctcactgaag ttgaaatgac tgcccacttc aaaatcttca ttgtgtttac acaccagtgt      60
atttatacaa atcagaggca ttttgtagat gctttgctga cttgttcagc tctgtaaaaa     120
cacagaaatc agacccattt tgtaaagcgg aaaatcatgt tacatggaac atgtcctgta     180
tatatcacat acatggtaat ggagtcttaa tgataagtgc aagataataa tttaatgatg     240
ggattagtct gatcgcttaa tatgcacaat cctggaagtg aattacttgc atcagatata     300
gtgatattta ttattctgta cagagagaaa aatacatata aaacatatgc ttacattaca     360
tgcacgcgga tttcatgctc cataatcttt tctatttttt aatttacctt tctgtaaatg     420
atgtgcatgg aatatgcctt atagaaaaat gctgttcata atttgactac gtggaaaagt     480

gcctatatgg tggtaatgct agtaaggca                                       509
```

<210> 13
<211> 1654
<212> DNA
<213> Homo sapiens

<400> 13

```
tggagcccga ggtccccgcg cggcccgggc ctggcgccct gaggggaaga gcggcccggc      60
ccgagccatg acggacggga tcctagggaa ggcagccaca atggagatcc ctatccacgg     120
gaacggcgaa gccaggcagc ttcctgaaga tgatgggctg gagcaggacc tccagcaggt     180
gatggtgtca ggacccaacc tcaatgaaac cagcattgtg tctggtggct atgggggctc     240
tggtgatgga ctcatcccca cagggtctgg ccgccatcca tctcacagca ccactccttc     300
tggccctgga gatgaggtgg ctcggggcat tgctggagaa aagtttgaca tcgtcaagaa     360
atggggcatc aacacctata agtgcacaaa gcaactgtta tcagaacgat ttggtcgagg     420
ctcacggact gtggacctgg agctagagct gcagattgag ttgctgcgtg agacgaagcg     480
caagtatgag agtgtcctgc agctgggccg ggcactgaca gcccacctct acagcctgct     540
gcagacccag catgcactgg gtgatgcctt tgctgacctc agccagaagt ccccagagct     600
tcaggaggaa tttggctaca atgcagagac acagaaacta ctatgcaaga atggggaaac     660
gctgctagga gccgtgaact tctttgtctc tagcatcaac acattggtca ccaagaccat     720
ggaagacacg ctcatgactg tgaaacagta tgaggctgcc aggctggaat atgatgccta     780
ccgaacagac ttagaggagc tgagtctagg cccccgggat gcagggacac gtggtcgact     840
tgagagtgcc caggccactt tccaggccca tcgggacaag tatgagaagc tgcggggaga     900
tgtggccatc aagctcaagt tcctggaaga aaacaagatc aaggtgatgc acaagcagct     960
gctgctcttc cacaatgctg tgtccgccta ctttgctggg aaccagaaac agctggagca    1020
gaccctgcag cagttcaaca tcaagctgcg gcctccagga gctgagaaac cctcctggct    1080
agaggagcag tgagctgctc ccagcccaac ttggctatca agaaagacat tgggaagggc    1140
agccccaggg tgtgggagat tggacatggt acatcctttg tcacttgccc tctggcttgg    1200
gctccttttt ctggctgggg cctgacacca gttttgccca cattgctatg gtgggaagag    1260
tgcctggagg cccagaagtt gctgccctgt ctatcttcct ggccacaggg cttcattccc    1320
agatcttttc cttccacttc acagccaacg gctatgacaa aaccactccc tggccaatgg    1380
catcactctt caggctgggg tgtgctccct gaccaatgac agagcctgaa aatgccctgt    1440
cagccaatgg cagctcttct cggactcccc tgggccaatg atgttgcgtc taatacccttt   1500
tgtctctcct ctatgcgtgc ccattgcaga gaaggggact gggaccaaag gggtggggat    1560
aatggggagc cccattgctg gccttgcatc tgaataggcc taccctcacc cacccaccca    1620

gtttaattgt gcttagagcc caagaagatt ggga                                1654
```

<210> 14
<211> 652
<212> DNA
<213> Homo sapiens

<220>
<221> misc_feature
<222> (188)..(188)

<223> n is a, c, g, or t

<400> 14

```
tttttttttt ttttttttaa agtagtttaa taaactccac aaaataatag cagatgcatt    60
gaaatattta cataattcga ttttcaaatc tctcattcaa ataaaaggga taaaaataaa   120
atttctgcct ttacggcagc agaacctctt tcctgaaatg gattggtaaa ataagatact   180
tcactggnag aggaactaat ttatgtttaa gaggtattca tattcagcta agaaaataca   240
acccttttc agctatatag attagggaat ataaaatgat attttctaca ttttttgacc    300
tgtattcaaa gttctaaatt caactttgac ttgaagagag aaggtgattt tggtacccat   360
acagagtaga tcatcacaat tacaatggaa agataattaa cgttttatat gctgtttatt   420
tgcttttgaa agtttgggtc agaaaggctg tgataataat tctggcccaa acaggtatgc   480
ttatacctga cacaaatttc actaaaacct aacacttttg gcttggagtt cttgggattt   540
cgactttctg agtcccttcc atttccaaag catgtttcat tgagagcagg caatgtttgg   600
ggatcaggtg tatgattcaa gactaattaa gatgccaaag ttttccaagc tc           652
```

<210> 15
<211> 1990
<212> DNA
<213> Homo sapiens

<400> 15

```
attgtggcgg tgaggaacag gaagccctga agggtcaaaa gaaatacaaa agcaaaggct    60
attttctttt tttttttctt tctttcattc cttccttcct ctgtttcttt ctttcttcct   120
ttcatttttt tttcttttt aagagcgagc ggctctgcgg tggcggtttg gggtgggcgc    180
cgccgaggtg aggtcgtctc gcctcccgcg cgccggtaga ttggttgttt cattatggat   240
ggaggggatg atggtaacct tattatcaaa aagaggtttg tgtctgaggc agaactagat   300
gaacggcgca aaaggaggca agaagaatgg gagaaagttc gaaaacctga agatccagaa   360
gaatgtccag aggaggttta tgaccctcga tctctatatg aaaggctaca ggaacagaag   420
gacaggaagc agcaggagta cgaggaacag ttcaaattca aaaacatggt aagaggctta   480
gatgaagatg agaccaactt ccttgatgag gtttctcgac agcaggaact aatagaaaag   540
```

caacgaagag aagaagaact gaaagaactg aaggaataca gaaataacct caagaaggtt 600
ggaatttctc aagagaacaa gaaggaagtg gaaaagaaac tgactgtgaa gcctatagaa 660
accaagaaca agttctccca ggcgaagctg ttggcaggag ctgtgaagca taagagctca 720
gagagtggca acagtgtgaa aagactgaaa ccggaccctg agccagatga caagaatcaa 780
gagccctcat cctgcaagtc tctcggaaac acctccctga gtggcccctc catccactgc 840
ccctctgctg cagtatgtat cggcatcctc ccaggcctgg gtgcctactc tgggagcagc 900
gactccgagt ccagctcaga cagcgaaggc accatcaatg ccaccggaaa gattgtctcc 960
tccatcttcc gaaccaacac cttcctcgag gcccccctagt ttctccgtcc ctacacaggg 1020
agctcctccc caagggtaga tcggaccgtt catgctgcct ataggcatta tgtccctcaa 1080
aaaaaaactc ctttgcctgc atcctgtgta caacatgaca tttttaacca atccaatcta 1140
aaaatgtgcc agaatccacc tgtggcccga atcgtgtttg gttcctcttt ctactccact 1200
gcagatgacc aaacctgtcc cgctgccact ttcctcactg atattgggag gagggcaagg 1260
cccagccgaa gttccactaa aaatgcccca ggagaatagg caccggctgg cttgccaaag 1320
ggtttgggtt ttattgcttt ctgtttttc ttttcccgac agcacaaaga agtaagggca 1380
gttattggac aggtgttatt taaacattct attgtaaatg aatgtgttgt ttggttctac 1440
tgcattgtgg agcatgcggg ggaagagaac tgacccaggt aatgaaatgg agcccttccc 1500
tggaactaac cagtccttga tgttgtgtga ctaagtaaag atgataaacc ccatctgctg 1560
ggggtgtcac ttcacactcg gcatgcattg tgaaagcttt ccataccctt ggccattccc 1620
tctctcctct ctctccaacc ccatttatgc aggaggggac tgctaacaag aacgcttcca 1680
tctcaaacct tttctctgcc tgggaaatta ttttatgttt gtttttgaaa taaaggattt 1740
agtttaagat tctaaatttt agagaaacaa acgtaggcct tgtttactaa tagccagaca 1800
tcagaactgc aggtaggtat gttaatgaga tgacttattt ctggcagctc ctggaatcct 1860
aatattgtaa atgagtggga cacacttgca tattgtgacc attctattga ggcccttctc 1920
tgtttaatgc atattatact tgtgctttta actgtggaat ctatttctaa cctaaaaaaa 1980
aaaaaaaaaa 1990

<210> 16
<211> 450
<212> DNA
<213> Homo sapiens

<220>
<221> misc_feature
<222> (355)..(355)
<223> n is a, c, g, or t

<220>
<221> misc_feature
<222> (361)..(361)
<223> n is a, c, g, or t

<220>
<221> misc_feature
<222> (375)..(375)
<223> n is a, c, g, or t

<220>
<221> misc_feature
<222> (380)..(380)
<223> n is a, c, g, or t

<220>
<221> misc_feature
<222> (404)..(404)
<223> n is a, c, g, or t

<220>
<221> misc_feature
<222> (437)..(437)
<223> n is a, c, g, or t

<220>
<221> misc_feature
<222> (445)..(445)
<223> n is a, c, g, or t

<400> 16

```
aaatttctgt attttagttt tgaagtgctt tctatttaaa aataaaaaac atgatttatt      60
ttcatttctg acacagaagt gtttctttta aaaaaaaaag accacatttt aaatttctgc     120
ttaaatgtat ataaagtata catttaagta tactggcact cgcaacaaat gaatccttcc     180
ccagggataa atggattgga aaatttgttt ttcattcaac atttggaaag agaacaaacc     240
tgaaatatgt aatttttaaa attatgtgaa aataatgtga aaaatttcat atagtatttg     300
tgtgaaaatc aggtggaaaa aaacttccat gaagaaccca atttaccaaa attcnccatc     360
nttttaagat ttacnttttn aataccatac tactggtttt aacnggaatt ggggtgtggt     420
atgagggggg ttttgcnggg gagangggga                                      450
```

<210> 17
<211> 508
<212> DNA
<213> Homo sapiens

<400> 17

```
tatttccatg aattcaaagc cttttaatga tgtgaacact tactccccat ttcttttta       60
cattgttaca aaaaatttac atacagtttt ctgaaagtgg cattttgttg gttgttatta     120
tactgatgac acatattaac actttgtatt gaagaagtat cataaaaatc acagggcatt     180
acagattttt gataagaagt agtaatagca ttgtctttta acagctggag gctcccaggc     240
atactctttg gtgagaaatg attaatttta tattttcatt ttgatgagaa tcttttcttg     300
```

tttttaccag ttataaaaac aaagcttttt ctttgttgtg atactgtgca ctaagactta 360

gtttcttgag ctgatgctaa ataaaatgag atcaatagga atattccggg aggtcgtgag 420

aagtttttag aaaggatggc atctacatat atatggagct ctgaaaactg ttggagagta 480

tgacctggga ctgaaactgt ggagcaca 508

<210> 18
<211> 475
<212> DNA
<213> Homo sapiens

<220>
<221> misc_feature
<222> (475)..(475)
<223> n is a, c, g, or t

<400> 18

tcgacatcta atcctcattc ttatgaactt ggtattatgt gaatccattg ggaaatgaag 60

actcagagag gttaaaccat ttgcccaagg tcacacagct actaagcggt gctaggatta 120

aaccctggca gtttcagtcc ttaaccaaag ggttaagcgc tttacatata tatacctgta 180

tattatcaat tttcaaataa tttgaaatag taaaatgcag ttcctctggt cctgaatatg 240

aggtaatctt cctatggttc agaagacatt tcagcatttc ctaatatatc ataatgagtc 300

cattttggtt gtaccatgat gtagtcattt tgttttatag tatattaaag aatgcagaaa 360

agcatagctc atagttccag tgtcttgctc tgaggttttt ccattcagta gacattttaa 420

gtatatgtac caggcacata aatatccaga taattaaagt ttatatcatt aagcn 475

<210> 19
<211> 1061
<212> DNA
<213> Homo sapiens

<400> 19

gcgccatcaa tcgccgccgc ctcgtcccgc ttctcggctg aggcgccgcg cggccaggca 60
gcgggtccag gcctcagccg cgcgcccagg ggcctccggg gccctcccgg gtcagcatgc 120
ccggggtgaa actgaccacc caggcctact gcaagatggt gctgcacggc gccaagtacc 180
cgcactgcgc cgtcaacggg ctcctggtgg ccgagaagca gaagccgcgt aaggagcacc 240
tccccctggg cggccccggc gcccaccaca ccctcttcgt ggactgcatc cccctcttcc 300
acggcaccct ggccctcgcc cccatgctgg aggtggctct caccctgatt gattcatggt 360
gcaaagatca tagctacgtg attgctggtt attatcaagc taatgagcga gtaaaggatg 420
ccagtccaaa ccaggttgca gagaaggtgg cctccagaat cgccgagggc ttcagcgaca 480
ctgcgctcat catggtagac aacaccaagt ttacgatgga ctgcgtagcg cctacgatcc 540
acgtgtacga gcaccatgag aacagatggc ggtgcagaga cccacaccat gactactgtg 600

aagactggcc agaggcacag aggatctcag cctcgctcct ggacagccgg tcctacgaga 660
cgctcgtgga tttcgataac cacctggatg acattcggaa tgactggaca aacccagaga 720
tcaataaagc tgtcctacac ttgtgctagg caggcaccgc tgtgactggg ctccgggcct 780
ttcccactac gttgaagaag aaaacctatt tttaaatgta aataaaatat ctggtagcct 840
gtgtggaaag ctgaccgttt taagaagtgg catgtgcctt gaaagggggc agaatgttca 900
gtcggtcgtg tttttaacac agagtctcta gaagaggtgc agacatcccg tctgactgtc 960
cctgtggact ctctcagttg tatgttgcta taatcctcca aatcaaagct ctttctgctt 1020
gtgcaagatt gttcctatta aacagtttta actaaccttt a 1061

<210> 20
<211> 2040
<212> DNA
<213> Homo sapiens

<400> 20

```
gaattccggg cagctcctct tccatctcca gaaatgacct ccaccttcaa cccccgagaa     60
tgtaaactgt ccaagcaaga agggcaaaac tatggcttct tcctgcgaat tgagaaggac    120
accgagggcc acctggtccg ggtggttgag aagtgtagcc cagcagagaa ggctggcctt    180
caagatggag acagagttct taggatcaat ggtgtctttg tggacaaaga agaacatatg    240
caggttgtgg atctggtcag aaagagtggg aattcagtga ctttactagt tctggatggg    300
gattcctatg agaaagcagt gaaaacacgg gtggacttga aagagttggg tcaaagtcag    360
aaggagcaag gtttgagtga taatatactt tcccctgtga tgaatggagg tgtgcaaact    420
tggacccagc cccggctctg ctatctcgtg aaggaaggag gcagctatgg cttctctctg    480
aaaactgtcc aaggtaaaaa gggggtgtac atgactgata ttacacctca aggtgtggct    540
atgagagctg gagttctggc tgatgatcac ttgattgaag tgaatggaga gaatgtagag    600
gatgccagcc atgagaaagt ggttgaaaag gtgaagaagt caggaagccg tgtcatgttc    660
ctgctggtgg acaaagaaac tgacaagcgt catgttgagc agaagataca attcaaaaga    720
gaaacagcca gtttgaaact gttaccccac cagccccgaa ttgtggagat gaagaaagga    780
agcaatggct atggtttcta tctgagggca ggctcagaac agaaaggtca aatcatcaag    840
gacatagatt ctggaagtcc agcagaggag gctggcttga agaacaatga tctggtagtt    900
gctgtcaacg gcgagtctgt ggaaaccctg gatcatgaca gtgtggtaga aatgattaga    960
aagggtggag atcagacttc actgttggtg gtagacaaag agacggacaa catgtacaga   1020
ctggctcatt tttctccatt tctctactat caaagtcaag aactgcccaa tggctctgtc   1080
aaggaggctc cagctcctac tcccacttct ctggaagtct caagtccacc agatactaca   1140
gaggaagtag atcataagcc taaactctgc aggctggcta aaggtgaaaa tggctatggc   1200
```

tttcacttaa atgcgattcg gggtctgcca ggctcattca tcaaagaggt acagaagggc 1260
ggtcctgctg acttggctgg gctagaggat gaggatgtca tcattgaagt gaatggggtg 1320
aatgtgctag atgaacccta tgagaaggtg gtggatagaa tccagagcag tgggaagaat 1380
gtcacacttc tagtctgtgg aaagaaggcc tatgattatt tccaagctaa gaaaatccct 1440
attgtttcct ccctggctga tccacttgac acccctccag attctaaaga aggaatagtg 1500
gtggagtcaa accatgactc gcacatggca aaagaacggg cccacagtac agcctcacat 1560
tcttcttcca attctgaaga tacagagatg tgatgaaaac aagtaatagc tttggctgtt 1620
tatttgatag ctgtttctgg gtatttaata ggaatccttt ctcaaggaat gagttgtgac 1680
ctgtttactg tctctttaga agaaaaactc cactggaaac cattcaccat gtgtgactgt 1740
cttctgttat catttgtctt acaggcggct attgcagacg gctaatttat gcttaactta 1800
ggaagagata aggcaagagc tagatttttt tcatgtgatc ttttccaagc ttcaacttaa 1860
cttaactaca tttctctgta tgatgatgtc tcttacttct acaggttcct tgagcaccaa 1920
agatgattca taactctgta taggtgacag ctgcttataa aagcatctta gcagataagc 1980
ctattaaaat tgtgcttttg taacaatgtt gtggttgcta gaataaatac catgaacccg 2040

<210> 21
<211> 633
<212> DNA
<213> Homo sapiens

<400> 21

tttaaaagcc actaattatc tgttttttat tttgtaagta acaagatata gacatttgaa 60
tgccaatgtc ttattctgga gagacactgg agctgaagtt caacaatgat cacacttatt 120
acctggcaat aaaaacacaa ccatctttcc agtcaggtca aaatatccta ctttttgcct 180
ttctaccaaa tcccaaacat tcacagtttt tcaaggacca ctaataaaat acaggaagct 240
tttaaagaca gtaagagaac acctagtgta agttaggtga attaaagatg gcaaaggaga 300
ttacatcctc aacactgaca gcttccaaga cttagaaaag agattgttcc ttgcttctaa 360
aattgttcta ttttcctctg taggaaaatg aaagtttttt cttacaaata ttaaataatc 420
aaagtactta cgcaaaatta atctgctcct caatgagatg agcactccat ttaaatgatc 480
tttacagatc cctgaagttg ctgtcctgtc actgtattta agtgatggat attcaattga 540
attattctgc ataaataatt ctggtcaacc cagacgtata gtagtatgat gggtcagata 600
cagtcaactg ttcaataaaa atgcagatgt ctg 633

<210> 22
<211> 1878
<212> DNA
<213> Homo sapiens

<400> 22

```
ctgcgtttct cctcaaacct aacgatgccg ccggagcgga ggagacgaat gaaactggac     60
cggagaaccg gagcgaagcc gaagcggaag cccggaatga ggccggactg gaaagccgga    120
gcggggccag gcgggcctcc ccaaaagcct gccccttcat cccagcggaa accgccggcc    180
cggccgagcg cggcggccgc tgcgattgca gtcgcggcgg cggaggaaga gagacggctc    240
cggcagcgga accgcctgag gctggaggag gacaaaccgg ccgtggagcg gtgcttggag    300
gagctggtct tcggcgacgt cgagaacgac gaggatgcgt tgctgcggcg tctgcgaggc    360
ccgagggttc aagaacatga agactcgggt gactcagaag tggagaatga agcaaaaggt    420
aattttccac ctcaaaagaa gccagtttgg gtggatgaag aagatgaaga tgaggaaatg    480
gttgacatga tgaacaatcg gtttcggaag gatatgatga aaaatgctag tgaaagtaaa    540
ctttcgaaag acaaccttaa aaagagactt aaagaagaat tccaacatgc catgggagga    600
gtacctgcct gggcagagac tactaagcgg aaaacatctt cagatgatga aagtgaagag    660
gatgaagatg atttgttgca aaggactggg aatttcatat ccacatcaac ttctcttcca    720
agaggaatct tgaagatgaa gaactgccag catgcgaatg ctgaacgtcc tactgttgct    780
cggatctcat ctgtgcagtt ccatcccggt gcacagattg tgatggttgc tggattagat    840
aatgctgtat cactatttca ggttgatggg aaaacaaatc ctaaaattca gagcatctat    900
ttggaaaggt ttccaatctt taaggcttgt tttagtgcta atggggaaga agttttagcc    960
acgagtaccc acagcaaggt tctttatgtc tatgacatgc tggctggaaa gttaattcct   1020
gtgcatcaag tgagaggttt gaaagagaag atagtgagga gctttgaagt ctccccagat   1080
gggtccttct tgctcataaa tggcattgct ggatatttgc atttgctagc aatgaagacc   1140
aaagaactga ttggaagcat gaaaattaat ggaagggttg cagcatccac attctcttca   1200
gatagtaaga aagtatacgc ctcttcgggg gatggagaag tttatgtttg ggatgtgaac   1260
tcaaggaagt gccttaacag atttgttgat gaaggcagtt tatatggatt aagcattgcc   1320
acatctagga atggacagta tgttgcttgt ggttctaatt gtggagtggt aaatatatac   1380
aatcaagatt cttgtctcca agaaacaaac ccaaagccaa taaaagctat aatgaacttg   1440
gttacaggtg ttacttctct gaccttcaat cctactacag aaatcttggc aattgcttca   1500
gaaaaaaatg aaagaagcag tcagattggt tcatcttcct tcctgtacag tattttcaaa   1560
cttcccagtc attaaaaata agaatatttc tcatgttcat accatggatt tttctccgag   1620
aagtggatac tttgccttgg ggaatgaaaa gggcaaggcc ctgatgtata ggttgcacca   1680
ttactcagac ttctaaagag actatttgaa gtccagttga gtcacaagag aagcctgtct   1740
tgatatatca tctcagaaac tttcctgaat atgtgataat atatggaaaa tgatttatag   1800
atccagctgt gcttaagagc cagtaatgtc ttaataaaca tgtggcagct tttgtttgaa   1860

aaaaaaaaaa aaaaaaaa                                                 1878
```

<210> 23
<211> 3426

<212> DNA
<213> Homo sapiens

<400> 23

```
aattccgggc ggcggcggcc gaggctgaag gaagatggcg gacggcgtgg accacataaa     60
catttacgcg gatgtcggcg aagagttcaa ccaggaagct gaatatggtg ggcatgatca    120
gatagatttg tatgacgatg tcatatctcc atctgcaaat aatggagatg ccccagaaga    180
ccgagattac atggatactc tcccaccaac tgttggtgat gatgtgggta aaggagcagc    240
accaaatgtt gtctatacat atactggaaa gagaattgca ttatatattg gaaatctaac    300
atggtggaca acagatgaag acttaactga agcagttcat tctttgggag taaatgatat    360
tttggagata aaattttttg aaaatcgagc aaatggccag tcaaaggggt ttgcccttgt    420
tggtgttgga tctgaagcat cttcaaaaaa gttaatggat ctgttaccta aaagagaact    480
tcatggtcag aatcctgttg taactccatg caataaacag ttcctgagtc aatttgaaat    540
gcagtccagg aaaactacac aatcaggaca aatgtctggg gaaggtaaag ctggtcctcc    600
aggaggcagt tcccgtgcag catttccaca aggtggtaga ggacggggcc gttttccagg    660
ggctgttcct ggtggggaca gatttcctgg gccagcagga ccaggagggc caccccccacc    720
ttttccagct ggacagactc caccacgtcc acccttaggt cctccaggcc cacctggtcc    780
accaggtcct ccacctcctg gtcaggttct gcctcctcct ctagctgggc ctcctaatcg    840
aggagatcgc cctccaccac cagttctttt tcctggacaa ccttttgggc agcctccatt    900
gggtccactt cctcctggcc ctccacctcc agttccaggc tacggccccc ctcctggccc    960
accacctcca caacagggac cacctccacc tccaggcccc tttccacctc gtccacccgg   1020
tccacttggg ccaccccctta cactagctcc tcctccgcat cttcctggac cacctccagg   1080
tgccccaccg ccagctccgc atgtgaaccc agctttcttt cctccaccaa ctaacagtgg   1140
catgcctaca tcagatagcc gaggtccacc accaacagat ccatatgggc gacctccacc   1200
atatgatagg ggtgactatg gcccccctgg aagggaaatg gatactgcaa gaacgccatt   1260
gagtgaagct gaatttgaag aaatcatgaa tagaaatagg gcaatctcaa gcagtgctat   1320
ttcgagagct gtgtctgatg ccagtgctgg tgattatggg agtgctattg agacactggt   1380
aactgcaatt tctttaatta aacaatccaa agtatctgct gatgatcgtt gcaaagttct   1440
tattagttct ttgcaagatt gccttcatgg aattgagtcc aagtcttatg gttctggatc   1500
aagacgtgaa cgatcaagag agagggacca tagtagatca cgagaaaaga gtcgacgtca   1560
taaatcccgt agtagagacc gtcatgacga ttattacaga gagagaagca gagaacgaga   1620
```

gaggcaccgg gatcgtgacc gagaccgtga ccgagagcgt gaccgagagc gcgaatatcg 1680
tcatcgttag aagctgaagg aagaggatca ccttccaaga caaaacagtc ttcatgggcc 1740
aaaaatgacg cttgtccagc agtttgcttc ttgtgattga actgaacctg taaggattca 1800
tggataaaat gaacaggaat agatctgaat aaagcaaatc tgcataaatg gtaaccagta 1860
gctctacttt tattttttat gttgcttaac tgttttattt gaaggaaacc tgtgtgattt 1920
aaaaagttat agcttttgca actttattac tggttatata catttggcca ttatgatgtg 1980
caagcaattg gaaaaaaagt caagtaaatg cttgtttttg tagtagtttg ttcttgttaa 2040
aaatgtttat atgataatgt ctgtaaacag catcactttg attacaatag atgtagtgtt 2100
gtaataaact gtttaatggg gctgatgtgt aaagctgttc aagttatttg atgtttacac 2160
ctcagggaaa gtcttgtgtt cagcaatatc taaagataat gttactatga caacattttt 2220
actgtccttt aaagcattgc aatagcgttt ttggatatgc ctcaatctaa tcttgcgttc 2280
agtgaattaa acatagtaat taagtgtctt ttgcccttga ttttgatatt agaataggtg 2340
attacatgga tatttaatat ttctatattc tgcttttcta gctgttttta cctagttagc 2400
ttgtgacttt gctgaatggt atgtaaactt gtaaaaatag agatttgaca gacatagcaa 2460
tctagtcaat gtgtaagggg tcaaaaaaaa cagaggtttt aacacataag taaaaacccg 2520
tacatatttg atgtgtaatg caggttaatt acaacacaga tgtaccgaaa cacttaattg 2580
tgaaccgcta acattgaaga aattttgaca attccgattt gatgctgcaa ttacttgctg 2640
tttttattga tcttatggtt tatttcttaa gccatagtca gtgtaaatac agccctgcag 2700
caggtaaatg tgagtaaaga gagccttata ttttccaatt ggtataaaat ttttgaagga 2760
tgtgatgttc attaacattc ggttgtattc cccagtattt gtaatgggaa attacagata 2820
aaccgtgtct gcacagttta aggaatacta tgtatattca tgcaccgtat tgattcatgc 2880
tatagttact taatcaaaga tttttttcaa acctgcctta catataggcc cactttaaaa 2940
gcacctgact agcatgtgtt cttgattgca aaattggcag aggcagggtg tcaacttgat 3000
taggtgtttt tatgggaatg taatttgaaa tcactacttc agaaatttga cttaaaattc 3060
ttgagcacgt taatatgttt ttaagatctg attatctttg agagatcttc tgttaataca 3120
cattggttgt taaagagtac ccaaattcta ggacaatgct taaagtgtta aaatacccta 3180
gatactgtgt tatgtgcaac tgtagaaacc ctccagaaat ttccactgct gttcttcact 3240
ttcatcttgt ctgctatcaa accacttctg acaaaattag ctgttttgaa ttacccatat 3300
cactgccagt tttattttaa aatattttgt gtttgaagta tctgtgcatg ggatcgttga 3360
tgtttatcag aactgttcac tttcagaaat gattttttaa agcattttgt tgaaatgcgg 3420
ttgctt 3426

<210> 24
<211> 5401
<212> DNA
<213> Homo sapiens

<400> 24

```
tcactctcgc tgccgctgct ccgccccatc cccttctgtt tttctctctc attctccagt     60
ggcggcggcg gggaaggcgg aggcagaggc agcagcagcc gcgctggctg caatgaatga    120
tcccccagct tggggggagg actccaggtg agcctctgcc ctcgggaggc ccgggacccc    180
cggccgccca cgaccggcag cccacgctat ggatccctag aggaaggagg agaagacagc    240
tcgccgccca cccccatccc attttcctct tcctttatct cattgttgcc gaagctgttt    300
acggcagcgc tccctctgct ccagcatggg gcgggctccg ggcacggctg ctcggcaggc    360
gctgctcccg cggcgactgg gggattctgc ctaattcacc tcccagccgg tgcagagagg    420
accggagagc ggtggaggcc cggactgcag cagcgttggg gccacctccc agcgtcccca    480
ccctaggagg ctgcatgcgg attgaagagc tgcgcctggg ggctgggccg gccccgctga    540
tcccgaccta gcgagcagga tagcaggacc gcccaggctg cggaggggct cgggggcagg    600
aaggtcagag cagcaagatg gccagtaaga ccaaggccag cgaggccctc aaggtggtgg    660
cccggtgccg ccccctcagc aggaaggagg aggctgctgg tcacgagcag atcctgacca    720
tggacgtgaa actgggccag gtgaccctgc ggaacccccg cgccgccccg ggggagctgc    780
ccaagacctt cacctttgac gccgtgtatg atgccagctc caagcaggcc gacctgtatg    840
acgaaaccgt gaggcccctg atagactccg tgctccaggg tttcaatggc acggtgtttg    900
cctatggcca gacgggcact ggcaagacct ataccatgca ggggacctgg gtggagcccg    960
agctgcgcgg ggtcatcccg aatgcctttg agcacatctt cacccacatc tcccgctccc   1020
agaaccaaca gtacctggtc cgggcctcct atttggagat ctaccaggaa gagattcgag   1080
acctgctctc caaggagccg ggcaagaggc tagagctgaa agagaacccc gagactggcg   1140
tctacatcaa ggacctctcc tccttcgtca ccaagaatgt caaggagatt gagcatgtga   1200
tgaacctggg gaaccagacc cgggctgtgg gcagcaccca catgaatgag gtcagctccc   1260
gctcccatgc catcttcatc atcactgtgg agtgcagcga acgtggctct gatggccagg   1320
accacatccg agtgggcaag ctcaacctcg tggacctggc tggcagcgag aggcagaaca   1380
aggcaggccc caacacagcg ggaggggcag ccacaccatc ctcgggtggc ggtggtggcg   1440
gtggaggcag tggtggtggt gctggtggag agaggcctaa ggaagcctcc aaaatcaacc   1500
tctcattatc tgccctgggc aacgtgattg ctgccctggc gggcaacagg agcacccaca   1560
ttccctaccg ggactccaag ctgacccggc tgctccagga ctccctgggg gggaatgcca   1620
agaccatcat ggtagccaca ctggggccag cttctcacag ctacgatgag agcctctcca   1680
```

ccttgcgctt tgccaaccga gccaagaaca tcaagaacaa gccccgggtg aacgaggacc 1740
ccaaggacac actgctgcgg gaattccaag aggagattgc ccgcctgaag gcccagctgg 1800
agaagagggg gatgctgggg aagcggcccc ggaggaagag cagccgcagg aagaaggccg 1860
tgtccgcccc gcctgggtac cctgagggcc cagtgattga ggcctgggtg gcagaagagg 1920
aggatgacaa caacaacaac caccgcccgc cccagcccat cctggagtca gccttggaga 1980
agaacatgga gaattacctg caggaacaga aggagcggct ggaggaggag aaggcagcca 2040
tccaggatga ccgcagcctg gtgagcgagg agaagcagaa gctgctggag gagaaggaga 2100
agatgctgga ggacctgcgg cgggaacagc aggccacaga gctgcttgcg gccaagtaca 2160
aggccatgga gagcaagctc ctcatcgggg gcaggaacat catggatcac accaacgaac 2220
agcagaagat gttggaactg aagaggcagg agattgccga gcagaaacgt cgtgagcggg 2280
agatgcagca ggagatgatg ctccgggacg aggagactat ggagctccgg ggcacctaca 2340
catccctgca gcaggaggtg gaggtcaaaa ccaagaaact caagaagctc tacgccaagc 2400
tgcaggcggt gaaggcggag atccaggacc agcatgatga gtatatccgc gtgcggcagg 2460
acctggagga ggcgcagaac gagcagaccc gcgaactcaa gctcaagtac ctaatcatcg 2520
agaacttcat cccgccggag gagaagaaca agatcatgaa ccggcttttc ctggactgtg 2580
aggaggagca gtggaagttc cagccactgg tgccagccgg cgtcagtagc agccagatga 2640
agaagcggcc aacatctgca gtgggctaca agaggcctat cagccagtat gctcgggttg 2700
ccatggcaat ggggtcccac cccaggtaca gggctgaaaa cataatgttt ctggagttgg 2760
atgtgtcccc tccagctgtc tttgagatgg aattctctca cgaccaagaa caagaccctc 2820
gtgcgctaca catggagagg ctcatgcgat tggacagctt tctggaaaga ccttccacgt 2880
ctaaagtccg aaagtccaga tcctggtgcc agagtcctca gcggcctcca ccttccacca 2940
cacatgcctc cctggcctct gcttctctgc gccctgcaac agtggcggac catgagtgac 3000
aaccatcacg tcaggctgcc catccaatag actcctggga tggggcagcc aaccctggct 3060
catctcatct gccgcttggt gcgtgtgcgt gtgcgtgcat gtgcgtgtgc gtgtgtgcag 3120
gggtgagaat ctggcagatg gtgcctctgc ctgctcttct tcgcctcctt tatttaattc 3180
atgttattta ttcgcggagc tctgttcgtg ttggggagat gccctcgcct gagccgtctg 3240
ggcctaccgt ggtcactgcg tagcctcttt ttcttctgac ttgagagctc ccccagtcag 3300
atctcaggct tgtccccctg tcagctgcct ccagaaggga aggtagccag tgcctgagaa 3360
gacagtccct tttctaccca ccgcactcca taacctccat cttctcccac actgatggcg 3420
agcagcccct gagcactttc tgggactggg agactgcttg gtgttccctg aggacaagag 3480
acatcctgac agtgttgggc atctgctccc cgtggacaca gccccactct ccactttctg 3540
agcctcagac aacctcattc agcctcttgg gctccttttc aaggacatta ataacctcac 3600

caacatagct catgcccttc agctttgaca agaactcacg gcttcccaaa ctctgctttc 3660
tgcccacctt ggatgggaac tgtggaccaa gcaattacca tcgccttgga acctgcagga 3720
aatggaacag caattgagac aacttgaaca gtcatcaacg gaagtccctc cactggattc 3780
ctttgtttct gtcccctccg aggagtcatt ttggtcgaca ggctctcaag gcaactcccc 3840
attttcaaga ggctgctcct gcctgcttcg atcatttctc cctgcagctg cctagacccc 3900
gttcacagtg ggaggagtca atgtcattct acccctcgct aaacgaagat attaacatct 3960
attgcttttt cccttcatct gtcacaggaa acagaagccc aggcacaatc ttttccagct 4020
ttgcctgtta cccctgtttc tgaattgcat ctttaaggta ttattttgtt gacaatagat 4080
cctttattca ctagttacgc aaattggttc ctagggggat actccttacc ttcctttgtg 4140
atggcccaaa atgtctctag gtatctcaag tgataagtaa atttctacaa aaaaaaatgg 4200
ttaatgttca ttgactggct ttttaagtgt atattttgga ggacgggtga agaggtcata 4260
acgaaagcaa gcgagtgaat taggatttca aagtgcccta atagtgtgag tctccagttc 4320
ctagaatatg aagagtgctg tcgttggggt gaaaccatga gactgacaga tctgcctgaa 4380
atggggggtg tgggaggtgg tggcgggggt tattctcttt ccttcaggaa atgaaccctt 4440
cttacatcat tcaagttctg ctctgaggat caagcttggg tctgatttaa ctcagcgaca 4500
ctgtcatttc tgcttcatta ctggactaga gggttgagcc acccacttgc catttgctcc 4560
tgtccttcca ggaaatcaca attttcatca gagcccaaga gattatttga gactcaggat 4620
tcagatcaga ggttcgactg tggctgggac aggagttgtg tgtagaaatt caccaggtgg 4680
cctgagcgca gggggacctc cagggctgcg ttgagcagcc tctcccactg acctctttct 4740
cgtttgtgga caaagcagca cgtatcacct cattcatcac ttggacacat cgcctttgca 4800
ttgtcttgtc acacctccct cacagtctta tagcacaata tacccaaatc agcccccca 4860
gtccgagggc tgggcccaag gtatggtcgg aggaggagct cctgcctgcg gttttgtgta 4920
tgtgtgtatg tgtgtgcgtg tttgtgtgcg tgtttacctc cacaggggac actctacact 4980
cagtgtaaga tctgctggga acagggccac caggagtggc tggatctcag tctctctgtc 5040
tctctttctc tccttttcct tttggtgtat caaatatttg attgacaaag taagggcctt 5100
gattaggacc aaattctcgt gtgttgctat ggtctttatt taggacaaca attaacaatg 5160
cagtggccca ttcttgtcac tctacacata tgactatacg ggacatatgt aatatataaa 5220
tatatatata aaacattccc ctctgtcccc ttggcttcgg atggaggcct ttctgttgag 5280
ctgaaatgca cctgcagctg ggtgctgcca gcagcttgca ggccccagcc ctgttccaat 5340
caatgcagtt gacaataaag gaatgagtat cgtcacggaa aaaaaaaaaa aaaaaaaaaa 5400
a 5401

<210> 25
<211> 1280
<212> DNA
<213> Homo sapiens

<400> 25

```
taaacaatgg tatcaacgca aagtaagcgg gcagccgcct gcatctgtat ccagcgccag      60
gtcccgccag tcccagtgcg cgcgcccccc agtcccgcac ccgttcggcc aggctaagtt     120
agccctcacc atgcggtcaa aggaggcagc aagtgcatca aatacctgct gttcggattt     180
aacttcatct tctggcttgc cgggattgct gtccttgcca ttggactatg ggtccgattc     240
gactctcaga ccaagagcat cttcgagcaa gaaatttcct aataataata attccagctt     300
ctacacagga gtctatattc tgatcggagc cggcgccctc atgatgctgg tgggcttcct     360
gggctgctgc ggggctgtgc aggagtccca gtgcatgctg ggactgttct tcggcttcct     420
cttggtgata ttcgccattg aaatagctgc ggccatctgg ggatattccc acaaggatga     480
ggtgattaag gaagtccagg agttttacaa ggacacctac aacaagctga aaaccaagga     540
tgagccccag cgggaaacgc tgaaagccat ccactatgcg ttgaactgct gtggtttggc     600
tgggggcgtg gaacagttta tctcagacat ctgccccaag aaggacgtac tcgaaacctt     660
caccgtgaag tcctgtcctg atgccatcaa agaggtcttc gaccaataaa ttccacatca     720
tcggcgcagt gggcatcggc attgccgtgg tcatgatatt tggcatgatc ttcagtatga     780
tcttgtgctg tgctatccgc aggaaccgcg agatggtcta gagtcagctt acatccctga     840
gcaggaaagt ttacccatga agattggtgg gatttttttgt ttgtttgttt tgttttgttt     900
gttgtttgtt gtttgttttt ttgccactaa ttttagtatt cattctgcat tgctagataa     960
aagctgaagt tactttatgt ttgtctttta atgcttcatt caatattgac atttgtagtt    1020
gagcgggggg tttggtttgc tttggtttat attttttcag ttgtttgttt ttgcttgtta    1080
tattaagcag aaatcctgca atgaaaggta ctatatttgc tagactctag acaagatatt    1140
gtacataaaa gaattttttt gtctttaaat agatacaaat gtctatcaac tttaatcaag    1200
ttgtaactta tattgaagac aatttgatac ataataaaaa attatgacaa tggccaaaaa    1260
aaaaaaaaaa aaaaaaaaaa                                                1280
```

<210> 26
<211> 3345
<212> DNA
<213> Homo sapiens

<400> 26

```
gcggccgggg cctggggctg cctgccgggc ggccgggcgc ggcgagccca gggaggcagc      60
gtccatggag caaaaggaat gccaggatcc tgcacaggca gacgcgggcc agcctcagca     120
ccgacagccg acgcgcagat agcagagcca tccttggggt tgaaccatga ttccggtgac     180
```

agagctccgc tactttgcgg acacgcagcc agcataccgg atcctgaagc cgtggtggga 240
tgtgttcaca gactacatct ctatcgtcat gctgatgatt gccgtcttcg gggggacgct 300
gcaggtcacc caagacaaga tgatctgcct gccttgtaag tgggtcacca aggactcctg 360
caatgattcg ttccggggct gggcagcccc tggcccggag cccacctacc ccaactccac 420
cattctgccg acccctgaca cgggccccac aggcatcaag tatgacctgg accggcacca 480
gtacaactac gtggacgctg tgtgctatga gaaccgactg cactggtttg ccaagtactt 540
cccctacctg gtgcttctgc acacgctcat cttcctggcc tgcagcaact tctggttcaa 600
attcccgcgc accagctcga agctggagca ctttgtgtct atcctgctga agtgcttcga 660
ctcgccctgg accacgaggg ccctgtcgga gacagtggtg gaggagagcg accccaagcc 720
ggccttcagc aagatgaatg ggtccatgga caaaaagtca tcgaccgtca gtgaggacgt 780
ggaggccacc gtgcccatgc tgcagcggac caagtcacgg atcgagcagg gtatcgtgga 840
ccgctcagag acgggcgtgc tggacaagaa ggagggggag caagccaagg cgctgtttga 900
gaaggtgaag aagttccgga cccatgtgga ggagggggac attgtgtacc gcctctacat 960
gcggcagacc atcatcaagg tgatcaagtt catcctcatc atctgctaca ccgtctacta 1020
cgtgcacaac atcaagttcg acgtggactg caccgtggac attgagagcc tgacgggcta 1080
ccgcacctac cgctgtgccc acccccctggc cacactcttc aagatcctgg cgtccttcta 1140
catcagccta gtcatcttct acggcctcat ctgcatgtat acactgtggt ggatgctacg 1200
gcgctccctc aagaagtact cgtttgagtc gatccgtgag gagagcagct acagcgacat 1260
ccccgacgtc aagaacgact tcgccttcat gctgcacctc attgaccaat acgacccgct 1320
ctactccaag cgcttcgccg tcttcctgtc ggaggtgagt gagaacaagc tgcggcagct 1380
gaacctcaac aacgagtgga cgctggacaa gctccggcag cggctcacca agaacgcgca 1440
ggacaagctg gagctgcacc tgttcatgct cagtggcatc cctgacactg tgtttgacct 1500
ggtggagctg gaggtcctca agctggagct gatccccgac gtgaccatcc cgcccagcat 1560
tgcccagctc acgggcctca aggagctgtg gctctaccac acagcggcca agattgaagc 1620
gcccgcgctg gccttcctgc gcgagaacct gcgggcgctg cacatcaagt tcaccgacat 1680
caaggagatc ccgctgtgga tctatagcct gaagacactg gaggagctgc acctgacggg 1740
caacctgagc gcggagaaca accgctacat cgtcatcgac gggctgcggg agctcaaacg 1800
cctcaaggtg ctgcggctca agagcaacct aagcaagctg ccacaggtgg tcacagatgt 1860
gggcgtgcac ctgcagaagc tgtccatcaa caatgagggc accaagctca tcgtcctcaa 1920
cagcctcaag aagatggcga acctgactga gctggagctg atccgctgtg acctggagcg 1980
catcccccac tccatcttca gcctccacaa cctgcaggag attgacctca aggacaacaa 2040

cctcaagacc atcgaggaga tcatcagctt ccagcacctg caccgcctca cctgccttaa 2100
gctgtggtac aaccacatcg cctacatccc catccagatc ggcaacctca ccaacctgga 2160
gcgcctctac ctgaaccgca acaagatcga gaagatcccc acccagctct tctactgccg 2220
caagctgcgc tacctggacc tcagccacaa caacctgacc ttcctccctg ccgacatcgg 2280
cctcctgcag aacctccaga acctagccat cacggccaac cggatcgaga cgctccctcc 2340
ggagctcttc cagtgccgga agctgcgggc cctgcacctg ggcaacaacg tgctgcagtc 2400
actgccctcc agggtgggcg agctgaccaa cctgacgcag atcgagctgc ggggcaaccg 2460
gctggagtgc ctgcctgtgg agctgggcga gtgcccactg ctcaagcgca gcggcttggt 2520
ggtggaggag gacctgttca acacactgcc acccgaggtg aaggagcggc tgtggagggc 2580
tgacaaggag caggcctgag cgaggccggc ccagcacagc aagcagcagg accgctgccc 2640
agtcctcagg cccggagggg caggcctagc ttctcccaga actcccggac agccaggaca 2700
gcctcgtggc tgggcaggag cctggggccg cttgtgagtc aggccagagc gagaggacag 2760
tatctgtggg gctggcccct tttctccctc tgagactcac gtcccccagg gcaagtgctt 2820
gtggaggaga gcaagtctca agagcgcagt atttggataa tcagggtctc ctccctggag 2880
gccagctctg ccccaggggc tgagctgcca ccagaggtcc tgggaccctc actttagttc 2940
ttggtattta tttttctcca tctcccacct ccttcatcca gataacttat acattcccaa 3000
gaaagttcag cccagatgga aggtgttcag ggaaaggtgg gctgcctttt ccccttgtcc 3060
ttatttagcg atgccgccgg gcatttaaca cccacctgga cttcagcaga gtggtccggg 3120
gcgaaccagc catgggacgg tcacccagca gtgccgggct gggctctgcg gtgcggtcca 3180
cgggagagca ggcctccagc tggaaaggcc aggcctggag cttgcctctt cagtatttgt 3240
ggcagtttta gtttttttgtt tttttttttt taatcaaaaa acaatttttt taaaaaaaaa 3300
gctttgaaaa tggatggttt gggtattaaa aaaaaaaaaa aaaaa 3345

<210> 27
<211> 4762
<212> DNA
<213> Homo sapiens

<400> 27

atgcggcgcg gccccggagg cagcagcagc ggcggcggca gccggagcag taggcacccg 60
agcagcgcca gcggccgagc gggcggcttc ctggcctggg cgctccggtg gcggcggagg 120
tgcgcgcgga gccatggtta tcatgtcgga gttcagcgcg gaccccgcgg gccagggtca 180
gggccagcag aagcccctcc gggtgggttt ttacgacatc gagcggaccc tgggcaaagg 240
caacttcgcg gtggtgaagc tggcgcggca tcgagtcacc aaaacgcagg ttgcaataaa 300
aataattgat aaaacacgat tagattcaag caatttggag aaaatctatc gtgaggttca 360

gctgatgaag cttctgaacc atccacacat cataaagctt taccaggtta tggaaacaaa 420
ggacatgctt tacatcgtca ctgaatttgc taaaaatgga gaaatgtttg attatttgac 480
ttccaacggg cacctgagtg agaacgaggc gcggaagaag ttctggcaaa tcctgtcggc 540
cgtggagtac tgtcacgacc atcacatcgt ccaccgggac ctcaagaccg agaacctcct 600
gctggatggc aacatggaca tcaagctggc agattttgga tttgggaatt tctacaagtc 660
aggagagcct ctgtccacgt ggtgtgggag ccccccgtat gccgccccgg aagtctttga 720
ggggaaggag tatgaaggcc cccagctgga catctggagc ctgggcgtgg tgctgtacgt 780
cctggtctgc ggttctctcc ccttcgatgg gcctaacctg ccgacgctga gacagcgggt 840
gctggagggc cgcttccgca tccccttctt catgtctcaa gactgtgaga gcctgatccg 900
ccgcatgctg gtggtggacc ccgccaggcg catcaccatc gcccagatcc ggcagcaccg 960
gtggatgcgg gctgagccct gcttgccggg acccgcctgc cccgccttct ccgcacacag 1020
ctacacctcc aacctgggcg actacgatga gcaggcgctg ggtatcatgc agaccctggg 1080
cgtggaccgg cagaggacgg tggagtcact gcaaaacagc agctataacc actttgctgc 1140
catttattac ctcctccttg agcggctcaa ggagtatcgg aatgcccagt gcgcccgccc 1200
cgggcctgcc aggcagccgc ggcctcggag ctcggacctc agtggtttgg aggtgcctca 1260
ggaaggtctt tccaccgacc ctttccgacc tgccttgctg tgcccgcagc cgcagacctt 1320
ggtgcagtcc gtcctccagg ccgagatgga ctgtgagctc cagagctcgc tgcagtggcc 1380
cttgttcttc ccggtggatg ccagctgcag cggagtgttc cggccccggc ccgtgtcccc 1440
aagcagcctg ctggacacag ccatcagtga ggaggccagg caggggccgg gcctagagga 1500
ggagcaggac acgcaggagt ccctgcccag cagcacgggc cggaggcaca ccctggccga 1560
ggtctccacc cgcctctccc cactcaccgc gccatgtata gtcgtctccc cctccaccac 1620
ggcaagtcct gcagagggaa ccagctctga cagttgtctg accttctctg cgagcaaaag 1680
ccccgcgggg ctcagtggca ccccggccac tcaggggctg ctgggcgcct gctccccggt 1740
caggctggcc tcgcccttcc tggggtcgca gtccgccacc ccagtgctgc aggctcaggg 1800
gggcttggga ggagctgttc tgctccctgt cagcttccag gagggacggc gggcgtcgga 1860
cacctcactg actcaagggc tgaaggcctt tcggcagcag ctgaggaaga ccacgcggac 1920
caaagggttt ctgggactga acaaaatcaa ggggctggct cgccaggtgt gccaggtccc 1980
tgccagccgg gccagcaggg gcggcctgag cccccttccac gcccctgcac agagcccagg 2040
cctgcacggc ggcgcagccg gcagccggga gggctggagc ctgctggagg aggtgctaga 2100
gcagcagagg ctgctccagt tacagcacca cccggccgct gcacccggct gctcccaggc 2160
cccccagccg gcccctgccc cgtttgtgat cgccccctgt gatggccctg gggctgcccc 2220
gctccccagc accctcctca cgtcggggct cccgctgctg ccgcccccac tcctgcagac 2280

cggcgcgtcc ccggtggcct cagcggcgca gctcctggac acacacctgc acattggcac 2340
cggccccacc gccctccccg ctgtgccccc accacgcctg gccaggctgg ccccaggttg 2400
tgagcccctg gggctgctgc agggggactg tgagatggag gacctgatgc cctgctccct 2460
aggcacgttt gtcctggtgc agtgagggca gccctgcatc ctggcacgga cactgactct 2520
tacagcaata acttcagagg aggtgaagac atctggcctc aaagccaaga actttctaga 2580
agcgaaataa gcaatacgtt aggtgttttg gctttttagt ttatttttgt tttatttttt 2640
tcttgcactg agtgacctca actttgagta gggactggaa actttaggaa gaaagataat 2700
tgaggggcgt gtctgggggc gggggcagga ggggagcggg gtggagggaa cacgtgcagt 2760
gccgtggtgt ggggatctcg gcccctctct ctgggttcgt cgtggttgag atgattacct 2820
cggacgtcta cggaaacgag cgggcgcatt gttgtccgct tgtgtgtgtg tgtgtgtgtg 2880
tgtgtgtgcg cgtgcattga ttactatcca tttctttagt caacgctctc cacttcctga 2940
tttctgcttt aaggaaaact gtgaactttc tgcttcatgt atcagtttta aagcagccca 3000
ggcaaagatc atctacagat tctaggaatt ctctcccctg aaatcaaaac ctggaagact 3060
tttttttctt attttagttg agaagtttca taaactgctc aaggattagt tttccaggac 3120
tctgcggagg aacggcagga agaacctcag agagggcaga ggtgacttca aagtgctggg 3180
gactccgtcc tgagggtcac ttggccctga gcccctgcgt gcccttgcgg aagcccagaa 3240
gcttcttcct gctgcacctc ccgtttccgc tgctgctgac gtttatgcat ttcatgatgg 3300
ggtccaacaa gaacacctga cttgggtgaa gttgtgcaat attggaggct gactgtaggg 3360
ctgggcagct gggagacagg ctcatggctc atggctcatg gctcagggcg gtgcctgcca 3420
tgggccggga cccccctccc cacccccac ctaggctttt tgggttttgt tcaaggaagg 3480
taaagtgaga ggtttaggtc agtgttttta agtttttgtt tttttttaa agcaaatcct 3540
gtatatgtat ctacatggga gacaggtaga cactacttat ttgttacatt ttgtactaca 3600
cgtttgtgtt ccaggtttca gcttccctcg ctcctgttgt taagaagcgt ccctgtcagc 3660
acaggtgtgc attgaggaag gggccccagg gccttcgctc cctcagcact ggggtggagg 3720
cggcaggaag gggcggccct tacctggcag gtctgggcgc acctttagca ggtggactcc 3780
gtggggctcc accagccaga agcctttgga aggcaacgaa ggcaatgctg ctccctgagt 3840
ccagtccccg cccccaaacc cagcccaggt gccttcagct acttcggctt cttaaacccct 3900
gcagtgttaa acagaggcat tgagaaaggg gaaaggcggg tatttttaaa agccaaagat 3960
tgacccagtt acttgagggt agggaggcgg gcccagtgca ggaggctgca tccctggcct 4020
gctggtgccc accgggggct gtgcctgtgc cgggccgcag ggaagctggc tgcccccatt 4080
cctgctgctg ctgctgctgc tgctctgtgg ctgtttcaaa gactgggcga aaggctgtcc 4140

ggagggcaga ccaggtgcct tgccgcagag aaaacaccaa agtctcctgt tcgctcataa 4200
agaagttttt gggatgggag agaatccaga ccatcttggg gcagccaggc ccttgccttc 4260
atttttacag aggtagcaca attgattcca acacaaaact tccccttttt aaaatgattt 4320
ctgttctaat gccatagatc aaaggcctca gaaaccattg tgtgtttcct ctttgaagca 4380
atgacaagca ctttactttc acggtggttt ttgtttttc ttattgctgt ggaacctctt 4440
ttggaggacg ttaaaggcgt gttttacttg ttttttaag agtgtgtgat gtgtgttttg 4500
tagatttctt gacagtgctg taatacagac ggcaatgcaa tagcctattt aaagacacta 4560
cgtgatctga ttgagatgta catagttttt tttttacca taactgaatt attttatctc 4620
ttatgttaac atgagaaatg tatgccaaat gattagttga tgtatgtttt ttaatttaat 4680
atttaaataa aatatttggg agtataaaaa aaaaaaaaaa aaaaaaaaaa aaaaaaaaaa 4740
aaaaaaaaaa aaaaaaaaaa aa 4762

<210> 28
<211> 3327
<212> DNA
<213> Homo sapiens

<400> 28

cacacctttc caaggacccc caaactctgc tccgtgcacg tcaaatgctc ctttcccttg 60
tgtccaaccc cctacccctc tccctaacac ccctcttctc aacaagactc agcctctccc 120
cgaggtgggt gagcatcctt gaggtttccc acccttaact gctgtgtccc cggatggagc 180
cagagaaatg tggtgggggg gccggggcag agtttcaaca ttgcccccca gaaggaggag 240
ccagagatgg ggtctgtcca ggaaaacagg atgccggagc ccaggagtcg tcagcctagc 300
agttgcctgg cctccagatg cctcccaggg gagcagatcc tagcatgggc cccaggggtg 360
aggaagggcc tggaaccaga attgtctgga accctgatct gtaccaactt tagggtcacc 420
ttccagccct gtggatggca gtggaatcag gacactccct tgaacagtga atacgatttt 480
gccctggtca acattggacg attagaggct gtgagcggct tgtcccgagt ccagctcctc 540
cgtccagggt ccctgcataa atttatccct gaggagattc tgattcatgg ccgagacttc 600
cggctgctca gagttggttt tgaggctgga ggcctagagc ctcaggcttt tcaggtgacc 660
atggccattg tccaagccag agctcagagc aatcaagccc aacagtattc ggggataacc 720
ctgagcaagg ctggccaggg ttctggctcc agaaaaccac caattcctct catggagaca 780
gcggaagact gggagactga gcggaagaag caggcagcca gaggctggag ggtcagcacg 840
gtcaacgaga ggttcgacgt agccaccagc ctcccccgtt acttctgggt ccctaaccga 900
attctggaca gtgaggtcag gagagcattt ggccactttc atcagggccg tggaccggtc 960
agtgtgatgg ttagggtaat ggctgtggat tagagggtca tgtgggccag ggacatcgtg 1020

gagggaggaa cctctgtgag gtcagtgtgg gggcaagggt agcgtggagc taggcatttc 1080
tcccacaatg accctcttct gccccatgtg aagcgcttgt cctggcatca ccctgggggc 1140
agtgatcttc tccgctgtgg aggcttctat acagccagtg accctaacaa ggaggatatc 1200
agagcagtgg agttgatgca ccaggctggg cattcagatg ttgtcctggt agacactatg 1260
gatgagctgc ccagccttgc agatgtccaa cttgcccacc tgaggctgag ggccctctgc 1320
ctgcctgatt catctgtagc tgaggataaa tgctttcagc cctggaagga acacgatggc 1380
tggactatgt cagggcttgt cttcgaaagg ccagtgacat ttcagtatta gtgacatcca 1440
gggttcgttc tgtaatactt caaggctccg gtgtttctcc tcttccttga ttgtgtctgg 1500
cagctcctcc agcagtttcc agctgatttt gaattctctg agtttttcct tcttgctctt 1560
catgacagtg tcagggttcc tgacaccctt accttcctga gaaatacccc ctgggagcgc 1620
ggaaagcaga gcggacaggt cagtgacttc tatttttgac tcgtgttttt ttttccattg 1680
agatgtactc tctgaagttt ggtcttgatt tgttttatga gaagtgaggt ctgtgagtgg 1740
ggaggggggag atttattctc attttcagga cgagactttt gccctacatc tttcctagaa 1800
taagaggtga gaatctcatg atttgtctct agatgtggga ggattgtgtg taaccatcct 1860
ttttcttgct tcctctgtcc agttaaactc ctatacacaa gtctacaccc caggatactc 1920
cagcctccag ctgggaactc ttttaacctg cagctgtctg tctgggactg ggatttacgt 1980
tatagcaatg cacagatact acaattccag aatcctggct atgacccaga acactgtcca 2040
gattcctggc tccctagacc acagccaagc ttcatggttc ctggaccccc cagttttgtg 2100
tggctcttct ctagaggagc attgaccccc ctgaatcagc tctgtccttg gcgggacagt 2160
ccttccctgc tggcagtctc ttctcgttgg ctccctcgac ctgctatctc ctctgaaagc 2220
tggctgacca ggaatggggt ctccccctcac attggggagc ttgcccttta cctccagggc 2280
tgctgctgcc tgggtatctg ggaccccaga tcaggctctg gagacgctgc tacctgaggg 2340
gaaggcctga ggtccaggta agaagggaaa atagactggg agtgggacaa gggacttgac 2400
tctgctgaac cagatgaaca ggagctggaa aggcaaggag ctgaagcctc tgggagtctg 2460
ggaagtgaag ttctactcct cttggcatca aacaaggttt gggagtgtag gaggtgcggg 2520
aaagtgcttg tggcttagat taagtggaat ttagggcata gctgaaaggg gaaacagaat 2580
taaagacacc agaagtagca gagaagcagg gggccagagc tacaacagta ttcttctctg 2640
ttcctctttg cctcctcccc agatgggcct ctcatctccc acaatctctg gcctccagga 2700
tgagctatcc catcttcagg agttattacg gaaaggacac caagaatatc tcctgaggat 2760
cactccaaga aaagagatcc acataccatt ctcaatccca ctgaaattgc tggcattctc 2820
aaaggcaggg cagaggggga tctggggtag agggagggtt ctgtctaatc tttttttttt 2880
cttttgtatc tgcacttgca gcctcagctt tcatacttca gcccttaagt tcactaagaa 2940

ggtctgagtt tctgctgcag atagtggtgt taactgctcc aactcttgtc ttgcttagtt 3000
tctacaaata tttttgcttc ttgtcatttg aaggattaag aaacaaaaac aatccagaaa 3060
ttgatcggtt tttttaggcc aatcccatcc cttctggata accagatgtt aaatcatgag 3120
atcagagatg ctgttcatca gtcccaacaa gatggcctag aaatcgcatt ctcacctcgc 3180
cttgctgctg ctttaattcc aagttctatt tcttccctta tagttttcta tgggaatgag 3240
gcggatacag gaaacacccct atctcctctg tatttttgta gtggaatttc tatttaaggg 3300
gctcattaaa gcatagtatt tatacac 3327

<210> 29
<211> 3061
<212> DNA
<213> Homo sapiens

<400> 29

gagcaattga ttaatagctc ggcgagggga ctcactgact gttataataa cactacacca 60
gcaactcctg gcttcccagc agccggaaca cagacaggag agagtcagtg gcaaatagac 120
atttttctta tttcttaaaa aacagcaact tgtttgctac ttttatttct gttgattttt 180
ttttcttggt gtgtgtggtg gttgttttta agtgtggagg gcaaaaggag ataccatccc 240
aggctcagtc caacccctct ccaaaacggc ttttctgaca ctccaggtag cgagggagtt 300
gggtctccag gttgtgcgag gagcaaatga tgaccgccaa ggccgtagac aaaatcccag 360
taactctcag tggttttgtg caccagctgt ctgacaacat ctacccggtg gaggacctcg 420
ccgccacgtc ggtgaccatc tttcccaatg ccgaactggg aggccccttt gaccagatga 480
acggagtggc cggagatggc atgatcaaca ttgacatgac tggagagaag aggtcgttgg 540
atctcccata tcccagcagc tttgctcccg tctctgcacc tagaaaccag accttcactt 600
acatgggcaa gttctccatt gaccctcagt accctggtgc cagctgctac ccagaaggca 660
taatcaatat tgtgagtgca ggcatcttgc aaggggtcac ttccccagct tcaaccacag 720
cctcatccag cgtcacctct gcctccccca acccactggc cacaggaccc ctgggtgtgt 780
gcaccatgtc ccagacccag cctgacctgg accacctgta ctctccgcca ccgcctcctc 840
ctccttattc tggctgtgca ggagacctct accaggaccc ttctgcgttc ctgtcagcag 900
ccaccacctc cacctcttcc tctctggcct acccaccacc tccttcctat ccatccccca 960
agccagccac ggacccaggt ctcttcccaa tgatcccaga ctatcctgga ttctttccat 1020
ctcagtgcca gagagaccta catggtacag ctggcccaga ccgtaagccc tttccctgcc 1080
cactggacac cctgcgggtg ccccctccac tcactccact ctctacaatc cgtaagccct 1140
ttccctgccc actggacacc ctgcgggtgc cccctccact cactccactc tctacaatcc 1200
gtaactttac cctgggggc cccagtgctg gggtgaccgg accaggggcc agtggaggca 1260

gcgagggacc ccggctgcct ggtagcagct cagcagcagc agcagccgcc gccgccgccg 1320
cctataaccc acaccacctg ccactgcggc ccattctgag gcctcgcaag taccccaaca 1380
gacccagcaa gacgccggtg cacgagaggc cctacccgtg cccagcagaa ggctgcgacc 1440
ggcggttctc ccgctctgac gagctgacac ggcacatccg aatccacact gggcataagc 1500
ccttccagtg tcggatctgc atgcgcaact tcagccgcag tgaccacctc accacccata 1560
tccgcaccca caccggtgag aagcccttcg cctgtgacta ctgtggccga aagtttgccc 1620
ggagtgatga gaggaagcgc cacaccaaga tccacctgag acagaaagag cggaaaagca 1680
gtgccccctc tgcatcggtg ccagccccct ctacagcctc ctgctctggg ggcgtgcagc 1740
ctgggggtac cctgtgcagc agtaacagca gcagtcttgg cggagggccg ctcgcccctt 1800
gctcctctcg gacccggaca ccttgagatg agactcaggc tgatacacca gctcccaaag 1860
gtcccggagg ccctttgtcc actggagctg cacaacaaac actaccaccc tttcctgtcc 1920
ctctctccct ttgttgggca aagggctttg gtggagctag cactgccccc tttccaccta 1980
gaagcaggtt cttcctaaaa cttagcccat tctagtctct cttaggtgag ttgactatca 2040
acccaaggca aaggggaggc tcagaaggag gtggtgtggg gacccctggc caagagggct 2100
gaggtctgac cctgctttaa agggttgttt gactaggttt tgctacccca cttcccctta 2160
ttttgaccca tcacaggttt ttgaccctgg atgtcagagt tgatctaaga cgttttctac 2220
aataggttgg gagatgctga tcccttcaag tggggacagc aaaaagacaa gcaaaactga 2280
tgtgcacttt atggcttggg actgatttgg gggacattgt acagtgagtg aagtatagcc 2340
tttatgccac actctgtggc cctaaaatgg tgaatcagag catatctagt tgtctcaacc 2400
cttgaagcaa tatgtattat aaactcagag aacagaagtg caatgtgatg ggaggaacat 2460
agcaatatct gctccttttc gagttgtttg agaaatgtag gctatttttt cagtgtatat 2520
ccactcagat tttgtgtatt tttgatgtac actgttctct aaattctgaa tctttgggaa 2580
aaaatgtaaa gcatttatga tctcagaggt taacttattt aagggggatg tacatatatt 2640
ctctgaaact aggatgcatg caattgtgtt ggaagtgtcc ttggtgcctt gtgtgatgta 2700
gacaatgtta caaggtctgc atgtaaatgg gttgccttat tatggagaaa aaaatcactc 2760
cctgagttta gtatggctgt atatttctgc ctattaatat ttggaatttt ttttagaaag 2820
tatatttttg tatgctttgt tttgtgactt aaaagtgtta cctttgtagt caaatttcag 2880
ataagaatgt acataatgtt accggagctg atttgtttgg tcattagctc ttaatagttg 2940
tgaaaaaata aatctattct aacgcaaaac cactaactga agttcagata atggatggtt 3000
tgtgactata gtgtaaataa atacttttca acaataaaaa aaaaaaaaaa aaaaaaaaaa 3060
a 3061

<210> 30
<211> 1677
<212> DNA
<213> Homo sapiens

<400> 30

ggccaagcaa gcttctatct gcacctgctc tcaatcctgc tctcaccatg agcctccgcc 60
tgcagagctc ctctgccagc tatggaggtg gtttcggggg tggctcttgc cagctgggag 120
gaggccgtgg tgtctctacc tgttcaactc ggtttgtgtc tgggggatca gctgggggct 180
atggaggcgg cgtgagctgt ggttttggtg gaggggctgg tagtggcttt ggaggtggct 240
atggaggtgg ccttggaggt ggctatggag gtggccttgg aggtggcttt ggtgggggtt 300
ttgctggtgg ctttgttgac tttggtgctt gtgatggcgg cctcctcact ggcaatgaga 360
agatcaccat gcagaacctc aacgaccgcc tggcttccta cctggagaag gtgcgcgccc 420
tggaggaggc caacgctgac ctggaggtga agatccgtga ctggcacctg aagcagagcc 480
cagctagccc tgagcgggac tacagcccct actacaagac cattgaagag ctccgggaca 540
agatcctgac cgccaccatt gaaaacaacc gggtcatcct ggagattgac aatgccaggc 600
tggctgtgga cgacttcagg ctcaagtatg agaatgagct ggccctgcgc cagagcgtgg 660
aggccgacat caacggcctg cgccgggtgc tggatgagct cactctgtct aagactgacc 720
tggagatgca gatcgagagc ctgaatgaag agctagccta catgaagaag aaccatgaag 780
aggagatgaa ggaatttagc aaccaggtgg tcggccaggt caacgtggag atggatgcca 840
ccccaggcat tgacctgacc cgcgtgctgg cagagatgag ggagcagtac gaggccatgg 900
cagagaggaa ccgccgggat gctgaggaat ggttccacgc caagagtgca gagctgaaca 960
aggaggtgtc taccaacact gccatgattc agaccagcaa gacagagatc acggagctca 1020
ggcgcacgct ccaaggcctg gagattgagc tgcagtccca gctgagcatg aaagcggggc 1080
tggagaacac ggtggcagag acggagtgcc gctatgccct gcagctgcag cagatccagg 1140
gactcatcag cagcatcgag gcccagctga gcgagctccg cagtgagatg gagtgccaga 1200
accaagagta caagatgctg ctggacatca agacacgtct ggagcaggag atcgccacct 1260
accgcagcct gctcgagggc caggacgcca agaagcgtca gcccccgtag cacctctgtt 1320
accacgactt ctagtgcctc tgttaccacc acctctaatg cctctggtcg ccgcacttct 1380
gatgtccgta ggccttaaat ctgcctggcg tcccctccct ctgtcttcag cacccagagg 1440
aggagagagc cggcagttcc ctgcaggaga gaggaggggc tgctggaccc aaggctcagt 1500
ccctctgctc tcaggacccc ctgtcctgac tctctcctga tggtgggccc tctgtgctct 1560
tctcttccgg tcggatctct ctcctctctg acctggatac gctttggttt ctcaacttct 1620
ctaccccaaa gaaaagatta ttcaataaag tttcctgcct ttctgcaaac ataaaaa 1677

<210> 31
<211> 8191
<212> DNA
<213> Homo sapiens

<400> 31

```
tttgcttgca acactggcac ctctgccctg caccccggga gtgagcagtg agtgaggctc      60
gggtctgggc gctggctccg aatcttcggg ctgggagaga ctccaccatc tgggggcggc     120
ctgggggagc agccttagtg tcttcctgct gatgcaatcc gctaggtcgc gagtctccgc     180
cgcgagaggg ccggtctgca atccagcccg ccacgtgtac tcgccgccgc ctcgggcact     240
gccccaggtc ttgctgcagc cgggaccgcg ctctgcagcc gcagacccgg tccacacggc     300
caggggctac gacccttggg atctgccctc cgctcagctc gagcttccct cgtggccgac     360
ggaacaatga aggattgcag taacggatgc tccgcagagt gtaccggaga aggaggatca     420
aaagaggtgg tggggacttt taaggctaaa gacctaatag tcacaccagc taccatttta     480
aaggaaaaac cagaccccaa taatctggtt tttggaactg tgttcacgga tcatatgctg     540
acggtggagt ggtcctcaga gtttggatgg gagaaacctc atatcaagcc tcttcagaac     600
ctgtcattgc accctggctc atcagctttg cactatgcag tggaattatt tgaaggattg     660
aaggcatttc gaggagtaga taataaaatt cgactgtttc agccaaacct caacatggat     720
agaatgtatc gctctgctgt gagggcaact ctgccggtat ttgacaaaga agagctctta     780
gagtgtattc aacagcttgt gaaattggat caagaatggg tcccatattc aacatctgct     840
agtctgtata ttcgtcctac attcattgga actgagcctt ctcttggagt caagaagcct     900
accaaagccc tgctctttgt actcttgagc ccagtgggac cttatttttc aagtggaacc     960
tttaatccag tgtccctgtg ggccaatccc aagtatgtaa gagcctggaa aggtggaact    1020
ggggactgca agatgggagg gaattacggc tcatctcttt ttgcccaatg tgaagcagta    1080
gataatgggt gtcagcaggt cctgtggctc tatggagagg accatcagat cactgaagtg    1140
ggaactatga atctttttct ttactggata aatgaagatg gagaagaaga actggcaact    1200
cctccactag atggcatcat tcttccagga gtgacaaggc ggtgcattct ggacctggca    1260
catcagtggg gtgaatttaa ggtgtcagag agatacctca ccatggatga cttgacaaca    1320
gccctggagg ggaacagagt gagagagatg tttggctctg gtacagcctg tgttgtttgc    1380
ccagtttctg atatactgta caaaggcgag acaatacaca ttccaactat ggagaatggt    1440
cctaagctgg caagccgcat cttgagcaaa ttaactgata tccagtatgg aagagaagag    1500
agcgactgga caattgtgct atcctgaatg gaaaatagag gatacaatgg aaaatagagg    1560
ataccaactg tatgctactg ggacagactg ttgcatttga attgtgatag atttctttgg    1620
ctacctgtgc ataatgtagt ttgtagtatc aatgtgttac aagagtgatt gtttcttcat    1680
```

gccagagaaa atgaattgca atcatcaaat ggtgtttcat aacttggtag tagtaactta 1740
ccttacctta cctagaaaaa cattaatgta agccatataa catgggattt tcctcaatga 1800
ttttagtgcc tccttttgta cttcactcag atactaaata gtagtttatt ctttaatata 1860
agttacattc tgctcctcaa acaaatgcaa ttttttgtgt gtgtttgaaa gctaatttga 1920
gaaaatttca taggttacat ttcctgcagc ctatctttat ccacagaaag tgttttcttt 1980
tttttaaatc aagactttta aaactggatt tcctcccatc actgtttttt gaaggtcctc 2040
caagtccgtg ttaaggtaaa tatctgtttt cttcctgatg tcacagcctg agcatactct 2100
gtgcattagg aagacctgag tgcatttccc accattgtcc tttccacatt atgttgtagc 2160
tggctggctg tcaggcgact acaagactga gggtcttgtg ccttatagat ctttgtatcc 2220
cccatggctg acatatagta ggtactcagt aaatggtttt ataatgaatc agtgaacatt 2280
ttgcttctat agaagtgtac cttctttgtt tctatattat gaaacctctt tattagaatt 2340
tgtgattgat tctgacagtg tatagattta ccttatattg tctttatttt ccatgagcta 2400
ctaagtcatt agagatactc tgaagcatag ttagtttagg aaatcacttc atattgattg 2460
tattagaatt atcttggaat tgaagatata tccctagagc aggggacccc aacccccagg 2520
ccatgggcca cacagcagga agaggtgagt ggtgggccat tgaggagctt catctgtatt 2580
tatggctact tcccatcact cgaattacca cctgaactcc acctcttgtc agctcagtgg 2640
cagcattaga ttctcatagg agcacaaatc ctattgtgaa ctctgcatgc aagggatcta 2700
ggctatgcgc tccttatgag aatctaatgc ttgatgacct gaggtgtaac agtttcatcc 2760
tgaaaccacc cttcaccctg cagtctgtgg aaaaattgtc ttccacaaaa ctggtccctg 2820
gtgccaaaaa tgttggggac cactgctcta gagagaggtc atgatatcat accaaccaaa 2880
tggaaatgac aaatgtttta tgtcaagtgt taattgcaga aataaatctt tttttttttt 2940
ttttggtaga aaacaaagag gcatactctg atttttatac tctgtttttg caggtgctct 3000
tttctttgaa tggagatttg atgagcaagt ggttaggatg cagggagagc tactatgggt 3060
gatattttcc ttgtttagga gctgtgagtt aaaattgtat cctttgtggt ttatctaagg 3120
aaagtcaaat cttgacagaa aacatttttc cttggaaggt caactctcag acattgtatt 3180
ttggtttccc tcagtcctca taacttcctt cttgctgaac atattttatt ctcttttcag 3240
agaaggaaaa taaaaaggat tctaaaagtt tgatgcattg gaaaaatttc cttgaggcat 3300
ttagcaacac atagaaaatg ggctttgatt cttttccaaa acttttagcc atagggtctt 3360
ttatagacag ggatagtaaa atgaaaattg agaaatataa gatgaaaagg aatgataaaa 3420
atatctttta gggggctttt aattggtgat ctgaaatctt gggagaagct gttcttttca 3480
ggcctgaggt gctcttgact gtcgcctgcg cactgtgtac cccgagcaac attctaaggg 3540
tgtgctttcg ccttggctaa ctcctttgac ctcattcttc atatagtagt ctaggaaaaa 3600

gttgcaggta atttaaactg tctagtggta catagtaact aaatttctat tcctatgaga 3660
aatgagaatt atttatttgc catcaacaca ttttatactt tgcatctcca aatttattgt 3720
ggcgagactt gtccattgtg aaagttagag aacattatgt ttgtatcatt tctttcataa 3780
aacctcaaga gcatttttaa gccctttttca tcagacccag tgaaaactaa ggatagatgt 3840
ttaaaaactg gaggtctcct gataaggaga acacaatcca ccattgtcat ttaagtaata 3900
agacaggaaa ttgaccttga cgctttcttg ttaaatagat ttaacaggaa catctgcaca 3960
tctttttcc ttgtgcacta tttgtttaat tgcagtggat taatacagca agagtgccac 4020
attataacta ggcaattatc cattcttcaa gacttagtta ttgtcacact aattgatcgt 4080
ttaaggcata agatggtcta gcattaggaa catgtgaagc taatctgctc aaaaagatca 4140
acaaattaat attgttgctg atatttgcat aattggctgc aattatttaa tgtttaattg 4200
ggttgatcaa atgagattca gcaattcaca agtgcattaa tataaacaga actggtggca 4260
cttaaaatga taatgattaa cttatattgc atgttctctt cctttcactt ttttcagttt 4320
ctacatttca gaccgagctt gtcagctttt ttgaaaacac atcagtagaa accaagattt 4380
taaaatgaag tgtcaagaca aaggcaaaac ctgagcagtt cctaaaaaga tttgctgtta 4440
gaaattttct ttgtggcagt catttattaa ggattcaact cgtgatacac caaaagaaga 4500
gttgacttca gagatgtgtt ccatgctctc tagcacagga atgaataaat ttataacacc 4560
tgctttagcc tttgttttca aaagcacaaa ggaaaagtga aagggaaaga gaaacaagtg 4620
actgagaagt cttgttaagg aatcaggttt tttctacctg gtaaacattc tctattcttt 4680
tctcaaaaga ttgctgtaag aaaaaatgta agacaaaaaa aaaaaaaaaa aacaaacaga 4740
ggcagaggca ggcagtagca agaaagcaga gcgtaacatc agctagatgg taacatgcaa 4800
tgtcagctct cttgaagaca tgggaaacct aagttacacc ttgggttaaa attcttcacc 4860
atattagttt tgttgcttca taaaatttac ctaagcaagt ggtcttgctt gcctcaaatc 4920
caagcagtct tgaacacttg gaggcaatta atgagtatat cttagtcaaa agaattgttg 4980
gagcttttta ttaaagctac agtttcagtt ctgcttttgg ggaattgtgc tatgaaagca 5040
gctgccaaaa taagctcatt tattttcttc aatcccactc agtgctcagt cactatattc 5100
tgtttccttt tttttttca agttgcatat ttggtttccc cttatgattg ggaaagatga 5160
attttcagca gaaaacattg tttgttcact ttcaaagagt gatagtttct aaaacattta 5220
gagcaataaa tattcatcag aggtaccaag taagccggca gaagagttaa gggttagaga 5280
aatcccttat ttcatgtctt gactctaaaa ttatcaaagt acttttcctt gtaatgtgga 5340
tttcttctta tgcggatatg caaaaacttc agttatacgt agtaatgcta gcaggtaatt 5400
ttagtagaca ttttataaca actgtcactt tgtttcgcca catgtagagt ttgttcagct 5460

atttccaga tatctcccca caaaaggagg caaagggtac cagcttttca atgagcatta 5520
cctattactt ggcaaagatg atgaagactc tattaatagt tcatttgata aatgttgaca 5580
taaccaacaa tagagattag gaagttagtt ttaagaaatc aatggcatat agacattacc 5640
ctcatggagt ttgtattcta ctacttgaac tgattgtagc tataaaagca tagttagata 5700
gctgaatagt tagatcataa gcaaagaagg ccagaacaca tctcttatca agaaatcaat 5760
gaatagttta tctcattttt aaagcaactt tatccttctt taattccttc ctttcttcta 5820
gtgcaaaact acttaataag gttggtgttt aggttagtgt tcacaccatt cctcatctgg 5880
tgtgaattac cttctctttc tttactattt actaccaacc tagtacatgt gttgactgaa 5940
ttcttttcaa acaatgttga gttatcatgg tgcacctaat aaattaacac cacagattac 6000
agcatccttg ctgattttct cagcaaagcc agattagatg gaaataaaca aagaaaatga 6060
tcctagagtg aatttttcta gaaaatatct attatgaacc atgctgttta aagtattagc 6120
ttgaaggtga tggatccagc tattcagaaa ataactttca tataaccatg attttgcaca 6180
gtatgaggtc ttaaatgtgt ggaaagagat aaatttttta tcattaccac aaaccccttt 6240
taaagattca aaggtggaag aaagtgattt attttttctc ttcagcatac atatataaaa 6300
gacttgtcag atgtttaatt tggggaggtt gataatgaaa catatcaaca gagtatagta 6360
gttatagtag tgtttgtggg taaataattt cctggggtca gacatatata aacatatttg 6420
cttcaaaatg ataaaggcat gaaatcagtc ttaaaaattg aaatgggggt gatgggggag 6480
aaaaagaaga acaaatttga agtgcccttt caaatctgct ggatacaagt attgaagttt 6540
taagtcatct tattctgtct gaaagtgtat ttttcattct acaatagacc caatcaacaa 6600
gacgtataac ttgagttgca tgatgttcag tttatgtaat ctactgttgg gatggtaaga 6660
attgatgtag gctgtggtgt aagaatgaat taaaatatag tttcactggc ttttctctac 6720
atatccacta tcacaatggc taggtttcct gttgctcact attggattct ggagaaaaat 6780
ttaatgaaag atgatatcag aggaagaata agtggaggta gagaagaaag gaatgataga 6840
ggaggggaaa aaaacaaaac atatttttgt gttatccaaa ggagcttttt ccttattctg 6900
tcaagcattg agatcttctt cagctttcaa tgtagttgct aaatacaaat aatgctacta 6960
ggtagtgact aaatatagca aacacttcat cagatattag aattaggtca cactattgag 7020
gttataatct gaaggttgtg ttacatagaa accactttag attattatca acttggacta 7080
ggctttattt tataatagca tagtaagtaa tatctattgt gtcatttctt caaccatttt 7140
attctaagat ccatgaagct tcttgaggcc aaataaaata ataagtttag acaagaagta 7200
gattgtgact tttttccctt agagatacta tttactatct cctatcctga taggtggaag 7260
gtttactgaa ttggaaattg gttgactatt agtttttaac taaaatgtgc aataacacat 7320
tgcagtttcc tcaaactagt ttcctatgat cattaaactc attctcaggg ttaagaaagg 7380

```
aatgtaaatt tctgcctcaa tttgtacttc atcaataagt ttttgaagag tgcagatttt    7440
tagtcaggtc ttaaaaataa actcacaaat ctggatgcat ttctaaattc tgcaaatgtt    7500
tcctggggtg acttaacaag gaataatccc acaatatacc tagctaccta atacatggag    7560
ctggggctca acccactgtt tttaaggatt tgcgcttact tgtggctgag gaaaaataag    7620
tagttcgagg aagtagtttt taaatgtgag cttatagata gaaacagaat atcaacttaa    7680
ttatgaaatt gttagaacct gttctcttgt atctgaatct gattgcaatt actattgtac    7740
tgatagactc cagccattgc aagtctcaga tatcttagct gtgtagtgat tcttgaaatt    7800
ctttttaaga aaaattgagt agaaagaaat aaaccctttg taaatgaggc ttggcttttg    7860
tgaaagatca tccgcaggct atgttaaaag gattttagct cactaaaagt gtaataatgg    7920
aaatgtggaa aatatcgtag gtaaaggaaa ctacctcatg ctctgaaggt tttgtagaag    7980
cacaattaaa catctaaaat ggctttgtta caccagagcc atctggtgtg aagaactcta    8040
tatttgtatg ttgagagggc atggaataat tgtattttgc tggcaataga cacattcttt    8100
attatttgca gattcctcat caaatctgta attatgcaca gtttctgtta tcaataaaac    8160
aaaagaatcc tgtttgtgtg gtttcatgaa a                                  8191
```

<210> 32
<211> 2191
<212> DNA
<213> Homo sapiens

<400> 32

```
cacgggcgga gccggggcca tggagccgcc gctgccgggc taggcaggtc gtgccccgcc      60
gggccggcgg cgatgtcggg ctaccagcgc cacccgggcg ccaccccgct gtcccgagcc     120
cggagcctcg ccattcccga cgctccagcg ttctatgagc gccggtcttg tctcccccag     180
ctaaattgtg agcgccccca tggcagggac ctggactccc ccttcttcgg cattcggccg     240
gcctttatgt gctatgtgcc cagcccggtg ctggcttccg tgggagacac agatgacaga     300
tttgaagatc tggaagaggc aaatccattc tcttttagag agtttctgaa gaccaagaac     360
ctcggcctct cgaaagagga tccggccagc agaatttatg caaaggaagc ctcgaggcat     420
tccctgggac ttgaccacaa ctccccaccc tcccaaaccg gcgggtatgg cctggagtat     480
cagcagccat tttcgagga tccgacaggg gctggtgacc tcctggatgg ggaggaggat      540
gaggacaccg gatggagtgg ggcctacctg ccgtccgcca tcgagcagac tcaccccgag     600
agggtccctg ccggcacgtc gccctgcagc acatacctttt cctttttctc caccccgtcg    660
gagctggcag ggcctgagtc tctgccctcg tgggcgttga gtgacactga ttctcgcgtg     720
tctccggcct ctccggcagg gagtcctagc gcagactttg cggttcatgg agagtctctg     780
ggagacaggc acctgcggac gctgcagata agttacgacg cactgaaaga tgaaaattct     840
```

aagctgagaa gaaagctgaa tgaggttcag agcttctctg aagctcaaac agaaatggtg 900
aggacgcttg agcggaagtt agaagcaaaa atgatcaagg aggaaagcga ctaccacgac 960
ctggagtcgg tggttcagca ggtggagcag aacctggagc tgatgaccaa acgggctgta 1020
aaggcagaaa accacgtcgt gaaactaaaa caggaaatca gtttgctcca ggcgcaggtc 1080
tccaacttcc agcgagagaa tgaagccctg cggtgcggcc agggcgccag cctgaccgtg 1140
gtgaagcaga acgccgacgt ggccctgcag aacctccggg tggtcatgaa cagtgcacag 1200
gcttccatca agcaactggt ttccggagct gagacactga atcttgttgc cgaaatcctt 1260
aaatctatag acagaatttc tgaaattaaa gacgaggagg aagactcttg aggacccctg 1320
ggtgttctca gcatgaagct ccgtgtatac cctgaggtca ccaccgctcg atctaaatgt 1380
gcagttgtgt ccttaaatat gcagtcttca cccagagtaa agtgttgatc gcaagagtcc 1440
agtgtcgtgc cctcagccag ttcttggcca ccacaatggg agcagccctg gccgagttgt 1500
ctctgtggtt tctatgcagc ccttcttggc gaaattcctg cgatcttata gattctaatg 1560
agctcttgga agacattgtc ataaaagcca gtgattttaa gaaaaagagt ggttctggaa 1620
tcagtgtttt ccagtcccat cccagaacat cagttgtaag ataagtacaa ttggttgtcc 1680
ttgatttcat aagtagaaca aacactaaat gtgcctctga gatggccacc ccgggcaggg 1740
acctgtgcct tccaccgatg ctcagggctc cctctggctc ccgggtcact cttgtggccc 1800
cagtgggtgg tccctgcagt catggcctga gtgcgcaggg gccaccgcgt ggctgccgct 1860
gtcctcctcc gggacccacg gggaccaagg tcacacgttc cgtgctgtga agctgtccag 1920
atgtgcctct ttggctgggg gttctggtgg acgtttcaag tggcattttg tacaatgcag 1980
gttagaattc aggaatttca agtatgtgcc cgggtctgtc aggtcccagt tgcctttctg 2040
acggcccccc tcagagggac ggcgatgagc actaaatgct tttttgacta ttttcctata 2100
gattttttttt aaaacttttt tttcctcctg ttccaattga tagctttctt atttaataaa 2160
ttctgtagtt caccaaaaaa aaaaaaaaaa a 2191

<210> 33
<211> 463
<212> DNA
<213> Homo sapiens

<400> 33

atatattccc agctagttga aaatgatgat tcccacaaga agcataactc agcttgtttc 60
tgcttactga gtattttcta ctatggtata tattgataac atttcttcca ttatgtatgt 120
tgtataccag agttacagtt actgtgggaa tcataatttg aaattttgac tcctgtgttt 180
ctggaatctt tacaacaaat gttgcattaa catataactt ttttcagttg actttaccaa 240
aattaagccc atctttagta gatactgttt taacatgtga aagaaatacg ttataaacat 300

accacaagat atggctataa aacaatgaga tcagtatcca tttttgcttt aaagaattgg 360
ccttattgct tcagtgtcac atctcatact caagggcatt tactacaaag aaagagttct 420
ccaatattgc tgttctgttg ctgcctgccc tatttacaca tgt 463

<210> 34
<211> 393
<212> DNA
<213> Homo sapiens

<400> 34

tttacactta tagtagactt tatttagtga atccaaatga catgtgataa ttgtttggaa 60
aggcctattg attttatatc tgatcattca atccagagac attaaattca gttgattaat 120
ggagttcccc aactgtaaga cttctttacg agattatttt caagctttga aaagatcttc 180
tgagataaag gggatcagca aacagtaaga gtgtgttgct atacccaagc aaaagaaata 240
aatcttaatc tctcagcaaa tcattcaaaa tgtcagaaat gttagtgttt ctatatcttg 300
gtaaaatgga ttgattgaga agtatgaaaa gtataacagt ggcatgcaga atattgtttt 360
tatgaatatt cagaatttca gttgtttaca taa 393

<210> 35
<211> 10434
<212> DNA
<213> Homo sapiens

<400> 35

atggcgaacc ggcgagtggg gcgaggctgc tgggaagtga gcccgaccga gcggaggccg 60
cccgcggggc tgcggggccc cgcggccgag gaggaggcgt cttccccgcc ggtcctgtct 120
ctcagccact tctgcaggtc tcctttcctt tgcttcgggg acgttctcct gggagcctca 180
cggacgctgt ctctggccct agacaaccct aacgaggagg tggcagaagt gaagatctcc 240
cacttcccgg ccgcggacct gggcttcagt gtgtcgcagc gctgtttcgt gttgcagcct 300
aaagagaaaa ttgttatttc tgttaactgg acaccactca aagaaggccg agtaagagag 360
attatgacat ttcttgtaaa tgatgttctg aaacaccaag ctatattact aggaaatgca 420
gaagagcaga aaaagaaaaa gaggagtctt tgggatacca ttaaaaagaa gaaaatttca 480
gcctctacaa gtcacaacag aagggtttca aatattcaga atgttaataa aacatttagt 540
gtttcccaaa aagttgacag agttaggagc ccactacaag cttgtgaaaa cttggctatg 600
aatgaaggcg gtcccccaac agaaaacaat tctttaatac ttgaagaaaa taaaataccc 660
atatcaccta ttagccctgc tttcaatgaa tgccatggtg caacttgctt gccactctct 720
gtacgtcgat ctactaccta ctcatctctt catgcatcag aaaataggga actattaaat 780
gtacacagtg ccaacgtttc aaaagtttct tttaatgaga aagctgtaac tgaaacttcc 840

```
tttaattctg taaatgttaa tggccaaaga ggagagaata gtaaacttag tcttaccccc    900
aactgttctt caactttgaa cattacacaa agccaaatac attttctaag tccagattct    960
tttgtaaata atagtcatgg agctaataat gaactagaat tagtaacatg tctttcatca   1020
gatatgttta tgaaagataa ttcacagcct gtgcatttgg aatcaacaat tgcacatgaa   1080
atttatcaga aaattttaag tccagattct ttcataaaag ataattatgg actaaatcag   1140
gatctagaat cagagtcagt taatcctatt ttatccccta atcaattttt aaaagataac   1200
atggcatata tgtgtacatc tcagcaaaca tgtaaagtac cattatcaaa tgaaaattct   1260
caagtcccac agtctcctga agattggaga aaaagtgaag tttcgccacg tattcctgaa   1320
tgtcagggtt caaaatctcc caaagctatt tttgaagaac tagtagaaat gaagtcaaat   1380
tactacagtt ttataaaaca aaataatcct aaattttctg cagttcagga tatttctagt   1440
catagccaca ataaacaacc taagagacgt ccaatacttt ctgccactgt tactaaaagg   1500
aaggccacct gtaccagaga aaaccaaact gagattaata aaccaaaagc aaaaagatgt   1560
ctcaacagtg cagtgggtga acatgaaaaa gtaataaata atcaaaagga aaaagaagat   1620
tttcattctt atcttccaat tatagatcca atattaagta aatctaagag ttataaaaac   1680
gaggtaacac cctcttcgac aacagcttca gttgctcgga aaagaaagag cgatggaagc   1740
atggaagatg caaatgtgag agttgcaatt acagaacata cagaagtgcg agaaatcaaa   1800
agaatccatt tttctccctc agagcctaaa acatcagctg ttaagaaaac aaaaaatgtg   1860
acaacaccca tctcaaaacg tattagcaac agagagaaat taaacctgaa gaagaaaact   1920
gatttatcaa tattcagaac tccaatttct aaaacaaaca aaaggacaaa acccattatc   1980
gctgtggcac agtccagttt gaccttcata aaaccattaa aaacagatat tcccagacac   2040
ccgatgccat ttgctgcaaa aaacatgttt tatgatgaac gctggaagga aaagcaggaa   2100
cagggcttca cttggtggtt aaattttata ttaacccctg atgacttcac tgtaaaaaca   2160
aatatttctg aagtaaatgc tgctactctt cttttgggaa tagagaatca acataaaata   2220
agtgttccta gagcacctac aaaagaggaa atgtctctca gagcttatac tgctcggtgt   2280
aggttaaaca gactacgtcg tgcagcatgc cgtttgttta cttctgaaaa aatggttaaa   2340
gctattaaaa agcttgaaat tgaaattgaa gctaggcggt taattgttcg aaaagataga   2400
cacctatgga aagatgtggg agaacgtcag aaagtcctga attggctgtt gtcctacaat   2460
cctttgtggc ttcgaattgg tctagagaca acttatggag aactcatatc tttggaagat   2520
aacagtgatg tcacagggtt ggctatgttt attctgaatc gcctactttg gaatcctgat   2580
atagcagctg agtatagaca ccccactgtt cctcacctgt atagagatgg tcatgaagaa   2640
gctttgtcca agtttacatt gaaaaagtta ttgttgttgg tctgttttct tgattatgct   2700
aaaatttcca gactcattga tcatgatcct tgtctcttct gtaaagatgc cgaattcaag   2760
```

gctagtaaag aaatcctttt ggctttttca cgagatttcc taagtggtga aggtgacctt 2820
tcccgtcacc ttggcttatt gggattacct gttaaccatg ttcagacacc atttgatgaa 2880
tttgattttg ccgttacaaa tcttgccgta gacttgcaat gtggagtgcg ccttgtgcga 2940
accatggaac ttctcacaca gaactgggac ctctcaaaga aactcaggat tccggcaata 3000
agtcgtcttc aaaagatgca caatgttgac attgttcttc aagttcttaa atcacgagga 3060
attgaattaa gtgatgagca tggaaataca attctatcta aggatattgt ggataggcac 3120
agagaaaaaa ctctcaggtt gctttggaaa atagcgtttg cttttcaggt ggatatttcc 3180
cttaacttag atcaattaaa ggaagaaatt gcctttctaa aacacacaaa gagtataaag 3240
aaaacaatat ctctactatc atgccattct gatgatctta ttaataagaa aaaaggcaaa 3300
agggatagtg gttcctttga acaatatagt gaaaacataa agttattgat ggattgggta 3360
aatgctgttt gtgccttcta taataaaaag gtggagaatt ttacagtgtc tttctcagac 3420
ggccgtgtgt tatgttacct gatccaccat taccatcctt gctatgtgcc atttgacgct 3480
atatgtcagc gtactactca aactgtggaa tgtacgcaaa ctggttcagt ggtattaaat 3540
tcatcatctg aatctgatga cagttctctg gatatgtcac ttaaagcatt tgatcatgaa 3600
aatacttcag agctatacaa agagctccta gaaaatgaaa agaaaaattt tcacttggtt 3660
aggtctgcag ttagagacct tggtggaata cctgctatga ttaatcattc agatatgtca 3720
aatacaattc cagatgaaaa ggtggttatt acctatttgt catttctttg tgcaaggctt 3780
ttggatcttc gtaaagaaat aagagctgct cgactcatac aaacaacatg gagaaaatat 3840
aaactaaaaa cagatctcaa acgccatcag gagagagaga aagctgcaag aattattcaa 3900
ttggctgtaa tcaattttct agcaaaacaa agattgagaa aaagagttaa tgcagcactc 3960
gtcattcaga aatattggcg aagagtctta gcacagagaa aattattaat gttaaaaaag 4020
gaaaagctgg aaaaagttca aaataaagca gcatcactta ttcagggata ttggagaaga 4080
tattccacta gacaaagatt tctgaaattg aaatattatt caatcatcct gcaatctagg 4140
ataagaatga taattgctgt tacatcttat aaacgatatc tttgggctac agttacaatt 4200
cagaggcatt ggcgtgctta tttaagaaga aaacaagatc aacaaagata tgaaatgcta 4260
aaatcatcaa ctcttataat ccaatctatg ttcagaaaat ggaagcaacg taaaatgcaa 4320
tcacaagtaa aagctacagt aatattgcaa agagctttta gagaatggca tttaagaaaa 4380
caagctaaag aagaaaattc tgctattatc atacaatcat ggtatagaat gcataaagaa 4440
ttacggaagt atatttatat tagatcttgt gttgttatca ttcagaaaag atttcggtgc 4500
tttcaagccc aaaagttata taaaagaaga aaagagtcca tactaaccat ccagaagtac 4560
tacaaagcat atctgaaagg aaagattgag cgcaccaact atttgcagaa acgagctgca 4620

gccattcaat tacaagctgc ttttaggaga ctgaaagctc ataatttatg tagacaaatt 4680
agagctgctt gtgttattca gtcatactgg agaatgagac aagacagagt tcgattttta 4740
aaccttaaga agactattat caaatttcag gcacatgtaa gaaaacatca acaacgacag 4800
aaatataaga agatgaagaa agcagctgtt ataattcaga ctcatttccg agcttatatt 4860
tttgccatga aagttctagc atcttaccag aaaacacgct ctgctgtcat tgtgctgcag 4920
tctgcatata gagggatgca agccaggaaa atgtatattc acatcctcac atctgttata 4980
aagattcaat catattatcg tgcttatgtt tctaaaaagg aatttttgag cctaaaaaat 5040
gctacaataa aattgcagtc aactgttaag atgaaacaaa cacgtaaaca atatttgcat 5100
ttaagagcag ctgcactatt tatccagcaa tgttaccgtt ccaaaaaaat agctgcacaa 5160
aagagagaag agtatatgca gatgcgggaa tcttgtatca aactgcaagc atttgttaga 5220
ggatacccttg tccgaaagca gatgaggtta caaagaaaag ctgttatttc actacagtct 5280
tatttcagaa tgagaaaggc tcggcagtat tatctgaaaa tgtataaagc aattattgtc 5340
attcagaatt actatcatgc atacaaagca caggtcaatc agaggaagaa cttcttgcaa 5400
gtcaaaaaag cagctacttg cttgcaagca gcttacagag gttataaagt acgccagcta 5460
atcaaacaac aatctatagc tgctcttaaa attcagtctg cttttagagg ctataataaa 5520
agggtaaaat atcaatctgt gcttcaatct ataataaaga ttcagagatg gtacagggcg 5580
tacaagactc ttcatgatac aagaacacat tttttgaaga caaaggcagc tgtgatttcc 5640
ctccagtctg cttatcgtgg ctggaaggtt cggaaacaga ttagaaggga acatcaagct 5700
gccttgaaga ttcagtctgc ttttagaatg gccaaggccc agaaacagtt tagattgttt 5760
aaaacagcag cattagtcat ccagcaaaat ttcagagcat ggactgcagg aaggaagcaa 5820
tgtatggagt atattgaact ccgtcatgcg gtactggtgc ttcaatctat gtggaaggga 5880
aaaacactga gaagacagct tcaaaggcaa cataaatgtg ctatcatcat acagtcatac 5940
tatagaatgc atgtgcaaca aaagaagtgg aaaatcatga aaaaagctgc tcttctgatt 6000
caaaagtatt atagggctta cagtattgga agagaacaga atcatttata tttgaaaaca 6060
aaagcagctg tagtaacttt acagtcagct tatcgtggta tgaaagtgag aaaaagaata 6120
aaggattgca acaaagcagc agtcactata cagtctaaat acagagctta caaaaccaaa 6180
aagaaatatg caacctatag agcttcagct attataattc agagatggta tcgaggtatt 6240
aaaattacaa accatcagca taaggagtat cttaatttga agaagacagc aattaaaatc 6300
caatctgttt atagaggtat tagagttaga agacatattc aacacatgca cagggcagcc 6360
acttttatta aagccatgtt taaaatgcat cagtcaagaa taagttacca tacaatgaga 6420
aaagcagcta ttgttattca agtaagatgt agagcatatt atcaaggtaa aatgcagcgt 6480
gaaaagtacc tgacaatttt gaaagctgtt aaagtccttc aggcaagttt tagaggagta 6540

agagttagac ggactcttag aaagatgcag actgcagcaa cactcattca gtcaaactac 6600
agaagataca gacagcaaac atactttaat aagttaaaga aaataacaaa aacagtacag 6660
caaagatact gggcaatgaa agaaagaaac atacaatttc aaaggtataa caaactgagg 6720
cattctgtaa tatacattca ggctattttt aggggaaaga aagctagaag acatttaaaa 6780
atgatgcata tagccgcaac tctcattcag aggagattta gaactctaat gatgagaaga 6840
agattcctct ctctcaagaa aactgctatt ttgattcaga gaaaatatcg ggcacatctt 6900
tgtacaaagc atcacttaca gttccttcag gtacaaaatg cagttattaa aatccagtca 6960
tcatacagaa gatggatgat aaggaaaagg atgcgagaga tgcacagggc tgctactttc 7020
atccagtcta ctttcagaat gcacagatta catatgagat atcgagcttt gaaacaggcc 7080
tccgttgtga tccaacagca ataccaagca aatagagctg caaaactgca gaggcagcat 7140
tatctcagac aaagacactc tgctgtgatc cttcaggctg cattcagggg tatgaaaact 7200
agaagacatt tgaagagtat gcattcctct gcaaccctta ttcagagtag gtttagatca 7260
ttactggtga ggagaagatt catttccctc aaaaaagcta ctatttttgt tcagaggaaa 7320
tatcgagcca ccatttgtgc caaacataaa ttgtaccaat tcttgcactt aagaaaggca 7380
gccattacaa tacagtcatc ttacagaaga ctgatggtaa agaagaagtt acaagaaatg 7440
caaagggctg cagttctcat tcaggctact ttcaggatgc acagaacata tattacattt 7500
cagacttgga aacatgcttc aattctaatt cagcaacatt atcgaacata tagagctgca 7560
aaattgcaaa gagaaaatta tatcagacaa tggcattctg ctgtggttat tcaggctgca 7620
tataaaggaa tgaaagcaag acaactttta agggaaaaac acaaagcttc tatcgtaata 7680
caaagcacct acagaatgta taggcagtat tgtttctacc aaaagcttca gtgggctaca 7740
aaaatcatac aagaaaaata tagagcaaat aaaaagaaac agaaagtatt tcaacacaat 7800
gaacttaaga aagagacttg tgttcaggca ggttttcagg acatgaacat aaaaaaacag 7860
attcaggaac agcaccaggc tgccattatt attcagaagc attgtaaagc ctttaaaata 7920
aggaagcatt atctccacct tagagcaaca gtagtttcta ttcaaagaag atacagaaaa 7980
ctaactgcag tgcgtaccca agcagttatt tgtatacagt cttattacag aggctttaaa 8040
gtacgaaagg atattcaaaa tatgcaccgg gctgccacac taattcagtc attctatcga 8100
atgcacaggg ccaaagttga ttatgaaaca aagaaaactg caattgtggt tatacagaat 8160
tattataggt tgtatgttag agtaaaaaca gaaagaaaaa actttttagc agttcagaaa 8220
tctgtacgaa ctattcaggc tgcttttaga ggcatgaaag ttagacaaaa attgaaaaat 8280
gtatcagagg aaaagatggc agccattgtt aaccaatctg cactctgctg ttacagaagt 8340
aaaactcagt atgaagctgt tcaaagtgaa ggtgttatga ttcaagagtg gtataaagct 8400

tctggccttg cttgttcaca ggaagcagag tatcattctc aaagtagggc tgcagtaaca 8460
attcaaaaag ctttttgtag aatggtcaca agaaaactgg aaacacagaa atgtgctgcc 8520
ctacggattc agttcttcct tcagatggct gtgtatcgga gaagatttgt tcagcagaaa 8580
agagctgcta tcactttaca gcattatttt aggacgtggc aaaccagaaa acagttttta 8640
ctatatagaa aagcagcagt ggttttacaa aatcactaca gagcatttct gtctgcaaaa 8700
catcaaagac aagtctattt acagatcaga agcagtgtta tcattattca agctagaagt 8760
aaaggattta tacagaaacg gaagtttcag gaaattaaaa atagcaccat aaaaattcag 8820
gctatgtgga ggagatatag agccaagaaa tatttatgta aagtgaaagc tgcctgcaag 8880
attcaagcct ggtatagatg ttggagagca cacaaagaat atctagctat attaaaagct 8940
gttaaaatta ttcaaggttg cttctatacc aaactagaga gaacacggtt tttgaatgtg 9000
agagcatcag caattatcat tcagagaaaa tggagagcta tacttcctgc aaagatagct 9060
catgaacact tcttaatgat aaaaagacat cgagctgctt gtttgatcca agcacattat 9120
agaggatata aaggaaggca ggtctttctt cggcagaaat ctgctgcttt gatcatacaa 9180
aaatatatac gagccaggga ggctggaaag catgaaagga taaaatatat tgaatttaaa 9240
aaatctacag ttatcctaca agcactggtg cgtggttggc tagtacgaaa aagattttta 9300
gaacagagag ccaaaattcg acttcttcac ttcactgcag ctgcatatta tcacctgaat 9360
gctgttagaa ttcaaagagc ctataaactt tacctggctg tgaagaatgc taacaagcag 9420
gttaattcag tcatctgtat tcagagatgg tttcgagcaa gattacaaga aaagagattt 9480
attcagaaat atcatagcat caaaaagatt gagcatgaag gtcaagaatg tctgagccag 9540
cgaaataggg ctgcatcagt aatacagaaa gcagtgcgcc attttctcct ccgtaaaaag 9600
caggaaaaat tcactagtgg aatcattaaa attcaggcat tatggagagg ctattcttgg 9660
aggaagaaaa atgattgtac aaaaattaaa gctatacgac taagtcttca agttgttaat 9720
agggagattc gagaagaaaa caaactctac aaaagaactg cacttgcact tcattacctt 9780
ttgacatata agcacctttc tgccattctt gaggccttaa aacacctaga ggtagttact 9840
agattgtctc cactttgttg tgagaacatg gcccagagtg gagcaatttc taaaatattt 9900
gttttgatcc gaagttgtaa tcgcagtatt ccttgtatgg aagtcatcag atatgctgtg 9960
caagtcttgc ttaatgtatc taagtatgag aaaactactt cagcagttta tgatgtagaa 10020
aattgtatag atatactatt ggagcttttg cagatatacc gagaaaagcc tggtaataaa 10080
gttgcagaca aaggcggaag catttttaca aaaacttgtt gtttgttggc tattttactg 10140
aagacaacaa atagagcctc tgatgtacga agtaggtcca aagttgttga ccgtatttac 10200
agtctctaca aacttacagc tcataaacat aaaatgaata ctgaaagaat actttacaag 10260
caaaagaaga attcttctat aagcattcct tttatcccag aaacacctgt aaggaccaga 10320

atagtttcaa gacttaagcc agattgggtt ttgagaagag ataacatgga agaaatcaca 10380
aatcccctgc aagctattca aatggtgatg gatacgcttg gcattcctta ttag 10434

<210> 36
<211> 3581
<212> DNA
<213> Homo sapiens

<400> 36

atacgtggct gccgtctgtc cccgctgagg aggtgcagca gccggagatg gcggcggtgc 60
tgaacgcaga gcgactcgag gtgtccgtcg acggcctcac gctcagcccg gacccggagg 120
agcggcctgg ggcggagggc gccccgctgc tgccgccacc gctgccaccg ccctcgccac 180
ctggatccgg tcgcggcccg ggcgcctcag gggagcagcc cgagcccggg gaggcggcgg 240
ctgggggcgc ggcggaggag gcgcggcggc tggagcagcg ctggggtttc ggcctggagg 300
agttgtacgg cctggcactg cgcttcttca aagaaaaaga tggcaaagca tttcatccaa 360
cttatgaaga aaaattgaag cttgtggcac tgcataagca agttcttatg ggcccatata 420
atccagacac ttgtcctgag gttggattct ttgatgtgtt ggggaatgac aggaggagag 480
aatgggcagc cctgggaaac atgtctaaag aggatgccat ggtggagttt gtcaagctct 540
taaataggtg ttgccatctc ttttcaacat atgttgcgtc ccacaaaata gagaaggaag 600
agcaagaaaa aaaaaggaag gaggaagagg agcgaaggcg gcgtgaagag gaagaaagag 660
aacgtctgca aaaggaggaa gagaaacgta ggagagaaga agaggaaagg cttcgacggg 720
aggaagagga aaggagacgg atagaagaag aaaggcttcg gttggagcag caaaagcagc 780
agataatggc agctttaaac tcccagactg ccgtgcagtt ccagcagtat gcagcccaac 840
agtatccagg gaactacgaa cagcagcaaa ttctcatccg ccagttgcag gagcaacact 900
atcagcagta catgcagcag ttgtatcaag tccagcttgc acagcaacag gcagcattac 960
agaaacaaca ggaagtagta gtggctgggt cttccttgcc tacatcatca aaagtgaatg 1020
caactgtacc aagtaatatg atgtcagtta atggacaggc caaaacacac actgacagct 1080
ccgaaaaaga actggaacca gaagctgcag aagaagccct ggagaatgga ccaaaagaat 1140
ctcttccagt aatagcagct ccatccatgt ggacacgacc tcagatcaaa gacttcaaag 1200
agaagattca gcaggatgca gattccgtga ttacagtggg ccgaggagaa gtggtcactg 1260
ttcgagtacc cacccatgaa gaaggatcat atctcttttg ggaatttgcc acagacaatt 1320
atgacattgg gtttggggtg tattttgaat ggacagactc tccaaacact gctgtcagcg 1380
tgcatgtcag tgagtccagc gatgacgacg aggaggaaga agaaaacatc ggttgtgaag 1440
agaaagccaa aaagaatgcc aacaagcctt tgctggatga gattgtgcct gtgtaccgac 1500
gggactgtca tgaggaggtg tatgctggca gccatcaata tccagggaga ggagtctatc 1560

tcctcaagtt tgacaactcc tactctttgt ggcggtcaaa atcagtctac tacagagtct 1620
attatactag ataaaaatgt tgttacaaag tctggagtct agggttgggc agaagatgac 1680
atttaatttg gaaatttctt tttacttttg tggagcatta gagtcacagt ttaccttatt 1740
gatattggtc tgatggtttg tgaactcttg ctgggaatca aaatttcctt gagactcttt 1800
agcattcata ctttggggtt aaaggagatt cctcagactc atccagccct tgggtgctga 1860
ccagcagagt cactagtgga tgctgaagtt acatgagcta catgttaaat atttaaagtc 1920
tccaaaataa aacaccccaa cgttgacctt acccggctga tggttagccc cttgctgcct 1980
gctccatgtg tcttatgaga gcccgtagtt acagtgtcct ctaatttgaa atccataagt 2040
taacaagtct atatcaggtg cagctggctt tgattaaagg ccatttttaa aacttaaaaa 2100
ctcaacacct cacagattat aatagaaaaa gaaatggcct cagtttgatc tcgttcagaa 2160
tgacccagat tgtttctgct ttgggtgcag ctgtttagtt cagagttata ttacagagaa 2220
ttattttctg agataatctt aaactagaat gttcaaaact aattgataat tgaagtatca 2280
agatacgtag aacacctcag agatttttct tcaggaactt ccacaaactt tgaatccttg 2340
tatctttatt tggtattcat actactagta gcaaaataca ggtttttgt tttgttttgt 2400
tttgttttgg cttcatagag tatctcaaat tgaaactttt ctgcacaaag aataaaatta 2460
aggattttat aaactcaaat tggcacctac tgaattaaaa tacataaaat catttaaata 2520
taattcagca tatgggaagt aacattgcac taatatggaa atcactgcca gagacagtct 2580
attttctttt aatttgttac tacttagtca caaaccccac attattccag tttggaatta 2640
cttattaagg agaattggaa atacatatgc ccatgcttaa attttatagc tttaatttgt 2700
gttatttctt tattgacggg aagaggtaca tcttttttc cttactgaaa acaaatatgg 2760
attaattgcc tcaaatttgt ataagtgatt ggctagtgat tcttgttttc agaagggaga 2820
gtggtataga tagaaaatga caaagatggc aatatacact taatgttgtt attgtatgtt 2880
gttactgaag tacttagatt tttaaaattt caaatcctaa atcacttctt gtaggagggt 2940
tttcattaac tgcagtatat acagttcact acatatgggt tgtttgagtt ttttgtgtgc 3000
tgtatttctt tctgtttttt aatacctggt tttgtacata tctaactctg ttctcttttg 3060
gttgttcaga aactggattt ttttttctt aagcagtgct taatttgtgt tttttaattt 3120
tgattcagaa gtagtcccag ctcataggtg ttcatactgt tacatccaga acatttgtca 3180
ggctctctgt cagctttcat gtacatatgg tatagaaacc atggagttag gcacttcctg 3240
gatttttttt ttatgagaaa aatactgtat ttaaaatgta aaataaactt ttaaaaagca 3300
ggcactaata tatatttctt ccagcctttg attacaaatt tgtccttgca catgttaaga 3360
tgaattatct cctaaaaata tcattgttct tgggagcagt gtatgttact ttacatagca 3420
gcggttcctg tcatgtgttc atgtcagaat atttttggtt ttaaactttc ttattgcctt 3480
tggctgttga ttagtacagt acaagtgcga tttcaaaaag atcttgaaag taatatattt 3540
aatcaattaa aatgtttatc tgtaaaaaaa aaaaaaaaaa a 3581

<210> 37
<211> 609
<212> DNA
<213> Homo sapiens

<220>
<221> misc_feature
<222> (565)..(565)
<223> n is a, c, g, or t

<220>
<221> misc_feature
<222> (587)..(587)
<223> n is a, c, g, or t

<220>
<221> misc_feature
<222> (598)..(598)
<223> n is a, c, g, or t

<400> 37

```
tttttttaca gttttcaaat attttactga aaatgcatat tgtacaatta atgtataatg     60
acacaccagt gtgagaaacc tccataggta tcatttccac aaatatgcta tgaatataga    120
gttcctacac aaaactatac aacttaccag atgtaattcc tgttacgtac catactcaca    180
atcgtcttga agaatatgga gaaaaagtgc tgagtgacaa aaacaggagc catgtgtgat    240
tttaataaat ggaaaacacg gcatttcagc tcagtggtaa agcagtaaac caatcagatg    300
cttagctatc aagtaatcat gtgagaggaa acagaattag atcctacctc atactatatg    360
ttgtcagcta acactgtagc agtggtatat gaatcactaa attacctcca acaaaatgta    420
ttcctgtatt gaaaaaagga ggtatggcca acattgtgtc acgttccaag gtgaattttg    480
cggtcacgat atgacgttca ggaagctact tttattgttc agttgatttc tatgctcaac    540
tattaggtca attccgaaat aatcncatat cacagctaaa ataatgncta ccaagtcnct    600
ctgactgct                                                            609
```

<210> 38
<211> 2216
<212> DNA
<213> Homo sapiens

<400> 38

```
ctgttagcaa tgcttcctga tgttgtgcgt ggcccttttt ggttgattct ctccaaattc     60
gggtcagctg ctgccacctg gcaaataaca gaggatatgc tgaatctcct gtccatcctt    120
gtaacgatat ccttcttaat gaaattcttc aactggctga gcaattacaa atgtcatctg    180
```

```
tccagacaca tgggcttaag gatgtctaca aaattttaga catttttgca aatgggaaaa     240
aaaatagtct tgtaaatact gaaacagatt tccatgaact ttatcctact cttggaaaga     300
aaacaattct ccttggctgc agaaatcaaa taagctgggt ttgcaatgac caaggacata     360
aatgaagatg gattgaagtg gaaaaattct gtctcccaag tgatcagtga catctgccag     420
aggtcattac agctactttt aactgtgaac agtcaccagc taaactactc acttgccaca     480
acaaaataac ctctctcaaa gtaaatccag tgcatctgta tatatgtgta gatagcagca     540
acaaacaatc ctgaaacatt atttttggct gttaggtaag taaacgtgat gataattata     600
aacaacattc aaataacctt ggaccttggt gaaatgactt gtggtggcca gaatggtgca     660
acaagatgtt atttgcaagt ttttttaaga cacaaatatc tcagatacta ataatgagaa     720
taaagactgt tgaatatgaa attaaagcca agcaataatg tgccaaaaag aggcagttat     780
accagcaaat gcatctatta tgggcacacc attatataat gatggtttgc tttatgaaga     840
ctgactgtaa cccacaggat aaaataagca aaggcatagt ttctgctttc ttcctggaaa     900
aacttgttta gaagcttcat aaagaggtac agcactaatg agcattagtc aggatacagt     960
tggcatctat gtttttatgt gagcccagag ggaagaggag ccactcaaag tcttgctggt    1020
ttaaaactca agacagctgc aaccagaagt tttgttgaaa tggagacttt aaacttatgg    1080
taattactct ttctggacac tagcatgtag aaagcaattc agttaactct gcccagagga    1140
ttaccagctt tagctgtgaa aaaatgggct cccggatgta aaatcactaa aacatgagat    1200
cttgtatcca aagaggcttc aaatgatgcc ttacagaaaa cgatgctcca gatgggcact    1260
tctaaatgct aactcttcat caagtatctt tctggattca agctcaaaat taattggctg    1320
caaaatagta ggaataaaaa tcacatattt tacactttag aaaaggatat tgatgatcaa    1380
cctgcatggt gataattatg atgagatacc ccagtgattt aatgatgtta gaaagaatta    1440
aatgggagag aattgctaac agctttcttg atctcttaac tatggagatg tcattcattt    1500
atttctgggg tgaaaattat agcttgcttt ttgacattgc tgctagtatt gttctttgtt    1560
gctttaaaaa ttgtctctct ttagaaaaac tcttgagcag ttaaacagtt ctttttctga    1620
ttcatatcat tgcttttaat aacatgtaaa ggctgtgtgt agagcaaact atataaaatg    1680
agtagaaagg gcttgctcat gttaattggc atccttgatg attttagttg agattcctta    1740
acatttattt tagatcacat ctttacgtaa cttatttttc ctaatgtttt ccatcgtgtc    1800
ttaaaatgat gctggtatat caggagattg cagtattata gtcatactcc ccaatcccta    1860
gaggagagga aagactaatt cttgttttaa gggcccctgg agataccttt tattaaggtt    1920
gaaaaaggtc aacacagcct gaaaataaga aaaatatata ctagcaatta ctaattttct    1980
aaatgtgtgt atctctgctg tactaatgtg tgaacaatat gtcgtgcata atactgtagc    2040
tggtcgtggt atgtcaatac attctgtgag tgtgtacagt ctgagtgatc agttttctat    2100
ttttatgtgt aaaaaaaata acttgtcgta tcccatttaa aggccaattt ctgtattcag    2160
gcaggcatat gtacatacat gaataaagcc aacaaaagtg tgcacatgta ttcagt        2216
```

<210> 39
<211> 1705
<212> DNA
<213> Homo sapiens

<400> 39

```
aattcggcac gagaagactt ccagtttgga gtcgtttgct gcggggaggg aatgaatggg      60
cgctgggaac acgcccgcga ggtggggacg cgccggccgt agcgaggtcc ttagcgtgtg     120
agtggccggg gtcgggtcgc ttccccgcag catggaggac gatgcaccag tgatctacgg     180
gctggagttc caggcacgtg ccttaacacc tcaaactgca gaaacagatg ccattcggtt     240
tttggttggg acgcagtctc ttaaatatga taatcagatc catatcatag attttgacga     300
tgaaaacaac attataaata aaaatgtcct cctccatcaa gcgggtgaaa tctggcatat     360
tagcgctagc cctgcagaca gaggtgtgct gacgacctgc tacaacagaa cttcagacag     420
caaagtcctg acatgtgcag ccgtgtggag gatgccgaag gaattggaat caggcagcca     480
cgagtcccct gatgattcat ccagcactgc acagaccctg gagctgctct gtcaccttga     540
caacacagcc catggcaaca tggcctgtgt cgtgtgggag ccaatgggag atgggaagaa     600
aatcatttcc ttggctgata accatatcct gctgtgggat ttacaggaaa gctcgagcca     660
ggctgtgctg gccagctcag cgtccctgga agggaaggga caactgaagt tcacctcagg     720
acggtggagc ccacatcata actgcaccca ggtggccaca gcgaacgaca ccaccctccg     780
tggctgggac acccggagca tgagccagat ctactgcata gagaatgccc acggacagct     840
ggtgcgggac cttgacttta atcccaataa gcagtactac ttggccagct gcggagacga     900
ctgtaaggtg aagttctggg acacccgaaa tgtcaccgaa cccgtgaaga ccctggagga     960
gcactcccac tgggtgtgga acgtccgcta caaccactct catgaccagc tggtcctcac    1020
gggcagcagt gacagcagag tcatcctttc caacatggtg tccatctcgt cggagccctt    1080
cggccacttg gtagacgacg atgacatcag tgaccaggag gaccaccgtt ctgaagagaa    1140
gagcaaggag cccctgcagg acaacgtgat cgccacctac gaggagcacg aggacagcgt    1200
ctatgccgtg gactggtcct cggctgaccc gtggctgttt gcctccctga gctatgacgg    1260
gaggctcgtg atcaacaggg tgcccagggc cctgaagtac cacatcctgc tatgactccc    1320
gggcctgggt tatccaggtc ccattgagtg gttttcctct tggcagattc tcaaacagtc    1380
gcagctcttt ggaggtgact cgtgttccag gtggatccct ctctgggaga gccgctgttc    1440
ccttcctgta gcagcagcat ttatgaatgg ggtgaatggg gctattgtcg acggcacagc    1500
taatgcccga acccagcccc tgtcggcaga gacagagccc cacattatta tgtgaataac    1560
aatgttttct gttttaaggg tgtcaggagt ttcgcttttt aaaaaaatgt ctgttcctgc    1620
agtagtaact cttctttctc ttgagagtaa aaaatgaaat aaaataaatc cacgctgaca    1680
aaaaaaaaaa aaaaaaaaaa aaaaa                                          1705
```

<210> 40
<211> 1800
<212> DNA
<213> Homo sapiens

<400> 40

```
gaggaagatg gcggcgtccg cagctgccgc tgagctccag gcttctgggg gtccgcggca    60
cccagtgtgt ctgttggtgt tgggaatggc gggatccggg aaaaccactt ttgtacagag   120
gctcacagga cacctgcatg cccaaggcac tccaccgtat gtgatcaacc tggatccagc   180
agtacatgaa gttccctttc ctgccaatat tgatattcgt gatactgtaa agtataaaga   240
agtaatgaaa caatatggac ttggacccaa tggcggcata gtgacctcac tcaatctctt   300
tgctaccaga tttgatcagg tgatgaaatt tattgagaag gcccagaaca tgtccaaata   360
tgtgttgatt gacacacctg gacagattga ggtattcacc tggtcagctt ctgggacaat   420
tatcactgaa gcccttgcat cctcatttcc aacagttgtc atctatgtaa tggacacatc   480
gagaagtacc aacccagtga ccttcatgtc caacatgctc tatgcctgca gcatcttata   540
caaaaccaag ctgcctttca ttgtggtcat gaataaaact gacatcattg accacagctt   600
tgcagtggaa tggatgcagg attttgaggc tttccaagat gccttgaatc aagagactac   660
atacgtcagt aacctgactc gttcaatgag cctggtgtta gatgagtttt acagctcact   720
cagggtggtg ggtgtctctg ctgttctggg tactggatta gatgaactct ttgtgcaagt   780
taccagtgct gccgaagaat atgaaaggga gtatcgtcct gaatatgaac gtctgaaaaa   840
atcactggcc aacgcagaga gccaacagca gagagaacaa ctggaacgcc ttcgaaaaga   900
tatgggttct gtagccttgg atgcagggac tgccaaagac agcttatctc ctgtgctgca   960
cccttctgat ttgatcctga ctcgaggaac cttggatgaa gaggatgagg aagcagacag  1020
cgatactgat gacattgacc acagagttac agaggaaagc catgaagagc cagcattcca  1080
gaattttatg caagaatcga tggcacaata ctggaagaga aacaataaat aggagacttt  1140
agcacacttc acttgtttct agaagtccag aattttggac ctccacgtga aagaactgtt  1200
cttacctctg aactgggggc tcccataagg gataattttc ctcagagtag caaagtttct  1260
cttattagag aaatcttgtg actcagatga agtcagggat agaagaccct tggacctggc  1320
aggttaatgc tgattattcc ttggcctttc ccttgtattt atgcaaggaa ggatatactg  1380
agctgatact gttccaagcc tacaacttca agttttatca tttgaactca agtacttttg  1440
```

ctgctgagga atggaatcaa aagaacgtag tctcctggtg accacctcag atctctatta 1500
ttaggctaga tgtatagcct ctactccccc agcttcttgc tcttgaccct gcactgtaag 1560
ttgcccttct attagcagcc aaggaaaagg gaaacatgag cttatccaga acggtggcag 1620
agtctccttg gcaatcaacc aacgttgcta tgaaatatgc ctcacactgt atagctcatt 1680
ataggacgtc aggtttgttg aaaaaagtgg gcaagacatg attaatgaat cagaatcctg 1740
tttcattggt gacttggata aagacttttt aattttaaaa aaaaaaaaaa aaaaaaaaaa 1800

<210> 41
<211> 2297
<212> DNA
<213> Homo sapiens

<400> 41

ggctgaggcg cgatggcagg tgtcggggct gggcctctgc gggcgatggg gcggcaggcc 60
ctgctgcttc tcgcgctgtg cgccacaggc gcccaggggc tctacttcca catcggcgag 120
accgagaagc gctgtttcat cgaggaaatc cccgacgaga ccatggtcat cggcaactat 180
cgtacccaga tgtgggataa gcagaaggag gtcttcctgc cctcgacccc tggcctgggc 240
atgcacgtgg aagtgaagga ccccgacggc aaggtggtgc tgtcccggca gtacggctcg 300
gagggccgct tcacgttcac ctcccacacg cccggtgacc atcaaatctg tctgcactcc 360
aattctacca ggatggctct cttcgctggt ggcaaactgc gtgtgcatct cgacatccag 420
gttggggagc atgccaacaa ctaccctgag attgctgcaa aagataagct gacggagcta 480
cagctccgcg cccgccagtt gcttgatcag gtggaacaga ttcagaagga gcaggattac 540
caaaggtatc gtgaagagcg cttccgactg acgagcgaga gcaccaacca gagggtccta 600
tggtggtcca ttgctcagac tgtcatcctc atcctcactg gcatctggca gatgcgtcac 660
ctcaagagct tctttgaggc caagaagctg gtgtagtgcc ctctttgtat gacccttcct 720
ttttacctca tttatttggt actttcccca cacagtcctt tatccacctg gatttttagg 780
gaaaaaaatg aaaaagaata agtcacattg gttccatggc cacaaaccat tcagatcagc 840
cacttgctga ccctggttct taaggacaca tgacattagt ccaatctttc aaaatcttgt 900
cttagggctt gtgaggaatc agaactaacc caggactcag tcctgcttct tttgcctcga 960
gtgattttcc tctgtttttc actaaataag caaatgaaaa ctctctccat taccttctgc 1020
tttctctttg tccacttacg cagtaggtga ctggcatgtg ccacagagca ggccctgcct 1080
cactgtctgc tggtcagttc tgggttcact taatggcttt gtgaatgtaa ataaggggca 1140
ggtcttggcc ctagaggatt gagatgtttt tctatatctt agaactattt ttggataaat 1200
tatatatttt ccttcctagt agaagtgtta ctgcctgtaa ctagctcaaa ataccaatgc 1260
agtttctgca ttctgggttt tgtttttcct tttttttttt tttttttttt ttttgagttt 1320

tgctctcgtc gcccaggctg gagtgcaatg gcgtgatctc agctcactgg caacatctgc 1380
ctcccgggtt caaatgattc tcctgcctca gtctcctgag tagctgggat tacaggtgcc 1440
cgccaccacg ctcagctaat ttttgtattt ttagtagaga tggggtttta ccatgttggc 1500
caggctggtc ttagactcct gacctcagtt gatccacctg cctcagcctc tgcattcagt 1560
ttattcacat atttttggta actcccatgg cagctcctag gatttcagcg gtctgtgggc 1620
cagaaagcag gcaccagggc tgacctcaag gccgtatcag agggccaagc agagttcttt 1680
tggatacctg cttttcatcc cacagggcct tagagtcaga ggtaaggtag caacagagct 1740
agaatggggc aatgcactct taccctcctt ctcaactttt atttaagctg tgctaaatgt 1800
tttcttcaag ggaaccagat ttagttcttt acagaatttt ccagtgaaat aaaacatgtt 1860
gtaatagctg tgtttgagat gaaataagag gttgtgggta gaggggaggc acctaaagga 1920
aaagaggaaa ggtgcctggg ctacctatgc agataacctg gagtggactt cactgtggac 1980
tcgtggtact aaggcttggc ctggacaggc agtctagggg gtatgggaat acacggtgtg 2040
gttgttcaac tatttgcaaa ggtcaaccaa atagaccaca tgttcgcaaa gtatcatctg 2100
aggaaattaa gtaccttctt agccctctca gtcataaatt tgaacaaatt ttaatacact 2160
tccctcatgc ccttctatat aaaacttaat accattagtt ccccattctt gacattttat 2220
ttcagttttt attatatatt tatttgaaat atttattaaa ttatctgacc tacagaacta 2280
aaaaaaaaaa aaaaaaa 2297

<210> 42
<211> 653
<212> DNA
<213> Homo sapiens

<220>
<221> misc_feature
<222> (621)..(621)
<223> n is a, c, g, or t

<220>
<221> misc_feature
<222> (653)..(653)
<223> n is a, c, g, or t

<400> 42

gggacatttc caagggtatt taaactctca ctctgccacc tttctaaggg tgggaggctg 60
gcagagatgc tgcaatgctt gataatcatt tggccacact gaaatttcca aagggagctc 120
ttgccggtgc ttaaaaccaa aactcctgga cacttagaaa attccatgaa tctagcacaa 180
aatatccatt cttgcccaag tgtatcccct ttctctccag cttaatcttt tttttttttt 240
ttttttaaag cccaggccaa gggtactttt aactggaaac tggggaggag ggaagaacac 300
tagcagggag ctaagaggca ggttgctggg taagccatcc tgctcctacc tggtgcctgt 360

```
atctacattg ctgagtgctg tgcgccagtg cctttccttc atctgcagat ggagcccatc     420
tctttccacc tgggtgagga gaccctctgc tactccaggg gtaaacctta aagaaggtgt     480
cttgaagagc ccaaaggaca ctcacgtgct aaggtgtcca ttttatgcat ctttaaaata     540
ttttatttaa aaaaaaaaat agccctgccc tgtcttagtg ccactaacgg cccagattca     600
ttcattctga atggaaaaac ngagactgcc agcactttcc tttggtcctt ccn            653
```

<210> 43
<211> 1955
<212> DNA
<213> Homo sapiens

<400> 43

```
catggaggcg ctgctgctgg gcgcggggtt gctgctgggc gcttacgtgc ttgtctacta      60
caacctggtg aaggccccgc cgtgcggcgg catgggcaac ctgcggggcc gcacggccgt     120
ggtcacgggt gagtgcggag gcgggtgagt gcgagctggc ggggcgcgcg gagaggaggc     180
cgggccggcg gtagcagcgg cccgccgggc tcagctcagc tcggctcccg cccgcggtcc     240
gcaggcgcca acagcggcat cggaaagatg acggcgctgg agctggcgcg ccggggagcg     300
cgcgtggtgc tggcctgccg cagccaggag cgcggggagg cggctgcctt cgacctccgc     360
caggagagtg ggaacaatga ggtcatcttc atggccttgg acttggccag tctggcctcg     420
gtgcgggcct ttgccactgc ctttctgagc tctgagccac ggttggacat cctcatccac     480
aatgccggta tcagttcctg tggccggacc cgtgaggcgt ttaacctgct gcttcgggtg     540
aaccatatcg gtccctttct gctgacacat ctgctgctgc cttgcctgaa ggcatgtgcc     600
cctagccgcg tggtggtggt agcctcagct gcccactgtc ggggacgtct tgacttcaaa     660
cgcctggacc gcccagtggt gggctggcgg caggagctgc gggcatatgc tgacactaag     720
ctggctaatg tactgtttgc ccgggagctc gccaaccagc ttgaggccac tggcgtcacc     780
tgctatgcag cccacccagg gcctgtgaac tcggagctgt tcctgcgcca tgttcctgga     840
tggctgcgcc cacttttgcg cccattggct tggctggtgc tccgggcacc aagagggggt     900
gcccagacac ccctgtattg tgctctacaa gagggcatcg agcccctcag tgggagatat     960
tttgccaact gccatgtgga agaggtgcct ccagctgccc gagacgaccg ggcagcccat    1020
cggctatggg aggccagcaa gaggctggca gggcttgggc ctggggagga tgctgaaccc    1080
gatgaagacc cccagtctga ggactcagag gccccatctt ctctaagcac cccccaccct    1140
gaggagccca cagtttctca accttacccc agccctcaga gctcaccaga tttgtctaag    1200
atgacgcacc gaattcaggc taaagttgag cctgagatcc agctctccta accctcaggc    1260
caggatgctt gccatggcac ttcatggtcc ttgaaaacct cggatgtgtg cgaggccatg    1320
ccctggacac tgacgggttt gtgatcttga cctccgtggt tactttctgg ggccccaagc    1380
```

tgtgccctgg acatctcttt tcctggttga aggaataatg ggtgattatt tcttcctgag 1440
agtgacagta accccagatg gagagatagg ggtatgctag acactgtgct tctcggaaat 1500
ttggatgtag tattttcagg ccccaccctt attgattctg atcagctctg gagcagaggc 1560
agggagtttg caatgtgatg cactgccaac attgagaatt agtgaactga tccctttgca 1620
accgtctagc taggtagtta aattaccccc atgttaatga agcggaatta ggctcccgag 1680
ctaagggact cgcctagggt ctcacagtga gtaggaggag ggcctgggat ctgaacccaa 1740
gggtctgagg ccagggccga ctgccgtaag atgggtgctg agaagtgagt cagggcaggg 1800
cagctggtat cgaggtgccc catgggagta aggggacgcc ttccgggcgg atgcagggct 1860
ggggtcatct gtatctgaag cccctcggaa taaagcgcgt tgaccgccaa aaaaaaaaaa 1920
aaaaaaaaaa aaaaaaaaaa aaaaaaaaaa aaaaa 1955

<210> 44
<211> 3098
<212> DNA
<213> Homo sapiens

<400> 44

tcctgcttgt cggcatcgct ccccacaggc cgacgtcgag agggcctgct ttactcctcc 60
tctttctcct ccttctcccg cggcttctgc gcggagaggc gtcgcccggg atctgggttt 120
tggaagaagg atctttgtgg gaagacaggg tgaatttatc acagaggaat aacgagggag 180
aggagaaagg tttcctaaag acaaaaaaaa aaatggagga atctgtaaac caaatgcagc 240
cactgaatga gaagcagata gccaattctc aggatggata tgtatggcaa gtcactgaca 300
tgaatcgact acaccggttc ttatgtttcg gttctgaagg tgggacttat tatatcaaag 360
aacagaagtt gggccttgaa aatgctgaag ctttaattag attgattgaa gatggcagag 420
gatgtgaagt gatacaagaa ataaagtcat ttagtcaaga aggcagaacc acaaagcaag 480
agcctatgct ctttgcactt gccatttgtt cccagtgctc cgacataagc acaaaacaag 540
cagcatttaa agctgtttct gaagtttgtc gcattcctac ccatctcttt acttttatcc 600
agtttaagaa agatctgaag gaaagcatga aatgtggcat gtggggtcgt gccctccgga 660
aggctatagc ggactggtac aatgagaaag gtggcatggc ccttgctctg gcagttacaa 720
aatataaaca gagaaatggc tggtctcaca aagatctatt aagattgtca catcttaaac 780
cttccagtga aggacttgca attgtgacca aatatattac aaagggctgg aaagaagttc 840
atgaattgta taaagaaaaa gcactctctg tggagactga aaaattatta aagtatctgg 900
aggctgtaga gaaagtgaag cgcacaagag atgagctaga agtcattcat ctaatagaag 960
aacatagatt agttagagaa catcttttaa caaatcactt aaagtctaaa gaggtatgga 1020
aggctttgtt acaagaaatg ccgcttactg cattactaag gaatctagga aagatgactg 1080

ctaattcagt acttgaacca ggaaattcag aagtatcttt agtatgtgaa aaactgtgta 1140
atgaaaaact attaaaaaag gctcgtatac atccatttca tattttgatc gcattagaaa 1200
cttacaagac aggtcatggt ctcagaggga aactgaagtg gcgccctgat gaagaaattt 1260
tgaaagcatt ggatgctgct ttttataaaa catttaagac agttgaacca actggaaaac 1320
gtttcttact agctgttgat gtcagtgctt ctatgaacca aagagttttg ggtagtatac 1380
tcaacgctag tacagttgct gcagcaatgt gcatggttgt cacacgaaca gaaaaagatt 1440
cttatgtagt tgcttttttcc gatgaaatgg taccatgtcc agtgactaca gatatgacct 1500
tacaacaggt tttaatggct atgagtcaga tcccagcagg tggaactgat tgctctcttc 1560
caatgatctg ggctcagaag acaaacacac ctgctgatgt cttcattgta ttcactgata 1620
atgagacctt tgctggaggt gtccatcctg ctattgctct gagggagtat cgaaagaaaa 1680
tggatattcc agctaaattg attgtttgtg gaatgacatc aaatggtttc accattgcag 1740
acccagatga tagaggcatg ttggatatgt gcggctttga tactggagct ctggatgtaa 1800
ttcgaaattt cacattagat atgatttaac cataagcagc agcacgatcc agagatccat 1860
tgccatcagt gatctcacta aaaatataca gctacttccc agctaatctc cacccaatga 1920
atgatgatgg tatagtatgt gcataatgga aagttacctt actgaaaaaa aaaaaagaag 1980
gaaaaataag atgggcccaa aggtctatct actaaactag ctcttgggga aatagcttca 2040
ggatactgta gtttcctcta tctaatagag aactttttgt taacagacac tgtaaaatag 2100
ttttgctttg ttgaataata catgtgtacc taaaagaggt aagagcaaaa agtgtaattc 2160
cacatcatgt tacttgagaa gtgcttaacg ttttcttaaa tgttttcatt gggaaaggac 2220
agctttgata atgtccaaat actctgaaat gcactagacc atataactgt gatgaaatat 2280
gaaactcatc tgtaaacttt tataccaagg gggtaaaaaa aaaaactaag gcatttgatt 2340
aaattatgaa tgagttttac aaattccttt cagagtttta ctaagatcac acaaataaca 2400
gctttcttat tcagtgaaaa agatatttta tttctgatgt tttatttgca ctcgtggaat 2460
atgttaccat taatcagaaa catcatggca acccctaaga atagactaag tttgtgttgg 2520
ctgagggatt ctatttggtt tgcttttttt tttttgcttt gttatatttt attgctacaa 2580
ggggtgtgac ttgataatga tttcctctga attataataa catagccaga tgtagtctca 2640
cactgttttt catactctta agtgtaaata atataaaatg tttcaagcgc ttaactcccc 2700
ctcattcaca aagtataaca attaaaatct caactataac cagtttagct ttttccttac 2760
ttttaaaata aaattttta cttttaacta tttttttagt taatattttt aaaagtatac 2820
atgtcaatgg cctctttgtc cattattcat tttgtggcaa aatattcttc tttgatagtg 2880
taaacaaata ataaagcaat ctaggtcctt taggtttgaa aggcaatttt tgagtagcat 2940

attaccagct agccagtcac taggaatttt tttcagtatt atttgtatgt attaaacttt 3000
tcattacact aaagtgcatt attttattga gcaagtatcc ttcattgtga ggtttgacat 3060
taaagcaatc tgttgaaatg ccaaaaaaaa aaaaaaaa 3098

<210> 45
<211> 2689
<212> DNA
<213> Homo sapiens

<400> 45

```
gatcttgggc tgaggttccc gggcgggcgg gcgcggagag acgcgggaag caggggctgg     60
gcgggggtcg cggcgccgca gctagcgcag ccagcccgag ggccgccgcc gccgccgccc    120
agcgcgctcc ggggccgccg gccgcagcca gcacccgccg cgccgcagct ccgggaccgg    180
ccccggccgc cgccgccgcg atgggcaacg ccgccgccgc caagaagggc agcgagcagg    240
agagcgtgaa agaattctta gccaaagcca aagaagattt tcttaaaaaa tgggaaagtc    300
ccgctcagaa cacagcccac ttggatcagt ttgaacgaat caagaccctc ggcacgggct    360
ccttcgggcg ggtgatgctg gtgaaacaca aggagaccgg gaaccactat gccatgaaga    420
tcctcgacaa acagaaggtg gtgaaactga aacagatcga acacaccctg aatgaaaagc    480
gcatcctgca agctgtcaac tttccgttcc tcgtcaaact cgagttctcc ttcaaggaca    540
actcaaactt atacatggtc atggagtacg tgcccggcgg ggagatgttc tcacacctac    600
ggcggatcgg aaggttcagt gagccccatg cccgtttcta cgcggcccag atcgtcctga    660
cctttgagta tctgcactcg ctggatctca tctacaggga cctgaagccg gagaatctgc    720
tcattgacca gcagggctac attcaggtga cagacttcgg tttcgccaag cgcgtgaagg    780
gccgcacttg gaccttgtgc ggcacccctg agtacctggc ccctgagatt atcctgagca    840
aaggctacaa caaggccgtg gactggtggg ccctgggggt cttatctat gaaatggccg     900
ctggctaccc gcccttcttc gcagaccagc ccatccagat ctatgagaag atcgtctctg    960
ggaaggtgcg cttcccttcc cacttcagct ctgacttgaa ggacctgctg cggaacctcc   1020
tgcaggtaga tctcaccaag cgctttggga acctcaagaa tggggtcaac gatatcaaga   1080
accacaagtg gtttgccaca actgactgga ttgccatcta ccagaggaag gtggaagctc   1140
ccttcatacc aaagtttaaa ggccctgggg atacgagtaa ctttgacgac tatgaggaag   1200
aagaaatccg ggtctccatc aatgagaagt gtggcaagga gttttctgag ttttaggggc   1260
atgcctgtgc ccccatgggt tttctttttt ctttttcttt ttttttggtc ggggggggtgg  1320
gagggttgga ttgaacagcc agagggcccc agagttcctt gcatctaatt tcacccccac   1380
cccaccctcc agggttaggg ggagcaggaa gcccagataa tcagagggac agaaacacca   1440
gctgctcccc ctcatcccct tcaccctcct gccccctctc ccacttttcc cttcctcttt   1500
```

ccccacagcc ccccagcccc tcagccctcc cagcccactt ctgcctgttt taaacgagtt 1560
tctcaactcc agtcagacca ggtcttgctg gtgtatccag ggacagggta tggaaagagg 1620
ggctcacgct taactccagc ccccacccac acccccatcc cacccaacca caggccccac 1680
ttgctaaggg caaatgaacg aagcgccaac cttcctttcg gagtaatcct gcctgggaag 1740
gagagatttt tagtgacatg ttcagtgggt tgcttgctag aattttttta aaaaaacaac 1800
aatttaaaat cttatttaag ttccaccagt gcctccctcc ctccttcctc tactcccacc 1860
cctcccatgt ccccccattc ctcaaatcca ttttaaagag aagcagactg actttggaaa 1920
gggaggcgct ggggtttgaa cctccccgct gctaatctcc cctgggcccc tccccgggga 1980
atcctctctg ccaatcctgc gagggtctag gcccctttag gaagcctccg ctctcttttt 2040
ccccaacaga cctgtcttca cccttgggct ttgaaagcca gacaaagcag ctgcccctct 2100
ccctgccaaa gaggagtcat cccccaaaaa gacagagggg gagccccaag cccaagtctt 2160
tcctcccagc agcgtttccc cccaactcct taattttatt ctccgctaga ttttaacgtc 2220
cagccttccc tcagctgagt ggggagggca tccctgcaaa agggaacaga agaggccaag 2280
tccccccaag ccacggcccg gggttcaagg ctagagctgc tggggagggg ctgcctgttt 2340
tactcaccca ccagcttccg cctcccccat cctgggcgcc cctcctccag cttagctgtc 2400
agctgtccat cacctctccc ccactttctc atttgtgctt ttttctctcg taatagaaaa 2460
gtggggagcc gctggggagc caccccattc atccccgtat ttcccccctct cataacttct 2520
ccccatccca ggaggagttc tcaggcctgg ggtggggccc cgggtgggtg cgggggcgat 2580
tcaacctgtg tgctgcgaag gacgagactt cctcttgaac agtgtgctgt tgtaaacata 2640
tttgaaaact attaccaata aagttttgtt taaaaaaaaa aaaaaaaaa 2689

<210> 46
<211> 1936
<212> DNA
<213> Homo sapiens

<400> 46

ccggagcctc ctggaccagg agaactgtaa cgcgagcccc gagccatggg cgaaaggcgg 60
ggccgagacg ggttgggggc gccgacggtt tcccggccct ggctgcagct tggaggagaa 120
gctgagcctg tgcttccgcc cctcggatcc gggcgccgag cccgaggacg gccgtgcggc 180
catcacggag ctcaactcct gcagggggac gagatttgga atgccctgac agataattat 240
gggaatgtga tgcctgtaga ctggaagtca tcgcatacta ggaccttgca cttgcttact 300
ctgaacctct cagaaaaagg ggtaagtgac agtttgctct ttgatacatc agatgatgaa 360
gagctgagag aacagctgga tatgcactca atcatcgtct cctgtgttaa tgatgaaccc 420
ctcttcacgg cagaccaggt tattgaagaa attgaagaaa tgatgcagga atcaccggac 480

ccagaagatg atgaaacccc tacacagtca gatcggcttt caatgctttc ccaggaaatt 540
caaactctca agaggtctag taccggcagt tatgaagaga gagtgaaaag gctctcagtg 600
tctgagttaa atgaaatcct ggaagaaatt gagactgcca ttaaggagta ctctgaggag 660
ctggtgcagc agttggcttt acgagatgaa ctggagtttg aaaaggaagt gaaaaacagc 720
tttatttctg ttcttattga agtgcaaaac aaacagaaag agcacaaaga aacagcaaaa 780
aagaaaaaga aactaaaaaa tggcagctct cagaatggga agaatgagag aagtcatatg 840
cccggcacat atttgactac agtcattcct tatgagaaaa aaaacggacc accgtctgtt 900
gaagatcttc aaatattaac aaaaattctt cgtgccatga aggaggacag tgaaaaagtt 960
ccgagcttgt taactgatta tattctgaaa gttctgtgtc ctacatagag cagcaacttt 1020
atctgcggtg ggctccaagc tagatttccg acagcattat tctgagagct ggctaccatt 1080
acccttcttg ctattggaaa ctcagcacat ttgaacttgg gtttgattca gtattaacag 1140
atcttgacta cactaattct ttatattata gaaccaacgg aaatatgggc actattttga 1200
attctagaga tggtttttgt taaatctact aataaactgt tctcttagta gattaagaga 1260
gagtaatatt aattgtgcat gtgcagttgt atttctcatt aactgacagt atgcccattt 1320
gtttttatgg ctttcttatc taaactgcac tgatgaacta gattaaagcc ttgggagatt 1380
tatactataa attcagtgat ggcaagaacc aacactgttt ttttgtgaga attgtcagtg 1440
taactattac ctaccagtat tgttcagaga gattgaaaca gaataaacgg gctgttcttg 1500
aagaagcaaa accagaatat gcattacttt ggtttaatac ttagtgctaa cattgaaact 1560
gttggtggtg atggattttg tagcttgctg cttgtttcac cactggtcaa attttaacca 1620
ttaaattgcc attcactttt agaatcttgt atttaagtaa gttttgattt tcaaatgttc 1680
tgcttcatgt gtctgtgaag aattgtactt ttttaaaagt gtgtgtcctc tgaggtgctt 1740
gagaaagtgt acactgcaga actgcccatt ctcattactg tgtcctattt tattcatgcc 1800
tgtgtgtttt tcttaagtat gaattctaga tacagctact tatggattca tcaatatcat 1860
gagcactttt gctggttcca gtcaaatcaa tggcatttaa taaatttttt aagaagtaaa 1920
aaaaaaaaaa aaaaaa 1936

<210> 47
<211> 3990
<212> DNA
<213> Homo sapiens

<400> 47

ggagtttagg gcctgacaga agcccgcccc cgctggcgct cgtgcgcacg cgtggcgggc 60
tctcggcgca ctgagcaggc gcggcctcgt gtcggccgga gggggcgggc gcaacgacgc 120
gcgctgcgtc ccggcgctcg gctttccctc cgccggtccc gccctccgtc gcggcggcgc 180

```
ggtgtaccct gggataggga gcgatctccg agcgaggcgg caagatggac gcgggatttt     240
tccgcggaac aagtgcagaa caggataatc ggttcagcaa caaacagaag aaactactga     300
agcagctgaa atttgcagaa tgcctagaaa aaaaggtgga catgagcaaa gtaaatttgg     360
aggttataaa gccttggata acaaaaagag taacggaaat ccttgggttt gaagatgatg     420
ttgtgattga gtttatattc aaccagctgg aagtgaagaa tccagactcc aaaatgatgc     480
aaatcaacct gactggattt ttgaatggaa aaaatgctcg agaatttatg ggagaactgt     540
ggcccctgct gctaagtgca caagaaaaca tcgcgggaat cccttctgct ttcctagaac     600
tgaagaaaga agaaataaaa caaagacaga ttgaacaaga aaaactggca tctatgaaaa     660
agcaagatga agacaaagat aaaagagata aggaagaaaa agaaagcagc agagaaaaaa     720
gggagcggtc tcgtagccca agaagacgca aatccagatc tccttcccct agaagacgat     780
cttcccctgt caggagagag agaaagcgca gtcattctcg atctccccgt cacagaacca     840
agagccggag tccttcccct gctccagaaa agaaggaaaa aactccagag ctcccagaac     900
cttcagtgaa agtaaaagaa ccttcagtac aagaggctac ttctactagt gacattctga     960
aagttcccaa acctgaacct ataccagagc ctaaagaacc ttctccggaa aaaaattcca    1020
aaaaagaaaa ggagaaggag aagacccgac cacgatctcg gtcacgctcc aaatcaagat    1080
cccggacgcg gtcccgctct ccttctcaca ctcgacctag acggcgccat agatcccgat    1140
caagatcgta ttcacctaga aggcggccaa gcccaagaag gcggccatct cctcgaagaa    1200
gaactccgcc aagaagaatg cctcctccac caaggcatag aaggagtaga tctccagtaa    1260
gacgaagaag acgttcgtca gcatccttgt ctgggagtag ctcatcatcc tcttcatctc    1320
gttcacggtc accaccaaag aagcctccca agaggacatc cagcccccct cggaaaactc    1380
gtaggttatc tccttcagca agtcctccaa ggcgaaggca caggccatca cctcctgcaa    1440
ctccaccacc caaaactcgg cattccccta caccccagca gtcaaaccgt acaagaaaaa    1500
gtcgtgtttc tgtgtctcca gggagaactt caggtaaagt gacaaaacat aaaggtactg    1560
agaaaagaga atccccttca ccagcaccga agcctagaaa agtagagtta tctgaatcgg    1620
aagaagataa aggtggcaaa atggctgcag cagattctgt gcagcagaga cgccaataca    1680
gacgacaaaa ccagcagtct tcatctgact ctggctcctc ctcctcctca gaagatgaac    1740
gacccaagag atcccatgtg aagaatggtg aggttggcag gcggcggaga cattcccctt    1800
cccggagtgc ttctccatca ccacgaaagc gccaaaaaga gacttcccct cgtggtagac    1860
ggaggagaag tccatcccca ccacccacca gaaggcgacg gtctccttct cccgcccctc    1920
ctcctcgacg gcgcaggact cccacaccac caccacgacg aaggactcct tctcctcccc    1980
cacgtcggcg ctcaccttct cctagaagat actctcctcc aatacagagg agatactctc    2040
cttctccacc tccaaagaga agaacggctt cacctcctcc ccctcctaaa cgaagagcat    2100
```

```
caccatctcc accaccaaag cggcgggtct cccattctcc acctcccaaa caaagaagct   2160
ccccagtcac caagagacgt tcaccttcat tatcatccaa gcataggaaa gggtcttccc   2220
caagccgctc tacccgggag gcccgatcac cacaaccaaa caaacggcat tcgccctcac   2280
cacggcctcg agctcctcag acctcctcaa gtcctccacc cgttcgaaga ggagcgtcgt   2340
catcacccca aagaaggcag tccccgtctc caagtactag gcccattagg agagtctcca   2400
ggactccgga acctaaaaag ataaaaaagg ctgcttcccc aagcccacag tctgtaagaa   2460
gggtctcatc ctcccgatct gtctccgggt ctcctgagcc agcagctaaa aagcccccag   2520
cacctccatc ccccgtccag tctcagtcac cgtctacaaa ctggtcacca gctgtaccgg   2580
tcaaaaaggc caaaagccca acaccgagcc catcaccgcc aagaaattca gatcaggaag   2640
gaggtggaaa gaaaaagaag aaaaagaagg acaagaaaca caaaaaggat aagaagcaca   2700
agaagcacaa aaaacacaag aaggaaaagg ctgtggctgc agctgctgca gctgctgtga   2760
cccctgcagc cattgcagct gccacaacca cattagcaca ggaagagcca gtggcagcgc   2820
cagagccgaa gaaggagact gaaagtgaag ctgaagataa ccttgatgat ttagaaaagc   2880
acctgcgtga aaaggccctg agatcaatga ggaaggccca agtgtcccca cagtcttagg   2940
gggaaatgtt tgttatgatg taaattttat ttggtttgta cgcagttcaa tttcaaaatt   3000
gctaaaatgt gtttgagctt tagactataa catttgttgt aataattgct aggttgaagt   3060
tcaacatgta aaaaaagggg gcatggattt acattgcaaa aggtgtccac agtgtattag   3120
tgacattctt tcattgacag ctgacataat tcattgagtg aaatatttta agccaaaaaa   3180
aaattccctt tttaaaaaag ggggtttaaa tactgttggc atttttatgg ttcctttaaa   3240
tgccctagct attcccagag gggttttttt gtttgttttt ttggttttga ttttcttttt   3300
gtttttcttt cttcttctta tttttttcat ttgagtctta gctcccattt aagttatgct   3360
tctgaccttg tatggtctgt aagcttgccc agaaataaga ccactgtttt gaactaccac   3420
aaaagtataa atgaatattt taatgccaca atctttcctg ttgcctgtgg agtctctgct   3480
gaaatgaatc aggattcgag ctctaggatg agacagaaaa tgaaagcatg ttgtttgcca   3540
ggacactgtg ggtttatatt gatgtgtaac aagttgattt ggaacactgg actctcattc   3600
tgttattctg gttttgtttt ttttgttttg ttttttttct tttgtaaagg caatgagcta   3660
gtcccagaaa ggatccttca gttacataca atttgtttaa tgaaatgtca tggctctgtt   3720
catatttttg tcttgttctt ccaattggta tatacaactt tcagagcctc ttgtatttgg   3780
aaggctggaa gggcccagac tttggaatag tgtcttggtt tcactgtttt tgttttgatt   3840
tttttttgt tttgattttt tttaaactaa agctatataa agcttgtgga ttaaacagaa   3900
taaatttcta aatttaaaaa tttaaaaaaa aaaaaaaaaa aaaaaaaaaa aaaaaaaaaa   3960

aaaaaaaaaa aaggaaaaaa aaaaaaaaaa                                     3990
```

<210> 48
<211> 1502

<212> DNA
<213> Homo sapiens

<400> 48

```
cctgcgtccc cgccccgcgc agccgccgcg ctcctgcgct ccgaggtccg aggttcccga      60
gatgaaggtc tggctgctgc ttggtcttct gctggtgcac gaagcgctgg aggatgttac     120
tggccaacac cttcccaaga acaagcgtcc aaaagaacca ggagagaata gaatcaaacc     180
taccaacaag aaggtgaagc ccaaaattcc taaaatgaag gacagggact cagccaattc     240
agcaccaaag acgcagtcta tcatgatgca agtgctggat aaaggtcgct tccagaaacc     300
cgccgctacc ctgagtctgc tggcggggca aactgtagag cttcgatgta aagggagtag     360
aattgggtgg agctaccctg cgtatctgga cacctttaag gattctcgcc tcagcgtcaa     420
gcagaatgag cgctacggcc agttgactct ggtcaactcc acctcggcag acacaggtga     480
attcagctgc tgggtgcagc tctgcagcgg ctacatctgc aggaaggacg aggccaaaac     540
gggctccacc tacatctttt ttacagagaa aggagaactc tttgtacctt ctcccagcta     600
cttcgatgtt gtctacttga acccggacag acaggctgtg gttccttgtc gggtgaccgt     660
gctgtcggcc aaagtcacgc tccacaggga attcccagcc aaggagatcc cagccaatgg     720
aacggacatt gtttatgaca tgaagcgggg ctttgtgtat ctgcaacctc attccgagca     780
ccagggtgtg gtttactgca gggcggaggc cgggggcaga tctcagatct ccgtcaagta     840
ccagctgctc tacgtggcgg ttcccagtgg ccctccctca acaaccatct tggcttcttc     900
aaacaaagtg aaaagtgggg acgacatcag tgtgctctgc actgtcctgg gggagcccga     960
tgtggaggtg gagttcacct ggatcttccc agggcagaag gatgaaaggc ctgtgacgat    1020
ccaagacact tggaggttga tccacagagg actgggacac accacgagaa tctcccagag    1080
tgtcattaca gtggaagact tcgagacgat tgatgcagga tattacattt gcactgctca    1140
gaatcttcaa ggacagacca cagtagctac cactgttgag ttttcctgac ttggaaaagg    1200
aaatgtaatg aacttatgga aagcccattt gtgtacacag tcagctttgg ggttcctttt    1260
attagtgctt tgccagaggc tgatgtcaag caccacaccc caaccccagc gtctcgtgag    1320
tccgacccag acatccaaac taaaaggaag tcatccagtc tattcacaga agtgttaact    1380
tttctaacag aaagcatgat tttgattgct tacctacata cgtgttccta gtttttatac    1440
atgtgtaaac aattttatat aatcaatcat ttctattaaa tgagcacgtt tttgtaaaaa    1500
at                                                                  1502
```

<210> 49
<211> 385
<212> DNA
<213> Homo sapiens

<400> 49

aaaaaaatta aggctaacca agtgcatcca ttgttcaatg gcacaattga tttcagcaac 60
tatttggaat atcctaatta taggaaatgc ccatctaagt gatatattta aataatacaa 120
tcaatttttt aaggtgaata aactatgatg gtttctaaat agtgtacatg ttacctgaaa 180
aatcagaaaa cacaaagaat gattaatttc gaaagttctt gcctaaaggc accactgact 240
taaaaaacat tcaaaatcaa ataccacaag acataaagcc tcttcatgta tatattcata 300
tatgcaataa atgcattaaa tgtaacttta ttaaacatag tacactgtac ttgacttatg 360
gttaaatatt ttacacacag cttga 385

<210> 50
<211> 6313
<212> DNA
<213> Homo sapiens

<400> 50

gccaggtccc tgaggggcgg gcagatgagg cctaggggtg ccgatcccta gtgtcgacta 60
tgcgagatct gattccggag ctgccatgat tgaagtggta gcagagctca gccggggtcc 120
tgtatttttg gctggggagg cgctggagtg tgtagtgacc gtcaccaacc cccttccgcc 180
cacggccact tctgcatcca gtgaggccct ggcctgggcc agtgcccaaa tccactgcca 240
gttccatgcc agtgagagtc gagtagcact gcctcctcct gactctagtc agccagatgt 300
ccagcccgac agccagactg tctttctgcc acaccgaggt gagaggggcc agtgtatcct 360
ttctactcca ccgaaaattc tattctgtga cctgaggctt gatcctggag agtccaaatc 420
atactcctac agtgaagtgc tgcccataga gggaccaccc tcctttcggg gtcagtcagt 480
caagtacgtc tacaaactga ccattggctg ccagcgtgtc aactccccta tcactttact 540
cagagtccct ctgagggttc ttgtgctgac tggccttcag gatgtccggt ttccccagga 600
tgaggctgta gccccatcca gtccattctt ggaggaggat gaaggtggga agaaagattc 660
atggctagct gagctggctg gggaacgcct aatggctgcc acatcctgcc gcagcctcca 720
tctatacaat atcagtgatg gccgagggaa agttgggacg tttggcatct tcaaatctgt 780
gtacagactt ggcgaggacg tggtggggac cttaaactta ggggaaggaa ccgtagcttg 840
tttgcagttt tcagtcagct tacagaccga ggagcgtgta cagcctgagt accagcggcg 900
acgtggggca gggggtgtcc cctctgtgtc acatgtgact cacgcccggc accaggaatc 960
ctgcctacat acaactagaa ccagcttctc cctcccaatc cctctcagct ccaccccagg 1020
cttctgtaca gccattgtgt ccttgaagtg gagattgcat tttgaatttg taacgtcccg 1080
agaaccagga ttggtactcc tacccccctgt ggaacagccc gaacctacca cctggacagg 1140

acctgagcaa gtacctgtag acaccttcag ctgggacctg cccatcaagg tgctgcctac 1200
tagccccacc ctggcctcat atgctgcccc aggccccagc accagcacca taaccatctg 1260
aaactggccc accctggtgc tagttccttc cggatactga gaactcagca cctggactct 1320
aatgggaccc actttttcca cctggggtcc aatgtcgtgg acagtgagag tcgggctttc 1380
agctatagca ttaatttatt tgttcagaat acattggcag ctgctagtgg tttccctgga 1440
agtggcagca gcagtgagca gtcagcagat ggatgatcag ttgagtttag ctggagtggg 1500
gagcaggagc cccaggaaca ggggtgttgg ctgagcccca ttctgggtca ggccctcccc 1560
ctttgcaggg cagccgaggg tcagattttt gcaccaagga gaactggcag gttcctgcct 1620
cctgacgtac ctcacaccca gccgggaagt cgatgggatg ctgggacctg gggaaccaag 1680
gataggggaa ggagtcagca cagtgaaagg ctgcctttat ccctgcccac atgttccctc 1740
tctcacagtt ttccccccac agagcccctt tcagtggccc cttggtcctc ctaactaagc 1800
tgtcacctac catatgtggg ccttttttgtt ttataacagg agtattttct ctccaggtcc 1860
accccaacct cccctgattt atagcctgaa gccttatctt tcacactagt gttggtccct 1920
tcaggtttgg cccatcttgt attgctcttc tgttcattct tacatcacag caatctagtc 1980
actccctggt catccctcag tcactcatat cagagtcatt ctctctggcc atctttggtc 2040
actcacgtgt cacagcagcc cacgccaaca ggatgcagac aggtgcaatg gaaacagtcc 2100
ttgcggagcc aagactcacc cagggtaaaa tatttcccct catagtgaca gggggctagg 2160
gaagaacggg aaatgttagt aggtgtagga gtgctgatga gaggcagagg ctcttctggt 2220
ctggggtgga gacagtaagt acgcactatc cccgtattta gtttgtcttt cctgtttcac 2280
agctggagga agcctgggta ttttgacacg ggatcatctg taaggcccca tcctccctgt 2340
gccctctctg ctgctcctcc attcctaacg cttcacccca ctttaccttg agcttggaag 2400
tagcacttgc tgtagactcc tgggtgctgg aggagtagag acatcaccaa gcagatgatc 2460
ccccagcctc ctaggatccc cttggcctgt ccagcccaga gcatccttag ggccattgct 2520
gctgcacagc cctctcagac ccttcttggc ctctgctcag ctactctggt cttgactcct 2580
tgactttgct ttgcgttgct ccttgagtct tagtttctgt ctttctcccc tgggctcctg 2640
tctcacacta tctccctgcc ctctgctctc acaggctggg gatgtttata aagtgaggac 2700
cctggccccc tgctgagtag agctggaaaa gttgtaactc tgtttcctga ggtgagggca 2760
tgaaaacaag aggtctagct ttaacaagct gtgagagctg attcatgccc cggcacagct 2820
agagggaggg aggtggccat ggagggggca ctggactggg cacttcccca gcaaggaggc 2880
aggaggggcg agggccccca ggtggtcccc agatctcttc cctgacctgg agagaaggaa 2940
gcattccacc ttcccccttt ctcccccact gccaccacca ggggtgtgta tgctgggatc 3000

cctgcctgga ccggagggag gcatttcctg gggatggtta atcctgtgcc ccagccaaac 3060
ccaggagctg caatagggtg cgacggccag aagctccagg agagtgagca ggcacctgga 3120
gtggagactg tgtttccctc agatcctagg gcagggtttc cctaatgtat ccaagaaata 3180
gggctgcccc tcagagatgg tggggagggt ctcttttcct caggcattcc agaggtgaac 3240
tgtccattgc ttatcacctt caaacataca gcagatgtgg gatcacccca catctgggga 3300
tggttctttc ccctttcaaa gaggagcatc tctaagtgcc ctgatgggat gaatcactcc 3360
aggttcacag aggtgtcctc tctttcctcc catatataat ggagtgaggt ttttaggaat 3420
ttatcatttg gcatcctctg agtttcccac aggttctgga ggagcccagg atggattatt 3480
gagagcatgg gctgtagaga cagtcttctt ggattcagat cctgactcca cttagctatg 3540
taacctggtc agattacttc acctctctga gcctgtttcc tcatctataa attggggata 3600
gtaatgccaa ctcattgggc tgttatgagg attactgaga taatgcgtgc agtgctctta 3660
tcaccatctc tggtgcgtaa gcgtcaggaa atagcagttg ctgtgattgg ggctaaagct 3720
ctgaggcaaa atgggcgaca ttattttctt tgaatgacat taagcagttt gtgcatagct 3780
gagggcttct attggggatg gctgtctcct ggcatagacc tctgcacctt tcacactcat 3840
actccttgtc agcagtcccc aacctttttg gtaccaggga ccggttttgt ggaaaacaat 3900
ttttccacca gtggatggag gggatagca gcggggagat gattttggga tgaaactgtt 3960
tcatctcaga tcatcaggca ttagattctc ataaggagtg tgcaatctag atcccttgca 4020
tgcggagttc acagtggggt ttgcactcct gtgagaatct aatgcctctg ctgatctgcc 4080
aggaggagga gctcaggcgg taatgctcac tcgcctgccg cccacctcct gctttgtgct 4140
cccgcttcct aacaggccac agactggtac tggcctgtgg cctgggggat ggagacccct 4200
aatccatgtc acctttccca cctctttcaa aaacaggtac ctccaggaac attttggttt 4260
tggcccttgt attgacttct gaatgtctag tttgagaaac tgttcccaaa taagccttct 4320
tcccccagat ctgcaccctc gcctctaccc taggacaaga tgtccttttc tcatcatcct 4380
gccaggctaa ctttaagtct cctgcttttt ctcacttgga tttggatcca tttcttccta 4440
tttccgctca tgtgaactct ccagttctcc tttctcacca ctctcctgct agccatctct 4500
ttggcactaa aggccctggt caaattggat ttctttcatt tttccacact tcaaagaccc 4560
atgttctagg tattctccat agggatagtc tctttggcat ttatttggtt tttctacgtt 4620
ttcagtccca tttactccaa gactcactcc ctgccaccta gtgcatcaga tacagctact 4680
tctggctgac ttttcaaggg ggaccacccct acctgtcatc tcttcactgt tcagaaatga 4740
ctgtgtcagt gcacctcaaa ctcccttgct gtccttttcc aaggagacag ctaaggtgga 4800
tggagatgca gaatggacct cacgttcgcc ctagtcagga ctgataccct ttccgtttca 4860
gaggattgcc aagaaaaaac tcacagttga ggcagggtgc tctgaggtcg gctgcggtgt 4920

```
gggaggcacg gcctgggcct gctctctggg ctggagcagg tggattcgaa ggcctgtcta   4980
gcacgagggc ccaaaggtct tgtcagtggc cagtagctct gccgcctttc ccagagaggg   5040
ggtccagggg acatcctgga aggctgggcc ctgggccacc ttctgctctt gcaagctaga   5100
gccagcccaa tagggggcgg atgtgagtgg ggagctgggg cgcatgaagg tgggggtgat   5160
gccgaagggg aagggatcgc cagtggggat tggtgcgtgt gcggaaacgg ggacagaagt   5220
gaaggttcat cgcctataac gaagatgagg taggcatata ggggcttctg gaaagctaga   5280
ggctgggctg agccaggagt cctctcccag aagttggggg gcggtgcaga ggtgtgggtc   5340
gagcccgcat gcgtgcctgc tggggagggg gtgagtggtg aggaccaggc ccgctgggtc   5400
ctgggggcgc ggtggctggc gcgcaggtcc cggagggggc ggctggcgcg cactacacgc   5460
ttgggaacaa ggaaaacatc cgccggaggc ccggccgggc ggcgctccag cctcggggca   5520
ggtgcgcgga gaggaagtga gagcattccg gcccccccac cccaaccccg gccgctggcc   5580
ctctggtgag tcacagccga cccccgccgc cggagggaga ggggagctgc gggccagagc   5640
cccggagggt ctggaggagc caggagggtt tctgggagca gagggtcact tagtgggctt   5700
ctgtcgtggt gtcgctacgg gcgcgaaacg gacactgaac acagtctgac tgtatggagg   5760
caggtgggga gggatcccct gggagaactt ggcgggccga gagcagaccc cagggcaagg   5820
aggggccccc gagggggaaa ccgggagtcg ggcaggtggc gtaacccaga aagggaagga   5880
gagccggatt gattggggtg agagaggaag gaagcacgcc aagttaggcc tgggagaact   5940
gagggacctg aggagggagg agggagacca acacagggtg ggaaggcgga aatggccaaa   6000
ccccaggcat caggtctgtc cagaggctga cgtagacagt gaagggtgaa gggtaggttt   6060
taggagtagg gggagttatg attatttggt tacattttgg gattatttgg tctcacaggt   6120
agaagggagc ctgctggtct ctgtgtaacg gatggcttaa aagcaaggtt gtctgcgtct   6180
tggattactg tctgccattc agcctttgcc aaaaaatttg gcactgatct gcacattttt   6240
atagtcattt aaaattgtat gactctgtca aatgatttaa gtaattttgg tggattttta   6300
aaaataaaaa aat                                                      6313
```

<210> 51
<211> 1133
<212> DNA
<213> Homo sapiens

<400> 51

```
ggtaaggggt cctccctgcg ccacacggcc gtcgccatgg tgaagctgag caaagaggcc     60
aagcagagac tacagcagct cttcaagggg agccagtttg ccattcgctg gggctttatc    120
cctcttgtga tttacctggg atttaagagg ggtgcagatc ccggaatgcc tgaaccaact    180
gttttgagcc tactttgggg ataaaggatt atttggtctt ctggatttgg aggcaatcag    240
```

cggacagcat ggaagatgtg tgctctggct cggataagag atgggacatc attcagtcac 300
tagttggatg gcacaaggct cttcacagac gcatctgtag cagagtggat cttgtactaa 360
cttatgatag aatgtatcag aataaatgtt tttaacagtg taaacaccac aaacaaaaaa 420
cacaacacac acatcataca cacaaaaaac acaaaaaaaa caaacaaatc acacaaaagc 480
tacggtagac ctactattat gcggtgggcc gaaacaagac gggtattata gacaagggaa 540
acgagtcgtc aaatcgtcgt agcctgacac acatcatatt gttagaccca gcgtgtgcaa 600
tatctcgccg gggtagctcc ctcatatgag ggacacgtta tatatgtctc agatagggcg 660
ccggggtata acctgcagtt ttatagatat gctggcaaca gaaaaaagcg atgtaaaaaa 720
aaaaatgaag acaacataaa cacacacaca aagatactat cacatatata ctataaccaa 780
aaaatctcaa agcgtaaatc aaaaatacac taaaacaatt cacagccata ttcactacac 840
cctatccacc ccacacaaaa aaaataagac acaaaacatc acacatatac acactaccta 900
tcattttata ctttaatcta atataattaa gtaacaaatc aacacaaata tacacacgat 960
cgatagatac actgataaaa ttcaacaaac aaaataccaa ataaaatata ctaaacacac 1020
cactagacga gcatcttata ttgcactttt acgtagacct ctgatcaata acaacagacc 1080
tactccacaa atatactact aacacacaaa caatgcaaac agcacagaat aac 1133

<210> 52
<211> 20
<212> DNA
<213> Artificial

<220>
<223> Oligonucleotide primer

<400> 52
gccataagtg gtcccacagt 20

<210> 53
<211> 23
<212> DNA
<213> Artificial

<220>
<223> Oligonucleotide primer

<400> 53
gtcttctagt ccgtcatctc cct 23

<210> 54
<211> 22
<212> DNA
<213> Artificial

<220>
<223> Oligonucleotide primer

<400> 54
ccatagctgc ctttatgtct gc 22

<210> 55
<211> 22
<212> DNA
<213> Artificial

<220>
<223> Oligonucleotide primer

<400> 55
ctttttacct tcgtgcacct tt 22

<210> 56
<211> 23
<212> DNA
<213> Artificial

<220>
<223> oligonucleotide primer

<400> 56
taatctgctg aggacctttt gtc 23

<210> 57
<211> 23
<212> DNA
<213> Artificial

<220>
<223> Oligonucleotide primer

<400> 57
taattcactg tcctcttctg gga 23

<210> 58
<211> 21
<212> DNA
<213> Artificial

<220>
<223> oligonucleotide primer

<400> 58
gctcacagca gtaaatgcct a 21

<210> 59
<211> 23
<212> DNA
<213> Artificial

<220>
<223> Oligonucleotide primer

<400> 59
tgctatgctg taaacactgg cta 23

<210> 60
<211> 23
<212> DNA

<213> Artificial

<220>
<223> Oligonucleotide primer

<400> 60
ggatccttta ttggtggtag agc 23

<210> 61
<211> 23
<212> DNA
<213> Artificial

<220>
<223> oligonucleotide primer

<400> 61
ccagagtgac cctgaagata aat 23

<210> 62
<211> 23
<212> DNA
<213> Artificial

<220>
<223> oligonucleotide primer

<400> 62
acctgattct ctaggtgcag ttt 23

<210> 63
<211> 23
<212> DNA
<213> Artificial

<220>
<223> oligonucleotide primer

<400> 63
gtcgtttcaa ccaggtagtt ttg 23

<210> 64
<211> 20
<212> DNA
<213> Artificial

<220>
<223> Oligonucleotide primer

<400> 64
gggcgcctta agttattgga 20

<210> 65
<211> 22
<212> DNA
<213> Artificial

<220>

<223> Oligonucleotide primer

<400> 65
ggatggtaga aaagcaaact gg 22

<210> 66
<211> 23
<212> DNA
<213> Artificial

<220>
<223> Oligonucleotide primer

<400> 66
tgtaatggag attgtacagg ttg 23

<210> 67
<211> 23
<212> DNA
<213> Artificial

<220>
<223> Oligonucleotide primer

<400> 67
aggaacagta caaatgctgt ggt 23

<210> 68
<211> 23
<212> DNA
<213> Artificial

<220>
<223> Oligonucleotide primer

<400> 68
gcactccttg aaggtacact aac 23

<210> 69
<211> 22
<212> DNA
<213> Artificial

<220>
<223> Oligonucleotide primer

<400> 69
atttgtattc actcagccat gc 22

<210> 70
<211> 23
<212> DNA
<213> Artificial

<220>
<223> Oligonucleotide primer

<400> 70

acccaacttc aaaactagga ctc 23

<210> 71
<211> 23
<212> DNA
<213> Artificial

<220>
<223> oligonucleotide primer

<400> 71
acagcttgat gtcctttcta tgc 23

<210> 72
<211> 22
<212> DNA
<213> Artificial

<220>
<223> Oligonucleotide primer

<400> 72
tcatgaaagg cactgagttt tg 22

<210> 73
<211> 23
<212> DNA
<213> Artificial

<220>
<223> Oligonucleotide primer

<400> 73
gttagctgaa gcagctttat tgc 23

<210> 74
<211> 22
<212> DNA
<213> Artificial

<220>
<223> oligonucleotide primer

<400> 74
atatgcacaa tcctggaagt ga 22

<210> 75
<211> 23
<212> DNA
<213> Artificial

<220>
<223> Oligonucleotide primer

<400> 75
tgccttacta gcattaccac cat 23

<210> 76

<211> 21
<212> DNA
<213> Artificial

<220>
<223> Oligonucleotide primer

<400> 76
gaggtgatag cattgctttc g 21

<210> 77
<211> 21
<212> DNA
<213> Artificial

<220>
<223> Oligonucleotide primer

<400> 77
caagtcagtg tacaggtaag c 21

<210> 78
<211> 21
<212> DNA
<213> Artificial

<220>
<223> Oligonucleotide primer

<400> 78
tcttacaccc agtggagaag c 21

<210> 79
<211> 20
<212> DNA
<213> Artificial

<220>
<223> Oligonucleotide primer

<400> 79
gtctttgtgt tcccggacat 20

<210> 80
<211> 20
<212> DNA
<213> Artificial

<220>
<223> oligonucleotide primer

<400> 80
actatgtccg ggaacacaaa 20

<210> 81
<211> 18
<212> DNA
<213> Artificial

<220>
<223> Oligonucleotide primer

<400> 81
ttccgtcata tggcttgg 18

<210> 82
<211> 23
<212> DNA
<213> Artificial

<220>
<223> oligonucleotide primer

<400> 82
cgtcgctatc aaggaattaa gag 23

<210> 83
<211> 23
<212> DNA
<213> Artificial

<220>
<223> oligonucleotide primer

<400> 83
gtagctccag acatcactct ggt 23